(19) Europäisches Patentamt
European Patent Office
Office européen des brevets

(11) EP 0 503 548 B1

(12) **EUROPÄISCHE PATENTSCHRIFT**

(45) Veröffentlichungstag und Bekanntmachung des Hinweises auf die Patenterteilung:
**04.06.1997 Patentblatt 1997/23**

(51) Int Cl.[6]: **C07D 233/32**, A61K 31/415, C07D 233/70, C07D 233/72, C07D 249/12, C07D 285/10

(21) Anmeldenummer: **92104045.7**

(22) Anmeldetag: **10.03.1992**

(54) **Cyclische Harnstoffderivate, diese Verbindungen enthaltende Arzneimittel und Verfahren zu ihrer Herstellung**

Cyclic urea derivatives, medicaments contaning them and process for their preparation

Dérivés de l'urée cyclique, médicaments les contenant et procédé pour leur préparation

(84) Benannte Vertragsstaaten:
**AT BE CH DE DK ES FR GB GR IT LI LU NL PT SE**

(30) Priorität: **12.03.1991 DE 4107857**

(43) Veröffentlichungstag der Anmeldung:
**16.09.1992 Patentblatt 1992/38**

(73) Patentinhaber: **Dr. Karl Thomae GmbH**
**D-88397 Biberach (DE)**

(72) Erfinder:
- **Himmelsbach, Frank, Dr. Dipl.-Chem.**
  **W-7951 Mittelbiberach (DE)**
- **Pieper, Helmut, Dr. Dipl.-Chem.**
  **W-7950 Biberach 1 (DE)**
- **Austel, Volkhard, Dr. Dipl.-Chem.**
  **W-7950 Biberach 1 (DE)**
- **Linz, Günter, Dr. Dipl.-Chem.**
  **W-7951 Mittelbiberach (DE)**
- **Müller, Thomas, Dr. Dipl.-Chem.**
  **W-7950 Biberach (DE)**
- **Weisenberger, Johannes, Dr. Dipl.-Chem.**
  **W-7950 Biberach 1 (DE)**
- **Eisert, Wolfgang, Prof. Dr.**
  **W-7950 Biberach 1 (DE)**

(56) Entgegenhaltungen:
**EP-A- 0 222 664**
**EP-A- 0 301 946**
**CH-A- 362 916**
**US-A- 3 989 707**
**US-A- 4 724 261**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

In der EP-A-0,381,033 werden u. a. Alkansäure-Derivate beschrieben, welche durch einen substiutierten Phenyl-, Pyridyl- oder Thienylrest substituiert sind. Desweiteren fallen unter die allgemeine Formel III der US-A-3,989,707 u.a. im Benzimidazolteil substituierte 1-Piperidino-benzimidazol-2-one, die in 3-Stellung durch eine Alkoxycarbonylalkylgruppe substituiert sind, wobei keine derartige Verbindung expressis verbis beschrieben wird.

Es wurde nun gefunden, daß die cyclischen Harnstoffderivate der allgemeinen Formel

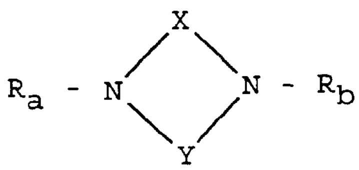 , (I)

deren Tautomere, deren Stereoisomere einschließlich deren Gemische und deren Salze, insbesondere deren physiologisch verträgliche Salze mit anorganischen Säuren oder Basen, wertvolle Eigenschaften aufweisen, u. a. wertvolle pharmakologische Eigenschaften, vorzugsweise aggregationshemmende Wirkungen.

Gegenstand der vorliegenden Erfindung sind somit die obigen Verbindungen der allgemeinen Formel I, diese Verbindungen enthaltende Arzneimittel und Verfahren zu ihrer Herstellung.

In der obigen allgemeinen Formel I bedeutet

X eine gegebenenfalls am Stickstoffatom durch eine Alkyl-, Aralkyl-, Aryl-, Heteroaryl- oder Cyanogruppe substituierte Carbiminogruppe, eine Carbonyl-, Thiocarbonyl-, Sulfinyl- oder Sulfonylgruppe,

Y eine gegebenenfalls durch $R_c$ oder $R_d$ oder $R_c$ und $R_d$ substituierte geradkettige Alkylen- oder Alkenylengruppe mit jeweils 2 bis 4 Kohlenstoffatomen, in denen jedes Kohlenstoffatom durch ein Fluor-, Chlor- oder Bromatom, durch eine Alkyl-, Trifluormethyl-, Aralkyl-, Aryl-, Heteroaryl- oder Alkylcarbonylgruppe mono- oder disubstituiert sein kann, wobei die Substituenten gleich oder verschieden sein können und zusätzlich eine oder zwei Methylengruppen jeweils durch eine Carbonylgruppe ersetzt sein können, oder eine gegebenenfalls durch $R_c$ oder $R_d$ oder $R_c$ und $R_d$ substituierte 1,2-Cycloalkylengruppe mit 4 bis 7 Kohlenstoffatomen,

eine 1,2-Cycloalkenylengruppe mit 4 bis 7 Kohlenstoffatomen oder eine 1,2-Phenylengruppe, in der eine oder zwei Methingruppen durch ein Stickstoffatom ersetzt sein können und die im Kohlenstoffgerüst durch ein Fluor-, Chlor- oder Bromatom, durch eine Alkylgruppe mit 1 bis 4 Kohlenstoffatomen, durch eine Trifluormethyl-, Hydroxy-, Alkoxy-, Alkylsulfenyl-, Alkylsulfinyl-, Alkylsulfonyl-, Alkylcarbonyl-, Arylcarbonyl-, Alkoxycarbonyl-, Carboxy-, Nitro-, $(R_1)_2N$-, $(R_1)_2NCO$- oder $(R_1)_2NSO_2$- Gruppe, wobei die Reste $R_1$ gleich oder verschieden sein können und jeweils ein Wasserstoffatom, eine Alkyl-, Aralkyl-, Aryl-oder Heteroarylgruppe bedeuten, oder durch eine durch eine Alkylcarbonyl-, Arylcarbonyl-, Aralkylcarbonyl-, Heteroarylcarbonyl-, Alkylsulfonyl-, Aralkylsulfonyl- oder Arylsulfonylgruppe substituierte $R_1NH$-Gruppe substituiert sein kann und in der zusätzlich eine oder zwei -CH=CH-Gruppen jeweils durch eine -CO-$NR_1$-Gruppe ersetzt sein können,

eine gegebenenfalls durch $R_c$ oder $R_d$ substituierte -CO-NH-, -NH-CO-, -CH=N- oder -N=CH-Gruppe,

einer der Reste $R_a$ bis $R_d$ eine Gruppe der Formel

A - B - C -,

in der

A eine geradkettige oder verzweigte Aminoalkylgruppe mit 1 bis 5 Kohlenstoffatomen, eine Amino-, Amidino- oder Guanidinogruppe, wobei in jeder der vorstehend erwähnten Gruppen jeweils an einem der Stickstoffatome ein oder zwei Wasserstoffatome jeweils durch eine Alkylgruppe mit 1 bis 4 Kohlenstoffatomen oder ein Wasserstoffatom durch eine Alkoxycarbonylgruppe mit insgesamt 2 bis 5 Kohlenstoffatomen, durch eine Alkylcarbonyl-, Arylcarbonyl-, Aryloxycarbonyl- oder Aralkoxycarbonylgruppe ersetzt sein kann, eine Cyanogruppe, eine Cyanoalkylgruppe mit 1 bis 4 Kohlenstoffatomen im Alkylteil oder auch, falls A an ein Stickstoffatom der Reste B oder C

gebunden ist, das nicht Teil einer Lactamgruppe ist, ein Wasserstoffatom oder eine Alkylgruppe,

B eine Bindung,

eine Alkylen- oder Alkenylengruppe,

eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, $(R_1)_2N$-, $(R_1)_2NCO$-, $(R_1)_2NSO_2$- oder Nitrogruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1$NH-Gruppen mono-oder disubstituiert sein kann, wobei die Substituenten gleich oder verschieden sein können,

eine Pyridinylen-, Pyrimidinylen-, Pyrazinylen-, Pyridazinylen-oder Triazinylengruppe, die jeweils im Kohlenstoffgerüst durch eine Alkylgruppe substituiert sein können, wobei zusätzlich eine oder zwei -CH=N-Gruppen jeweils durch eine -CO-$NR_1$-Gruppe ersetzt sein können und eines der Stickstoffatome statt an den Rest $R_1$ auch an den Rest C gebunden sein kann, sofern dieser nicht mit einem Heteroatom oder einer Carbonylgruppe an den Rest B anschließt,

eine gegebenenfalls durch eine Alkyl-, Aralkyl- oder Arylgruppe substituierte Cyclopropylengruppe,

eine gegebenenfalls durch eine Alkyl-, Aralkyl- oder Arylgruppe substituierte Cycloalkylengruppe mit 4 bis 5 Kohlenstoffatomen, in der eine CH-Einheit durch ein Stickstoffatom und zusätzlich eine zum Stickstoffatom benachbarte Methylengruppe durch eine Carbonylgruppe ersetzt sein kann,

eine gegebenenfalls durch eine Alkyl-, Aralkyl- oder Arylgruppe substituierte Cycloalkylengruppe mit 6 oder 7 Kohlenstoffatomen, in der eine oder zwei zueinander in 1,4-Stellung befindliche CH-Einheiten je durch ein Stickstoffatom ersetzt sein können, wobei zusätzlich jeweils eine oder zwei der zu einem Stickstoffatom benachbarten Methylengruppen durch eine Carbonylgruppe ersetzt sein können,

eine Biphenylengruppe, die durch Fluor-, Chlor- oder Bromatome, durch Alkyl-, Trifluormethyl-, Hydroxy-, Alkoxy-, Alkylsulfenyl-, Alkylsulfinyl-, Alkylsulfonyl-, Alkylcarbonyl-$NR_1$- oder Alkylsulfonyl-$NR_1$-Gruppen mono- oder disubstituiert sein kann, wobei die Substituenten gleich oder verschieden sein können und $R_1$ wie eingangs definiert ist, und

C eine gegebenenfalls durch eine Hydroxy-, Alkoxy- oder $(R_1)_2N$-Gruppe substituierte Alkylen- oder Alkenylengruppe,

eine über die Carbonylgruppe mit dem Rest B verbundene Alkylencarbonylgruppe,

eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, $(R_1)_2N$-, $(R_1)_2NCO$-, $(R_1)_2NSO_2$- oder Nitrogruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1$NH-Gruppen mono- oder disubstituiert sein kann, wobei die Substituenten gleich oder verschieden sein können,

eine Indanylen- oder 1,2,3,4-Tetrahydronaphthylengruppe, in denen jeweils der gesättigte Ring an den Rest A und der aromatische Ring an das cyclische Harnstoffgerüst gebunden ist, eine Pyridinylen-, Pyrimidinylen-, Pyrazinylen-, Pyridazinylen- oder Triazinylengruppe, die jeweils im Kohlenstoffgerüst durch eine Alkylgruppe substituiert sein können, wobei zusätzlich eine oder zwei -CH=N-Gruppen jeweils durch eine -CO-$NR_1$-Gruppe ersetzt sein können und eines der Stickstoffatome statt an den Rest $R_1$ auch an den Rest B gebunden sein kann, solange dieser nicht eine Bindung bedeutet oder nicht mit einem Heteroatom an den Rest C anschließt,

eine gegebenenfalls durch eine Alkyl-, Aralkyl- oder Arylgruppe substituierte Cycloalkylengruppe mit 4 bis 5 Kohlenstoffatomen, in der eine CH-Einheit durch ein Stickstoffatom und zusätzlich eine zum Stickstoffatom benachbarte Methylengruppe durch eine Carbonylgruppe ersetzt sein kann, oder

eine gegebenenfalls durch eine Alkyl-, Aralkyl- oder Arylgruppe substituierte Cycloalkylengruppe mit 6 oder 7 Kohlenstoffatomen, in der eine oder zwei zueinander in 1,4-Stellung befindliche CH-Einheiten je durch ein

Stickstoffatom ersetzt sein können, wobei zusätzlich jeweils eine oder zwei der zu einem Stickstoffatom benachbarten Methylengruppen durch eine Carbonylgruppe ersetzt sein können,

ein zweiter der Reste $R_a$ bis $R_d$ eine Gruppe der Formel

F - E - D -,

in der

D eine Alkylengruppe mit 1 bis 5 Kohlenstoffatomen oder eine Alkenylengruppe mit 2 bis 5 Kohlenstoffatomen,

eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, Carboxyalkoxy-, Alkoxycarbonylalkoxy-, Aralkoxycarbonylalkoxy-, $(R_1)_2N$-, $(R_1)_2NCO$-, $(R_1)_2NSO_2$- oder Nitrogruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1NH$-Gruppen mono- oder disubstituiert sein kann, wobei die Substituenten gleich oder verschieden sein können,

eine Pyridinylen-, Pyrimidinylen-, Pyrazinylen-, Pyridazinylen-oder Triazinylengruppe, die jeweils im Kohlenstoffgerüst durch eine Alkylgruppe substituiert sein können, wobei zusätzlich eine oder zwei -CH=N-Gruppen jeweils durch eine -CO-$NR_1$-Gruppe ersetzt sein können und eines der Stickstoffatome statt an den Rest $R_1$ auch an den Rest E gebunden sein kann, sofern dieser keine Bindung darstellt oder nicht mit einem Heteroatom an den Rest D gebunden ist,

eine gegebenenfalls durch eine Alkyl-, Aralkyl- oder Arylgruppe substituierte Cycloalkylengruppe mit 4 bis 5 Kohlenstoffatomen, in der eine CH-Einheit durch ein Stickstoffatom und zusätzlich eine zum Stickstoffatom benachbarte Methylengruppe durch eine Carbonylgruppe ersetzt sein kann,

eine gegebenenfalls durch eine Alkyl-, Aralkyl- oder Arylgruppe substituierte Cycloalkylengruppe mit 6 oder 7 Kohlenstoffatomen, in der eine oder zwei zueinander in 1,4-Stellung befindliche CH-Einheiten je durch ein Stickstoffatom ersetzt sein können, wobei zusätzlich jeweils eine oder zwei der zu einem Stickstoffatom benachbarten Methylengruppen durch eine Carbonylgruppe ersetzt sein können, oder

eine durch den Rest W unterbrochene Alkylengruppe mit 2 bis 6 Kohlenstoffatomen, in der W ein Sauerstoff- oder Schwefelatom, eine Sulfinyl-, Sulfonyl-, $R_1N$-, (Alkylcarbonyl)N-, (Aralkylcarbonyl) N-, (Arylcarbonyl)N-, (Heteroarylcarbonyl)N-, (Alkylsulfonyl)N-, (Arylsulfonyl)N-, Aminocarbonyl- oder Carbonylaminogruppe darstellt,

E eine Bindung,

eine Alkylengruppe mit 1 bis 5 Kohlenstoffatomen oder eine Alkenylengruppe mit 2 bis 5 Kohlenstoffatomen, die jeweils durch eine oder zwei Alkylgruppen, durch eine Hydroxy-, Alkoxy-, Amino-, Alkylamino-, Aralkylamino-, Dialkylamino-, Bis(aralkyl)amino-, Carboxyalkyl-, Alkoxycarbonylalkyl- oder Aralkoxycarbonylalkylgruppe substituiert sein können,

eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, $(R_1)_2N$-, $(R_1)_2NCO$-, $(R_1)_2NSO_2$- oder Nitrogruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1NH$-Gruppen mono-oder disubstituiert sein kann, wobei die Substituenten gleich oder verschieden sein können,

eine Pyridinylen-, Pyrimidinylen-, Pyrazinylen-, Pyridazinylen-oder Triazinylengruppe, die jeweils im Kohlenstoffgerüst durch eine Alkylgruppe substituiert sein können, wobei zusätzlich eine oder zwei -CH=N-Gruppen jeweils durch eine -CO-$NR_1$-Gruppe ersetzt sein können und eines der Stickstoffatome statt an den Rest $R_1$ auch an den Rest D gebunden sein kann,

eine gegebenenfalls durch eine Alkyl-, Aralkyl- oder Arylgruppe substituierte Cycloalkylengruppe mit 4 bis 5

Kohlenstoffatomen, in der eine CH-Einheit durch ein Stickstoffatom und zusätzlich eine zum Stickstoffatom benachbarte Methylengruppe durch eine Carbonylgruppe ersetzt sein kann,

eine gegebenenfalls durch eine Alkyl-, Aralkyl- oder Arylgruppe substituierte Cycloalkylengruppe mit 6 oder 7 Kohlenstoffatomen, in der eine oder zwei zueinander in 1,4-Stellung befindliche CH-Einheiten je durch ein Stickstoffatom ersetzt sein können, wobei zusätzlich jeweils eine oder zwei der zu einem Stickstoffatom benachbarten Methylengruppen durch eine Carbonylgruppe ersetzt sein können,

eine über den Arylteil mit dem Rest D verknüpfte Alkylenarylengruppe oder

eine über den Rest W mit dem Rest D verknüpfte Alkylengruppe, in der W wie eingangs definiert ist, und

F eine Carbonylgruppe, die durch eine Hydroxy- oder Alkoxygruppe mit 1 bis 6 Kohlenstoffatomen substituiert ist, wobei ein Alkoxyrest mit 1 bis 3 Kohlenstoffatomen in 1-, 2- oder 3-Stellung durch eine Aryl- oder Heteroarylgruppe oder in 2- oder 3-Stellung durch eine Pyrrolidin-2-on-1-yl-, Morpholino-, Thiomorpholino- oder 1-Oxido-thiomorpholinogruppe substituiert sein kann, eine Sulfo-, Phosphono-, O-Alkylphosphono- oder Tetrazol-5-ylgruppe darstellen, wobei, sofern A eine Cyanogruppe oder eine gegebenenfalls am Stickstoffatom benzoylierte oder benzyloxycarbonylierte Amino- oder Aminoalkylgruppe darstellt, der kürzeste Abstand zwischen dem Stickstoffatom dieser Gruppen und dem Rest F mindestens 10 Bindungen beträgt,

der dritte der Reste $R_a$ bis $R_d$ ein Wasserstoffatom, eine Alkyl-, Aralkyl-, Aryl- oder Heteroarylgruppe oder auch, wenn der dritte der Reste $R_a$ bis $R_d$ mit einem ungesättigten Kohlenstoffatom des Restes Y verbunden ist, eine Alkoxy-, Alkylsulfenyl-oder $(R_1)_2N$-Gruppe und

der vierte der Reste $R_a$ bis $R_d$ ein Wasserstoffatom, eine Alkyl-, Aralkyl-, Aryl- oder Heteroarylgruppe oder $R_a$ oder $R_b$ zusammen mit einem benachbarten Rest $R_c$ oder $R_d$ auch eine Bindung mit der Maßgabe darstellen, daß die A-B-C-Gruppe keine 4-Piperidinylgruppe darstellt, wenn gleichzeitig die F-E-D-Gruppe eine Alkoxycarbonylalkylgruppe und Y eine durch ein Fluor-, Chlor- oder Bromatom, durch eine Alkyl- oder Trifluormethylgruppe substituierte 1,2-Phenylengruppe bedeuten,

wobei, soweit nichts anderes erwähnt wurde,

die vorstehend erwähnten Alkyl-, Alkylen-, Alkenylen- oder Alkoxyteile jeweils 1 bis 3 Kohlenstoffatome enthalten können, sowie

unter dem vorstehend erwähnten Begriff "eine Arylgruppe" eine Phenylgruppe, die durch eine Trifluormethyl-, Carboxy-, $(R_1)_2NCO$-, Alkoxycarbonyl-, Alkylcarbonyl-, Alkylsulfenyl-, Alkylsulfinyl-, Alkylsulfonyl-, Nitro-, $(R_1)_2N$-, Alkylcarbonyl-$NR_1$-, Aralkylcarbonyl-$NR_1$-, Arylcarbonyl-$NR_1$-, Heteroarylcarbonyl-$NR_1$-, Alkylsulfonyl-$NR_1$-, Aralkylsulfonyl-$NR_1$-, Arylsulfonyl-$NR_1$- oder $(R_1)_2N$-sulfonyl-Gruppe monosubstituiert oder durch Fluor-, Chlor- oder Bromatome, durch Hydroxy-, Alkoxy- oder Alkylgruppen mit 1 bis 4 Kohlenstoffatomen mono-, di-oder trisubstituiert sein kann, und

unter dem vorstehend erwähnten Begriff "eine Heteroarylgruppe" ein 5-gliedriger heteroaromatischer Ring, welcher ein Sauerstoff-, Schwefel- oder Stickstoffatom, ein Stickstoffatom und ein Sauerstoff-, Schwefel- oder Stickstoffatom oder zwei Stickstoffatome und ein Sauerstoff-, Schwefel- oder Stickstoffatom enthält oder ein 6-gliedriger heteroaromatischer Ring, welcher ein, zwei oder drei Stickstoffatome enthält und in dem zusätzlich eine oder zwei -CH=N-Gruppen durch eine -CO-$NR_1$-Gruppe ersetzt sein können, wobei die vorstehend erwähnten heteroaromatischen Ringe zusätzlich durch eine oder zwei Alkylgruppen oder durch ein Fluor-, Chlor- oder Bromatom, durch eine Hydroxy- oder Alkoxygruppe substituiert sein können, zu verstehen ist.

Bevorzugte Verbindungen der obigen allgemeinen Formel I sind diejenigen, in denen

X eine gegebenenfalls am Stickstoffatom durch eine Alkyl-, Aralkyl-, Aryl-, Heteroaryl- oder Cyanogruppe substituierte Carbiminogruppe, eine Carbonyl-, Thiocarbonyl-, Sulfinyl- oder Sulfonylgruppe,

Y eine gegebenenfalls durch $R_c$ oder $R_d$ oder $R_c$ und $R_d$ substituierte geradkettige Alkylen- oder Alkenylengruppe mit jeweils 2 oder 3 Kohlenstoffatomen, die durch ein Fluor-, Chlor- oder Bromatom, durch eine Alkyl-, Trifluormethyl-, Aralkyl-, Aryl-, Heteroaryl- oder Alkylcarbonylgruppe mono- oder disubstituiert sein kann, wobei die Substituenten gleich oder verschieden sein können und zusätzlich eine oder zwei Methylengruppen jeweils durch eine Carbonylgruppe ersetzt sein können, oder eine gegebenenfalls durch $R_c$ oder $R_d$ oder $R_c$ und $R_d$ substituierte 1,2-Cyclohexylengruppe,

eine 1,2-Cyclohexenylengruppe oder eine 1,2-Phenylengruppe, in der eine oder zwei CH-Gruppen jeweils durch ein Stickstoffatom ersetzt sein können und die im Kohlenstoffgerüst durch ein Fluor-, Chlor- oder Bromatom, durch eine Alkylgruppe mit 1 bis 4 Kohlenstoffatomen, durch eine Trifluormethyl-, Hydroxy-, Alkoxy-, Alkylsulfenyl-, Alkylsulfinyl-, Alkylsulfonyl-, Alkylcarbonyl-, Arylcarbonyl-, Alkoxycarbonyl-, Carboxy-, Nitro-, $(R_1)_2N$-, $(R_1)_2NCO$- oder $(R_1)_2NSO_2$-Gruppe, wobei die Reste $R_1$ gleich oder verschieden sein können und jeweils ein Wasserstoffatom, eine Alkyl-, Aralkyl-, Aryl- oder Heteroarylgruppe bedeuten, oder durch eine durch eine Alkylcarbonyl-, Arylcarbonyl-, Aralkylcarbonyl-, Heteroarylcarbonyl-, Alkylsulfonyl-, Aralkylsulfonyl- oder Arylsulfonylgruppe substituierte $R_1$NH-Gruppe substituiert sein kann, und in der zusätzlich eine oder zwei -CH=CH-Gruppen jeweils durch eine -CO-NR$_1$-Gruppe ersetzt sein können,

eine gegebenenfalls durch $R_c$ oder $R_d$ substituierte -CO-NH-, -NH-CO-, -CH=N- oder -N=CH-Gruppe darstellen,

einer der Reste $R_a$ bis $R_d$ eine Gruppe der Formel

A - B - C -,

in der

A eine geradkettige oder verzweigte Aminoalkylgruppe mit 1 bis 5 Kohlenstoffatomen, eine Amino-, Amidino- oder Guanidinogruppe, wobei in jeder der vorstehend erwähnten Gruppen jeweils an einem der Stickstoffatome ein oder zwei Wasserstoffatome jeweils durch eine Alkylgruppe mit 1 bis 4 Kohlenstoffatomen oder ein Wasserstoffatom durch eine Alkoxycarbonylgruppe mit insgesamt 2 bis 5 Kohlenstoffatomen, durch eine Alkylcarbonyl-, Arylcarbonyl-, Aryloxycarbonyl- oder Aralkoxycarbonylgruppe ersetzt sein kann, eine Cyanogruppe, eine Cyanoalkylgruppe mit 1 bis 4 Kohlenstoffatomen im Alkylteil oder auch, falls A an ein Stickstoffatom der Reste B oder C gebunden ist, das nicht Teil einer Lactamgruppe ist, ein Wasserstoffatom oder eine Alkylgruppe,

B eine Bindung,

eine Alkylen- oder Alkenylengruppe,

eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, $(R_1)_2N$-, $(R_1)_2NCO$-, $(R_1)_2NSO_2$- oder Nitrogruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1$NH-Gruppen mono- oder disubstituiert sein kann, wobei die Substituenten gleich oder verschieden sein können,

eine Pyridinylen-, Pyrimidinylen-, Pyrazinylen- oder Pyridazinylengruppe, die jeweils im Kohlenstoffgerüst durch eine Alkylgruppe substituiert sein können, wobei zusätzlich eine oder zwei -CH=N-Gruppen jeweils durch eine -CO-NR$_1$-Gruppe ersetzt sein können und eines der Stickstoffatome statt an den Rest $R_1$ auch an den Rest C gebunden sein kann, sofern dieser nicht mit einem Heteroatom oder einer Carbonylgruppe an den Rest B anschließt,

eine Cycloalkylengruppe mit 3 bis 5 Kohlenstoffatomen,

eine Cyclohexylengruppe, in der eine oder zwei CH-Einheiten, die sich zueinander in 1,4-Stellung befinden, durch Stickstoffatome ersetzt sein können, wobei zusätzlich jeweils eine oder zwei der zu einem Stickstoffatom benachbarten Methylengruppen durch eine Carbonylgruppe ersetzt sein können, oder

eine Biphenylengruppe und

C eine gegebenenfalls durch eine Hydroxygruppe substituierte Alkylen- oder Alkenylengruppe,

eine über die Carbonylgruppe mit dem Rest B verbundene Alkylencarbonylgruppe,

eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, $(R_1)_2N$-, $(R_1)_2NCO$-, $(R_1)_2NSO_2$- oder Nitrogruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1$NH-Gruppen mono- oder disubstituiert sein kann, wobei die Substituenten gleich oder verschieden sein können,

eine Indanylen- oder 1,2,3,4-Tetrahydronaphthylengruppe, in denen jeweils der gesättigte Ring an den Rest A und der aromatische Ring an das cyclische Harnstoffgerüst gebunden ist,

eine Pyridinylen-, Pyrimidinylen-, Pyrazinylen- oder Pyridazinylengruppe, die jeweils im Kohlenstoffgerüst durch eine Alkylgruppe substituiert sein können, wobei zusätzlich eine oder zwei -CH=N-Gruppen jeweils durch eine -CO-$NR_1$-Gruppe ersetzt sein können und eines der Stickstoffatome statt an den Rest $R_1$ auch an den Rest B gebunden sein kann, solange dieser keine Bindung bedeutet oder nicht mit einem Heteroatom an den Rest C anschließt,

eine Cyclohexylengruppe, in der eine oder zwei CH-Einheiten, die sich zueinander in 1,4-Stellung befinden, durch Stickstoffatome ersetzt sein können, wobei zusätzlich jeweils eine oder zwei der zu einem Stickstoffatom benachbarten Methylengruppen durch eine Carbonylgruppe ersetzt sein und die Stickstoffatome nicht an ein Stickstoffatom des cyclischen Harnstoffs gebunden sein können,

ein zweiter der Reste $R_a$ bis $R_d$ eine Gruppe der Formel

F - E - D -,

in der

D eine Alkylengruppe mit 1 bis 5 Kohlenstoffatomen oder eine Alkenylengruppe mit 2 bis 5 Kohlenstoffatomen,

eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, Carboxyalkoxy-, Alkoxycarbonylalkoxy-, Aralkoxycarbonylalkoxy-, $(R_1)_2N$-, $(R_1)_2NCO$-, $(R_1)_2NSO_2$- oder Nitrogruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1$NH-Gruppen mono- oder disubstituiert sein kann, wobei die Substituenten gleich oder verschieden sein können,

eine Pyridinylen-, Pyrimidinylen-, Pyrazinylen- oder Pyridazinylengruppe, die jeweils im Kohlenstoffgerüst durch eine Alkylgruppe substituiert sein kann, wobei zusätzlich eine oder zwei -CH=N-Gruppen jeweils durch eine -CO-$NR_1$-Gruppe ersetzt sein können und eines der Stickstoffatome statt an den Rest $R_1$ auch an den Rest E gebunden sein kann, sofern dieser keine Bindung darstellt oder nicht mit einem Heteroatom an den Rest D gebunden ist,

eine Cyclohexylengruppe, in der eine oder zwei CH-Einheiten, die sich zueinander in 1,4-Stellung befinden, durch Stickstoffatome ersetzt sein können, wobei zusätzlich jeweils eine oder zwei der zu einem Stickstoffatom benachbarte Methylengruppen durch eine Carbonylgruppe ersetzt sein können, oder

eine durch den Rest W unterbrochene Alkylengruppe mit 3 bis 6 Kohlenstoffatomen, in der W ein Sauerstoff- oder Schwefelatom, eine Sulfinyl-, Sulfonyl-, $R_1$N-, (Alkylcarbonyl)N-, (Aralkylcarbonyl)N-, (Arylcarbonyl)N-, (Heteroarylcarbonyl)N-, (Alkylsulfonyl)N- oder (Arylsulfonyl)N-Gruppe darstellt und die mit einem Stickstoffatom des cyclischen Harnstoffs verknüpfte Alkylengruppe 2 oder 3 Kohlenstoffatome enthält,

E eine Bindung,

eine Alkylengruppe mit 1 bis 5 Kohlenstoffatomen oder eine Alkenylengruppe mit 2 bis 5 Kohlenstoffatomen, die jeweils durch eine oder zwei Alkylgruppen, durch eine Hydroxy-, Alkoxy-, Amino-, Alkylamino-, Aralkylamino-, Dialkylamino-, Bis-(aralkyl)amino-, Carboxyalkyl-, Alkoxycarbonylalkyl- oder Aralkoxycarbonylalkylgruppe substituiert sein können,

eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, $(R_1)_2N$-, $(R_1)_2NCO$-, $(R_1)_2NSO_2$- oder Nitrogruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1$NH-Gruppen mono- oder disubstituiert sein kann, wobei die Substituenten gleich oder verschieden sein können,

eine Pyridinylen-, Pyrimidinylen-, Pyrazinylen- oder Pyridazinylengruppe, die jeweils im Kohlenstoffgerüst durch eine Alkylgruppe substituiert sein können, wobei zusätzlich eine oder zwei -CH=N-Gruppen jeweils durch eine -CO-$NR_1$-Gruppe ersetzt sein können und eines der Stickstoffatome statt an dem Rest $R_1$ auch an den Rest D gebunden sein kann,

eine Cyclohexylengruppe, in der eine oder zwei CH-Einheiten, die sich zueinander in 1,4-Stellung befinden, durch Stickstoffatome ersetzt sein können, wobei zusätzlich jeweils eine oder zwei der zu einem Stickstoffatom benachbarten Methylengruppen durch eine Carbonylgruppe ersetzt sein können,

eine über den Arylrest mit dem Rest D verknüpfte Alkylenarylengruppe oder

eine über den Rest W' mit dem Rest D verknüpfte Alkylengruppe, in der W' ein Sauerstoff- oder Schwefelatom, eine Sulfinyl-, Sulfonyl-, $R_1$N-, (Alkylcarbonyl)N-, (Aralkylcarbonyl)N-, (Arylcarbonyl)N-, (Heteroarylcarbonyl) N-, (Alkylsulfonyl)N-, (Arylsulfonyl)N- oder Aminocarbonylgruppe, in der das Stickstoffatom an die Alkylengruppe gebunden ist, darstellt,

F eine Carbonylgruppe, die durch eine Hydroxy- oder Alkoxygruppe mit 1 bis 6 Kohlenstoffatomen substituiert ist, wobei ein Alkoxyrest mit 1 bis 3 Kohlenstoffatomen in 1-, 2- oder 3-Stellung durch eine Aryl- oder Heteroarylgruppe oder in 2- oder 3-Stellung durch eine Pyrrolidin-2-on-1-yl-, Morpholino-, Thiomorpholino- oder 1-Oxido-thiomorpholinogruppe substituiert sein kann, eine Sulfo-, Phosphono-, O-Alkylphosphono- oder Tetrazol-5-ylgruppe darstellen, wobei, sofern A eine Cyanogruppe oder eine gegebenenfalls am Stickstoffatom benzoylierte oder benzyloxycarbonylierte Amino- oder Aminoalkylgruppe darstellt,

der kürzeste Abstand zwischen dem Stickstoffatom dieser Gruppen und dem Rest F mindestens 10 Bindungen beträgt,

der dritte der Reste $R_a$ bis $R_d$ ein Wasserstoffatom, eine Alkyl-, Aralkyl-, Aryl- oder Heteroarylgruppe oder auch, wenn der dritte der Reste $R_a$ bis $R_d$ mit einem ungesättigten Kohlenstoffatom des Restes Y verbunden ist, eine Alkoxy-, Alkylsulfenyl-oder $(R_1)_2N$-Gruppe und

der vierte der Reste $R_a$ bis $R_d$ ein Wasserstoffatom, eine Alkyl-, Aralkyl-, Aryl- oder Heteroarylgruppe oder $R_a$ oder $R_b$ zusammen mit einem benachbarten Rest $R_c$ oder $R_d$ auch eine Bindung mit der Maßgabe darstellen, daß die A-B-C-Gruppe keine 4-Piperidinylgruppe darstellt, wenn gleichzeitig die F-E-D-Gruppe eine Alkoxycarbonylalkylgruppe und Y eine durch ein Fluor-, Chlor- oder Bromatom, durch eine Alkyl- oder Trifluormethylgruppe substituierte 1,2-Phenylengruppe bedeuten,

insbesondere diejenigen Verbindungen der allgemeinen Formel I, in denen

X eine gegebenenfalls am Stickstoffatom durch eine Methyl-, Phenyl- oder Pyridylgruppe substituierte Carbiminogruppe, eine Carbonyl-, Thiocarbonyl- oder Sulfonylgruppe,

Y eine gegebenenfalls durch $R_c$ oder $R_d$ oder $R_c$ und $R_d$ substituierte geradkettige Alkylen- oder Alkenylengruppe mit jeweils 2 oder 3 Kohlenstoffatomen, die durch ein Chloratom, durch eine oder zwei Methylgruppen oder durch eine Trifluormethyl-, Phenyl- oder Acetylgruppe substituiert sein kann, wobei zusätzlich eine Methylengruppe durch eine Carbonylgruppe ersetzt sein kann,

eine gegebenenfalls durch $R_c$ oder $R_d$ substituierte -CO-NH-, -NH-CO-, -CH=N- oder -N=CH-Gruppe, eine 1,2-Phenylen- oder 2,3-Pyridinylengruppe,

einer der Reste $R_a$ bis $R_d$ eine Gruppe der Formel

A - B - C -,

in der

A eine geradkettige oder verzweigte Aminoalkylgruppe mit 1 bis 4 Kohlenstoffatomen, eine Amino-, Amidino- oder Guanidinogruppe, wobei in jeder der vorstehend erwähnten Gruppen jeweils an einem der Stickstoffatome ein oder zwei Wasserstoffatome jeweils durch eine Alkylgruppe mit 1 bis 4 Kohlenstoffatomen oder ein Wasserstoffatom durch eine Alkoxycarbonylgruppe mit insgesamt 2 bis 5 Kohlenstoffatomen oder durch eine Benzyloxycarbonylgruppe ersetzt sein kann, oder auch, falls A an ein Stickstoffatom des Restes C gebunden ist, das nicht Teil einer Lactamgruppe ist, ein Wasserstoffatom oder eine Methylgruppe,

B eine Bindung,

eine Phenylengruppe, die durch eine oder zwei Methylgruppen, durch ein Fluor-, Chlor- oder Bromatom, durch eine Methoxy-, Methylsulfenyl-, Methylsulfinyl-, Methylsulfonyl-, Nitro-, Amino-, Acetylamino-, Benzoylamino- oder Methansulfonylaminogruppe substituiert sein kann,

eine Cycloalkylengruppe mit 3 bis 6 Kohlenstoffatomen,

eine Pyridinylen-, Pyrimidinylen-, Pyrazinylen-, Pyridazinylen- oder Biphenylengruppe,

C eine gegebenenfalls durch eine Hydroxygruppe substituierte Ethylengruppe,

eine über die Carbonylgruppe mit dem Rest B verbundene Methylencarbonylgruppe,

eine Phenylengruppe, die durch eine oder zwei Methylgruppen, durch ein Fluor-, Chlor- oder Bromatom, durch eine Methoxy-, Methylsulfenyl-, Methylsulfinyl-, Methylsulfonyl-, Nitro-, Amino-, Acetylamino-, Benzoylamino- oder Methansulfonylaminogruppe substituiert sein kann,

eine Indanylen- oder 1,2,3,4-Tetrahydronaphthylengruppe, in denen jeweils der gesättigte Ring an den Rest A und der aromatische Ring an das cyclische Harnstoffgerüst gebunden ist,

eine Pyridinylen-, Pyrimidinylen-, Pyrazinylen-, Pyridazinylen-, Cyclohexylen- oder Piperidinylengruppe, wobei das Stickstoffatom nicht an ein Stickstoffatom des cyclischen Harnstoffs gebunden sein kann,

ein zweiter der Reste $R_a$ bis $R_d$ eine Gruppe der Formel

F - E - D -,

in der

D eine Alkylengruppe mit 1 bis 4 Kohlenstoffatomen,

eine Phenylengruppe, die durch ein Fluor-, Chlor- oder Bromatom, durch eine Methyl-, Methoxy-, Methylsulfenyl-, Methylsulfinyl-, Methylsulfonyl-, Carboxymethoxy-, Methoxycarbonylmethoxy-, Nitro-, Amino-, Acetylamino-, Benzoylamino- oder Methansulfonylaminogruppe substituiert sein kann,

eine Pyridinylen-, Cyclohexylen- oder Piperidinylengruppe, wobei zusätzlich in einer Pyridinylengruppe eine -CH=N-Gruppe durch eine -CO-NH-Gruppe ersetzt sein kann, wobei das Stickstoffatom statt an das Wasserstoffatom auch den Rest E gebunden sein kann, sofern dieser keine Bindung darstellt oder nicht mit einem Heteroatom an den Rest D gebunden ist, oder

eine durch den Rest W unterbrochene Alkylengruppe mit 3 bis 5 Kohlenstoffatomen, in der W ein Sauerstoff- oder Schwefelatom, eine Sulfinyl-, Sulfonyl-, Imino-, Methylimino-, Acetylimino-, Benzoylimino- oder Methansulfonyliminogruppe darstellt und die mit dem cyclischen Harnstoff verknüpfte Alkylengruppe 2 oder 3 Kohlenstoffatome enthält,

E eine Bindung,

eine gegebenenfalls durch eine oder zwei Methylgruppen, durch eine Hydroxy-, Methoxy-, Amino-, Dimethylamino-, Dibenzylamino-, Carboxymethyl- oder Methoxycarbonylmethylgruppe substituierte Alkylengruppe mit ein bis drei Kohlenstoffatomen oder eine Alkenylengruppe mit zwei oder drei Kohlenstoffatomen,

eine Phenylengruppe oder

eine durch den Rest W' mit der Gruppe D verknüpfte Alkylengruppe mit 1 oder 2 Kohlenstoffatomen, in der W' ein Sauerstoff- oder Schwefelatom, eine Sulfinyl-, Sulfonyl- oder Aminocarbonylgruppe, wobei die Aminogruppe an die Alkylengruppe gebunden ist, darstellt, und

F eine Carbonylgruppe, die durch eine Hydroxygruppe, durch eine Alkoxygruppe mit 1 bis 4 Kohlenstoffatomen oder durch eine Phenylalkoxygruppe mit 1 oder 2 Kohlenstoffatomen im Alkoxyteil substituiert ist, eine Phosphono-, O-Methylphosphono- oder Tetrazol-5-yl-gruppe darstellen, wobei, sofern A eine gegebenenfalls am Stickstoffatom benzyloxycarbonylierte Amino- oder Aminoalkylgruppe darstellt, der kürzeste Abstand zwischen dem Stickstoffatom dieser Gruppe und dem Rest F mindestens 10 Bindungen beträgt,

der dritte der Reste $R_a$ bis $R_d$ ein Wasserstoffatom, eine Methyl-, Ethyl-, Trifluormethyl- oder Phenylgruppe und

der vierte der Reste $R_a$ bis $R_d$ ein Wasserstoffatom oder eine Methylgruppe bedeuten,

deren Tautomere, deren Stereoisomere einschließlich deren Gemische und deren Additionssalze.

Besonders bevorzugte Verbindungen der allgemeinen Formel I sind diejenigen, in denen

X eine Carbonyl- oder Sulfonylgruppe,

Y eine gegebenenfalls durch $R_c$ oder $R_d$ oder $R_c$ und $R_d$ substituierte geradkettige Alkylen- oder Alkenylengruppe mit jeweils 2 oder 3 Kohlenstoffatomen, die durch eine oder zwei Methylgruppen oder durch eine Trifluormethyl- oder Phenylgruppe substituiert sein kann, wobei zusätzlich eine Methylengruppe durch eine Carbonylgruppe ersetzt sein kann, oder eine gegebenenfalls durch $R_c$ oder $R_d$ substituierte -N=CH- oder -CH=N-Gruppe,

einer der Reste $R_a$ bis $R_d$ eine Gruppe der Formel

$$A - B - C -,$$

in der

A eine geradkettige oder verzweigte Aminoalkylgruppe mit 1 bis 4 Kohlenstoffatomen, eine Amino- oder Amidinogruppe, wobei in jeder der vorstehend erwähnten Gruppen jeweils an einem der Stickstoffatome ein Wasserstoffatom durch eine Alkoxycarbonylgruppe mit insgesamt 2 bis 5 Kohlenstoffatomen oder durch eine Benzyloxycarbonylgruppe ersetzt sein kann,

B eine Bindung,

eine Phenylengruppe, die durch ein Fluor- oder Chloratom substituiert sein kann,

eine Cyclopropylen-, Pyridinylen-, Pyrimidinylen-, Pyrazinylen- oder Pyridazinylengruppe,

C eine Phenylengruppe, die durch eine oder zwei Methylgruppen, durch ein Fluor-, Chlor- oder Bromatom, durch eine Methoxy-, Methylsulfenyl-, Methylsulfinyl-, Methylsulfonyl-, Amino-, Acetylamino-, Benzoylamino- oder Methansulfonylaminogruppe substituiert sein kann, oder auch, wenn A eine Aminogruppe und B eine Bindung darstellen, eine Indanylen- oder 1,2,3,4-Tetrahydronaphthylengruppe, in denen jeweils der gesättigte Ring an den Rest A und der aromatische Ring an das cyclische Harnstoffgerüst gebunden ist,

eine Pyridinylen-, Pyrimidinylen-, Pyrazinylen-, Pyridazinylen-, Cyclohexylen- oder Piperidinylengruppe, wobei

das Stickstoffatom nicht an ein Stickstoffatom des cyclischen Harnstoffs gebunden sein kann,

ein zweiter der Reste $R_a$ bis $R_d$ eine Gruppe der Formel

F - E - D -,

in der

D eine Alkylengruppe mit 1 bis 4 Kohlenstoffatomen,

eine Phenylengruppe, die durch ein Fluor-, Chlor- oder Bromatom, durch eine Methyl-, Methoxy-, Methylsulfenyl-, Methylsulfinyl- oder Methylsulfonylgruppe substituiert sein kann,

eine Pyridinylen-, Cyclohexylen- oder Piperidinylengruppe, wobei zusätzlich in einer Pyridinylengruppe die -CH=N-Gruppe durch eine -CO-NH-Gruppe ersetzt sein kann und das Stickstoffatom statt an das Wasserstoffatom auch an den Rest E gebunden sein kann, sofern dieser keine Bindung darstellt oder nicht mit einem Heteroatom an den Rest D gebunden ist, oder

eine $-CH_2CH_2-N(COCH_3)-CH_2-$Gruppe, in der der Ethylenteil an den cyclischen Harnstoff gebunden ist,

E eine Bindung,

eine gegebenenfalls durch eine oder zwei Methylgruppen oder durch eine Amino- oder Dibenzylaminogruppe substituierte Ethylengruppe,

eine Ethenylen- oder Phenylengruppe oder

eine durch den Rest W' mit der Gruppe D verknüpfte Methylengruppe, in der W' ein Sauerstoff- oder Schwefelatom, eine Sulfinyl- oder Sulfonylgruppe darstellt, und

F eine Carbonylgruppe, die durch eine Hydroxygruppe oder durch eine Alkoxygruppe mit 1 bis 4 Kohlenstoffatomen substituiert ist, eine Phosphono-, O-Methylphosphono- oder Tetrazol-5-yl-Gruppe darstellen, wobei, sofern A eine gegebenenfalls am Stickstoffatom benzyloxycarbonylierte Amino- oder Aminoalkylgruppe darstellt, der kürzeste Abstand zwischen dem Stickstoffatom dieser Gruppe und dem Rest F mindestens 10 Bindungen beträgt,

der dritte der Reste $R_a$ bis $R_d$ ein Wasserstoffatom, eine Methyl-, Ethyl- oder Phenylgruppe und

der vierte der Reste $R_a$ bis $R_d$ ein Wasserstoffatom oder eine Methylgruppe bedeuten,

deren Tautomere, deren Stereoisomere einschließlich deren Gemische und deren Additionssalze.

Ganz besonders bevorzugte Verbindungen der obigen allgemeinen Formel I sind diejenigen, in denen

X eine Carbonyl- oder Sulfonylgruppe,

Y eine gegebenenfalls durch $R_c$ oder $R_d$ und gegebenenfalls durch eine Methyl- oder Phenylgruppe substituierte Ethylen- oder Ethenylengruppe oder eine gegebenenfalls durch eine Methylgruppe substituierte Carbonylmethylen- oder Methylencarbonylgruppe, oder eine gegebenenfalls durch $R_c$ oder $R_d$ substituierte -CH=N- oder -N=CH-Gruppe,

einer der Reste $R_a$ bis $R_d$ eine Gruppe der Formel

A - B - C -,

in der

A eine gegebenenfalls durch eine Alkoxycarbonylgruppe mit insgesamt 2 bis 5 Kohlenstoffatomen substituierte Aminomethyl-, Aminoethyl- oder Amidinogruppe,

B eine Bindung oder eine 1,4-Phenylengruppe und

C eine gegebenenfalls durch eine Methylgruppe substituierte 1,4-Phenylengruppe, eine 3,6-Pyridazinylen- oder 1,4-Piperidinylengruppe, wobei jeweils das Stickstoffatom nicht an ein Stickstoffatom des cyclischen Harnstoffs gebunden sein kann,

oder, wenn A eine Aminogruppe und B eine Bindung darstellen, eine Indanylengruppe, in der der gesättigte Ring an den Rest A und der aromatische Ring an das cyclische Harnstoffgerüst gebunden ist,

ein zweiter der Reste $R_a$ bis $R_d$ eine Gruppe der Formel

F - E - D -,

in der

D eine Alkylengruppe mit 1 bis 4 Kohlenstoffatomen, eine 1,4-Phenylen- oder 1,4-Cyclohexylengruppe,

E eine Bindung,

eine gegebenenfalls durch eine Amino- oder Dibenzylaminogruppe substituierte Ethylengruppe oder eine Ethenylengruppe,

eine 1,4-Phenylengruppe oder

eine durch den Rest W' mit der Gruppe D verknüpfte Methylengruppe, in der W' ein Sauerstoff- oder Schwefelatom, eine Sulfinyl- oder Sulfonylgruppe darstellt,

F eine Carbonylgruppe, die durch eine Hydroxygruppe oder durch eine Alkoxygruppe mit 1 bis 4 Kohlenstoffatomen substituiert ist, darstellen, wobei, sofern A eine Aminomethylgruppe darstellt, der kürzeste Abstand zwischen dem Stickstoffatom dieser Gruppe und dem Rest F mindestens 10 Bindungen beträgt,

der dritte der Reste $R_a$ bis $R_d$ ein Wasserstoffatom oder eine Methyl-, Ethyl- oder Phenylgruppe und

der vierte der Reste $R_a$ bis $R_d$ ein Wasserstoffatom oder eine Methylgruppe bedeuten,

insbesondere diejenigen Verbindungen der allgemeinen Formel I, in denen sich zwischen den Anknüpfungsstellen derjenigen der Reste $R_a$ bis $R_d$, die die A-B-C- und F-E-D-Gruppen darstellen, am cyclischen Harnstoff ein weiteres Ringglied befindet,

deren Tautomere, deren Stereoisomere einschließlich deren Gemische und deren Additionssalze.

Die neuen Verbindungen lassen sich beispielsweise nach folgenden Verfahren herstellen:

a) Zur Herstellung von Verbindungen der allgemeinen Formel I, in der F eine Carboxygruppe darstellt: Umwandlung einer Verbindung der allgemeinen Formel

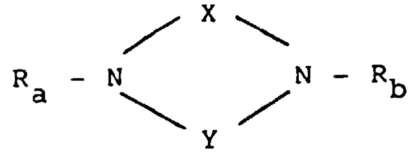

,(II)

in der
$R_a$, $R_b$, X und Y mit der Maßgabe wie eingangs definiert sind, daß einer der Reste $R_a$ bis $R_d$ eine Gruppe der Formel

F' - E - D -

darstellt, in der

E und D wie eingangs definiert sind und
F' eine mittels Hydrolyse, Behandeln mit Säuren, Thermolyse oder Hydrogenolyse in eine Carboxylgruppe überführbare Gruppe bedeutet, in eine Verbindung der allgemeinen Formel I, in der F eine Carboxylgruppe darstellt.

Beispielsweise können funktionelle Derivate der Carboxylgruppe wie deren unsubstituierte oder substituierte Amide, Ester, Thioester, Trimethylsilylester, Orthoester, Iminoester, Amidine oder Anhydride, oder die Nitrilgruppe mittels Hydrolyse in eine Carboxylgruppe,
Ester mit tertiären Alkoholen, z.B. der tert. Butylester, mittels Behandlung mit einer Säure oder Thermolyse in eine Carboxylgruppe und
Ester mit Aralkanolen, z.B. der Benzylester, mittels Hydrogenolyse in eine Carboxylgruppe übergeführt werden.
Die Hydrolyse wird zweckmäßigerweise entweder in Gegenwart einer Säure wie Salzsäure, Schwefelsäure, Phosphorsäure, Trichloressigsäure oder Trifluoressigsäure, in Gegenwart einer Base wie Lithiumhydroxid, Natriumhydroxid oder Kaliumhydroxid in einem geeigneten Lösungsmittel wie Wasser, Wasser/Methanol, Wasser/Äthanol, Wasser/Isopropanol, Methanol, Äthanol, oder Wasser/Dioxan bei Temperaturen zwischen -10 und 120°C, z.B. bei Temperaturen zwischen Raumtemperatur und der Siedetemperatur des Reaktionsgemisches, durchgeführt. Bei der Behandlung mit einer organischen Säure wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.
Bedeutet F' in einer Verbindung der Formel II eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen O und 50°C in die Carboxylgruppe übergeführt werden.
Bedeutet F' in einer Verbindung der Formel II beispielsweise die tert. Butyloxycarbonylgruppe, so kann die tert. Butylgruppe auch durch Behandlung mit einer Säure wie Trifluoressigsäure, Ameisensäure, p-Toluolsulfonsäure, Schwefelsäure, Phosphorsäure oder Polyphosphorsäure gegebenenfalls in einem inerten Lösungsmittel wie Methylenchlorid, Chloroform, Benzol, Toluol, Tetrahydrofuran oder Dioxan vorzugsweise bei Temperaturen zwischen -10 und 120°C, z.B. bei Temperaturen zwischen 0 und 60°C, oder auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z. B. bei Temperaturen zwischen 40 und 100°C, abgespalten werden.
Bedeutet F' in einer Verbindung der Formel II beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Ethanol, Ethanol/Wasser, Eisessig, Essigsäureäthylester, Dioxan oder Dimethylformamid vorzugsweise bei Temperaturen zwischen 0 und 50°C, z.B. bei Raumtemperatur, und einem Wasserstoffdruck von 1 bis 5 bar abgespalten werden. Bei der Hydrogenolyse können gleichzeitig andere Reste, z.B. eine Nitrogruppe zur Aminogruppe, oder eine Benzyloxygruppe zur Hydroxygruppe mitreduziert werden.

b) Zur Herstellung von Verbindungen der allgemeinen Formel I, in der A eine gegebenenfalls durch eine Alkylgruppe substituierte $H_2N$-C(=NH)-Gruppe darstellt:
Umsetzung einer gegebenenfalls im Reaktionsgemisch gebildeten Verbindung der allgemeinen Formel

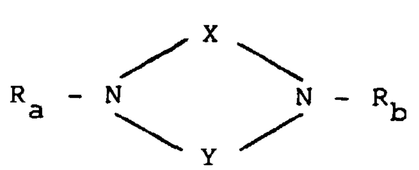

,(III)

in der

$R_a$, $R_b$, X und Y mit der Maßgabe wie eingangs definiert sind, daß einer der Reste $R_a$ bis $R_d$ eine Gruppe der Formel

$$Z_1 - C(=NH) - B -$$

darstellt, in der
B und C wie eingangs definiert sind und
$Z_1$ eine Alkoxy- oder Aralkoxygruppe wie die Methoxy-, Ethoxy-, n-Propoxy-, Isopropoxy- oder Benzyloxygruppe oder eine Alkylthio- oder Aralkylthiogruppe wie die Methylthio-, Ethylthio-, n-Propylthio- oder Benzylthiogruppe oder eine Aminogruppe darstellt, mit einem Amin der allgemeinen Formel

$$R_4 - NH_2 \qquad ,(IV)$$

in der
$R_4$ ein Wasserstoffatom oder eine Alkylgruppe mit 1 bis 4 Kohlenstoffatomen darstellt, oder mit deren Säureadditionssalzen.

Die Umsetzung wird zweckmäßigerweise in einem Lösungsmittel wie Methanol, Ethanol, n-Propanol, Wasser, Methanol/Wasser, Tetrahydrofuran oder Dioxan bei Temperaturen zwischen 0 und 150°C, vorzugsweise bei Temperaturen zwischen 20 und 120°C, mit einem entsprechenden freien Amin oder mit einem entsprechenden Säureadditionssalz wie beispielsweise den entsprechenden Ammoniumcarbonaten, -acetaten oder -chloriden durchgeführt.

Eine Verbindung der allgemeinen Formel III erhält man beispielsweise durch Umsetzung eines entsprechenden Nitrils mit einem entsprechenden Alkohol wie Methanol, Ethanol, n-Propanol, Isopropanol oder Benzylalkohol in Gegenwart einer Säure wie Salzsäure oder durch Umsetzung eines entsprechenden Amids mit einem Trialkyloxoniumsalz wie Triethyloxonium-tetrafluorborat in einem Lösungsmittel wie Methylenchlorid, Tetrahydrofuran oder Dioxan bei Temperaturen zwischen 0 und 50°C, vorzugsweise jedoch bei 20°C, oder eines entsprechenden Nitrils mit Schwefelwasserstoff zweckmäßigerweise in einem Lösungsmittel wie Pyridin oder Dimethylformamid und in Gegenwart einer Base wie Triethylamin und anschließender Alkylierung des gebildeten Thioamids mit einem entsprechenden Alkyl- oder Aralkylhalogenid oder durch Umsetzung eines entsprechenden Nitrils mit einem Alkoholat wie Natriummethylat in einem Lösungsmittel wie Dioxan oder Tetrahydrofuran, vorzugsweise jedoch in dem betreffenden Alkohol.

c) Zur Herstellung von Verbindungen der allgemeinen Formel I, in der mindestens einer der Reste B, C, D oder E eine Sulfinyl- oder Sulfonylgruppe enthält:
Oxidation einer Verbindung der allgemeinen Formel

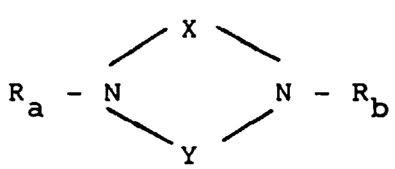

,(V)

in der
$R_a$, $R_b$, X und Y mit der Maßgabe wie eingangs definiert sind, daß mindestens einer der Reste Y, B, C, D oder E eine Sulfenyl- oder Sulfinylgruppe enthält.

Die Oxidation wird vorzugsweise in einem Lösungsmittel oder Lösungsmittelgemisch, z.B. in Wasser, Wasser/Pyridin, Aceton, Methylenchlorid, Eisessig, Eisessig/Acetanhydrid, verdünnter Schwefelsäure oder Trifluoressigsäure, je nach dem verwendeten Oxidationsmittel zweckmäßigerweise bei Temperaturen zwischen -80 und 100°C durchgeführt.

Zur Herstellung einer entsprechenden S-Oxidverbindung der allgemeinen Formel I wird die Oxidation zweckmäßigerweise mit einem Äquivalent des verwendeten Oxidationsmittels durchgeführt, z.B. mit Wasserstoffperoxid in Eisessig, Trifluoressigsäure oder Ameisensäure bei 0 bis 20°C oder in Aceton bei 0 bis 60°C, mit einer Persäure wie Perameisensäure in Eisessig oder Trifluoressigsäure bei 0 bis 50°C oder mit m-Chlorperbenzoesäure in Methylenchlorid oder Chloroform bei -20 bis 60°C, mit Natriummetaperjodat in wäßrigem Methanol oder Äthanol bei -15 bis 25°C, mit Brom in Eisessig oder wäßriger Essigsäure gegebenenfalls in Gegenwart einer schwachen Base wie Natriumacetat, mit N-Brom-succinimid in Äthanol, mit tert.Butyl-hypochlorit in Methanol bei -80 bis -30°C, mit Jodbenzodichlorid in wäßrigem Pyridin bei 0 bis 50°C, mit Salpetersäure in Eisessig bei 0 bis 20°C, mit Chromsäure in Eisessig oder in Aceton bei 0 bis 20°C und mit Sulfurylchlorid in Methylenchlorid bei -70°C, der hierbei erhaltene Thioäther-Chlor-Komplex wird zweckmäßigerweise mit wäßrigem Äthanol drolysiert.

Zur Herstellung einer S,S-Dioxidverbindung der allgemeinen Formel I wird die Oxidation ausgehend von einer entsprechenden Alkylsulfinylverbindung zweckmäßigerweise mit einem oder mehr Äquivalenten des verwendeten Oxidationsmittels oder ausgehend von einer entsprechenden Alkylsulfenylverbindung zweckmäßigerweise mit zwei oder mehr Äquivalenten des verwendeten Oxidationsmittels durchgeführt, z.B. mit Wasserstoffperoxid in Eisessig/Acetanhydrid, Trifluoressigsäure oder in Ameisensäure bei 20 bis 100°C oder in Aceton bei 0 bis 60°C, mit einer Persäure wie Perameisensäure oder m-Chlorperbenzoesäure in Eisessig, Trifluoressigsäure, Methylenchlorid oder Chloroform bei Temperaturen zwischen 0 und 60°C, mit Salpetersäure in Eisessig bei 0 bis 20°C, mit Chromsäure oder Kaliumpermanganat in Eisessig, Wasser/Schwefelsäure oder in Aceton bei 0 bis 20°C.

d) Zur Herstellung von Verbindungen der allgemeinen Formel I, in der Y eine gegebenenfalls durch $R_c$ oder $R_d$ oder $R_c$ und $R_d$ substituierte geradkettige Alkylengruppe mit 2 bis 4 Kohlenstoffatomen darstellt, die durch Alkyl-, Trifluormethyl-, Aralkyl-, Aryl- oder Heteroarylgruppen mono- oder disubstituiert sein kann:

Hydrierung einer Verbindung der allgemeinen Formel

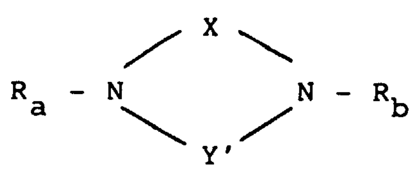

,(VI)

in der

$R_a$, $R_b$ und X wie eingangs definiert sind und
Y' eine gegebenenfalls durch $R_c$ oder $R_d$ oder $R_c$ und
$R_d$ substituierte geradkettige Alkenylengruppe mit 2 bis 4 Kohlenstoffatomen darstellt, die durch Alkyl-, Trifluormethyl-, Aralkyl-, Aryl- oder Heteroarylgruppen mono- oder disubstituiert sein kann.

Die Hydrierung erfolgt in einem Lösungsmittel wie Methanol, Ethanol, Essigsäureethylester oder Eisessig gegebenenfalls unter Zusatz einer Säure wie Salzsäure mit Wasserstoff in Gegenwart eines Katalysators wie Palladium/Kohle oder Platin bei Temperaturen zwischen 0 und 100°C, vorzugsweise jedoch zwischen 20 und 50°C, und bei einem Wasserstoffdruck von 1 bis 7 bar, vorzugsweise jedoch von 3 bis 5 bar.

e) Zur Herstellung von Verbindungen der allgemeinen Formel I, in der A eine durch eine Alkoxycarbonylgruppe mit insgesamt 2 bis 5 Kohlenstoffatomen, durch eine Aralkoxycarbonyl-, Aryloxycarbonyl-, Alkylcarbonyl- oder Arylcarbonylgruppe substituierte Aminoalkyl-, Amidino- oder Guanidinogruppe darstellt:

Umsetzung einer Verbindung der allgemeinen Formel

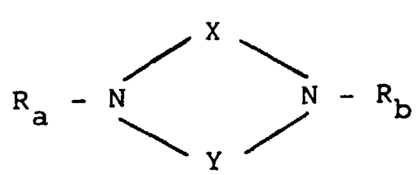

,(VII)

in der

$R_a$, $R_b$, X und Y mit der Maßgabe wie eingangs definiert sind, daß einer der Reste $R_a$ bis $R_d$ eine Gruppe der Formel

A' - B - C -

darstellt, in der
B und C wie eingangs definiert sind und
A' eine $H_2N$-$C_{1-5}$alkyl-, $H_2N$-C(=NH)- oder $H_2N$-C(=NH)-NH-Gruppe darstellt, mit einer Verbindung der allgemeinen Formel

$Z_2$ - $R_5$ ,(VIII)

in der
$R_5$ eine Alkoxycarbonylgruppe mit insgesamt 2 bis 5 Kohlenstoffatomen, eine Aralkoxycarbonyl-, Aryloxycarbonyl-, Alkylcarbonyl- oder Arylcarbonylgruppe und
$Z_2$ eine nukleophile Austrittsgruppe wie ein Halogenatom, z. B. ein Chlor- oder Bromatom, eine Aryloxy-, Arylthio-, Alkoxycarbonyloxy-, Aralkoxycarbonyloxy- oder Imidazolylgruppe darstellen.

Die Acylierung wird zweckmäßigerweise in einem Lösungsmittel wie Tetrahydrofuran, Methylenchlorid, Chloroform, Dimethylformamid, Wasser oder Gemischen aus diesen Lösungsmitteln gegebenenfalls in Gegenwart einer Base wie Natriumcarbonat, Kaliumcarbonat oder Natronlauge oder in Gegenwart einer tertiären organischen Base wie Triethylamin, N-Ethyl-diisopropylamin, N-Methyl-morpholin oder Pyridin, welche gleichzeitig als Lösungsmittel dienen können, bei Temperaturen zwischen -30 und 100°C, vorzugsweise jedoch bei Temperaturen zwischen -10 und 80°C, durchgeführt.

f) Zur Herstellung von Verbindungen der allgemeinen Formel I, in der F eine durch eine Alkoxygruppe mit 1 bis 6 Kohlenstoffatomen substituierte Carbonylgruppe darstellt, wobei ein Alkoxyrest mit 1 bis 3 Kohlenstoffatomen in 1-, 2- oder 3-Stellung durch eine Aryl- oder Heteroarylgruppe oder in 2- oder 3-Stellung durch eine Pyrrolidin-2-on-1-yl-, Morpholino-, Thiomorpholino- oder 1-Oxido-thiomorpholinogruppe substituiert sein kann:
Umsetzung einer Verbindung der allgemeinen Formel

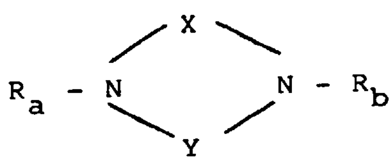 ,(IX)

in der

$R_a$, $R_b$, X und Y mit der Maßgabe wie eingangs definiert sind, daß einer der Reste $R_a$ bis $R_d$ eine Gruppe der Formel

F" - E - D -

darstellt, in der
E und D wie eingangs definiert sind und
F" eine Carboxy- oder Alkoxycarbonylgruppe darstellt, mit einem Alkohol der allgemeinen Formel

HO - $R_6$ ,(X)

in der

$R_6$ eine Alkylgruppe mit 1 bis 6 Kohlenstoffatomen, die in 1-, 2- oder 3-Stellung durch eine Aryl- oder Heteroarylgruppe oder in 2- oder 3-Stellung durch eine Pyrrolidin-2-on-1-yl-, Morpholino-, Thiomorpholino- oder 1-Oxido-thiomorpholinogruppe substituiert sein kann, darstellt.

Die Umsetzung wird zweckmäßigerweise in einem Lösungsmittel oder Lösungsmittelgemisch wie Methylenchlorid, Dimethylformamid, Dimethylsulfoxid, Benzol, Toluol, Chlorbenzol, Tetrahydrofuran, Benzol/Tetrahydrofuran oder Dioxan gegebenenfalls in Gegenwart einer Säure wie Salzsäure oder in Gegenwart eines wasserentziehenden Mittels, z.B. in Gegenwart von Chlorameisensäureisobutylester, Thionylchlorid, Trimethylchlorsilan, Salzsäure, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol und gegebenenfalls zusätzlich in Gegenwart von 4-Dimethylamino-pyridin, N,N'-Carbonyldiimidazol oder N,N'-Thionyldiimidazol oder Triphenylphosphin/Tetrachlorkohlenstoff, zweckmäßigerweise bei Temperaturen zwischen 0 und 150°C, vorzugsweise bei Temperaturen zwischen 0 und 50°C, durchgeführt.

Die Umsetzung einer entsprechende Alkoxyverbindung mit einem Alkohol der allgemeinen Formel X wird vorzugsweise in einem entsprechenden Alkohol als Lösungsmittel gegebenenfalls in Gegenwart eines weiteren Lösungsmittels wie Methylenchlorid oder Ether vorzugsweise in Gegenwart einer Säure wie Salzsäure bei Temperaturen zwischen 0 und 100°C, vorzugsweise bei Temperaturen zwischen 20 und 80°C, durchgeführt.

g) Zur Herstellung von Verbindungen der allgemeinen Formel I, in der A eine $NH_2$-C(=NH)-Gruppe und B oder, falls B eine Bindung darstellt, C eine gegebenenfalls durch eine Alkyl-, Aralkyl- oder Arylgruppe substituierte Cycloalkylengruppe mit 4 oder 5 Kohlenstoffatomen, in der eine CH-Einheit durch ein Stickstoffatom ersetzt ist, oder eine gegebenenfalls durch eine Alkyl-, Aralkyl- oder Arylgruppe substituierte Cycloalkylengruppe mit 6 oder 7 Kohlenstoffatomen, in der eine oder zwei zueinander in 1,4-Stellung befindliche CH-Einheiten jeweils durch ein Stickstoffatom ersetzt sind, wobei B oder, falls B eine Bindung darstellt, C über eines der genannten Stickstoffatome mit dem Rest A verknüpft ist, darstellen:

Umsetzung einer Verbindung der allgemeinen Formel

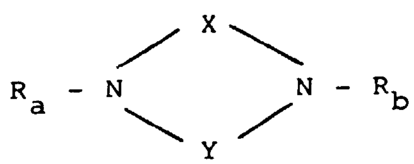

,(XI)

in der

$R_a$, $R_b$, X und Y mit der Maßgabe wie eingangs definiert sind, daß einer der Reste $R_a$ bis $R_d$ eine Gruppe der Formel

H - B' - C - oder H - C' -

darstellt, in der

C wie eingangs definiert ist und

B' oder C' eine gegebenenfalls durch eine Alkyl-, Aralkyl- oder Arylgruppe substituierte Cycloalkylengruppe mit 4 oder 5 Kohlenstoffatomen, in der eine CH-Einheit durch ein Stickstoffatom ersetzt ist, oder eine gegebenenfalls durch eine Alkyl-, Aralkyl- oder Arylgruppe substituierte Cycloalkylengruppe mit 6 oder 7 Kohlenstoffatomen, in der eine oder zwei zueinander in 1,4-Stellung befindliche CH-Einheiten jeweils durch ein Stickstoffatom ersetzt sind, wobei das Wasserstoffatom mit einem Stickstoffatom des Restes B' oder C' verknüpft ist, bedeuten, mit einer Verbindung der allgemeinen Formel

$Z_3$-C(=NH)-$NH_2$ ,(XII)

in der

$Z_3$ eine nukleophile Austrittsgruppe wie eine Alkoxy- oder Alkylthiogruppe, z. B. eine Methyl- oder Äthylthiogruppe, darstellt.

Die Umsetzung wird zweckmäßigerweise in einem Lösungsmittel oder Lösungsmittelgemisch wie Dimethylformamid, Dimethylsulfoxid, Benzol, Toluol, Chlorbenzol, Tetrahydrofuran, Benzol/Tetrahydrofuran oder Dioxan vorzugsweise in Gegenwart eines säurebindenden Mittels, z.B. eines Alkoholats wie Kaliumtert.butylat, eines Alkalihydroxids wie Natrium- oder Kaliumhydroxid, eines Alkalicarbonats wie Natriumcarbonat oder Kaliumcarbonat oder eines Alkalihydrids wie Natriumhydrid zweckmäßigerweise bei Temperaturen zwischen 50 und 150°C, vorzugsweise bei Temperaturen zwischen 75 und 125°C, durchgeführt.

h) Zur Herstellung von Verbindungen der allgemeinen Formel I, in der A eine $H_2N-CH_2-V$-Gruppe, in der V eine Bindung oder eine geradkettige oder verzweigte Alkylengruppe mit 1 bis 4 Kohlenstoffatomen bedeutet, darstellt:
Reduktion einer Verbindung der allgemeinen Formel

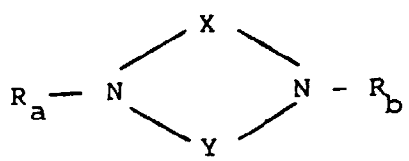

,(XIII)

in der

$R_a$, $R_b$, X und Y mit der Maßgabe wie eingangs definiert sind, daß einer der Reste $R_a$ bis $R_d$ eine Gruppe der Formel

NC - V - B - C -

darstellt, in der
B und C wie eingangs definiert sind und
V eine Bindung oder eine geradkettige oder verzweigte Alkylengruppe mit 1 bis 4 Kohlenstoffatomen darstellt.

Die Reduktion wird vorzugsweise in einem geeigneten Lösungsmittel wie Methanol, Methanol/Wasser, Methanol/Wasser/Ammoniak, Ethanol, Ether, Tetrahydrofuran, Dioxan oder Dimethylformamid gegebenenfalls unter Zusatz einer Säure wie Salzsäure in Gegenwart von katalytisch angeregtem Wasserstoff, z.B. von Wasserstoff in Gegenwart von Raney-Nickel, Platin oder Palladium/Kohle, oder in Gegenwart eines Metallhydrids wie Natriumborhydrid, Lithiumborhydrid oder Lithiumaluminiumhydrid bei Temperaturen zwischen 0 und 100°C, vorzugsweise bei Temperaturen zwischen 20 und 80°C, durchgeführt.

i) Zur Herstellung von Verbindungen der allgemeinen Formel I, in der C eine durch eine Hydroxygruppe substituierte Alkylengruppe darstellt:
Reduktion einer Verbindung der allgemeinen Formel

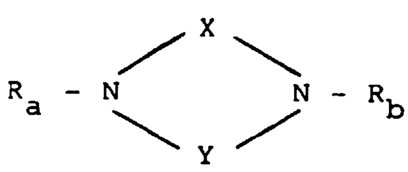

,(XIV)

in der

$R_a$, $R_b$, X und Y mit der Maßgabe wie eingangs definiert sind, daß einer der Reste $R_a$ bis $R_d$ eine Gruppe der Formel

A - B - C'' -

darstellt, in der
A und B wie eingangs definiert sind und
C'' eine Alkylengruppe darstellt, in der eine Methylengruppe durch eine Carbonylgruppe ersetzt ist.

Die Reduktion wird in einem geeigneten Lösungsmittel wie Methanol, Methanol/Wasser, Ethanol, Ether, Tetrahydrofuran, Dioxan oder Eisessig in Gegenwart von katalytisch angeregtem Wasserstoff, z.B. von Wasserstoff in Gegenwart von Platin oder Palladium/Kohle, oder in Gegenwart eines Metallhydrids wie Natriumborhydrid, Lithiumborhydrid oder Lithiumaluminiumhydrid bei Temperaturen zwischen -5 und 20°C, vorzugsweise jedoch bei Temperaturen zwischen 0 und 10°C, durchgeführt.

j) Zur Herstellung von Verbindungen der allgemeinen Formel I, in der A eine $H_2N-C(=NH)-NH$-Gruppe darstellt:
Umsetzung einer Verbindung der allgemeinen Formel

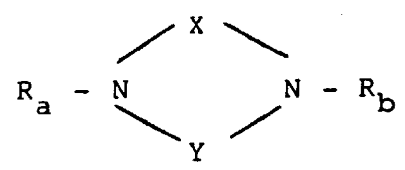

,(XV)

in der

$R_a$, $R_b$, X und Y mit der Maßgabe wie eingangs definiert sind, daß einer der Reste $R_a$ bis $R_d$ eine Gruppe der Formel

$H_2N$ - B - C -

darstellt, in der
B und C wie eingangs definiert sind, mit Cyanamid oder dessen Säureadditionssalz oder mit einem S-Alkylisothioharnstoff, O-Methylisothioharnstoff oder 1-Amidino-3,5-dimethylpyrazol.

Die Umsetzung wird zweckmäßigerweise in einem Lösungsmittel wie Dioxan, Dioxan/Wasser oder Tetrahydrofuran vorzugsweise bei Temperaturen zwischen 60 und 120°C, z.B. bei der Siedetemperatur des Reaktionsgemisches, durchgeführt.

k) Cyclisierung einer Verbindung der allgemeinen Formel

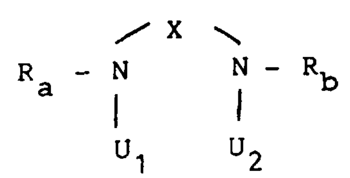

,(XVI)

in der

$R_a$, $R_b$ und X wie eingangs definiert sind,
einer der Reste $U_1$ oder $U_2$ ein Wasserstoffatom und der andere der Reste $U_1$ oder $U_2$ eine Gruppe der Formel

- Y'' - $Z_4$,

in der
Y'' eine gegebenenfalls durch $R_c$ oder $R_d$ oder $R_c$ und $R_d$ substituierte geradkettige Alkylen- oder Alkenylengruppe mit jeweils 2 bis 4 Kohlenstoffatomen, in denen jedes Kohlenstoffatom durch eine Alkyl-, Trifluormethyl-, Aralkyl-, Aryl-, Heteroaryl- oder Alkylcarbonylgruppe mono- oder disubstituiert sein kann, wobei die Substituenten gleich oder verschieden sein können, oder eine gegebenenfalls durch $R_c$ oder $R_d$ oder $R_c$ und $R_d$ substituierte 1,2-Cycloalkylengruppe mit 4 bis 7 Kohlenstoffatomen oder eine 1,2-Cycloalkenylengruppe mit 4 bis 7 Kohlenstoffatomen, eine gegebenenfalls durch die Reste $R_c$ oder $R_d$ substituierte -CH=N-Gruppe, in der der Stickstoffatom mit einem der Stickstoffatome in Formel XVI verknüpft ist, oder eine gegebenenfalls durch $R_c$ oder $R_d$ substituierte $-CH_2$-NH-Gruppe und
$Z_4$ eine nukleophile Austrittsgruppe wie ein Halogenatom, eine Hydroxy-, Alkoxy- oder Sulfonsäureestergruppe, z.B. ein Chlor-, Brom- oder Jodatom, eine Methoxy-, Ethoxy-, Isopropyloxy-, Methansulfonyloxy- oder p-Toluolsulfonyloxygruppe, oder zusammen mit einer benachbarten Methylengruppe des Restes Y'' eine Carbonyl-, Carboxy-, Alkoxycarbonyl-, Aralkoxycarbonyl-, Aryloxycarbonyl- oder Dialkoxymethylgruppe bedeuten.

Die Umsetzung wird vorzugsweise in einem Lösungsmittel wie Ethanol, Isopropanol, Methylenchlorid, Dioxan, Toluol, Dimethylformamid oder Dimethylsulfoxid gegebenenfalls in Gegenwart einer Base wie Pyridin, einer Säure wie Salzsäure, Schwefelsäure, Polyphosphorsäure oder Trifluoressigsäure und eines wasserentziehenden Mittels wie N,N'-Dicyclohexylcarbodiimid bei Temperaturen zwischen 20 und 200°C durchgeführt. Die Umsetzung kann jedoch auch ohne Lösungsmittel durchgeführt werden.

Bedeutet $Z_4$ eine nukleophile Austrittsgruppe wie ein Halogenatom oder eine Sulfonestergruppe, so wird die Umsetzung vorzugsweise in Gegenwart einer Base wie Kaliumcarbonat, Natriumhydrid oder Kalium-tert.butylat bei Temperaturen swischen 20 und 60°C,
bedeutet der Rest $Z_4$ eine Hydroxy- oder Alkoxygruppe oder zusammen mit einer benachbarten Methylengruppe des Restes Y'' eine Carbonyl-, Carboxy-, Alkoxycarbonyl-, Aralkoxycarbonyl-, Aryloxycarbonyl- oder Dialkoxymethylgruppe, so wird die Umsetzung vorzugsweise in Gegenwart einer Säure wie Salzsäure oder Trifluoressigsäure, welche gleichzeitig auch als Lösungsmittel dienen können, bei Temperaturen zwischen 20 und 80°C, oder auch in der Schmelze bei Temperaturen zwischen 50 und 250°C, vorzugsweise bei Temperaturen zwischen 100 und 200°C, durchgeführt.

l) Zur Herstellung von Verbindungen der allgemeinen Formel I, in der $R_a$ oder $R_c$ eine E-F-D-Gruppe darstellt:
Cyclisierung einer gegebenenfalls im Reaktionsgemisch gebildeten Verbindung der allgemeinen Formel

$$R_c - CO - CHR_d - NR_a - CO - NHR_b \quad ,(XVII)$$

in der
$R_a$ bis $R_d$ wie eingangs definiert sind, und gegebenenfalls anschließende Hydrierung.

Die Cyclisierung wird vorzugsweise in einem Lösungsmittel wie Wasser, Ethanol/Wasser, Ethanol, Benzol, Toluol oder Dioxan und zweckmäßigerweise in Gegenwart einer Base wie Pyridin, welche gleichzeitig als Lösungsmittel dienen kann, bei erhöhten Temperaturen, z.B. bei der Siedetemperatur des verwendeten Lösungsmittels, durchgeführt.

Die gegebenenfalls anschließende Hydrierung erfolgt vorzugsweise mit Wasserstoff in Gegenwart eines Katalysators wie Palladium/Kohle oder Platin in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

m) Zur Herstellung von Verbindungen der allgemeinen Formel I, in der X eine Carbonylgruppe darstellt:
Umsetzung einer Verbindung der allgemeinen Formel

$$R_a - NH - Y - NH - R_b \quad ,(XVIII)$$

in der

$R_a$, $R_b$ und Y wie eingangs definiert sind, mit einer Verbindung der allgemeinen Formel

$$Z_5 - CO - Z_6 \quad ,(XIX)$$

in der

$Z_5$ und $Z_6$, die gleich oder verschieden sein können, nukleophile Austrittsgruppen wie Halogenatome, Alkoxy- oder Aryloxygruppen, z.B. jeweils ein Chloratom oder eine Methoxy-, Ethoxy- oder Phenyloxygruppe, darstellen.

Die Umsetzung wird vorzugsweise in einem Lösungsmittel wie Methylenchlorid, Chloroform, Toluol oder Dioxan gegebenenfalls in Gegenwart einer Base wie Triethylamin oder Pyridin bei Temperaturen zwischen 0 und 50°C, vorzugsweise bei Raumtemperatur, durchgeführt.

n) Zur Herstellung von Verbindungen der allgemeinen Formel I, in der $R_a$ bis $R_d$ wie eingangs mit der Maßgabe definiert sind, daß mindestens einer der Reste $R_a$ und $R_b$ kein Wasserstoffatom darstellt:

Umsetzung einer Verbindung der allgemeinen Formel

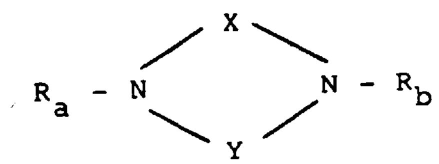

,(XX)

in der

X und Y wie eingangs definiert sind,
einer der Reste $R_a$ oder $R_b$ ein Wasserstoffatom darstellt und
der andere der Reste $R_a$ oder $R_b$ wie eingangs definiert ist, mit einer Verbindung der allgemeinen Formel

$$Z_7 - R' \quad ,(XXI)$$

in der

R' mit Ausnahme eines Wasserstoffatoms die für $R_a$ oder $R_b$ eingangs erwähnten Bedeutungen besitzt und
$Z_7$ eine nukleophile Austrittsgruppe wie ein Halogenatom oder einen Sulfonsäureesterrest, z.B. ein Chlor-, Brom- oder Jodatom oder eine Methansulfonyloxy- oder p-Toluolsulfonyloxygruppe

oder, falls $R_a$ oder $R_b$ eine -D-COO-alkylgruppe mit der Maßgabe darstellt, daß sich zwischen dem Stickstoffatom des cyclischen Harnstoffs und der Alkoxycarbonylgruppe zwei Kohlenstoffatome befinden, Umsetzung mit einer Verbindung der allgemeinen Formel

- D' -COO-alkyl ,(XXII)

in der
D' die für D eingangs erwähnten Bedeutungen mit der Maßgabe besitzt, daß der Alkoxycarbonylgruppe unmittelbar eine Kohlenstoff-Kohlenstoff-Doppel- oder Dreifachbindung vorausgeht.

Die Alkylierung wird zweckmäßigerweise in einem Lösungsmittel oder Lösungsmittelgemisch wie Methylenchlorid, Dimethylformamid, Dimethylsulfoxid, Benzol, Toluol, Chlorbenzol, Tetrahydrofuran, Benzol/Tetrahydrofuran oder Dioxan vorzugsweise in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid oder eines Alkalihydrids wie Natriumhydrid oder einer tertiären organischen Base wie Ethyl-diisopropylamin zweckmäßigerweise bei Temperaturen zwischen 0 und 150°C, vorzugsweise bei Temperaturen zwischen 0 und 50°C, durchgeführt.

Die Arylierung wird zweckmäßigerweise mit einer Arylverbindung der allgemeinen Formel XXI, in der $Z_7$ ein Jodatom darstellt, vorzugsweise in einem Lösungsmittel wie Toluol oder Xylol und vorzugsweise in Gegenwart

von einem oder mehreren Reaktionsbeschleunigern wie Tris-[2-(2-methoxy-ethoxy)ethyl]amin, Kupfer(I)chlorid oder Kupfer(II)chlorid bei erhöhten Temperaturen, z.B. bei Temperaturen zwischen 100 und 200°C, vorzugsweise jedoch bei der Siedetemperatur des Reaktionsgemisches, durchgeführt. Die Umsetzung kann jedoch auch ohne Lösungsmittel durchgeführt werden.

Die Addition einer Alkenylverbindung der allgemeinen Formel XXII wird vorzugsweise in einem Lösungsmittel wie Dimethylformamid und in Gegenwart einer Base wie Natriumhydrid bei Temperaturen zwischen 0 und 50°C, vorzugsweise bei Raumtemperatur, durchgeführt.

o) Zur Herstellung von Verbindungen der allgemeinen Formel I, in der F eine Carboxy-, Alkoxycarbonyl-, Aralkoxycarbonyl- oder Aryloxycarbonylgruppe darstellt:

Oxidation einer Verbindung der allgemeinen Formel

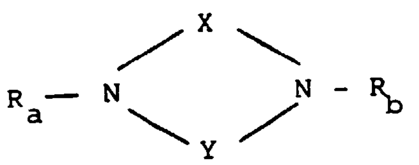

,(XXIII)

in der

$R_a$, $R_b$, X und Y mit der Maßgabe wie eingangs definiert sind, daß einer der Reste $R_a$ bis $R_d$ eine Gruppe der Formel

$$CH_2 = CH - E - D -$$

darstellt, wobei E und D wie eingangs definiert sind, und gegebenenfalls anschließende Veresterung.

Die Oxidation wird in einem Lösungsmittel wie Methylenchlorid, Acetonitril, Acetonitril/Wasser, Methylenchlorid/Acetonitril/Wasser oder Tetrachlorkohlenstoff/Acetonitril/Wasser in Gegenwart eines Oxidationsmittels wie Kaliumpermanganat oder Rutheniumtetroxid, wobei das Rutheniumtetroxid vorzugsweise im Reaktionsgemisch durch Umsetzung eines Rutheniumsalzes wie Rutheniumtrichlorid mit einem Oxidationsmittel wie Natriumperjodat gebildet wird, bei Temperaturen zwischen -10 und 50°C, vorzugsweise bei Temperaturen zwischen 15 und 30°C, durchgeführt.

Die gegebenenfalls anschließende Veresterung wird zweckmäßigerweise in einem geeigneten Lösungsmittel, z.B. in einem entsprechenden Alkohol, Pyridin, Toluol, Methylenchlorid, Tetrahydrofuran oder Dioxan, in Gegenwart eines säureaktivierenden und/oder wasserentziehenden Mittels wie Chlorwasserstoff, konzentrierte Schwefelsäure, Thionylchlorid, Chlorameisensäureäthylester, Carbonyldiimidazol oder N,N'-Dicyclohexyl-carbodiimid oder dessen Isoharnstoffestern, gegebenenfalls in Gegenwart eines Reaktionsbeschleunigers wie Kupferchlorid, oder durch Umesterung, z.B. mit einem entsprechenden Kohlensäurediester, bei Temperaturen zwischen 0 und 100°C, vorzugsweise jedoch bei Temperaturen zwischen 20°C und der Siedetemperatur des betreffenden Lösungsmittels, durchgeführt.

p) Zur Herstellung von Verbindungen der allgemeinen Formel I, in der F eine O-Alkyl-phosphonogruppe darstellt:

Umsetzung einer Verbindung der allgemeinen Formel

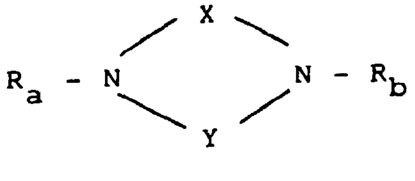

,(XXIV)

in der

$R_a$, $R_b$, X und Y mit der Maßgabe wie eingangs definiert sind, daß F eine Dialkoxyphosphorylgruppe darstellt, mit einem Alkalijodid.

Die Umsetzung wird vorzugsweise in einem Lösungsmittel wie Methylethylketon in Gegenwart eines Alkalijodids wie Natriumjodid bei Temperaturen zwischen 25 und 100°C, vorzugsweise bei der Siedetemperatur des Reaktionsgemisches, durchgeführt.

q) Zur Herstellung von Verbindungen der allgemeinen Formel I, in der F eine Phosphonogruppe darstellt:
Umsetzung einer Verbindung der allgemeinen Formel

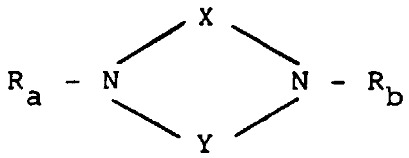

,(XXV)

in der
$R_a$, $R_b$, X und Y mit der Maßgabe wie eingangs definiert sind, daß F eine O-Alkylphosphono- oder Dialkoxyphosphorylgruppe darstellt, mit einem Alkalijodid in Gegenwart von einem Trialkylhalogensilan.

Die Umsetzung wird vorzugsweise in einem Lösungsmittel wie Acetonitril in Gegenwart eines Alkalijodids wie Natriumjodid und eines Trialkylhalogensilans wie Trimethylchlorsilan bei Temperaturen zwischen 25 und 80°C, vorzugsweise jedoch bei Temperaturen zwischen 30 und 50°C, durchgeführt.

r) Zur Herstellung von Verbindungen der allgemeinen Formel I, in der W eine $R_1N$-Gruppe darstellt:
Umsetzung einer Verbindung der allgemeinen Formel

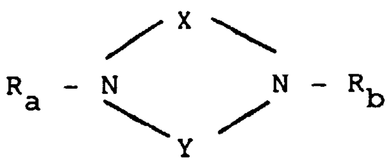

,(XXVI)

in der

$R_a$, $R_b$, X und Y mit der Maßgabe wie eingangs definiert sind, daß einer der Reste $R_a$ bis $R_d$ eine $Z_8$-D''-Gruppe darstellt, wobei D'' eine Alkylengruppe mit 1 bis 3 Kohlenstoffatomen und
$Z_8$ eine nukleophile Austrittsgruppe wie ein Halogenatom oder einen Sulfonsäureesterrest, z.B. ein Chlor-, Brom- oder Jodatom oder eine Methansulfonyloxy- oder p-Toluolsulfonyloxygruppe, darstellen, mit einer Verbindung der allgemeinen Formel

$$R_1NH - E' - F \qquad ,(XXVII)$$

in der
F und $R_1$ wie eingangs definiert sind und
E' eine Alkylengruppe mit 1 bis 3 Kohlenstoffatomen bedeutet.

Die Umsetzung wird zweckmäßigerweise in einem Lösungsmittel oder Lösungsmittelgemisch wie Methylenchlorid, Dimethylformamid, Dimethylsulfoxid, Benzol, Toluol, Chlorbenzol, Tetrahydrofuran, Benzol/Tetrahydrofuran oder Dioxan vorzugsweise in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid oder eines Alkalihydrids wie Natriumhydrid oder einer tertiären organischen Base wie Ethyl-diisopropylamin zweckmäßigerweise bei Temperaturen zwischen 0 und 150°C, vorzugsweise bei Temperaturen zwischen 0 und 50°C, durchgeführt.

s) Zur Herstellung von Verbindungen der allgemeinen Formel I, in der A eine Amino- oder Aminoalkylgruppe darstellt:
Umsetzung einer Verbindung der allgemeinen Formel

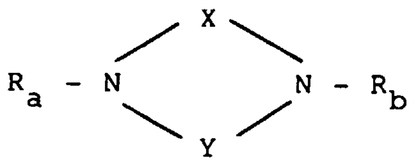

,(XXVIII)

in der

$R_a$, $R_b$, X und Y mit der Maßgabe wie eingangs definiert sind, daß einer der Reste $R_a$ bis $R_d$ eine $H_2N$-CO-T-B-C-Gruppe darstellt, wobei B und C wie eingangs definiert sind und
T eine Bindung oder eine Alkylengruppe mit 1 bis 5 Kohlenstoffatomen darstellt, mit einer Phenyljod(III)verbindung der allgemeinen Formel

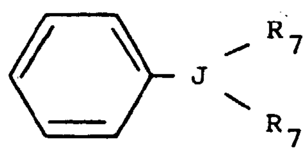

,(XXIX)

in der
$R_7$ jeweils den Acylrest einer organischen Carbonsäure wie die Acetoxy- oder Trifluoracetoxygruppe darstellt.

Die Umsetzung wird vorzugsweise in einem wässrigen Lösungsmittel wie Wasser oder Wasser/Acetonitril bei Temperaturen zwischen 0 und 50°C, vorzugsweise jedoch bei Raumtemperatur, durchgeführt.

t) Zur Herstellung von Verbindungen der allgemeinen Formel I, in der A eine durch eine oder zwei Alkylgruppen am Stickstoffatom substituierte Amino- oder Aminoalkylgruppe darstellt:
Umsetzung einer Verbindung der allgemeinen Formel

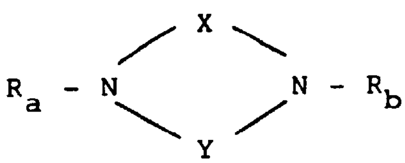

,(XXX)

in der

$R_a$, $R_b$, X und Y mit der Maßgabe wie eingangs definiert sind, daß einer der Reste $R_a$ bis $R_d$ eine Gruppe der Formel

$$A'' - B - C -$$

darstellt, in der
B und C wie eingangs definiert sind und
A'' eine Amino-, Alkylamino-, Aminoalkyl- oder Alkylaminoalkylgruppe darstellt, mit einer Verbindung der allgemeinen Formel

$$Z_9 - (R_8\text{-C-}R_9) - Z_{10} \qquad ,(XXXI)$$

in der
$R_8$ und $R_9$, die gleich oder verschieden sein können, Wasserstoffatome oder Alkylgruppen,
eine der Gruppen $Z_9$ oder $Z_{10}$ eine nukleophile Austrittsgruppe wie ein Halogenatom, z.B. ein Chlor-, Brom- oder Jodatom, oder eine Sulfonsäureestergruppe, z.B. eine Methansulfonyloxy- oder p-Toluolsulfonyloxygruppe, und
die andere der Gruppen $Z_9$ oder $Z_{10}$ ein Wasserstoffatom oder eine Alkylgruppe oder
$Z_9$ und $Z_{10}$ zusammen ein Sauerstoffatom bedeuten.

Die Alkylierung mit einer Verbindung der Formel XXXI, in der $Z_9$ oder $Z_{10}$ eine nukleophile Austrittsgruppe darstellt, wird zweckmäßigerweise in einem Lösungsmittel wie Tetrahydrofuran, Dioxan, Dimethylsulfoxid oder Dimethylformamid gegebenenfalls in Gegenwart einer Base wie Natriumcarbonat, Kaliumcarbonat oder Natronlauge oder in Gegenwart einer tertiären organischen Base wie N-Ethyl-diisopropylamin oder N-Methylmorpholin, welche gleichzeitig als Lösungsmittel dienen können, bei Temperaturen zwischen -30 und 100°C, vorzugsweise jedoch bei Temperaturen zwischen -10 und 80°C, durchgeführt.

Die Alkylierung mit einer Carbonylverbindung der allgemeinen Formel XXXI wird vorzugsweise in Gegenwart eines komplexen Metallhydrids wie Natriumborhydrid, Lithiumborhydrid oder Natriumcyanborhydrid zweckmäßigerweise bei einem pH-Wert von 6-7 und bei Raumtemperatur oder in Gegenwart eines Hydrierungskatalysators, z.B. mit Wasserstoff in Gegenwart von Palladium/Kohle, bei einem Wasserstoffdruck von 5 bar durchgeführt.

u) Zur Herstellung von Verbindungen der allgemeinen Formel I, in der A eine Cyanogruppe darstellt:
Umsetzung einer Verbindung der allgemeinen Formel

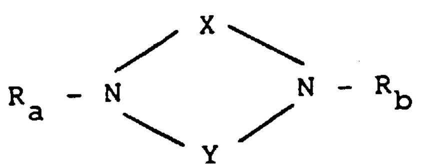
,(XXXII)

in der

$R_a$, $R_b$, X und Y mit der Maßgabe wie eingangs definiert sind, daß einer der Reste $R_a$ bis $R_d$ eine Gruppe der Formel

$$A''' - B - C -$$

darstellt, in der
B und C wie eingangs definiert sind und
A''' ein Halogenatom, z.B. ein Brom- oder Jodatom, darstellt, mit Kupfer(I)cyanid.

Die Umsetzung wird vorzugsweise in einem Lösungsmittel wie Dimethylformamid, Dimethylacetamid oder N-Methyl-pyrrolidon bei Temperaturen zwischen 100 und 250°C, vorzugsweise zwischen 150°C und der Siedetemperatur der Reaktionsmischung, durchgeführt.

v) Zur Herstellung von Verbindungen der allgemeinen Formel I, in der A eine Aminoalkylgruppe, in der die Aminogruppe nicht an ein quartäres Kohlenstoffatom gebunden ist, oder eine Aminogruppe darstellt, die an eine CH- oder $CH_2$-Gruppe des Restes B oder C gebunden ist, darstellt:
Reduktion einer Verbindung der allgemeinen Formel

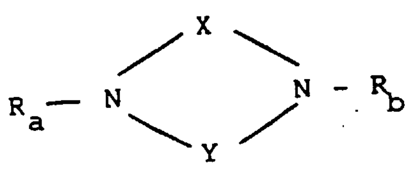
,(XXXIII)

in der

$R_a$, $R_b$, X und Y mit der Maßgabe wie eingangs definiert sind, daß einer der Reste $R_a$ bis $R_d$ eine Gruppe der Formel

A'''' - B - C -

darstellt, in der
B und C wie eingangs definiert sind und
A'''' eine N-Hydroxy-iminogruppe enthält.

Die Reduktion wird vorzugsweise in einem geeigneten Lösungsmittel wie Methanol, Methanol/Wasser, Methanol/Wasser/Ammoniak, Ethanol, Ether, Tetrahydrofuran, Dioxan oder Dimethylformamid, gegebenenfalls unter Zusatz einer Säure wie Salzsäure in Gegenwart von katalytisch angeregtem Wasserstoff, z.B. von Wasserstoff in Gegenwart von Raney-Nickel, Platin oder Palladium/Kohle, bei Temperaturen zwischen 0 und 100°C, vorzugsweise bei Temperaturen zwischen 20 und 80°C, durchgeführt.

Erhält man erfindungsgemäß eine Verbindung der allgemeinen Formel I, so kann diese mittels Bromierung in eine entsprechende Bromverbindung der allgemeinen Formel I übergeführt werden oder

eine Verbindung der allgemeinen Formel I, so kann diese mittels Nitrierung in eine entsprechende Nitroverbindung der allgemeinen Formel I übergeführt werden oder

eine Verbindung der allgemeinen Formel I, die eine Nitrogruppe enthält, so kann diese mittels Reduktion in eine entsprechende Aminoverbindung übergeführt werden oder

eine Verbindung der allgemeinen Formel I, die eine $R_1$NH-Gruppe enthält oder in der W eine Iminogruppe darstellt, so kann diese mittels Acylierung oder Sulfonierung in eine entsprechende Verbindung der allgemeinen Formel I übergeführt werden, die eine durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1$NH-Gruppen enthält, oder

eine Verbindung der allgemeinen Formel I, in der X eine Carbonylgruppe darstellt, so kann diese mittels einem Schwefelungsmittel in eine entsprechende Thiocarbonylverbindung übergeführt werden.

Die nachträgliche Bromierung wird vorzugsweise in einem Lösungsmittel wie Eisessig mit einem Bromierungsmittel wie Brom bei Temperaturen zwischen 0 und 40°C, vorzugsweise bei Raumtemperatur, durchgeführt.

Die nachträgliche Nitrierung wird mit einem Nitrierungsmittel wie konzentrierte Schwefelsäure/Salpetersäure oder rauchende Salpetersäure, wobei diese zweckmäßigerweise als Lösungsmittel dienen, gegebenenfalls in einem Lösungsmittel wie Nitrobenzol bei Temperaturen zwischen 0 und 50°C, vorzugsweise bei Raumtemperatur, durchgeführt.

Die nachträgliche Reduktion der Nitrogruppe wird vorzugsweise in einem Lösungsmittel wie Wasser, Wasser/Ethanol, Methanol, Eisessig, Essigsäurethylester oder Dimethylformamid zweckmäßigerweise mit Wasserstoff in Gegenwart eines Hydrierungskatalysators wie Raney-Nickel, Platin oder Palladium/Kohle, mit Metallen wie Eisen, Zinn oder Zink in Gegenwart einer Säure wie Zink/Essigsäure oder Zink/Calciumchlorid, mit Salzen wie Eisen(II)sulfat, Zinn(II)chlorid, Natriumsulfid, Natriumhydrogensulfit oder Natriumdithionit, oder mit Hydrazin in Gegenwart von Raney-Nickel bei Temperaturen zwischen 0 und 100°C, vorzugsweise jedoch bei Temperaturen zwischen 20 und 80°C, durchgeführt.

Die nachträgliche Acylierung oder Sulfonylierung einer $R_1$NH-gruppe wird zweckmäßigerweise in einem Lösungsmittel wie Methylenchlorid, Chloroform, Tetrachlorkohlenstoff, Ether, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureethylester, Thionylchlorid, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, gegebenenfalls in Gegenwart einer anorganischen Base wie Natriumcarbonat oder einer tertiären organischen Base wie Triethylamin, Pyridin oder 4-Dimethylaminopyridin, welche gleichzeitig als Lösungsmittel dienen können, bei Temperaturen zwischen -25 und 150°C, vorzugsweise jedoch bei Temperaturen zwischen -10°C und der Siedetemperatur des verwendeten Lösungsmittels, durchgeführt. Die nachträgliche Acylierung oder Sulfonylierung wird jedoch wie vorstehend beschrieben vorzugsweise mit einem entsprechenden Säurehalogenid oder Säureanhydrid durchgeführt, wobei diese auch ohne Lösungsmittel

durchgeführt werden kann.

Die Umsetzung wird mit einem Schwefelungsmittel wie Phosphorpentasulfid oder 2,4-Bis-(4-methoxyphenyl)-1,3-di-thia-2,4-diphosphetan-2,4-disulfid zweckmäßigerweise in einem Lösungsmittel wie Pyridin, Toluol oder Xylol bei Temperaturen zwischen 50 und 150°C, z.B. bei der Siedetemperatur des Reaktionsgemisches, durchgeführt.

Bei den vorstehend beschriebenen Umsetzungen a) bis v) und den nachträglichen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe,

als Schutzreste für eine Carboxylgruppe die Trimethylsilyl-, Methyl-, Ethyl-, tert.Butyl-, Benzyl- oder Tetrahydropyranylgruppe und
als Schutzrest für eine Amino-, Alkylamino- oder Iminogruppe die Acetyl-, Benzoyl-, Ethoxycarbonyl-, tert.Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder mittels Etherspaltung, z.B. in Gegenwart von Jodtrimethylsilan, bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen.

Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestes 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)- oder

(-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxylgruppe enthalten, gewünschtenfalls anschließend in ihre Additionssalze mit anorganischen oder organischen Basen, insbesondere für die pharmazeutische Anwendung in ihre physiologisch verträglichen Additionssalze, überführen. Als Basen kommen hierbei beispielsweise Natriumhydroxid, Kaliumhydroxid, Cyclohexylamin, Äthanolamin, Diäthanolamin und Triäthanolamin in Betracht.

Die als Ausgangsstoffe verwendeten Verbindungen sind teilweise literaturbekannt oder man erhält diese nach literaturbekannten Verfahren (siehe Beispiele), z.B. nach den in der deutschen Offenlegungsschriften DE-A-4.035.961 und DE-A-4.102.024 beschriebenen Verfahren.

Beispielsweise erhält man die cyclischen Harnstoffderivate durch Cyclisierung eines entsprechend substituierten Harnstoffs, welcher seinerseits nach bekannten Verfahren erhalten wird, oder durch Umsetzung eines entsprechend substituierten Diamins mit Phosgen und gegebenenfalls anschließende Schwefeleinführung und Oxidation einer so erhaltenen Thioverbindung.

Wie bereits eingangs erwähnt, weisen die neuen cyclischen Harnstoffderivate der allgemeinen Formel I und deren Additionssalze, insbesondere deren physiologisch verträgliche Additionssalze mit anorganischen oder organischen Säuren oder Basen, wertvolle Eigenschaften auf. So weisen die neuen Verbindungen der allgemeinen Formel I, in denen A eine gegebenenfalls substituierte Amino-, Amidino- oder Guanidinogruppe oder eine gegebenenfalls in vivo in eine gegebenenfalls substituierte Amino-, Amidino- oder Guanidinogruppe überführbare Gruppe, z.B. eine durch eine Alkoxycarbonylgruppe substituierte Amino-, Amidino- oder Guanidinogruppe, enthält und -D-E-F Carboxyl-, Sulfo-, Phosphono-, O-Alkyl-phosphono- oder 5-Tetrazolylgruppen oder in vivo in eine Carboxyl-, Sulfo-, Phosphono-, O-Alkyl-phosphono- oder Tetrazolylgruppe überführbare Gruppen, z.B. durch eine Alkoxygruppe substituierte Carbonylgruppen, enthält, wertvolle pharmakologische Eigenschaften auf, neben einer entzündungshemmenden und den Knochenabbau hemmenden Wirkung insbesondere antithrombotische, antiaggregatorische und tumor- bzw. metastasenhemmende Wirkungen.

Die Verbindungen der allgemeinen Formel I, in der A eine Cyano- oder Cyanoalkylgruppe darstellt, stellen wertvolle Zwischenprodukte zur Herstellung der entsprechenden Aminomethyl- und Amidinoverbindungen der allgemeinen Formel I dar.

Beispielsweise wurden die Verbindungen der allgemeinen Formel I auf ihre biologischen Wirkungen wie folgt untersucht:

Fibrinogen-Bindung an Humanthrombozyten

Das durch Punktion einer Antekubitalvene gewonnene Blut wird mit Trinatriumcitrat (Endkonzentration: 13 mM) antikoaguliert und 10 Minuten bei 170 ∗g zentrifugiert. Das überstehende plättchenreiche Plasma wird auf eine Sepharose 2B-Säule (Pharmacia) gegeben und mit einer Lösung aus 90 mM Kochsalz, 14 mM Trinatriumcitrat, 5 mM Glucose und 50 mM Tris(hydroxymethyl)aminomethan, eingestellt auf pH 7,4, eluiert. Die vor den Plasmaproteinen erscheinenden gelfiltrierten Plättchen (GFP) werden für die Bindungsversuche verwendet.

50 µl einer 60 mM Calziumchlorid-Lösung, 50 µl einer 0,6 mM Adenosindiphosphat-Lösung, 100 µl Substanzlösung bzw. Lösungsmittel und 50 µl Fibrinogenlösung (enthaltend 3 µg 125-J-Fibrinogen) werden zu 750 µl GFP gegeben und bei Raumtemperatur 20 Minuten inkubiert. Die unspezifische Bindung wird in Gegenwart von 3 mg/ml kaltem Fibrinogen bestimmt.

900 µl des Inkubates werden vorsichtig auf 250 µl Silikonöl (AP 38: AR 20, 1:2 v/v, Wacker Chemie) in Eppendorf-Gefäße pipettiert und 2 Minuten bei 10 000 ∗g zentrifugiert. Der wäßrige Überstand und ein Teil des Öls werden abgezogen, die Gefäßspitze mit dem Plättchenpellet abgeschnitten und im Gamma-Zähler die Menge des gebundenen Fibrinogens bestimmt. Aus einer Konzentrationsreihe wird die Substanzkonzentration ermittelt, welche die Fibrinogenbindung zu 50 % hemmt und als $IC_{50}$ angegeben.

2. Antithrombotische Wirkung

Methodik: Die Thrombozytenaggregation wird nach der Methode von Born und Cross (J. Physiol. 170, 397 (1964)) in plättchenreichem Plasma gesunder Versuchspersonen gemessen. Zur Gerinnungshemmung wird das Blut mit Natriumcitrat 3,14 % im Volumenverhältnis 1:10 versetzt.

Collagen-induzierte Aggregation: Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München. Vor der Collagen-Zugabe wird das Plasma jeweils 10 Minuten mit der Substanz bei 37°C inkubiert.

Aus den erhaltenen Meßzahlen wird graphisch eine $EC_{50}$ bestimmt, die sich auf eine 50%ige Änderung der "optical density" im Sinne einer Aggregationshemmung bezieht.

Die nachfolgende Tabelle enthält die gefundenen Ergebnisse:

| Substanz (Beispiel Nr.) | Fibrinogen-Bindungstest $IC_{50}$[nM] | Hemmung der Plättchenaggregation $EC_{50}$[nM] |
|---|---|---|
| 1 | 1 800 | 9 900 |
| 1(1) | 45 | 1 500 |
| 1(2) | 96 | 320 |
| 1(3) | 190 | 1 700 |
| 1(4) | 3 900 | >100 000 |
| 1(5) | 6 100 | 32 000 |
| 1(6) | 17 | 70 |
| 1(7) | 2 400 | 10 000 |
| 1(21) | 31 | 620 |
| 1(24) | 470 | 1 100 |
| 1(28) | 52 | 390 |
| 1(36) | 37 | 100 |
| 1(46) | 11 | 40 |
| 1(48) | 210 | 1 100 |

| Substanz (Beispiel Nr.) | Fibrinogen-Bindungstest $IC_{50}$[nM] | Hemmung der Plättchenaggregation $EC_{50}$[nM] |
|---|---|---|
| 1(49) | 26 | 140 |
| 1(50) | 45 | 290 |
| 1(51) | 3 600 | 13 000 |
| 1(55) | 860 | 60 000 |
| 1(59) | 150 | 350 |
| 1(62) | 13 | 40 |
| 1(66) | 9,1 | 50 |
| 1(67) | 30 | 60 |
| 1(77) | 4 900 | 8 000 |
| 1(82) | 17 000 | 29 000 |
| 1(94) | 310 | 400 |
| 1(117) | 230 | 5 400 |
| 1(118) | 170 | 460 |
| 1(119) | 210 | 730 |
| 1(137) | | 280 |
| 1(138) | 21 | 40 |
| 1(139) | 6,8 | 30 |
| 1(140) | 21 | 30 |
| 1(141) | 310 | 630 |
| 1(143) | >10 000 | 22 000 |
| 1(144) | | 600 |
| 1(145) | 7,7 | 50 |
| 1(146) | 6,5 | 50 |
| 1(147) | 27 | 160 |
| 1(148) | 25 | 110 |

(fortgesetzt)

| Substanz (Beispiel Nr.) | Fibrinogen-Bindungstest $IC_{50}$[nM] | Hemmung der Plättchenaggregation $EC_{50}$[nM] |
|---|---|---|
| 1(149) | 470 | 1 300 |
| 1(150) | 370 | 9 900 |
| 1(153) | 150 | 380 |
| 1(154) | 28 | 310 |
| 1(156) | 3 600 | 4 100 |
| 2 | 6 000 | 12 000 |
| 2(2) | 25 000 | 630 |
| 2(3) | 18 000 | 3 100 |

| Substanz (Beispiel Nr.) | Fibrinogen-Bindungstest $IC_{50}$[nM] | Hemmung der Plättchenaggregation $EC_{50}$[nM] |
|---|---|---|
| 2(4) | 15 000 | 42 000 |
| 2(5) | 5 600 | 25 000 |
| 2(6) | 240 | 160 |
| 2(20) | 5 700 | 690 |
| 2(27) | 2 500 | 490 |
| 2(34) | 7 400 | 350 |
| 2(43) | 420 | 100 |
| 2(45) | 370 | 280 |
| 2(47) | 32 000 | >100 000 |
| 2(48) | 22 000 | >100 000 |
| 2(53) | 4 500 | 200 |
| 2(57) | 640 | 320 |
| 2(58) | 4 700 | 140 |
| 2(71) | 13 000 | 14 000 |
| 2(75) | 8 000 | 27 000 |
| 2(81) | 19 000 | 1 500 |
| 2(104) | 7 100 | 2 100 |
| 2(105) | 28 000 | 1 100 |
| 2(106) | 2 700 | 6 600 |
| 2(115) | 530 | 80 |
| 2(116) | 59 000 | 49 000 |
| 2(117) | | 630 |
| 2(118) | 2 000 | 70 |
| 2(119) | 280 | 40 |
| 2(122) | | 1 200 |
| 2(123) | 3 100 | 70 |
| 2(124) | 1 200 | 130 |
| 2(127) | 5 600 | 18 000 |
| 4(9) | 2 600 | 9 500 |
| 4(11) | 45 000 | 2 300 |
| 4(13) | 32 | 310 |
| 4(14) | 41 | 200 |
| 4(15) | 42 | 300 |

| Substanz (Beispiel Nr.) | Fibrinogen-Bindungstest $IC_{50}$[nM] | Hemmung der Plättchenaggregation $EC_{50}$[nM] |
|---|---|---|
| 4(16) | 1 500 | 1 900 |

(fortgesetzt)

| Substanz (Beispiel Nr.) | Fibrinogen-Bindungstest $IC_{50}$[nM] | Hemmung der Plättchenaggregation $EC_{50}$[nM] |
|---|---|---|
| 4(18) | 48 | 210 |
| 5 | 9 300 | 32 000 |
| 5(1) | >100 000 | 20 000 |
| 5(8) | 8 000 | 31 000 |
| 5(11) | 5 700 | |
| 5(12) | 3 700 | |
| 5(13) | >10 000 | 24 000 |
| 5(18) | >10 000 | 8 060 |
| 8 | 750 | 600 |
| 8(1) | 68 000 | 21 000 |
| 8(2) | 450 | 370 |
| 8(3) | 29 000 | 6 200 |
| 8(5) | 3 000 | 5 900 |
| 11(11) | | 210 |
| 11(12) | 43 | 30 |
| 18 | 1 900 | 240 |
| 18(5) | 420 | 120 |
| 30 | 4 400 | 8 300 |
| 31 | 250 | 500 |
| 31(1) | 170 | 370 |

Außerdem hemmt beispielsweise die Verbindung des Beispiels 5(18) die durch Collagen induzierte Thrombocytenaggregation ex vivo am Rheusussaffen nach oraler Gabe von 1 mg/kg bis zu 8 Stunden.

Die neuen Verbindungen sind gut verträglich, da beispielsweise nach intravenöser Gabe von 30 mg/kg der Verbindung des Beispiels 1(138) an der Maus keines der drei getesteten Tiere verstarb. Ähnliche Resultate ergaben die Verbindungen der Beispiele 1(66) und 1(139) bei einer Dosis von 30 mg/kg, wobei jedoch in beiden Fällen ein Tier sediert war.

Auf Grund ihrer Hemmwirkung auf Zell-Zell- bzw. Zell-Matrix-Wechselwirkungen eignen sich die neuen cyclischen Harnstoffderivate der allgemeinen Formel I und ihre physiologisch verträglichen Additionssalze zur Bekämpfung bzw. Verhütung von Krankheiten, bei denen kleinere oder größere Zell-Aggregate auftreten oder Zell-Matrixinteraktionen eine Rolle spielen, z.B. bei der Bekämpfung bzw. Verhütung von venösen und arteriellen Thrombosen, von zerebrovasculären Erkrankungen, von Lungenembolien, des Herzinfarktes, der Arteriosklerose, der Osteoporose und der Metastasierung von Tumoren. Weiterhin eignen sich diese zur Begleittherapie bei der Thrombolyse mit Fibrinolytica oder Gefäßinterventionen wie transluminaler Angioplastie oder auch bei der Therapie von Schockzuständen, des Diabetes und von Entzündungen.

Für die Bekämpfung bzw. Verhütung der vorstehend erwähnten Krankheiten liegt die Dosis am Erwachsenen zwischen 0,1 µg und 20 mg/kg Körpergewicht, vorzugsweise bei 1 µg bis 10 mg/kg Körpergewicht, bei bis zu 4 Gaben pro Tag. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Stearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen, Lösungen, Sprays oder Zäpfchen einarbeiten.

Die nachfolgenden Beispiele sollen die Erfindung näher erläutern:

Beispiel I

1-(3-Buten-1-yl)-3-(4'-cyano-4-biphenylyl)-imidazolidin-2-on

9 g 1-(4'-Cyano-4-biphenylyl)-imidazolidin-2-on werden bei 50°C in 300 ml Dimethylformamid gelöst und portionsweise mit 1,6 g einer 55%igen Suspension von Natriumhydrid in Öl versetzt. Man rührt noch 45 Minuten nach, läßt auf

Raumtemperatur abkühlen und tropft zu der erhaltenen Suspension innerhalb von 10 Minuten eine Lösung von 4,16 ml 1-Brom-3-buten in 15 ml Dimethylformamid zu und rührt 3 Tage bei Raumtemperatur. Die Reaktionsmischung wird auf 400 ml Wasser gegossen und der ausgefallene Niederschlag nach Waschen mit Wasser säulenchromatographisch an Kieselgel (Elutionsmittel: Methylenchlorid/Essigester = 9:1) gereinigt.
Ausbeute: 3,6 g (33 % der Theorie),
Schmelzpunkt: 171-175°C
$R_f$-Wert: 0,54 (Kieselgel; Cyclohexan/Essigester = 1:1)

Analog werden folgende Verbindungen erhalten:

(1) N-[4-(2-Methoxycarbonyl-ethyl)-phenyl]-N-[2-(2,3,5,6-tetrahydro-2-pyranyloxy)-ethyl]-trifluoracetamid
Man erwärmt 20 Stunden auf 70-80°C
$R_f$-Wert: 0,47 (Kieselgel; Diisopropylether)

(2) N-[4-(2-Methoxycarbonyl-ethyl)-phenyl]-N-[3-(2,3,5,6-tetrahydro-2-pyranyloxy)-propyl]-trifluoracetamid
Man erwärmt 40 Stunden auf 60°C
$R_f$-Wert: 0,49 (Kieselgel; Diisopropylether)

(3) 1-Methoxycarbonylmethyl-3H-benzimidazol-2-on
Als Base dient Kalium-tert.butylat, als Lösungsmittel Methanol
$R_f$-Wert: 0,89 (Kieselgel; Methylenchlorid/Methanol = 85:15)

(4) 1-(3-Buten-1-yl)-3-(4'-cyano-3'-fluor-4-biphenylyl)-imidazolidin-2-on

(5) 1-(3-Buten-1-yl)-3-(3'-chlor-4'-cyano-4-biphenylyl)-imidazolidin-2-on

(6) 1-(3-Buten-1-yl)-3-(4'-cyano-3-methoxy-4-biphenylyl)-imidazolidin-2-on

(7) 1-(3-Buten-1-yl)-3-(4'-cyano-3-methylthio-4-biphenylyl)imidazolidin-2-on

(8) 1-(3-Buten-1-yl)-3-(4'-cyano-2,3-dimethyl-4-biphenylyl)imidazolidin-2-on

(9) 1-(3-Buten-1-yl)-3-[4-(5-cyano-2-pyridyl)-phenyl]-imidazolidin-2-on

(10) 1-(3-Buten-1-yl)-3-[4-(5-cyano-2-pyrazinyl)-phenyl]-imidazolidin-2-on

(11) 1-(3-Buten-1-yl)-3-[4-(5-cyano-2-pyrimidinyl)-phenyl]-imidazolidin-2-on

(12) 1-(3-Buten-1-yl)-3-[6-(4-cyano-phenyl)-3-pyridazinyl)-imidazolidin-2-on

(13) 1-(3-Buten-1-yl)-3-[2-(4-cyano-phenyl)-5-pyrimidinyl)-imidazolidin-2-on

(14) N-[2-Fluor-4-(2-methoxycarbonyl-ethyl)-phenyl]-N-[2-(2,3,5,6-tetrahydro-2-pyranyloxy)-ethyl]-trifluoractamid

(15) N-[2-Chlor-4-(2-methoxycarbonyl-ethyl)-phenyl]-N-[2-(2,3,5,6-tetrahydro-2-pyranyloxy)-ethyl]-trifluoractamid

(16) N-[2-Methoxy-4-(2-methoxycarbonyl-ethyl)-phenyl]-N-[2-(2,3,5,6-tetrahydro-2-pyranyloxy)-ethyl]-trifluoractamid Den für die Trifluoracetylierung mit Trifluoressigsäureanhydrid erforderlichen 3-(4-Amino-3-methoxy-phenyl)-propionsäuremethylester erhält man aus 3-(3-Methoxy-phenyl)-propionsaüre durch Nitrierung, Veresterung und Reduktion mit Palladiumkohle in Methanol.

(17) N-[4-(2-Methoxycarbonyl-ethyl)-2-methyl-phenyl]-N-[2-(2,3,5,6-tetrahydro-2-pyranyloxy)-ethyl]-trifluoractamid Die 3-(4-Amino-3-methyl-phenyl)-propionsäure erhält man aus 3-(3-Methyl-phenyl)-propionsäure analog Beispiel I (16).

(18) N-[4-(2-Methoxycarbonyl-ethyl)-2-methylthio-phenyl]-N-[2-(2,3,5,6-tetrahydro-2-pyranyl-oxy)-ethyl]-trifluoractamid Die 3-(4-Amino-3-methylthio-phenyl)-propionsäure erhält man aus 3-(4-Amino-phenyl)-propionsäure analog Beispiel III (10).

(19) 1-[6-(4-Cyano-phenyl)-3-pyridazinyl]-imidazolidin-2-on Hergestellt aus Imidazolidin-2-on und 3-Chlor-6-[4-cyano-phenyl)-pyridazin in Dimethylsulfoxid
$R_f$-Wert: 0,30 (Kieselgel; Methylenchlorid/Methanol = 19:1)

(20) 1-(4-Cyano-phenyl)-3-(ethoxycarbonylmethyl)-imidazolidin-2-on
Schmelzpunkt: 112-115°C

Beispiel II

1-(4'-Cyano-4-biphenylyl)-imidazolidin-2-on

Zu einer Lösung von N-(2-Chlorethyl)-N'-(4'-cyano-4-biphenylyl)-harnstoff in 100 ml Dimethylformamid wird bei Raumtemperatur innerhalb von 10 Minuten eine Lösung von 5,7 g Kalium-tert.butylat in 15 ml Dimethylformamid getropft. Man rührt eine Stunde bei Raumtemperatur, gießt auf 300 ml Wasser und filtriert das ausgefallene Produkt ab.
Ausbeute: 13 g (98 % der Theorie),
Schmelzpunkt: über 200°C
$R_f$-Wert: 0,12 (Kieselgel; Cyclohexan/Essigester = 1:1)

Analog werden folgende Verbindungen erhalten:

(1) 1-(4'-Cyano-4-biphenylyl)-3,4,5,6-tetrahydro-1H-pyrimidin-2-on
Schmelzpunkt: über 200°C
$R_f$-Wert: 0,44 (Kieselgel; Methylenchlorid/Methanol = 9:1)

(2) 1-(4'-Cyano-3'-fluor-4-biphenylyl)-imidazolidin-2-on

(3) 1-(3'-Chlor-4'-cyano-4-biphenylyl)-imidazolidin-2-on

(4) 1-(4'-Cyano-3-methoxy-4-biphenylyl)-imidazolidin-2-on

(5) 1-(4'-Cyano-3-methylthio-4-biphenylyl)-imidazolidin-2-on

(6) 1-(4'-Cyano-2,3-dimethyl-4-biphenylyl)-imidazolidin-2-on

(7) 1-[4-(5-Cyano-2-pyridyl)-phenyl]-imidazolidin-2-on

(8) 1-[4-(5-Cyano-2-pyrazinyl)-phenyl]-imidazolidin-2-on

(9) 1-[4-(5-Cyano-2-pyrimidinyl)-phenyl]-imidazolidin-2-on

(10) 1-[2-(4-Cyano-phenyl)-5-pyrimidinyl]-imidazolidin-2-on

(11) 1-[2-(4'-Cyano-4-biphenylyl)-ethyl]-imidazolidin-2-on

(12) 1-(1-Benzyl-4-piperidinyl)-3-[4-(2-methoxycarbonylethyl)-phenyl]-imidazolidin-2-on

(13) 1-(4-Cyano-phenyl)-imidazolidin-2-on
Als Base verwendet man Kaliumcarbonat, wobei man 6 Stunden auf 60°C erwärmt.
Schmelzpunkt: 172-175°C
$R_f$-Wert: 0,23 (Kieselgel; Cyclohexan/Essigester = 1:3)

(14) 1-(4-Cyano-phenyl)-3-[4-[2-(dimethoxy-phosphoryl)-ethyl]-phenyl]-imidazolidin-2-on
Hergestellt analog Beispiel 14.

(15) 1-[4-(2-Methoxycarbonyl-ethyl)-phenyl]-imidazolidin-2-on
Schmelzpunkt: 171-172°C

(16) 2-[4-(2-Methoxycarbonyl-ethyl)-phenyl]-3,4-dihydro-2H,5H-thiadiazol-1,1-dioxid

Schmelzpunkt: 110-112°C

(17) 1-(4-Brom-2-methyl-phenyl)-3-[4-(2-methoxycarbonylethyl)-phenyl]-imidazolidin-2-on
Hergestellt aus N-(4-Brom-2-methyl-phenyl)-N'-(2-hydroxyethyl)-N'-[4-(2-methoxycarbonyl-ethyl)-phenyl]-harnstoff über das Mesylat und Jodid ohne Reinigung dieser Produkte.
Schmelzpunkt: 164-166°C

Beispiel III

N-(2-Chlorethyl)-N'-(4'-cyano-4-biphenylyl)-harnstoff

Zu einer Lösung von 4-Amino-4'-cyano-biphenyl in 15 ml Dimethylformamid gibt man 1,1 ml 2-Chlorethyl-isocyanat und rührt 3 Stunden bei Raumtemperatur. Das Reaktionsgemisch wird auf 50 ml Wasser gegossen, 3 Stunden nachgerührt und das ausgefallene Produkt abfiltriert.
Ausbeute: 1,4 g (91 % der Theorie),
$R_f$-Wert: 0,40 (Kieselgel; Cyclohexan/Essigester = 1:1)

Analog werden folgende Verbindungen erhalten:

(1) N-(4'-Cyano-4-biphenylyl)-N-(2,2-diethoxy-ethyl)-N'-(2-ethoxycarbonyl-ethyl)-harnstoff
Man arbeitet in Dioxan als Lösungsmittel in Gegenwart von Ethyl-diisopropylamin bei 50°C. Die Reinigung erfolgt durch Chromatographie an Kieselgel (Elutionsmittel: Cyclohexan/Essigester = 1:1).
Den verwendeten 3-Isocyanato-propionsäure-ethylester erhält man aus β-Alanin-ethylester-hydrochlorid und Phosgen in Toluol als Lösungsmittel.
Schmelzpunkt: 85-88°C
$R_f$-Wert: 0,28 (Kieselgel; Cyclohexan/Essigester = 1:1)

(2) N-(tert.Butyloxycarbonylmethyl)-N-(4'-cyano-4-biphenylyl)-N'-(2-ethoxycarbonyl-ethyl)-harnstoff
Hergestellt analog Beispiel III (1).
Schmelzpunkt: 118-120°C (aus tert.Butyl-methylether)
$R_f$-Wert: 0,46 (Kieselgel; Cyclohexan/Essigester = 1:1)

(3) N-(4'-Cyano-4-biphenyl)-N-[3-(2,3,5,6-tetrahydro-2-pyranyloxy)-propyl]-N'-(2-ethoxycarbonyl-ethyl)-harnstoff
Hergestellt analog Beispiel III (1).
$R_f$-Wert: 0,49 (Kieselgel; Cyclohexan/Essigester = 1:3)

(4) N-(3-Chlorpropyl)-N'-(4'-cyano-4-biphenylyl)-harnstoff
$R_f$-Wert: 0,39 (Kieselgel; Cyclohexan/Essigester = 1:1)

(5) N-(4-Cyano-phenyl)-N'-(2-hydroxy-ethyl)-N'-[4-(2-methoxycarbonyl-ethyl)-phenyl]-harnstoff
Lösungsmittel: Dioxan
$R_f$-Wert: 0,32 (Kieselgel; Cyclohexan/Essigester = 3:7)

(6) N-(4-Cyano-phenyl)-N'-(3-hydroxy-propyl)-N'-[4-(2-methoxycarbonyl-ethyl)-phenyl]-harnstoff
Lösungsmittel: Dioxan
Schmelzpunkt: 86-89°C
$R_f$-Wert: 0,27 (Kieselgel; Cyclohexan/Essigester = 3:7)

(7) N-(2-Chlorethyl)-N'-(4'-cyano-3'-fluor-4-biphenylyl)-harnstoff
Das als Ausgangsmaterial verwendete 4-Amino-4'-cyano-3'-fluor-biphenyl erhält man aus 4-Brom-2-fluor-benzonitril durch Umsetzung mit Phenylboronsäure in Gegenwart von Palladium(II)acetat und tris-o-Tolyl-phosphin mit anschließender Nitrierung und Reduktion der Nitrogruppe mit 5%iger Palladiumkohle in Essigester.

(8) N-(3'-Chlor-4'-cyano-4-biphenylyl)-N'-(2-chlorethyl)-harnstoff
Das als Ausgangsmaterial verwendete 4-Amino-3'-chlor-4'-cyano-biphenyl erhält man analog Beispiel III (7).

(9) N-(2-Chlorethyl)-N'-(4'-cyano-3-methoxy-4-biphenylyl)-harnstoff
Das als Ausgangsmaterial dienende 4-Amino-4'-cyano-3-methoxy-biphenyl erhält man analog Beispiel III (7).

(10) N-(2-Chlorethyl)-N'-(4'-cyano-3-methylthio-4-biphenylyl)-harnstoff
Das Ausgangsmaterial 4-Amino-4'-cyano-3-methylthio-biphenyl erhält man aus 4'-Amino-4-biphenyl-carbonsäure durch Umsetzung mit Ammoniumrhodanid und Brom in Essigsäure, Verseifung des entstandenen 2-Amino-benzthiazolderivates mit verdünnter Kalilauge, Methylierung der Mercaptogruppe, Schützung der Aminogruppe über das Phthalylderivat, Umwandlung der Carboxylgruppe in eine Cyanogruppe (über das Säurechlorid und das Säureamid mit anschließender Entwässerung mit Phosphoroxychlorid/Pyridin) und Abspaltung des Phthalylrestes mit wäßriger Methylaminlösung.

(11) N-(2-Chlorethyl)-N'-(4'-cyano-2,3-dimethyl-4-biphenylyl)-harnstoff
Das Ausgangsmaterial 4-Amino-4'-cyano-2,3-dimethyl-biphenyl erhält man analog Beispiel III (7).

(12) N-(2-Chlorethyl)-N'-[4-(5-cyano-2-pyridyl)-phenyl]-harnstoff
Das Ausgangsmaterial 2-(4-Amino-phenyl)-5-cyano-pyridin erhält man analog Beispiel III (7), wobei 4-(2,2,5,5-Tetramethyl-1-aza-2,5-disila-1-cyclopentanyl)-phenylboronsäure (hergestellt aus 4-(2,2,5,5-Tetramethyl-1-aza-2,5-disilal-cyclopentanyl)-phenyl-lithium und Trimethoxyboran) verwendet und die Silylschutzgruppe hydrolytisch entfernt wird.

(13) N-(2-Chlorethyl)-N'-[4-(5-cyano-2-pyrazinyl)-harnstoff
Das Ausgangsmaterial 2-(4-Amino-phenyl)-5-cyano-pyrazin erhält man aus 4-Nitro-phenylglyoxal durch Kondensation mit Glycinamid, Behandeln des Produkts mit Phosphoroxytribromid, Bromaustausch mit Kupfer(I)cyanid und anschließende Reduktion der Nitrogruppe analog Beispiel III (7).

(14) N-(2-Chlorethyl)-N'-[4-(5-cyano-2-pyrimidinyl)-phenyl]-harnstoff
Das Ausgangsmaterial 2-(4-Amino-phenyl)-5-cyano-pyrimidin erhält man aus 4-Nitro-benzamidin und 3-Dimethylamino-2-formyl-acrylnitril mit anschließender Reduktion der Nitrogruppe analog Beispiel III (7).

(15) N-(2-Chlorethyl)-N'-[2-(4-cyano-phenyl)-5-pyrimidinyl]-harnstoff
Das Ausgangsmaterial 5-Amino-2-(4-cyano-phenyl)-pyrimidin erhält man, indem man zunächst 4-Amidino-benzoesäureethylester mit Malonsäurediethylester in Gegenwart von Natriummethylat kondensiert, das entstandene Pyrimidindion nitriert und mit Phosphoroxychlorid/Phosphorpentachlorid chloriert und katalytisch hydriert. Das erhaltene 5-Amino-2-(4-ethoxycarbonyl-phenyl)-pyrimidin wird in die Aminocarbonylverbindung überführt und mit Phosphoroxychlorid/Pyridin zum Nitril entwässert.

(16) N-[4-(4-Cyano-phenyl)-cyclohexyl]-N-(2,2-diethoxyethyl)-N'-(2-ethoxycarbonyl-ethyl)-harnstoff
Das Ausgangsmaterial N-[4-(4-Cyano-phenyl)-cyclohexyl]-N-(2,2-diethoxy-ethyl)-amin erhält man durch reduktive Aminierung von 4-(4-Cyano-phenyl)-cyclohexanon mit 2,2-Diethoxyethylamin in Gegenwart von Natriumcyanborhydrid.

(17) N-(2-Chlorethyl)-N'-[2-(4'-cyano-4-biphenylyl)-ethyl]-harnstoff
Das Ausgangsmaterial 2-(4'-Cyano-4-biphenylyl)-ethylamin erhält man aus 2-(4'-Cyano-4-biphenylyl)-ethylbromid und Kaliumphthalimid und anschließender Behandlung mit wäßriger Methylaminlösung.

(18) N-(4-Cyano-3-fluor-phenyl)-N'-(2-hydroxy-ethyl)-N'-[4'-(2-methoxycarbonyl-ethyl)-phenyl]-harnstoff
Das 4-Amino-2-fluor-benzonitril, aus dem man das Isocyanat analog Beispiel III (1) erhält, erhält man aus 2-Fluor-4-nitro-benzoesäure durch Überführen in das Säureamid, Wasserabspaltung durch Erwärmen mit Phosphoroxychlorid/Pyridin und Reduktion mit Wasserstoff in Gegenwart von 5%iger Palladiumkohle in Essigester

(19) N-(3-Chlor-4-cyano-phenyl)-N'-(2-hydroxy-ethyl)-N'-[4 (2-methoxycarbonyl-ethyl)-phenyl]-harnstoff
(Die Isocyanatherstellung erfolgt analog Beispiel III (1))

(20) N-(4-Cyano-2-methylthio-phenyl)-N'-(2-hydroxy-ethyl)-N'-[4-(2-methoxycarbonyl-ethyl)-phenyl]-harnstoff
Das 4-Amino-3-methylthio-benzonitril, das man analog Beispiel III (1) in das Isocyanat überführt, erhält man aus 4-Amino-3-methylthio-benzosäure analog Beispiel III (10).

(21) N-(4-Cyano-2-methyl-phenyl)-N'-(2-hydroxy-ethyl)-N'-[4-(2-methoxycarbonyl-ethyl)-phenyl]-harnstoff
(Die Isocyanatherstellung erfolgt analog Beispiel III (1))

(22) N-(4-Cyano-2-methoxy-phenyl)-N'-(2-hydroxy-ethyl)-N'-[4-(2-methoxycarbonyl-ethyl)-phenyl]-harnstoff

Das Ausgangsmaterial 4-Amino-3-methoxy-benzonitril, das man analog Beispiel III (1) in das Isocyanat überführt, erhält man aus 3-Methoxy-4-nitro-benzoesäure analog Beispiel III (18)

(23) N-(4-Cyano-phenyl)-N'-[2-fluor-4-(2-methoxycarbonyl-ethyl)-phenyl]-N'-(2-hydroxy-ethyl)-harnstoff

(24) N-[2-Chlor-4-(2-methoxycarbonyl-ethyl)-phenyl]-N-(2-hydroxy-ethyl)-N'-(4-cyano-phenyl)-harnstoff

(25) N-(4-Cyano-phenyl)-N'-(2-hydroxy-ethyl)-N'-[2-methoxy-4-(2-methoxycarbonyl-ethyl)-phenyl]-harnstoff

(26) N-(4-Cyano-phenyl)-N'-(2-hydroxy-ethyl)-N'-[4-(2-methoxycarbonyl-ethyl)-2-methyl-phenyl]-harnstoff

(27) N-(4-Cyano-phenyl)-N'-(2-hydroxy-ethyl)-N'-[4-(2-methoxycarbonyl-ethyl)-2-methylthio-phenyl]-harnstoff

(28) N-(4-Cyano-phenyl)-N-(2-hydroxy-ethyl)-N'-[5-(2-methoxy-carbonyl-ethyl)-2-pyridyl]-harnstoff

(29) N-(4-Cyano-phenyl)-N-(2-hydroxy-ethyl)-N'-[4-(2-methoxy-carbonyl-ethyl)-cyclohexyl]-harnstoff

(30) N-(4-Cyano-phenyl)-N'-(2-hydroxy-ethyl)-N'-(4-methoxy-carbonyloxymethyloxy-phenyl)-harnstoff

(31) N-(4-tert.Butyloxy-carbonylmethylthio-phenyl)-N-(2-hydroxy-ethyl)-N'-(4-cyano-phenyl)-harnstoff
Schmelzpunkt: 111-114°C

(32) N-(4-Cyano-phenyl)-N'-(2-hydroxy-ethyl)-N'-[4-(3-methoxycarbonyl-2-methyl-2-propyl)-phenyl]-harnstoff
Schmelzpunkt: 113-116°C

(33) N-(4-Cyano-phenyl)-N'-[4-[2-(dimethoxy-phosphoryl)-ethyl]-phenyl]-N'-(2-hydroxy-ethyl)-harnstoff

(34) N-(4-Cyano-phenyl)-N'-(2-hydroxy-ethyl)-N'-[2-(4-ethoxy-carbonyl-phenyl)-ethyl]-harnstoff

(35) N-(4-Cyano-phenyl)-N'-(2-hydroxy-ethyl)-N'-[4-(2-methoxycarbonyl-ethenyl)-phenyl]-harnstoff

(36) N-(5-Cyano-2-pyridyl)-N'-(2-hydroxy-ethyl)-N'-[4-(2-methoxycarbonyl-ethyl)-phenyl]-harnstoff

(37) N-(1-Benzyl-4-piperidinyl)-N'-(2-hydroxy-ethyl)-N'-[4-(2-methoxycarbonyl-ethyl)-phenyl]-harnstoff

(38) N-(4-Cyano-phenyl)-N'-(2-hydroxy-ethyl)-N'-[4-(3-methoxycarbonyl-propyl)-phenyl]-harnstoff

(39) N-(4-Cyano-phenyl)-N'-(2,2-diethoxy-ethyl)-N'-[4-(2-methoxycarbonyl-ethyl)-phenyl]-harnstoff
$R_f$-Wert: 0,60 (Kieselgel; Cyclohexan, Essigester = 1:1)

(40) N-(2-Chlorethyl)-N'-(4-cyano-phenyl)-harnstoff
$R_f$-Wert: 0,29 (Kieselgel; Methylenchlorid, Methanol = 40:1, dreifach entwickelt)

(41) N-(4-Cyano-phenyl)-N'-(2,2-dimethoxy-ethyl)-N'-[2-(4-methoxycarbonyl-phenyl)-ethyl]-harnstoff
Schmelzpunkt: 109-110°C

(42) N-(4-Cyano-phenyl)-N'-(2-hydroxy-propyl)-N'-[4-(2-methoxycarbonyl-ethyl)-phenyl]-harnstoff
Man arbeitet in Dioxan ohne Hilfsbase.
$R_f$-Wert: 0,40 (Kieselgel; Essigester/Cyclohexan = 2:1)

(43) N-Acetylamino-N-[4-(2-ethoxycarbonyl-ethyl)-phenyl]-N'-(4-cyano-phenyl)-harnstoff
Man arbeitet in Dioxan ohne Hilfsbase.
Schmelzpunkt: 126-128°C

(44) N-(4-Cyano-phenyl)-N'-(2-hydroxy-propyl)-N'-[4-(2-methoxycarbonyl-ethyl)-phenyl]-harnstoff
Man arbeitet in Dioxan ohne Hilfsbase.
$R_f$-Wert: 0,25 (Kieselgel; Cyclohexan/Essigester = 1:1)

(45) N-(4-Cyano-phenyl)-N'-formylamino-N'-[4-(2-methoxycarbonyl-ethyl)-phenyl]-harnstoff
Man arbeitet in Dioxan ohne Hilfsbase.
Schmelzpunkt: 166-168°C

(46) N-(4-Cyano-phenyl)-N-formylamino-N'-[4-(2-methoxycarbonyl-ethyl)-phenyl]-harnstoff
Schmelzpunkt: 180-183°C

(47) N-Acetylamino-N-(4-cyano-phenyl)-N'-[4-(2-methoxycarbonyl-ethyl)-phenyl]-harnstoff
Schmelzpunkt: 153-155°C

(48) N-(2-Chlor-ethyl)-N'-[4-(2-methoxycarbonyl-ethyl)-phenyl]-harnstoff
Schmelzpunkt: 124-125°C

(49) N-[1-(4-Cyano-phenyl)-4-piperidinyl]-N-(2,2-dimethoxyethyl)-N'-(2-ethoxycarbonyl-ethyl)-harnstoff
Schmelzpunkt: 90-92°C

(50) N-(4-Cyano-phenyl)-N-(propionylamino)-N'-[4-(2-ethoxy-carbonyl-ethyl)-phenyl]-harnstoff
Schmelzpunkt: 149-153°C

(51) N-(4-Cyano-phenyl)-N'-(2,2-diethoxy-ethyl)-N'-[4-(2-methoxycarbonyl-ethenyl)-phenyl]-harnstoff
$R_f$-Wert: 0,68 (Kieselgel; Cyclohexan/Essigester = 1:1)

(52) N-(4-Cyano-phenyl)-N'-[4-(2-dibenzylamino-2-methoxycarbonyl-ethyl)-phenyl]-N'-(2,2-diethoxy-ethyl)-harnstoff
$R_f$-Wert: 0,52 (Kieselgel; Cyclohexan/Essigester = 2:1)

(53) N-[4-(2-tert.Butyloxycarbonylamino-2-methoxycarbonyl-ethyl)-phenyl]-N'-(4-cyano-phenyl)-N'-(2,2-diethoxy-ethyl)-harnstoff
$R_f$-Wert: 0,15 (Kieselgel; Cyclohexan/Essigester = 2:1)

(54) N-(4-Brom-2-methyl-phenyl)-N'-(2-hydroxy-ethyl)-N'-[4-(2-methoxycarbonyl-ethyl)-phenyl]-harnstoff
Man arbeitet in Dioxan bei Raumtemperatur.
Schmelzpunkt: 101-104°C

## Beispiel IV

### N-(4'-Cyano-4-biphenylyl)-N-(2,2-diethoxy-ethyl)-amin

Eine Mischung aus 10 g 4-Amino-4'-cyano-biphenyl, 8,3 ml Bromacetaldehyd-diethylacetal, 13,9 ml Ethyl-diisopropylamin und 20 ml Dimethylformamid wird 16 Stunden bei einer Badtemperatur von 160°C gerührt. Man engt zur Trockne ein und reinigt den Rückstand säulenchromatographisch über Kieselgel (Elutionsmittel: Cyclohexan/Essigester = 2:1).
Ausbeute: 3 g (19 % der Theorie),
Schmelzpunkt: 88-90°C
$R_f$-Wert: 0,54 (Kieselgel; Cyclohexan/Essigester = 2:1)

Analog werden folgende Verbindungen erhalten:

(1) N-(tert.Butyloxycarbonyl-methyl)-N-(4'-cyano-4-biphenylyl)-amin
Man arbeitet bei Raumtemperatur und kristallisiert aus Cyclohexan/Essigester = 20:2 um.
Schmelzpunkt: 151-153°C
$R_f$-Wert: 0,76 (Kieselgel; Cyclohexan/Essigester = 1:1)

(2) N-(4'-Cyano-4-biphenylyl)-N-[3-(2,3,5,6-tetrahydro-2-pyranyl)-propyl]-amin
Man arbeitet bei 110°C.
Schmelzpunkt: 111-113°C
$R_f$-Wert: 0,65 (Kieselgel; Cyclohexan/Essigester = 1:1)

(3) N-(2,2-Diethoxy-ethyl)-N-[4-(2-methoxycarbonyl-ethyl)-phenyl]-amin
$R_f$-Wert: 0,35 (Kieselgel; Cyclohexan/Essigester = 8:2)

(4) N-(2,2-Dimethoxy-ethyl)-N-[2-(4-methoxycarbonyl-phenyl)-ethyl]-amin
Als Ausgangsmaterialien dienen 2,2-Dimethoxy-ethylamin und 4-(2-Chlor-ethyl)-benzoesäure-methylester. Man erwärmt 72 Stunden auf 50°C.
$R_f$-Wert: 0,46 (Kieselgel; Essigester/Methanol = 4:1)

(5) N-(4-Cyano-phenyl)-glycin-tert.butylester
Schmelzpunkt: 110-112°C

(6) 4-[(2,2-Diethoxy-ethyl)-amino]-zimtsäure-methylester
$R_f$-Wert: 0,79 (Kieselgel; Cyclohexan/Essigester = 1:1)

(7) 2-Dibenzylamino-3-[4-[(2,2-diethoxy-ethyl)-amino]-phenyl]-propionsäure-methylester
$R_f$-Wert: 0,59 (Kieselgel; Cyclohexan/Essigester = 2:1)

(8) 2-Dibenzylamino-3-(4-nitro-phenyl)-propionsäure-methylester
Man arbeitet in Methanol.
$R_f$-Wert: 0,65 (Kieselgel; Cyclohexan/Essigester = 2:1)

(9) 2-tert.Butyloxycarbonylamino-3-[4-(2,2-diethoxy-ethylamino)-phenyl]-propionsäure-methylester
$R_f$-wert: 0,45 (Kieselgel; Cyclohexan/Essigester = 2:1)

Beispiel V

N-(4'-Cyano-4-biphenylyl)-N-(3-methansulfonyloxy-propyl)-N'-(2-ethoxycarbonyl-ethyl)-harnstoff

Eine Mischung aus 2 g N-(4'-Cyano-4-biphenylyl)-N-(3-hydroxypropyl)-N'-(2-ethoxycarbonyl-ethyl)-harnstoff, 0,45 ml Methansulfonylchlorid und 15 ml Methylenchlorid wird auf 0°C abgekühlt und innerhalb von 15 Minuten mit 0,8 ml Triethylamin versetzt. Man rührt eine Stunde bei 0°C und eine Stunde bei Raumtemperatur nach, gibt 25 ml Methylenchlorid zu und extrahiert mit Wasser. Man dampft ein und kristallisiert den Rückstand durch Verreiben mit tert.Butylmethylether.
Ausbeute: 1,9 g (79 % der Theorie),
$R_f$-Wert: 0,47 (Kieselgel; Essigester)

Analog werden folgende Verbindungen erhalten:

(1) N-(4-Cyano-phenyl)-N'-(2-methansulfonyloxy-ethyl)-N'-[4-(2-methoxycarbonyl-ethyl)-phenyl]-harnstoff
$R_f$-Wert: 0,39 (Kieselgel; Cyclohexan/Essigester = 3:7)

(2) N-(4-Cyano-phenyl)-N'-(3-methansulfonyloxy-propyl)-N'-[4-(2-methoxycarbonyl-ethyl)-phenyl]-harnstoff
$R_f$-Wert: 0,43 (Kieselgel; Cyclohexan/Essigester = 3:7)

(3) N-(4-Cyano-3-fluor-phenyl)-N'-(2-methansulfonyloxy-ethyl)-N'-[4-(2-methoxycarbonyl-ethyl)-phenyl]-harnstoff

(4) N-(3-Chlor-4-cyano-phenyl)-N'-(2-methansulfonyloxy-ethyl)-N'-[4-(2-methoxycarbonyl-ethyl)-phenyl]-harnstoff

(5) N-(3-Cyano-2-methylthio-phenyl)-N'-(2-methansulfonyloxy-ethyl)-N'-[4-(2-methoxycarbonyl-ethyl)-phenyl]-harnstoff

(6) N-(4-Cyano-2-methyl-phenyl)-N'-(2-methansulfonyloxy-ethyl)-N'-[4-(2-methoxycarbonyl-ethyl)-phenyl]-harnstoff

(7) N-(4-Cyano-2-methoxy-phenyl)-N'-(2-methansulfonyloxy-ethyl)-N'-[4-(2-methoxycarbonyl-ethyl)-phenyl]-harnstoff

(8) N-(4-Cyano-phenyl)-N'-[2-fluor-4-(2-methoxycarbonyl-ethyl)-phenyl]-N'-(2-methansulfonyloxy-ethyl)-harnstoff

(9) N-[2-Chlor-4-(2-methoxycarbonyl-ethyl)-phenyl]-N-(2-methansulfonyloxy-ethyl)-N'-(4-cyano-phenyl)-harnstoff

(10) N-(4-Cyano-phenyl)-N'-(2-methansulfonyloxy-ethyl)-N'-[2-methoxy-4-(2-methoxycarbonyl-ethyl)-phenyl]-harnstoff

(11) N-(4-Cyano-phenyl)-N'-(2-methansulfonyloxy-ethyl)-N'-[4-(2-methoxycarbonyl-ethyl)-2-methyl-phenyl]-harnstoff

(12) N-(4-Cyano-phenyl)-N'-(2-methansulfonyloxy-ethyl)-N'-[4-(2-methoxycarbonyl-ethyl)-2-methylthio-phenyl]-harnstoff

(13) N-(4-Cyano-phenyl)-N-(2-methansulfonyloxy-ethyl)-N'-[5-(2-methoxycarbonyl-ethyl)-2-pyridyl]-harnstoff

(14) N-(4-Cyano-phenyl)-N-(2-methansulfonyloxy-ethyl)-N'-[4-(2-methoxycarbonyl-ethyl)-cyclohexyl]-harnstoff

(15) N-(4-Cyano-phenyl)-N'-(2-methansulfonyloxy-ethyl)-N'-(4-methoxycarbonylmethyloxy-phenyl)-harnstoff

(16) N-(4-tert.Butyloxycarbonylmethylthio-phenyl)-N-(2-methansulfonyloxy-ethyl)-N'-(4-cyano-phenyl)-harnstoff
$R_f$-Wert: 0,35 (Kieselgel; Methylenchlorid/Essigester = 95:5)

(17) N-(4-Cyano-phenyl)-N'-(2-methansulfonyloxy-ethyl)-N'-[4-(3-methoxycarbonyl-2-methyl-2-propyl)-phenyl]-harnstoff
$R_f$-Wert: 0,57 (Kieselgel; Cyclohexan/Essigester = 3:7)

(18) N-(4-Cyano-phenyl)-N'-[4-[2-(dimethoxy-phosphoryl)-ethyl]-phenyl]-N'-(2-methansulfonyloxy-ethyl)-harnstoff

(19) N-(4-Cyano-phenyl)-N'-[2-(4-ethoxycarbonyl-phenyl)-ethyl]-N'-(2-methansulfonyloxy-ethyl)-harnstoff

(20) N-(4-Cyano-phenyl)-N'-(2-methansulfonyloxy-ethyl)-N'-[4-(2-methoxycarbonyl-ethenyl)-phenyl]-harnstoff

(21) N-(5-Cyano-2-pyridyl)-N'-(2-methansulfonyloxy-ethyl)-N'-[4-(2-methoxycarbonyl-ethyl)-phenyl]-harnstoff

(22) N-(1-Benzyl-4-piperidinyl)-N'-(2-methansulfonyloxy-ethyl)-N'-[4-(2-methoxycarbonyl-ethyl)-phenyl]-harnstoff

(23) N-(4-Cyano-phenyl)-N'-(2-methansulfonyloxy-ethyl)-N'-[4-(3-methoxycarbonyl-propyl)-phenyl]-harnstoff

(24) 1-(4-Cyano-phenyl)-3-(2-methansulfonyloxy-ethyl)-imidazolidin-2-on
Schmelzpunkt: 98-100°C

(25) N'-(4-Cyanomethyl-phenyl)-N'-(2-methansulfonyloxyethyl)-N'-(4-methoxycarbonylmethyl-phenyl)-harnstoff
$R_f$-Wert: 0,19 (Kieselgel; Cyclohexan/Essigester = 6:4)

Beispiel VI

N-(4'-Cyano-4-biphenylyl)-N-(3-hydroxy-propyl)-N'-(2-ethoxycarbonyl-ethyl)-harnstoff

4,1 g N-(4'-Cyano-4-biphenylyl)-N-[3-(2,3,5,6-tetrahydro-2-pyranyloxy)-propyl]-N'-(2-ethoxycarbonyl-ethyl)-harnstoff werden in 15 ml Ethanol gelöst, mit 0,2 ml etherischer Salzsäure versetzt und 2 Stunden bei Raumtemperatur gerührt. Man engt zur Trockene ein, nimmt in 200 ml Methylenchlorid auf, extrahiert mit 10%iger Natriumbicarbonatlösung und dampft die organische Phase zur Trockene ein. Der Rückstand wird durch Säulenchromatographie an Kieselgel gereinigt (Elutionsmittel: Essigester).
Ausbeute: 2,1 g (64 % der Theorie),
Schmelzpunkt: 120-122°C
$R_f$-Wert: 0,36 (Kieselgel; Essigester)

Analog wird folgende Verbindung erhalten:

(1) 1-(4-Cyano-phenyl)-piperidin-4-on
Hergestellt aus dem entsprechenden Ethylenketal mit Pyridinium-toluolsulfonat in Aceton/Wasser bei 100°C
Schmelzpunkt: 102-104°C

Beispiel VII

N-(2-Hydroxy-ethyl)-N-[4-(2-methoxycarbonyl-ethyl)-phenyl]-amin

1,5 g N-[4-(2-Methoxycarbonyl-ethyl)-phenyl]-N-[2-(2,3,5,6-tetrahydro-2-pyranyloxy)-ethyl]-trifluoracetamid werden in einem Gemisch aus 20 ml Methanol und 2,5 ml 4n Natronlauge 3 Stunden bei Raumtemperatur gerührt. Man neutralisiert mit Eisessig, dampft ein und entfernt alles Wasser durch Kochen mit Toluol am Wasserabscheider. Man dampft ein, nimmt mit 20 ml Methanol auf, setzt 2 ml methanolische Salzsäure zu und läßt 16 Stunden bei Raumtemperatur stehen. Der vorhandene Niederschlag wird abfiltriert, das Filtrat eingeengt und mit 10 ml Methylenchlorid und 5 ml 0,1 n Natronlauge verrührt. Die organische Phase wird eingeengt und das verbleibende Öl direkt weiter verarbeitet.
Ausbeute: 0,65 g (78 % der Theorie),
$R_f$-Wert: 0,43 (Kieselgel; Cyclohexan/Essigester = 3:7)

Analog werden folgende Verbindungen erhalten:

(1) N-(3-Hydroxy-propyl)-N-[4-(2-methoxycarbonyl-ethyl)-phenyl]-amin
$R_f$-Wert: 0,44 (Kieselgel; Cyclohexan/Essigester = 3:7)

(2) N-[2-Fluor-4-(2-methoxycarbonyl-ethyl)-phenyl]-N-(2-hydroxy-ethyl)-amin

(3) N-[2-Chlor-4-(2-methoxycarbonyl-ethyl)-phenyl]-N-(2-hydroxy-ethyl)-amin

(4) N-(2-Hydroxy-ethyl)-N-[2-methoxy-4-(2-methoxycarbonyl-ethyl)-phenyl]-amin

(5) N-(2-Hydroxy-ethyl)-N-[4-(2-methoxycarbonyl-ethyl)-2-methyl-phenyl]-amin

(6) N-(2-Hydroxy-ethyl)-N-[4-(2-methoxycarbonyl-ethyl)-2-methylthio-phenyl]-amin

Beispiel VIII

3-(4-Cyano-phenyl)-3H-imidazo[4,5-b]pyridin-2-on

0,21 g 3-Amino-2-[(4-cyano-phenyl)-amino]-pyridin und 0,26 g N-Ethyl-diisopropylamin werden in 4,5 ml Methylenchlorid gelöst und unter Eiskühlung mit 0,5 ml einer 20%igen Lösung von Phosgen in Toluol portionsweise versetzt und 1,5 Stunden bei Raumtemperatur gerührt. Man gibt nochmals 0,3 ml der Phosgenlösung zu und rührt 20 Minuten weiter. Der ausgefallene Niederschlag wird abfiltriert und mit Methylenchlorid gewaschen.
Ausbeute: 0,17 g (72 % der Theorie),
$R_f$-Wert: 0,47 (Kieselgel; Methylenchlorid/Methanol = 19:1)

Analog wird folgende Verbindung erhalten:

(1) 3-(4'-Cyano-4-biphenylyl)-3H-imidazo[4,5-b]pyridin-2-on
$R_f$-Wert: 0,44 (Kieselgel; Methylenchlorid/Methanol = 9:1)

Beispiel IX

1-[4-(2-Methoxycarbonyl-ethyl)-phenyl]-3-(4-piperidinyl)-imidazolidin-2-on

Herstellung durch Behandeln von 1-(1-Benzyloxycarbonyl-4-piperidinyl)-3-[4-(2-methoxycarbonyl-ethyl)-phenyl]-imidazolidin-2-on mit Wasserstoff von 3 bar in Gegenwart von 5%iger Palladiumkohle in Methanol.

Beispiel X

2-[(2-Ethoxycarbonyl-ethyl)-aminosulfonyloxy]-phenol

1,54 g β-Alaninethylester-hydrochlorid und 1,9 g 1,2-Sulfonyldioxy-benzol werden in 10 ml Dimethylformamid gelöst, mit 1,55 g Ethyl-diisopropylamin versetzt und zwei Stunden bei Raumtemperatur gerührt. Das Lösungsmittel wird im Vakuum abdestilliert und der Rückstand säulenchromatographisch gereinigt (Kieselgel; Elutionsmittel: Cyclohexan/Essigester = 8:2) Ausbeute: 1,4 g (48 % der Theorie),

$R_f$-Wert: 0,31 (Kieselgel; Cyclohexan/Essigester = 7:3)

Analog wird folgende Verbindung erhalten:

(1) 4-Cyano-4'-[(2-ethoxycarbonyl-ethyl)-aminosulfonylamino]-biphenyl

Man arbeitet ohne Hilfsbase und erhitzt 15 Stunden auf 80°C
Schmelzpunkt: 103-105°C
$R_f$-Wert: 0,21 (Kieselgel; Cyclohexan/Essigester = 7:3)

Beispiel XI

3-[4-[(2-Hydroxy-propyl)-amino]-phenyl]-propionsäure-methylester

6 g 3-(4-Amino-phenyl)-propionsäure-methylester-hydrochlorid werden in Methylenchlorid suspendiert und 27,9 ml 1N Natronlauge zugefügt. Man trennt die organische Phase ab, extrahiert noch zweimal mit Methylenchlorid und dampft die vereinigten organischen Phasen ein. Der Rückstand wird in 50 ml Methanol aufgenommen und 1,9 ml Propylenoxid sowie 2 ml Wasser zugefügt. Man rührt 48 Stunden bei Raumtemperatur, dampft ein und reinigt über Kieselgel (Elutionsmittel: Cyclohexan/Essigester = 1:1).
Ausbeute: 3,5 g (54 % der Theorie),
$R_f$-Wert: 0,40 (Kieselgel; Cyclohexan/Essigester = 1:1)

Beispiel XII

4-[(2-Hydroxy-propyl)-amino]-benzonitril

12,1 g 4-Fluor-benzonitril, 7,5 g 2-Hydroxy-propylamin und 17,4 ml N-Ethyl-diisopropylamin werden zusammen 2,5 Stunden auf 100°C erhitzt. Man gießt auf 250 ml Wasser, extrahiert fünfmal mit je 50 ml Essigester und dampft die vereinigten organischen Phasen ein. Das verbleibende Produkt wird säulenchromatographisch gereinigt (Kieselgel; Elutionsmittel: Essigester) und durch Verreiben mit einer 1:1-Mischung aus tert.Butyl-methylether und Petrolether kristallisiert.
Ausbeute: 2,2 g (13 % der Theorie),
Schmelzpunkt: 70-73°C

Analog wird folgende Verbindung erhalten:

(1) 1-(4-Cyano-phenyl)-4,4-ethylendioxy-piperidin

Schmelzpunkt: 136-138°C

Beispiel XIII

1-[4-(2-Methoxycarbonyl-ethyl)-cyclohexyl]-imidazolidin-2-on

2 g 1-[4-(2-Methoxycarbonyl-ethyl)-phenyl]-imidazolidin-2-on werden in einer Mischung aus 20 ml Methanol und 30 ml Eisessig in Gegenwart eines Platin/Rhodium-Katalysators mit Wasserstoff von 5 bar bei 50°C 50 Minuten hydriert. Man filtriert vom Katalysator ab, dampft das Filtrat ein und verwendet das zurückbleibende Produkt ohne weitere Reinigung.
Ausbeute: 2,03 g (100 % der Theorie),
$R_f$-Wert: 0,29 (Kieselgel; Essigester/Cyclohexan = 9:1)

Beispiel XIV

3-[4-[(2-Chlor-ethyl)-aminosulfonylamino]-phenyl]-propionsäure-methylester

Zu einer Lösung von 8,6 g 3-(4-Amino-phenyl)-propionsäure-methylester-hydrochlorid und 12,9 g N-Ethyl-diisopropylamin in 40 ml Methylenchlorid tropft man bei -30°C 6,7 g N-(2-Chlorethyl)-N-chlorsulfonyl-amin, gelöst in 10 ml Methylenchlorid, zu. Man rührt noch 2 Stunden weiter, wobei man auf Raumtemperatur kommen läßt. Das Reaktionsgemisch wird mit Wasser gewaschen, eingeengt und säulenchromatographisch gereinigt (Kieselgel; Cyclohexan/Essigester = 7:3).
Ausbeute: 8,3 g (65 % der Theorie),
Schmelzpunkt: 97-99°C

Analog wird folgende Verbindung erhalten:

(1) N-(4-Cyano-phenyl)-N-methoxycarbonyl-glycin-tert.butylester

Man verwendet Kaliumcarbonat und 4-Dimethylamino-pyridin in Chloroform
$R_f$-Wert: 0,42 (Kieselgel; Cyclohexan/Essigester = 7:3)

Beispiel XV

1-(4-Cyano-phenyl)-4-[(2,2-dimethoxy-ethyl)-amino]-piperidin

11 g 1-(4-Cyano-phenyl)-piperidin-4-on werden in 90 ml Acetonitril gelöst, mit 10 ml Wasser und 6,9 g Aminoacetaldehyddimethylacetal versetzt. Man tropft 18,3 ml 3N Salzsäure zu und versetzt mit 4,5 g Natriumcyanborhydrid. Nach 20 Minuten Rühren bei Raumtemperatur wird das Acetonitril im Vakuum abgedampft und der Rückstand mit Essigester extrahiert. Der nach dem Eindampfen der Essigesterphasen verbleibende Rückstand wird durch Chromatographie an Kieselgel gereinigt (Elutionsmittel: Methylenchlorid/Methanol = 100:3).
Ausbeute: 10,8 g (74 % der Theorie),
$R_f$-Wert: 0,34 (Kieselgel; Methylenchlorid/Methanol/konzentriertes Ammoniak = 95:5:0,1)
Analog werden folgende Verbindungen erhalten:

(1) 3-[4-[(2-Hydroxy-ethyl)-amino]-phenyl]-3-methyl-buttersäure-methylester
$R_f$-Wert: 0,35 (Kieselgel; Cyclohexan/Essigester = 1:1)

(2) 4-[(2-Hydroxy-ethyl)-amino]-phenylessigsäure-methylester
$R_f$-Wert: 0,25 (Kieselgel; Cyclohexan/Essigester = 1:1)

Beispiel XVI

N-(4-Cyano-phenyl)-N-methoxycarbonyl-glycin-[4-(2-methoxycarbonyl-ethyl)-anilid]

Eine Mischung aus 3,7 g N-(4-Cyano-phenyl)-N-methoxycarbonylglycin, 2,4 g 1-Hydroxy-benztriazol, 150 ml Tetrahydrofuran, 2,81 g 3-(4-Amino-phenyl)-propionsäure-methylester, 4,2 g N,N'-Dicyclohexylcarbodiimid und 1,62 g Triethylamin wird bei Raumtemperatur 64 Stunden gerührt. Man filtriert den gebildeten Niederschlag ab, engt ein und reinigt den Rückstand saürlenchromatographisch über Kieselgel (Elutionsmittel: Cyclohexan/Essigester = 1:1 bis 2:3).
Ausbeute: 5,5 g (88 % der Theorie),
Schmelzpunkt: 100-110°C
Analog wird folgende Verbindung erhalten:
(1) N-(4-Cyano-phenyl)-N'-propionyl-hydrazin
Man verwendet Carbonyldiimidazol in Tetrahydrofuran.
Schmelzpunkt: 143-145°C

Beispiel XVII

N-(4-Cyano-phenyl)-N-methoxycarbonyl-glycin

7 g N-(4-Cyano-phenyl)-N-methoxycarbonyl-glycin-tert.butylester werden in 70 ml Methylenchlorid gelöst und tropfenweise mit einer Lösung von 18 ml Trifluoressigsäure in 18 ml Methylenchlorid versetzt. Man läßt 16 Stunden bei Raumtemperatur stehen, dampft ein, nimmt den Rückstand in tert.Butylmethylether auf, wäscht mit Wasser und dampft die organische Phase ein. Der Rückstand wird mit 50 ml Diethylether kurz aufgekocht und nach dem Abkühlen im Eisbad abfiltriert. Eine weitere Fraktion kann aus den Mutterlaugen gewonnen werden.
Ausbeute: 3,85 g (68 % der Theorie),
Schmelzpunkt: 134-137°C

Beispiel XVIII

3-(4-Amino-phenyl)-2-dibenzylamino-propionsäure-methylester

5,35 g 2-Dibenzylamino-3-(4-nitro-phenyl)-propionsäure-methylester, gelöst in 100 ml Methanol, werden in Gegenwart von 1 g Raney-Nickel 8 Stunden bei Raumtemperatur mit Wasserstoff von 5 bar Druck behandelt. Man filtriert vom Katalysator ab, dampft ein und verwendet das zurückbleibende Rohprodukt ohne Reinigung weiter.
Ausbeute: 4,9 g (92 % der Theorie),
$R_f$-Wert: 0,45 (Kieselgel; Cyclohexan/Essigester = 2:1)

Analog werden folgende Verbindungen erhalten:

(1) 3-Amino-2-[(4-cyano-phenyl)-amino]-pyridin
Man arbeitet in einem 3:1-Gemisch aus Essigester und Methanol mit 10%iger Palladiumkohle
$R_f$-Wert: 0,62 (Kieselgel; Methylenchlorid/Methanol = 9:1)
(2) 3-Amino-2-[(4'-cyano-4-biphenylyl)-amino]-pyridin
Man arbeitet in einem 3:1-Gemisch aus Essigester und Methanol mit 10%iger Palladiumkohle
$R_f$-Wert: 0,66 (Kieselgel; Methylenchlorid/Methanol = 19:1)

Beispiel XIX

1-(4-Cyano-phenyl)-3-(2-hydroxy-ethyl)-imidazolidin-2-on

Zu einer Lösung von 8,8 g 1-(4-Cyano-phenyl)-3-(ethoxycarbonylmethyl)-imidazolidin-2-on in 500 ml Tetrahydrofuran werden unter Rühren bei Raumtemperatur portionsweise 0,71 g Lithiumborhydrid gegeben. Man rührt eine Stunde bei Raumtemperatur und 2 Stunden bei 60°C, versetzt nach dem Abkühlen im Eisbad mit 16,5 ml 2N Salzsäure und dampft im Vakuum ein. Der Rückstand wird mit 50 ml Methanol digeriert, die Methanolphase eingedampft und der Rückstand über Kieselgel gereinigt (Elutionsmittel: Essigester/Methanol = 9:1)
Ausbeute: 5,0 g (67 % der Theorie),
Schmelzpunkt: 112-114°C

Beispiel XX

N-(4-Cyanomethyl-phenyl)-N'-(2-hydroxy-ethyl)-N'-(4-methoxycarbonylmethyl-phenyl)-harnstoff

1,46 g Carbonyldiimidazol und 1,04 g Imidazol werden in 20 ml Tetrahydrofuran gelöst. Man kühlt auf 0°C ab und fügt 1,18 g 4-Amino-benzylcyanid zu. Nach etwa 3 Minuten tropft man rasch eine Lösung von 4-[(2-Hydroxy-ethyl)-amino]-phenylessigsäuremethylester in 7 ml Tetrahydrofuran zu, entfernt die Eiskühlung und rührt 16 Stunden bei Raumtemperatur (ein anfänglich gebildeter Niederschlag wird abfiltriert). Man engt ein, nimmt den Rückstand in Essigester auf, wäscht mit 1N Salzsäure und Wasser und engt die organische Phase ein. Der Rückstand wird durch Chromatographie an Kieselgel gereinigt (Elutionsmittel: Essigester/Methylenchlorid = 1:1).
Ausbeute: 1,4 g (42 % der Theorie),
Schmelzpunkt: 140-142°C

Beispiel XXI

1-[4-(1-Hydroxyimino-ethyl)-phenyl]-3-[4-(2-methoxycarbonylethyl)-phenyl]-imidazolidin-2-on

1,1 g 1-(4-Acetyl-phenyl)-3-[4-(2-methoxycarbonyl-ethyl)-phenyl]-imidazolidin-2-on werden in einer Mischung aus 10 ml Dioxan und 10 ml Methanol suspendiert, mit einer Lösung von 0,25 g Hydroxylamin-hydrochlorid in 4 ml Wasser versetzt und 2,5 Stunden zum Rückfluß erhitzt. Anschließend dampft man im Vakuum ein und verreibt den Rückstand mit Wasser, wobei dieser kristallisiert.
Ausbeute: 1,1 g (92 % der Theorie),
Schmelzpunkt: 241-243°C (sintert ab 235°C)
Analog wird folgende Verbindung erhalten:
(1) 1-(1-Hydroxyimino-5-indanyl)-3-[4-(2-methoxycarbonylethyl)-phenyl]-imidazolidin-2-on
$R_f$-Wert: 0,13 (Kieselgel; Methylenchlorid/Essigester = 9:1)

Beispiel XXII

1-(4-Acetyl-phenyl)-3-[4-(2-methoxycarbonyl-ethyl)-phenyl]-imidazolidin-2-on

Hergestellt analog Beispiel 25 aus 1-[4-(2-Methoxycarbonylethyl)-phenyl]-imidazolidin-2-on und 4-Brom-acetophenon.
Schmelzpunkt: 198-200°C
$R_f$-Wert: 0,33 (Kieselgel; Methylenchlorid/Essigester = 25:1)
Analog wird folgende Verbindung erhalten:
(1) 1-[4-(2-Methoxycarbonyl-ethyl)-phenyl]-3-(1-oxo-5-indanyl)-imidazolidin-2-on

Schmelzpunkt: 204-206°C
$R_f$-Wert: 0,39 (Kieselgel; Methylenchlorid/Essigester = 9:1)

Beispiel 1

1-(4'-Amidino-4-biphenylyl)-3-carboxymethyl-imidazolidin-2-on

0,35 g 1-(4'-Amidino-4-biphenylyl)-3-methoxycarbonylmethylimidazolidin-2-on-hydrochlorid werden in 9 ml Methanol suspendiert. Man fügt 2,7 ml 1N Natronlauge hinzu und rührt 16 Stunden bei Raumtemperatur. Das Lösungsmittel wird im Vakuum abdestilliert, der Rückstand mit 10 ml Wasser versetzt. Man puffert durch Zugabe von Ammoniumchlorid ab. Das ausgefallene Produkt wird abfiltriert.
Ausbeute: 0,19 g (62 % der Theorie),
Schmelzpunkt: über 200°C
$R_f$-Wert: 0,61 (Reversed Phase Platte RP8; 10%ige Natriumchloridlösung/Methanol = 4:6)

| | | | | | | |
|---|---|---|---|---|---|---|
| Ber. x $H_20$: | C | 60,65 | H | 5,67 | N | 15,72 |
| Gef.: | | 61,20 | | 5,57 | | 15,98 |

Analog werden folgende Verbindungen erhalten:

(1) 1-(4'-Amidino-4-biphenylyl)-3-(2-carboxy-ethyl)-imidazolidin-2-on
Schmelzpunkt: über 200°C
$R_f$-Wert: 0,58 (Reversed Phase Platte RP8; 10%ige Natriumchloridlösung/Methanol = 4:6)

| | | | | | | |
|---|---|---|---|---|---|---|
| Ber. x 0,5 $H_20$: | C | 63,14 | H | 5,87 | N | 15,51 |
| Gef.: | | 63,37 | | 5,80 | | 15,13 |

(2) 1-(4'-Amidino-4-biphenylyl)-3-(2-carboxy-ethyl)-imidazolidin-2,4-dion
Als Ausgangsmaterial dient 1-(4'-Amidino-4-biphenylyl)-3-(2-ethoxycarbonyl-ethyl)-imidazolidin-2,4-dion-hydrochlorid Schmelzpunkt: über 200°C
$R_f$-Wert: 0,66 (Reversed Phase Platte RP8; 10%ige Natriumchloridlösung/Methanol = 4:6)

| | | | | | | |
|---|---|---|---|---|---|---|
| Ber. x 0,25 $H_20$: | C | 61,52 | H | 5,01 | N | 15,11 |
| Gef.: | | 61,89 | | 4,97 | | 14,97 |

(3) 1-(4'-Amidino-4-biphenylyl)-3-(2-carboxy-ethyl)-3H-imidazol-2-on
Als Ausgangsmaterial dient 1-(4'-Amidino-4-biphenylyl)-3-(2-ethoxycarbonyl-ethyl)-3H-imidazol-2-on-hydrochlorid
Schmelzpunkt: über 200°C
$R_f$-Wert: 0,68 (Reversed Phase Platte RP8; 5%ige Natriumchloridlösung/Methanol = 4:6)

| | | | | | | |
|---|---|---|---|---|---|---|
| Ber. x 0,25 $H_20$: | C | 64,30 | H | 5,24 | N | 15,79 |
| Gef.: | | 64,61 | | 5,34 | | 15,45 |

(4) 1-(4'-Amidino-4-biphenylyl)-3-(2-carboxy-ethyl)-3,4,5,6-tetrahydro-1H-pyrimidin-2-on
Als Ausgangsmaterial dient 1-(4'-Amidino-4-biphenylyl)-3-(2-ethoxycarbonyl-ethyl)-3,4,5,6-tetrahydro-1H-pyrimidin-2-on und man arbeitet in Ethanol als Lösungsmittel
Schmelzpunkt: über 200°C
$R_f$-Wert: 0,54 (Reversed Phase Platte RP8; 10%ige Natriumchloridlösung/Methanol = 4:6)

| | | | | | | |
|---|---|---|---|---|---|---|
| Ber. x 0,25 $H_20$: | C | 64,76 | H | 6,12 | N | 15,11 |
| Gef.: | | 64,62 | | 6,13 | | 14,82 |

(5) 1-(4'-Amidino-4-biphenylyl)-3-carboxymethyl-3,4,5,6-tetrahydro-1H-pyrimidin-2-on
Schmelzpunkt: über 200°C
$R_f$-Wert: 0,63 (Reversed Phase Platte RP8; 10%ige Natriumchloridlösung/Methanol = 4:6)

| | | | | | | |
|---|---|---|---|---|---|---|
| Ber. x 0,75 $H_2O$: | C | 62,37 | H | 5,92 | N | 15,31 |
| Gef.: | | 62,39 | | 5,94 | | 15,55 |

(6) 1-(4-Amidino-phenyl)-3-[4-(2-carboxy-ethyl)-phenyl]-imidazolidin-2-on
Man verwendet Lithiumhydroxid und als Lösungsmittel ein 5:4-Gemisch aus Tetrahydrofuran und Wasser.
Schmelzpunkt: über 270°C
$R_f$-Wert: 0,68 (Reversed Phase Platte RP8; 10%ige Natriumchloridlösung/Methanol = 4:6)

(7) 1-(4-Amidino-phenyl)-3-[4-(2-carboxy-ethyl)-phenyl]-3,4,5,6-tetrahydro-1H-pyrimidin-2-on
Man verwendet Lithiumhydroxid und als Lösungsmittel ein 5:4-Gemisch aus Tetrahydrofuran und Wasser.
Schmelzpunkt: über 270°C
$R_f$-Wert: 0,60 (Reversed Phase Platte RP8; 10%ige Natriumchloridlösung/Methanol = 4:6)

(8) 1-[2-(4'-Amidino-4-biphenylyl)-ethyl]-3-carboxymethyl-3H-benzimidazol-2-on
$R_f$-Wert: 0,55 (Kieselgel; Methylenchlorid, Methanol = 6:4)

(9) 1-(4'-Amidino-3'-fluor-4-biphenylyl)-3-(2-carboxy-ethyl)-imidazolidin-2-on

(10) 1-(4'-Amidino-3'-chlor-4-biphenylyl)-3-(2-carboxy-ethyl)-imidazolidin-2-on

(11) 1-(4'-Amidino-3-methoxy-4-biphenylyl)-3-(2-carboxy-ethyl)-imidazolidin-2-on

(12) 1-(4'-Amidino-3-brom-4-biphenylyl)-3-(2-carboxy-ethyl)-imidazolidin-2-on

(13) 1-(4'-Amidino-3-methylthio-4-biphenylyl)-3-(2-carboxy-ethyl)-imidazolidin-2-on

(14) 1-(4'-Amidino-3-methylsulfonyl-4-biphenylyl)-3-(2-carboxy-ethyl)-imidazolidin-2-on

(15) 1-(4'-Amidino-3-methylsulfinyl-4-biphenylyl)-3-(2-carboxy-ethyl)-imidazolidin-2-on

(16) 1-(4'-Amidino-2,3-dimethyl-4-biphenylyl)-3-(2-carboxy-ethyl)-imidazolidin-2-on

(17) 1-(4'-Amidino-3-nitro-4-biphenylyl)-3-(2-carboxy-ethyl)-imidazolidin-2-on

(18) 1-(4'-Amidino-3-amino-4-biphenylyl)-3-(2-carboxy-ethyl)-imidazolidin-2-on

(19) 1-(3-Acetamino-4'-amidino-4-biphenylyl)-3-(2-carboxy-ethyl)-imidazolidin-2-on

(20) 1-(4'-Amidino-3-benzoylamino-4-biphenylyl)-3-(2-carboxy-ethyl)-imidazolidin-2-on

(21) 2-(4'-Amidino-4-biphenylyl)-5-(2-carboxy-ethyl)-3,4-dihydro-2H,5H-1,2,5-thiadiazol-1,1-dioxid
Schmelzpunkt: über 260°C
$R_f$-Wert: 0,63 (Reversed Phase Platte RP8; Methanol/10%ige Natriumchloridlösung = 6:4)

| | | | | | | | | |
|---|---|---|---|---|---|---|---|---|
| Ber.: | C | 55,66 | H | 5,19 | N | 14,42 | S | 8,25 |
| Gef.: | | 55,42 | | 5,32 | | 14,56 | | 8,26 |

(22) 1-[4-(4-Amidino-phenyl)-cyclohexyl]-3-(2-carboxy-ethyl)-3H-imidazol-2-on

(23) 1-[4-(4-Amidino-phenyl)-cyclohexyl]-3-(2-carboxy-ethyl)-imidazolidin-2-on

(24) 1-[1-(4-Amidino-phenyl)-4-piperidinyl]-3-(2-carboxyethyl)-imidazolidin-2-on
Schmelzpunkt: über 275°C
$R_f$-Wert: 0,66 (Reversed Phase Platte RP8; Methanol/10%ige Natriumchloridlösung = 6:4)

| | | | | | | |
|---|---|---|---|---|---|---|
| Ber. x 0,5 $H_2O$: | C | 58,68 | H | 7,11 | N | 19,01 |
| Gef.: | | 58,16 | | 7,22 | | 18,76 |

(25) 1-[4-(5-Amidino-2-pyridyl)-phenyl]-3-(2-carboxy-ethyl)-imidazolidin-2-on

(26) 1-[4-(5-Amidino-2-pyrazinyl)-phenyl]-3-(2-carboxyethyl)-imidazolidin-2-on

(27) 1-[4-(5-Amidino-2-pyrimidinyl)-phenyl]-2-(2-carboxy-ethyl)-imidazolidin-2-on

(28) 1-[6-(4-Amidino-phenyl)-3-pyridazinyl]-3-(2-carboxy-ethyl)-imidazolidin-2-on
Schmelzpunkt: ab 300°C (Zers.)
$R_f$-Wert: 0,47 (Kieselgel; Butanol/Eisessig/Wasser = 4:1:1)

(29) 1-[2-(4-Amidino-phenyl)-5-pyrimidinyl]-3-(2-carboxyethyl)-imidazolidin-2-on

(30) 1-[2-(4'-Amidino-4-biphenylyl)-ethyl]-3-carboxymethyl-imidazolidin-2-on

(31) 1-(4-Amidino-3-fluor-phenyl)-3-[4-(2-carboxy-ethyl)-phenyl]-imidazolidin-2-on

(32) 1-(4-Amidino-3-chlor-phenyl)-3-[4-(2-carboxy-ethyl)-phenyl]-imidazolidin-2-on

(33) 1-(4-Amidino-2-methylthio-phenyl)-3-[4-(2-carboxy-ethyl)-phenyl]-imidazolidin-2-on

(34) 1-(4-Amidino-2-methylsulfinyl-phenyl)-3-[4-(2-carboxy-ethyl)-phenyl]-imidazolidin-2-on

(35) 1-(4-Amidino-2-methylsulfonyl-phenyl)-3-[4-(2-carboxy-ethyl)-phenyl]-imidazolidin-2-on

(36) 1-(4-Amidino-2-methyl-phenyl)-3-[4-(2-carboxy-ethyl)-phenyl]-imidazolidin-2-on
Schmelzpunkt: über 270°C
$R_f$-Wert: 0,56 (Reversed Phase Platte RP8; Methanol/5%ige Natriumchloridlösung = 6:4)

| | | | | | | |
|---|---|---|---|---|---|---|
| Ber.: | C | 65,56 | H | 6,05 | N | 15,29 |
| Gef.: | | 65,43 | | 6,04 | | 15,36 |

(37) 1-(4-Amidino-2-methoxy-phenyl)-3-[4-(2-carboxy-ethyl)-phenyl]-imidazolidin-2-on

(38) 1-(4-Amidino-phenyl)-3-[4-(2-carboxy-ethyl)-2-fluorphenyl]-imidazolidin-2-on

(39) 1-(4-Amidino-phenyl)-3-[4-(2-carboxy-ethyl)-2-chlorphenyl]-imidazolidin-2-on

(40) 1-(4-Amidino-phenyl)-3-[4-(2-carboxy-ethyl)-2-methoxyphenyl]-imidazolidin-2-on

(41) 1-(4-Amidino-phenyl)-3-[4-(2-carboxy-ethyl)-2-methylphenyl]-imidazolidin-2-on

(42) 1-(4-Amidino-phenyl)-3-[4-(2-carboxy-ethyl)-2-methylthio-phenyl]-imidazolidin-2-on

(43) 1-(4-Amidino-phenyl)-3-[4-(2-carboxy-ethyl)-2-methylsulfinyl-phenyl]-imidazolidin-2-on

(44) 1-(4-Amidino-phenyl)-3-[4-(2-carboxy-ethyl)-2-methylsulfonyl-phenyl]-imidazolidin-2-on

(45) 1-(4-Amidino-phenyl)-3-[5-(2-carboxy-ethyl)-2-pyridyl]-imidazolidin-2-on

(46) 1-(4-Amidino-phenyl)-3-[4-(2-carboxy-ethyl)-cyclohexyl]-imidazolidin-2-on
Schmelzpunkt: über 200°C
$R_f$-Wert: 0,59 (Reversed Phase Platte RP8; Methanol/5%ige Natriumchloridlösung = 6:4)

| | | | | | | |
|---|---|---|---|---|---|---|
| Ber. x 0,4 $H_2O$:<br>Gef.: | C | 62,41<br>62,43 | H | 7,38<br>7,31 | N | 15,32<br>15,18 |

(47) 1-(4-Amidino-phenyl)-3-(4-carboxymethyloxy-phenyl)-imidazolidin-2-on

(48) 1-(4-Amidino-phenyl)-3-(4-carboxymethylthio-phenyl)-imidazolidin-2-on
Schmelzpunkt: über 275°C
$R_f$-Wert: 0,56 (Reversed Phase Platte RP8; Methanol/10%ige Natriumchloridlösung = 6:4)

| | | | | | | | | |
|---|---|---|---|---|---|---|---|---|
| Ber. x 0,5 $H_2O$:<br>Gef.: | C | 56,98<br>56,94 | H | 5,05<br>5,15 | N | 14,76<br>14,98 | S | 8,45<br>8,42 |

(49) 1-(4-Amidino-phenyl)-3-(4-carboxymethylsulfinyl-phenyl)-imidazolidin-2-on
Schmelzpunkt: 252-254°C (Zers.)
$R_f$-Wert: 0,79 (Reversed Phase Platte RP8; Methanol/10%ige Natriumchloridlösung = 6:4)

| | | | | | | | | |
|---|---|---|---|---|---|---|---|---|
| Ber. x 1,5 $H_2O$:<br>Gef.: | C | 52,28<br>52,34 | H | 5,12<br>4,97 | N | 13,55<br>13,62 | S | 7,74<br>8,26 |

(50) 1-(4-Amidino-phenyl)-3-(4-carboxymethylsulfonyl-phenyl)-imidazolidin-2-on
Schmelzpunkt: über 260°C
$R_f$-Wert: 0,82 (Reversed Phase Platte RP8; Methanol/10%ige Natriumchloridlösung = 6:4)

| | | | | | | | | |
|---|---|---|---|---|---|---|---|---|
| Ber. x 0,5 $H_2O$:<br>Gef.: | C | 52,55<br>52,88 | H | 4,66<br>4,83 | N | 13,62<br>13,57 | S | 7,78<br>7,99 |

(51) 1-(4-Amidino-phenyl)-3-[4-(3-carboxy-2-methyl-2-propyl)-phenyl]-imidazolidin-2-on
Man verwendet Lithiumhydroxid
Schmelzpunkt: über 260°C
$R_f$-Wert: 0,76 (Reversed Phase Platte RP8; Methanol/10%ige Natriumchloridlösung = 6:4)

(52) 1-(4-Amidino-phenyl)-3-[4-(2-carboxy-2-methyl-propyl)-phenyl]-imidazolidin-2-on

(53) 1-(4-Amino-cyclohexyl)-3-[4-[(2-carboxy-ethyl)-aminocarbonyl]-phenyl]-imidazolidin-2-on

(54) 4-(4'-Amidino-4-biphenyl)-1-(2-carboxy-ethyl)-3-phenyl-imidazolidin-2-on

(55) 1-(4-Amidino-phenyl)-3-[2-(4-carboxyphenyl)-ethyl]-imidazolidin-2-on
Man arbeitet mit Lithiumhydroxid und einem 2:1-Gemisch aus Methanol und Dioxan.
Schmelzpunkt: 320-325°C (Zers.)
$R_f$-Wert: 0,55 (Reversed Phase Platte RP8; Methanol/5%ige Natriumchloridlösung = 6:4)

(56) 1-(4-Amidino-phenyl)-3-[4-(2-carboxy-ethenyl)-phenyl]-imidazolidin-2-on

(57) 1-(5-Amidino-2-pyridyl)-3-[4-(2-carboxy-ethyl)-phenyl]-imidazolidin-2-on

(58) 1-(1-Amidino-4-piperidinyl)-3-[4-(2-carboxy-ethyl)-phenyl]-imidazolidin-2-on

(59) 1-(4-Aminomethyl-phenyl)-3-[4-(2-carboxy-ethyl)-phenyl]-imidazolidin-2-on
Schmelzpunkt: über 260°C
$R_f$-Wert: 0,61 (Reversed Phase Platte RP8; Methanol/10%ige Natriumchloridlösung = 6:4)

| | | | | | | |
|---|---|---|---|---|---|---|
| Ber. x $H_2O$:<br>Gef.: | C | 63,85<br>64,17 | H | 6,49<br>6,50 | N | 11,76<br>11,59 |

(60) 1-(4-Amidino-phenyl)-3-[4-(2-carboxy-ethyl)-phenyl]-imidazolidin-2-thion

(61) 1-(4-Amidino-phenyl)-3-[4-(2-carboxy-ethyl)-phenyl]-2-imino-imidazolidin

(62) 1-(4-Amidino-phenyl)-3-[4-(2-carboxy-ethyl)-phenyl]-imidazolidin-2,4-dion
Man verwendet Lithiumhydroxid
Schmelzpunkt: über 260°C
$R_f$-Wert: 0,83 (Reversed Phase Platte RP8; Methanol/10%ige Natriumchloridlösung = 6:4)

| | | | | | | |
|---|---|---|---|---|---|---|
| Ber. x 0,5 $H_2O$: | C | 60,79 | H | 5,10 | N | 14,93 |
| Gef.: | | 60,69 | | 5,04 | | 15,12 |

(63) 3-(4-Amidino-phenyl)-1-[4-(2-carboxy-ethyl)-phenyl]-imidazolidin-2,4-dion

(64) 1-(4-Amidino-phenyl)-3-[4-(2-carboxy-ethyl)-phenyl]-4,4-dimethyl-imidazolidin-2,5-dion

(65) 3-(4-Amidino-phenyl)-1-[4-(2-carboxy-ethyl)-phenyl]-4,4-dimethyl-imidazolidin-2,5-dion

(66) 2-(4-Amidino-phenyl)-5-[4-(2-carboxy-ethyl)-phenyl]-3,4-dihydro-2H,5H-1,2,5-thiadiazol-1,1-dioxid
Schmelzpunkt: über 275°C
$R_f$-Wert: 0,53 (Reversed Phase Platte RP8; Methanol/10%ige Natriumchloridlösung = 6:4)

| | | | | | | | | |
|---|---|---|---|---|---|---|---|---|
| Ber.: | C | 55,66 | H | 5,19 | N | 14,42 | S | 8,25 |
| Gef.: | | 55,84 | | 5,24 | | 14,23 | | 8,01 |

(67) 1-(4-Amidino-phenyl)-3-[4-(2-carboxy-ethyl)-phenyl]-3H-imidazol-2-on
Man verwendet Lithiumhydroxid.
Schmelzpunkt: über 260°C
$R_f$-Wert: 0,58 (Reversed Phase Platte RP8; 10%ige Natriumchloridlösung/Methanol = 4:6)

(68) 1-(4-Amidino-phenyl)-3-[4-(2-carboxy-ethyl)-phenyl]-2-methylimino-imidazolidin

(69) 1-(4-Amidino-phenyl)-3-[4-(2-carboxy-ethyl)-phenyl]-2-phenylimino-imidazolidin

(70) 1-(4-Amidino-phenyl)-3-[4-(2-carboxy-ethyl)-phenyl]-2-(3-pyridylimino)-imidazolidin

(71) 3-(4-Amidino-phenyl)-1-[4-(2-carboxy-ethyl)-phenyl]-4-trifluormethyl-3H-imidazol-2-on

(72) 1-(4-Amidino-phenyl)-3-[4-(2-carboxy-ethyl)-phenyl]-4-phenyl-3H-imidazol-2-on

(73) 1-(4-Amidino-phenyl)-3-[4-(2-carboxy-ethyl)-phenyl]-4-phenyl-imidazolidin-2-on

(74) 1-[(4-Amidino-phenyl)-carbonylmethyl]-3-(3-carboxypropyl)-imidazolidin-2-on

(75) 1-[2-(4-Amidino-phenyl)-ethyl]-3-(3-carboxypropyl)-imidazolidin-2-on

(76) 1-[2-(4-Amidino-phenyl)-2-hydroxy-ethyl]-3-(3-carboxypropyl)-imidazolidin-2-on

(77) 1-(4-Amidino-phenyl)-3-(4-carboxy-butyl)-imidazolidin-2-on
$R_f$-Wert: 0,50 (Kieselgel; n-Butanol/Eisessig/Wasser = 4:1:1)

(78) 1-(4-Amidino-phenyl)-3-(2-carboxymethylthio-ethyl)-imidazolidin-2-on

(79) 1-(4-Amidino-phenyl)-3-(2-carboxymethylsulfinyl-ethyl)-imidazolidin-2-on

(80) 1-(4-Amidino-phenyl)-3-(2-carboxymethylsulfonyl-ethyl)-imidazolidin-2-on

(81) 1-(4-Amidino-phenyl)-3-(2-carboxymethyloxy-ethyl)-imidazolidin-2-on

(82) 1-(4-Amidino-phenyl)-3-(2-carboxymethylamino-ethyl)-imidazolidin-2-on
Man verwendet Lithiumhydroxid
Schmelzpunkt: 298°C (Zers., sintert ab 285°C)
$R_f$-Wert: 0,14 (Kieselgel; Methanol/2N Ammoniak = 5:1 bei dreifacher Entwicklung)

(83) 1-[2-(N-Acetyl-N-carboxymethyl-amino)-ethyl]-3-(4-amidino-phenyl)-imidazolidin-2-on

(84) 1-(4-Amidino-phenyl)-3-[2-(N-benzoyl-N-carboxymethyl-amino)-ethyl]-imidazolidin-2-on

(85) 1-(4-Amidino-phenyl)-3-[1-(2-carboxy-ethyl)-2-oxo-1H-4-pyridyl]-imidazolidin-2-on

(86) 1-[4-(2-carboxy-ethyl)-phenyl]-3-(4-ethoxycarbonylamidino-phenyl)-imidazolidin-2-on

(87) 1-(4-Amidino-phenyl)-3-[4-(3-carboxy-propyl)-phenyl]-imidazolidin-2-on

(88) 1-(4'-Amidino-3-methansulfonylamino-4-biphenylyl)-3-(2-carboxy-ethyl)-imidazolidin-2-on

(89) 1-(4-Amidino-phenyl)-3-[4-(2-carboxy-ethyl)-phenyl]-4H-1,2,4-triazol-5-on

(90) 1-(4-Amidino-phenyl)-3-[4-(2-carboxy-ethyl)-phenyl]-4-methyl-4H-1,2,4-triazol-5-on

(91) 3-(4-Amidino-phenyl)-1-[4-(2-carboxy-ethyl)-phenyl]-4H-1,2,4-triazol-5-on

(92) 3-(4-Amidino-phenyl)-1-[4-(2-carboxy-ethyl)-phenyl]-4-methyl-4H-1,2,4-triazol-5-on

(93) 4-(4-Amidino-phenyl)-1-[4-(2-carboxy-ethyl)-phenyl]-3H-imidazol-2-on

(94) 4-(4-Amidino-phenyl)-1-[4-(2-carboxy-ethyl)-phenyl]-3-methyl-3H-imidazol-2-on-hydrochlorid
Man verwendet Lithiumhydroxid
Schmelzpunkt: 288-294°C (Zers.)

(95) 4-(4-Amidino-phenyl)-1-[4-(2-carboxy-ethyl)-phenyl]-3-phenyl-3H-imidazol-2-on

(96) 4-(4-Amidino-phenyl)-1-[4-(2-carboxy-ethyl)-phenyl]-3H-imidazol-2-thion

(97) 4-(4-Amidino-phenyl)-1-[4-(2-carboxy-ethyl)-phenyl]-3-methyl-3H-imidazol-2-thion

(98) 4-(4-Amidino-phenyl)-1-[4-(2-carboxy-ethyl)-phenyl]-3-phenyl-3H-imidazol-2-thion

(99) 4-(4-Amidino-phenyl)-1-[4-(2-carboxy-ethyl)-phenyl]-5-methyl-3H-imidazol-2-on

(100) 4-(4-Amidino-phenyl)-1-[4-(2-carboxy-ethyl)-phenyl]-3,5-dimethyl-3H-imidazol-2-on

(101) 4-(4-Amidino-phenyl)-1-[4-(2-carboxy-ethyl)-phenyl]-5-methyl-3-phenyl-3H-imidazol-2-on

(102) 4-(4-Amidino-phenyl)-1-[4-(2-carboxy-ethyl)-phenyl]-imidazolidin-2-on

(103) 4-(4-Amidino-phenyl)-1-[4-(2-carboxy-ethyl)-phenyl]-3-methyl-imidazolidin-2-on

(104) 4-(4-Amidino-phenyl)-1-[4-(2-carboxy-ethyl)-phenyl]-3-phenyl-imidazolidin-2-on

(105) 1-(4-Amidino-phenyl)-4-[4-(2-carboxy-ethyl)-phenyl]-3H-imidazol-2-on

(106) 1-(4-Amidino-phenyl)-4-[4-(2-carboxy-ethyl)-phenyl]-3-methyl-3H-imidazol-2-on

(107) 1-(4-Amidino-phenyl)-4-[4-(2-carboxy-ethyl)-phenyl]-3-phenyl-3H-imidazol-2-on

(108) 1-(4-Amidino-phenyl)-4-[4-(2-carboxy-ethyl)-phenyl]-3H-imidazol-2-thion

(109) 1-(4-Amidino-phenyl)-4-[4-(2-carboxy-ethyl)-phenyl]-3-methyl-3H-imidazol-2-thion

(110) 1-(4-Amidino-phenyl)-4-[4-(2-carboxy-ethyl)-phenyl]-3-phenyl-3H-imidazol-2-thion

(111) 1-(4-Amidino-phenyl)-4-[4-(2-carboxy-ethyl)-phenyl]-5-methyl-3-phenyl-3H-imidazol-2-on

(112) 1-(4-Amidino-phenyl)-4-[4-(2-carboxy-ethyl)-phenyl]-3,5-dimethyl-3H-imidazol-2-on

(113) 1-(4-Amidino-phenyl)-4-[4-(2-carboxy-ethyl)-phenyl]-5-methyl-3H-imidazol-2-on

(114) 1-(4-Amidino-phenyl)-4-[4-(2-carboxy-ethyl)-phenyl]-imidazolidin-2-on

(115) 1-(4-Amidino-phenyl)-4-[4-(2-carboxy-ethyl)-phenyl]-3-methyl-imidazolidin-2-on

(116) 1-(4-Amidino-phenyl)-4-[4-(2-carboxy-ethyl)-phenyl]-3-phenyl-imidazolidin-2-on

(117) 4-(4'-Amidino-4-biphenylyl)-1-(2-carboxy-ethyl)-3H-imidazol-2-on-hydrochlorid
Schmelzpunkt: über 280°C

| | | | | | | | | |
|---|---|---|---|---|---|---|---|---|
| Ber. x $H_2O$:<br>Gef.: | C | 56,35<br>56,56 | H | 5,48<br>5,31 | N | 13,84<br>13,82 | Cl | 8,76<br>8,96 |

(118) 4-(4'-Amidino-4-biphenylyl)-1-(2-carboxy-ethyl)-3-methyl-3H-imidazol-2-on-hydrochlorid
Schmelzpunkt: über 280°C

| | | | | | | | | |
|---|---|---|---|---|---|---|---|---|
| Ber. x $H_2O$:<br>Gef.: | C | 57,34<br>57,37 | H | 5,53<br>5,91 | N | 13,37<br>13,39 | Cl | 8,46<br>8,79 |

(119) 4-(4'-Amidino-4-biphenylyl)-1-(2-carboxy-ethyl)-3-phenyl-3H-imidazol-2-on-hydrochlorid
Schmelzpunkt: über 280°C

| | | | | | | | | |
|---|---|---|---|---|---|---|---|---|
| Ber. x $H_2O$:<br>Gef.: | C | 62,43<br>62,66 | H | 5,24<br>5,14 | N | 11,65<br>11,83 | Cl | 7,37<br>7,67 |

(120) 4-(4'-Amidino-4-biphenylyl)-1-(2-carboxy-ethyl)-3H-imidazol-2-thion

(121) 4-(4'-Amidino-4-biphenylyl)-1-(2-carboxy-ethyl)-3-methyl-3H-imidazol-2-thion

(122) 4-(4'-Amidino-4-biphenylyl)-1-(2-carboxy-ethyl)-3-phenyl-3H-imidazol-2-thion

(123) 4-(4'-Amidino-4-biphenylyl)-1-(2-carboxy-ethyl)-5-methyl-3H-imidazol-2-on

(124) 4-(4'-Amidino-4-biphenylyl)-1-(2-carboxy-ethyl)-3,5-dimethyl-3H-imidazol-2-on

(125) 4-(4'-Amidino-4-biphenylyl)-1-(2-carboxy-ethyl)-5-methyl-3-phenyl-3H-imidazol-2-on

(126) 4-(4'-Amidino-4-biphenylyl)-1-(2-carboxy-ethyl)-imidazolidin-2-on

(127) 4-(4'-Amidino-4-biphenylyl)-1-(2-carboxy-ethyl)-3-methyl-imidazolidin-2-on

(128) 1-[4-(2-Carboxy-ethyl)-phenyl]-3-(3-guanidino-phenylimidazolidin-2-on

(129) 1-(4-Amidino-phenyl)-3-[1-(2-carboxy-ethyl)-4-piperidinyl]-imidazolidin-2-on

(130) 1-[4-(2-Carboxy-ethyl)-phenyl]-3-(4-methylamidino-phenyl)-imidazolidin-2-on

(131) 1-(4-n-Butylamidino-phenyl]-3-[4-(2-carboxy-ethyl)-phenyl]-imidazolidin-2-on

(132) 2-(4-Amidino-4'-biphenylyl)-4-(2-carboxy-ethyl)-4H-1,2,4-triazol-3-on

(133) 2-(4-Amidino-4'-biphenylyl)-4-(2-carboxy-ethyl)-5-methyl-4H-1,2,4-triazol-3-on

(134) 4-(4-Amidino-4'-biphenylyl)-2-(2-carboxy-ethyl)-4H-1,2,4-triazol-3-on

(135) 4-(4-Amidino-4'-biphenylyl)-2-(2-carboxy-ethyl)-5-methyl-4H-1,2,4-triazol-3-on

(136) 3-(4-Amidino-phenyl)-1-(4-carboxy-butyl)-3H-imidazo-[4,5-b]pyridin-2-on-hydrochlorid
$R_f$-Wert: 0,23 (Kieselgel; Methylenchlorid/Methanol = 19:1)

(137) 4-(4-Amidino-phenyl)-2-[4-(2-carboxy-ethyl)-phenyl]-5-methyl-4H-1,2,4-triazol-3-on
$R_f$-Wert: 0,60 (Reversed Phase Platte RP8; Methanol/5%ige Natriumchloridlösung = 6:4)

(138) 1-(4-Amidino-phenyl)-3-[4-(2-carboxy-ethyl)-phenyl]-4-methyl-3H-imidazol-2-on
$R_f$-Wert: 0,54 (Reversed Phase Platte RP8; Methanol/5%ige Natriumchloridlösung = 6:4)

| | | | | | | |
|---|---|---|---|---|---|---|
| Ber. x 1,8 $H_2O$: | C | 60,54 | H | 5,99 | N | 14,12 |
| Gef.: | | 60,95 | | 5,88 | | 14,15 |

(139) 2-(4-Amidino-phenyl)-4-[4-(2-carboxy-ethyl)-phenyl]-5-methyl-4H-1,2,4-triazol-3-on
Schmelzpunkt: 303-305°C
$R_f$-Wert: 0,63 (Reversed Phase Platte RP8; Methanol/5%ige Natriumchloridlösung = 6:4)

| | | | | | | |
|---|---|---|---|---|---|---|
| Ber.: | C | 62,45 | H | 5,24 | N | 19,17 |
| Gef.: | | 62,28 | | 5,28 | | 18,70 |

(140) 2-(4-Amidino-phenyl)-4-[4-(2-carboxy-ethyl)-phenyl]-5-ethyl-4H-1,2,4-triazol-3-on
Schmelzpunkt: über 250°C
$R_f$-Wert: 0,53 (Reversed Phase Platte RP8; Methanol/10%ige Natriumchloridlösung = 6:4)

| | | | | | | |
|---|---|---|---|---|---|---|
| Ber. x 1,5 $H_2O$: | C | 59,10 | H | 5,95 | N | 17,23 |
| Gef.: | | 58,71 | | 6,10 | | 17,03 |

(141) 1-(4-Aminomethyl-phenyl)-3-(4-carboxymethyloxy-phenyl)-imidazolidin-2-on
Schmelzpunkt: über 250°C
$R_f$-Wert: 0,58 (Reversed Phase Platte RP8; Methanol/5%ige Natriumchloridlösung = 6:4)

| | | | | | | |
|---|---|---|---|---|---|---|
| Ber. x 0,5 $H_2O$: | C | 61,70 | H | 5,75 | N | 11,99 |
| Gef.: | | 61,48 | | 5,81 | | 12,22 |

(142) 1-(4-Aminomethyl-phenyl)-3-[4-(2-carboxy-ethyl)-phenyl]-3,4,5,6-tetrahydro-1H-pyrimidin-2-on
Schmelzpunkt: 229-231°C
$R_f$-Wert: 0,56 (Reversed Phase Platte RP8; Methanol/10%ige Natriumchloridlösung = 6:4)

(143) 3-(4'-Amidino-4-biphenylyl)-1-(2-carboxy-ethyl)-3H-imidazo[4,5-b]pyridin-2-on
$R_f$-Wert: 0,28 (Kieselgel; Methylenchlorid/Methanol = 8:2)

(144) 3-(4-Amidino-phenyl)-1-[4-(2-carboxy-ethyl)-phenyl]-4-methyl-3H-imidazol-2-on
$R_f$-Wert: 0,54 (Reversed Phase Platte RP8; Methanol/5%ige Natriumchloridlösung = 6:4)

(145) 2-(4-Amidino-phenyl)-4-[4-(2-carboxy-ethyl)-phenyl]-4H-1,2,4-triazol-3-on

Schmelzpunkt: 310-313°C
$R_f$-Wert: 0,58 (Reversed Phase Platte RP8; Methanol/5%ige Natriumchloridlösung = 6:4)

| | | | | | | |
|---|---|---|---|---|---|---|
| Ber.: | C | 59,99 | H | 5,03 | N | 19,44 |
| Gef.: | | 60,03 | | 5,04 | | 19,15 |

(146) 4-(4-Amidino-phenyl)-2-[4-(2-carboxy-ethyl)-phenyl]-4H-1,2,4-triazol-3-on
Schmelzpunkt: 313-316°C
$R_f$-Wert: 0,61 (Reversed Phase Platte RP8; Methanol/5%ige Natriumchloridlösung = 6:4)

| | | | | | | |
|---|---|---|---|---|---|---|
| Ber.: | C | 61,53 | H | 4,88 | N | 19,93 |
| Gef.: | | 61,42 | | 4,97 | | 20,22 |

(147) 1-(4-Amidino-phenyl)-3-[4-(2-carboxy-ethenyl)-phenyl]-3H-imidazol-2-on
Schmelzpunkt: über 350°C
$R_f$-Wert: 0,45 (Reversed Phase Platte RP8; Methanol/10%ige Natriumchloridlösung = 6:4)

(148) 1-(4-Amidino-phenyl)-3-[4-(2-amino-2-carboxy-ethyl)-phenyl]-3H-imidazol-2-on
Man verwendet Lithiumhydroxid
$R_f$-Wert: 0,06 (Kieselgel; Methylenchlorid/Methanol/konz. Ammoniak = 8:4:1)

(149) 1-[4-(2-Amino-2-carboxy-ethyl)-phenyl]-3-(4-aminomethyl-phenyl)-imidazolidin-2-on
Man verwendet Lithiumhydroxid
$R_f$-Wert: 0,15 (Kieselgel; Methylenchlorid/Methanol/konz. Ammoniak = 16:4:1)

(150) 1-(4-Amidino-phenyl)-3-[4-(2-carboxy-2-dibenzylaminoethyl)-phenyl]-3H-imidazol-2-on
Man verwendet Lithiumhydroxid
$R_f$-Wert: 0,50 (Kieselgel; Methylenchlorid/Methanol = 4:1)

(151) 1-[4-(2-Amino-ethyl)-phenyl]-3-(4-carboxymethyl-phenyl)-imidazolidin-2-on
Schmelzpunkt: über 250°C
$R_f$-Wert: 0,41 (Reversed Phase Platte RP8; Methanol/5%ige Natriumchloridlösung = 6:4)

(152) 1-[4-(2-Amino-2-propyl)-phenyl]-3-[4-(2-carboxy-ethyl)-phenyl]-imidazolidin-2-on

(153) 1-[4-(1-Amino-ethyl)-phenyl]-3-(4-(2-carboxy-ethyl)-phenyl]-imidazolidin-2-on
Schmelzpunkt: 267-269°C (Zers.)
$R_f$-Wert: 0,40 (Reversed Phase Platte RP8; Methanol/5%ige Natriumchloridlösung = 6:4)

(154) 1-(1-Amino-5-indanyl)-3-[4-(2-carboxy-ethyl)-phenyl]-imidazolidin-2-on
Schmelzpunkt: 234-236°C (Zers.)

(155) 1-(1-Amino-1,2,3,4-tetrahydro-6-naphthyl)-3-[4-(2-carboxy-ethyl)-phenyl]-imidazolidin-2-on

(156) 1-(4-Aminomethyl-2-methyl-phenyl)-3-[4-(2-carboxyethyl)-phenyl]-imidazolidin-2-on
Schmelzpunkt: 237-239°C (Zers.)
$R_f$-Wert: 0,50 (Reversed Phase Platte RP8; Methanol/5%ige Natriumchloridlösung = 6:4)

| | | | | | | |
|---|---|---|---|---|---|---|
| Ber. x 0,8 $H_2O$: | C | 65,31 | H | 6,74 | N | 11,42 |
| Gef.: | | 65,31 | | 6,68 | | 11,43 |

(157) 1-(4-Amidino-phenyl)-3-[4-(2-carboxy-2-dimethylaminoethyl)-phenyl]-3H-imidazol-2-on

(158) 1-(4-Amidino-phenyl)-3-[4-(2-carboxy-1-carboxymethylethyl)-phenyl]-imidazolidin-2-on

(159) 1-(4-Amidino-phenyl)-3-(3,4-bis-carboxymethyloxy-phenyl]-imidazolidin-2-on

(160) 3-(4-Aminomethyl-phenyl)-1-[4-(2-carboxy-ethyl)-phenyl]-4-methyl-3H-imidazol-2-on

(161) 1-(4-Amino-cyclohexyl)-3-[4-(3-carboxy-propyl)-phenyl]-imidazolidin-2-on

(162) 1-(4-Amino-cyclohexyl)-3-[4-(2-carboxy-ethyl)-phenyl]-imidazolidin-2-on

(163) 1-(4'-Aminomethyl-4-biphenylyl)-3-(2-carboxy-ethyl)-imidazolidin-2-on

(164) 1-(4-Aminomethyl-3-fluor-phenyl)-3-[4-(2-carboxyethyl)-phenyl]-imidazolidin-2-on

(165) 1-(4-Amidino-phenyl)-3-[4-(2-carboxy-ethyl)-phenyl]-4-methyl-imidazolidin-2-on

(166) 1-(4-Aminomethyl-phenyl)-3-[3-(2-carboxy-ethyl)-phenyl]-imidazolidin-2-on

(167) 1-(4-Aminomethyl-phenyl)-3-[4-(2-carboxy-1-carboxymethyl-ethyl)-phenyl]-imidazolidin-2-on

(168) 4-(4-Aminomethyl-phenyl)-2-[4-(2-carboxy-ethyl)-phenyl]-4H-1,2,4-triazol-3-on

(169) 1-[4-(2-Amino-ethyl)-phenyl]-3-[4-(2-carboxy-ethyl)-phenyl]-imidazolidin-2-on
Man verwendet halbkonzentrierte Salzsäure bei Raumtemperatur. Schmelzpunkt: über 250°C
$R_f$-Wert: 0,57 (Reversed Phase Platte RP8; Eisessig/Wasser = 4:6)

(170) 1-[4-(1-Amino-2-methyl-2-propyl)-phenyl]-3-[4-(2-carboxy-ethyl)-phenyl]-imidazolidin-2-on

(171) 1-[4-[(2-Carboxy-ethyl)-aminocarbonyl]-phenyl]-3-(4-piperidinyl)-imidazolidin-2-on

(172) 1-[4-(2-Carboxy-ethyl)-phenyl]-3-[4-(dimethylamino-methyl)-phenyl]-imidazolidin-2-on

(173) 1-[4-(2-Carboxy-ethyl)-phenyl]-3-[4-(methylamino-methyl)-phenyl]-imidazolidin-2-on

(174) 1-[4-(2-Carboxy-ethyl)-phenyl]-3-[4-(n-propylamino-methyl)-phenyl]-imidazolidin-2-on

(175) 2-(4-Aminomethyl-phenyl)-4-[4-(2-carboxy-ethyl)-phenyl]-5-methyl-4H-1,2,4-triazol-3-on

(176) 1-[4-(1-Amino-cyclopropyl)-phenyl]-3-[4-(2-carboxyethyl)-phenyl]-imidazolidin-2-on

(177) 1-[4-(1-Amino-cyclopentyl)-phenyl]-3-[4-(2-carboxyethyl)-phenyl]-imidazolidin-2-on

(178) 1-(4-Amidino-phenyl)-3-[4-(2-amino-2-carboxy-ethyl)-phenyl]-imidazolidin-2-on
Man verwendet Lithiumhydroxid
$R_f$-Wert: 0,07 (Kieselgel; Methylenchlorid/Methanol/konz. Ammoniak = 8:4:1)

(179) 1-(4-Amidino-phenyl)-3-[4-(2-carboxy-2-hydroxy-ethyl)-phenyl]-3H-imidazol-2-on

(180) 1-(4-Amidino-phenyl)-3-[4-(2-carboxy-2-methoxy-ethyl)-phenyl]-3H-imidazol-2-on

(181) 1-[4-(2-Amino-ethyl)-phenyl]-3-(4-carboxymethyloxyphenyl)-imidazolidin-2-on
Man kocht das Hydrochlorid des Ethylesters mit Wasser. Schmelzpunkt: über 350°C
$R_f$-Wert: 0,58 (Reversed Phase Platte RP8; Eisessig/Wasser = 4:6)

(182) 1-[3-(2-Amino-ethyl)-phenyl]-3-[4-(2-carboxy-ethyl)-phenyl]-imidazolidin-2-on
Schmelzpunkt: >250°C
$R_f$-Wert: 0,42 (Reversed Phase Platte RP8; Methanol/5%ige Natriumchloridlösung = 6:4)

| | | | | | | |
|---|---|---|---|---|---|---|
| Ber. x 0,3 $H_2O$: | C | 66,95 | H | 6,63 | N | 11,71 |
| Gef.: | | 66,64 | | 6,65 | | 11,82 |

(183) 1-[4-[(2-Carboxy-ethyl)-aminocarbonyl]-phenyl]-3-(1-methyl-4-piperidinyl)-imidazolidin-2-on

(184) 1-(4-Aminomethyl-cyclohexyl)-3-[4-(2-carboxy-ethyl)-phenyl]-imidazolidin-2-on

(185) 2-(4-Amidino-phenyl)-4-[4-(2-carboxy-2-methyl-propyl)-phenyl]-5-methyl-4H-1,2,4-triazol-3-on

(186) 2-(4-Amidino-phenyl)-4-[4-(2-carboxy-ethyl)-phenyl]-5-trifluormethyl-4H-1,2,4-triazol-3-on

(187) 2-(4-Amidino-phenyl)-4-[4-(2-carboxy-ethyl)-phenyl]-5-phenyl-4H-1,2,4-triazol-3-on

Beispiel 2

1-(4'-Amidino-4-biphenylyl)-3-methoxycarbonylmethyl-imidazolidin-2-on-hydrochlorid

Zu 3,1 g 1-(4'-Cyano-4-biphenylyl)-3-methoxycarbonylmethylimidazolidin-2-on gibt man 85 ml einer unter Eiskühlung gesättigten Lösung von Chlorwasserstoff in Methanol. Die entstandene Suspension wird mit Petrolether überschichtet und 3,5 Stunden bei Raumtemperatur gerührt. Man engt zur Trockene ein und trocknet noch 15 Minuten bei 1 mbar nach. Der Rückstand wird in 80 ml absolutem Methanol suspendiert, mit 2,7 g Ammoniumcarbonat versetzt und 16 Stunden bei Raumtemperatur gerührt. Man filtriert vom Niederschlag ab, engt die Mutterlauge ein und reinigt den Rückstand durch Säulenchromatographie an Kieselgel (Elutionsmittel: Methylenchlorid/Methanol/konz.Ammoniak = 3:1:0,2).
Ausbeute: 0,7 g (20 % der Theorie),
Schmelzpunkt: über 200°C
$R_f$-Wert: 0,53 (Kieselgel; Methylenchlorid/Methanol/konz. Ammoniak = 3:1:0,2)

Analog werden folgende Verbindungen erhalten:

(1) 1-(4'-Amidino-4-biphenylyl)-3-(2-methoxycarbonyl-ethyl)-imidazolidin-2-on-hydrochlorid
Man arbeitet mit einem vierfachen Überschuß von Ammoniumchlorid, wobei man 6 Stunden zum Rückfluß erhitzt.
Schmelzpunkt: über 200°C
$R_f$-Wert: 0,40 (Kieselgel; Methylenchlorid/Methanol/konz. Ammoniak = 5:1:0,2)

(2) 1-(4'-Amidino-4-biphenylyl)-3-(2-ethoxycarbonyl-ethyl)-imidazolidin-2,4-dion-hydrochlorid
In der ersten Phase der Reaktion verwendet man ethanolische Salzsäure. Bei der Umsetzung mit Ammoniumcarbonat wird 4 Stunden auf 50°C erwärmt. Zur Reinigung wird der Eindampfrückstand mit Wasser verrührt.
Schmelzpunkt: über 200°C
$R_f$-Wert: 0,50 (Kieselgel; Methylenchlorid/Methanol = 4:1)

| | | | | | | | | |
|---|---|---|---|---|---|---|---|---|
| Ber. x HCl x $H_2O$:<br>Gef.: | C | 56,18<br>56,41 | H | 5,61<br>5,70 | N | 12,48<br>12,29 | Cl | 7,90<br>7,78 |

(3) 1-(4'-Amidino-4-biphenylyl)-3-(2-ethoxycarbonyl-ethyl)-3H-imidazol-2-on-hydrochlorid
In der ersten Phase der Reaktion verwendet man ethanolische Salzsäure. Bei der Umsetzung mit Ammoniumcarbonat dient Ethanol als Lösungsmittel.
Schmelzpunkt: über 200°C
$R_f$-Wert: 0,27 (Kieselgel; Methylenchlorid/Methanol/konz. Ammoniak = 3:1:0,2)

| | | | | | | | | |
|---|---|---|---|---|---|---|---|---|
| Ber. x 1,2 HCl x $H_2O$:<br>Gef.: | C | 57,30<br>57,36 | H | 5,77<br>5,83 | N | 12,73<br>12,38 | Cl | 9,66<br>9,26 |

(4) 1-(4'-Amidino-4-biphenylyl)-3-(2-ethoxycarbonyl-ethyl)-3,4,5,6-tetrahydro-1H-pyrimidin-2-on-hydrochlorid
In der ersten Phase der Reaktion verwendet man ethanolische Salzsäure. Bei der Umsetzung mit Ammoniumcarbonat wird 4 Stunden auf 50°C erwärmt. Zur Reinigung wird der Eindampfrückstand mit Wasser verrührt.
Schmelzpunkt: 212-215°C
$R_f$-Wert: 0,47 (Kieselgel; Methylenchlorid/Methanol/konz. Ammoniak = 3:1:0,2)

| | | | | | | | | |
|---|---|---|---|---|---|---|---|---|
| Ber. x HCl: | C | 61,32 | H | 6,32 | N | 13,00 | Cl | 8,23 |

(fortgesetzt)

| | | | | | | | | |
|---|---|---|---|---|---|---|---|---|
| Gef.: | | 60,71 | | 6,40 | | 12,85 | | 8,04 |

(5) 1-(4'-Amidino-4-biphenylyl)-3-methoxycarbonylmethyl-3,4,5,6-tetrahydro-1H-pyrimidin-2-on-hydrochlorid
Schmelzpunkt: über 200°C
$R_f$-Wert: 0,50 (Kieselgel; Methylenchlorid/Methanol/konz. Ammoniak = 3:1:0,2)

(6) 1-(4-Amidino-phenyl)-3-[4-(2-methoxycarbonyl-ethyl)-phenyl]-imidazolidin-2-on-hydrochlorid
Schmelzpunkt: ab 260°C (Zers.)
Das Produkt erhält man auch durch Umsetzung von 1-(4-Amidinophenyl)-3-[4-(2-carboxy-ethyl)-phenyl]-imidazolidin-2-on mit methanolischer Salzsäure.
$R_f$-Wert: 0,49 (Reversed Phase Platte RP8; 10%ige Natriumchloridlösung/Methanol = 4:6)

(7) 1-(4-Amidino-phenyl)-3-[4-(2-methoxycarbonyl-ethyl)-phenyl]-3,4,5,6-tetrahydro-1H-pyrimidin-2-on-hydrochlorid
$R_f$-Wert: 0,08 (Kieselgel; Methylenchlorid/Methanol = 95:5)

(8) 1-[2-(4'-Amidino-4-biphenylyl)-ethyl]-3-methoxycarbonylmethyl-3H-benzimidazol-2-on
$R_f$-Wert: 0,18 (Kieselgel; Methylenchlorid/Methanol = 8:2)

(9) 1-(4'-Amidino-3'-fluor-4-biphenylyl)-3-(2-methoxycarbonyl-ethyl)-imidazolidin-2-on-hydrochlorid

(10) 1-(4'-Amidino-3'-chlor-4-biphenylyl)-3-(2-methoxycarbonyl-ethyl)-imidazolidin-2-on-hydrochlorid

(11) 1-(4'-Amidino-3-methoxy-4-biphenylyl)-3-(2-methoxycarbonyl-ethyl)-imidazolidin-2-on-hydrochlorid

(12) 1-(4'-Amidino-3-brom-4-biphenylyl)-3-(2-methoxycarbonylethyl)-imidazolidin-2-on-hydrochlorid

(13) 1-(4'-Amidino-3-methylthio-4-biphenylyl)-3-(2-methoxycarbonyl-ethyl)-imidazolidin-2-on-hydrochlorid

(14) 1-(4'-Amidino-3-methylsulfonyl-4-biphenylyl)-3-(2-methoxycarbonyl-ethyl)-imidazolidin-2-on-hydrochlorid

(15) 1-(4'-Amidino-2,3-dimethyl-4-biphenylyl)-3-(2-methoxycarbonyl-ethyl)-imidazolidin-2-on-hydrochlorid

(16) 1-(4'-Amidino-3-nitro-4-biphenylyl)-3-(2-methoxycarbonyl-ethyl)-imidazolidin-2-on-hydrochlorid

(17) 1-(4'-Amidino-3-amino-4-biphenylyl)-3-(2-methoxycarbonyl-ethyl)-imidazolidin-2-on-hydrochlorid

(18) 1-(3-Acetamino-4'-amidino-4-biphenylyl)-3-(2-methoxycarbonyl-ethyl)-imidazolidin-2-on-hydrochlorid

(19) 1-(4'-Amidino-3-benzoylamino-4-biphenylyl)-3-(2-methoxycarbonyl-ethyl)-imidazolidin-2-on-hydrochlorid

(20) 2-(4'-Amidino-4-biphenylyl)-5-(2-methoxycarbonyl-ethyl)-3,4-dihydro-2H,5H-1,2,5-thiadiazol-1,1-dioxid-hydrochlorid
Schmelzpunkt: 243-245°C
$R_f$-Wert: 0,43 (Reversed Phase Platte RP8; Methanol/10%ige Natriumchloridlösung = 6:4)

(21) 1-[4-(4-Amidino-phenyl)-cyclohexyl]-3-(2-methoxycarbonyl-ethyl)-3H-imidazol-2-on-hydrochlorid

(22) 1-[4-(4-Amidino-phenyl)-cyclohexyl]-3-(2-methoxycarbonyl-ethyl)-imidazolidin-2-on-hydrochlorid

(23) 1-[1-(4-Amidino-phenyl)-4-piperidinyl]-3-(2-methoxycarbonyl-ethyl)-imidazolidin-2-on-hydrochlorid

(24) 1-[4-(5-Amidino-2-pyridyl)-phenyl]-3-(2-methoxycarbonyl-ethyl)-imidazolidin-2-on-hydrochlorid

(25) 1-[4-(5-Amidino-2-pyrazinyl)-phenyl]-3-(2-methoxycarbonyl-ethyl)-imidazolidin-2-on-hydrochlorid

(26) 1-[4-(5-Amidino-2-pyrimidinyl)-phenyl]-3-(2-methoxycarbonyl-ethyl)-imidazolidin-2-on-hydrochlorid

(27) 1-[6-(4-Amidino-phenyl)-3-pyridazinyl]-3-(2-methoxycarbonyl-ethyl)-imidazolidin-2-on-hydrochlorid
Schmelzpunkt: 303-305°C (Zers., sintert ab 240°C)
$R_f$-Wert: 0,42 (Kieselgel; Methylenchlorid/Methanol/konz. Ammoniak = 8:2:0,1)

(28) 1-[2-(4-Amidino-phenyl)-5-pyrimidinyl]-3-(2-methoxycarbonyl-ethyl)-imidazolidin-2-on-hydrochlorid

(29) 1-[2-(4'-Amidino-4-biphenylyl)-ethyl]-3-methoxycarbonyl-methyl-imidazolidin-2-on-hydrochlorid

(30) 1-(4-Amidino-3-fluor-phenyl)-3-[4-(2-methoxycarbonyl-ethyl)-phenyl]-imidazolidin-2-on-hydrochlorid

(31) 1-(4-Amidino-3-chlor-phenyl)-3-[4-(2-methoxycarbonyl-ethyl)-phenyl]-imidazolidin-2-on-hydrochlorid

(32) 1-(4-Amidino-2-methylthio-phenyl)-3-[4-(2-methoxycarbonyl-ethyl)-phenyl]-imidazolidin-2-on-hydrochlorid

(33) 1-(4-Amidino-2-methylsulfonyl-phenyl)-3-[4-(2-methoxycarbonyl-ethyl)-phenyl]-imidazolidin-2-on-hydrochlorid

(34) 1-(4-Amidino-2-methyl-phenyl)-3-[4-(2-methoxycarbonyl-ethyl)-phenyl]-imidazolidin-2-on-hydrochlorid
Schmelzpunkt: 143-146°C
$R_f$-Wert: 0,37 (Reversed Phase Platte RP8; Methanol/5%ige Natriumchloridlösung = 6:4)

(35) 1-(4-Amidino-2-methoxy-phenyl)-3-[4-(2-methoxycarbonyl-ethyl)-phenyl]-imidazolidin-2-on-hydrochlorid

(36) 1-(4-Amidino-phenyl)-3-[2-fluor-4-(2-methoxycarbonyl-ethyl)-phenyl]-imidazolidin-2-on-hydrochlorid

(37) 1-(4-Amidino-phenyl)-3-[2-chlor-4-(2-methoxycarbonyl-ethyl)-phenyl]-imidazolidin-2-on-hydrochlorid

(38) 1-(4-Amidino-phenyl)-3-[2-methoxy-4-(2-methoxycarbonyl-ethyl)-phenyl]-imidazolidin-2-on-hydrochlorid

(39) 1-(4-Amidino-phenyl)-3-[4-(2-methoxycarbonyl-ethyl)-2-methyl-phenyl]-imidazolidin-2-on-hydrochlorid

(40) 1-(4-Amidino-phenyl)-3-[4-(2-methoxycarbonyl-ethyl)-2-methylthio-phenyl]-imidazolidin-2-on-hydrochlorid

(41) 1-(4-Amidino-phenyl)-3-[4-(2-methoxycarbonyl-ethyl)-2-methylsulfonyl-phenyl]-imidazolidin-2-on-hydrochlorid

(42) 1-(4-Amidino-phenyl)-3-[5-(2-methoxycarbonyl-ethyl)-2-pyridyl]-imidazolidin-2-on-hydrochlorid

(43) 1-(4-Amidino-phenyl)-3-[4-(2-methoxycarbonyl-ethyl)-cyclohexyl]-imidazolidin-2-on-hydrochlorid
Schmelzpunkt: über 200°C
$R_f$-Wert: 0,44 (Reversed Phase Platte RP8; Methanol/5%ige Natriumchloridlösung = 6:4)

(44) 1-(4-Amidino-phenyl)-3-(4-methoxycarbonylmethyloxy-phenyl)-imidazolidin-2-on-hydrochlorid

(45) 1-(4-Amidino-phenyl)-3-(4-methoxycarbonylmethylthiophenyl)-imidazolidin-2-on
Schmelzpunkt: ab 197°C (Zers.)
$R_f$-Wert: 0,25 (Reversed Phase Platte RP8; Methanol/10%ige Natriumchloridlösung = 6:4)

(46) 1-(4-Amidino-phenyl)-3-(4-methoxycarbonylmethylsulfonyl-phenyl)-imidazolidin-2-on-hydrochlorid
Schmelzpunkt: ab 249°C (Zers.)
$R_f$-Wert: 0,43 (Kieselgel; Methylenchlorid/Methanol = 8:2)

(47) 1-(4-Amidino-phenyl)-3-[4-(3-methoxycarbonyl-2-methyl-2-propyl)-phenyl]-imidazolidin-2-on-hydrochlorid
Schmelzpunkt: 228-236°C (Zers.)
$R_f$-Wert: 0,19 (Kieselgel; Methylenchlorid/Methanol = 9:1)

(48) 1-(4-Amidino-phenyl)-3-[2-(4-methoxycarbonyl-phenyl)-ethyl]-imidazolidin-2-on
Schmelzpunkt: 226-228°C (Zers.)
$R_f$-Wert: 0,40 (Reversed Phase Platte RP8; Methanol/5%ige Natriumchloridlösung = 6:4)

(49) 1-(4-Amidino-phenyl)-3-[4-(2-methoxycarbonyl-ethenyl)-phenyl]-imidazolidin-2-on-hydrochlorid

(50) 1-(5-Amidino-2-pyridyl)-3-[4-(2-methoxycarbonyl-ethyl)-phenyl]-imidazolidin-2-on-hydrochlorid

(51) 1-(4-Amidino-phenyl)-3-[4-(2-methoxycarbonyl-ethyl)-phenyl]-imidazolidin-2-thion-hydrochlorid

(52) 1-(4-Amidino-phenyl)-2-imino-3-[4-(2-methoxycarbonyl-ethyl)-phenyl]-imidazolidin-hydrochlorid

(53) 1-(4-Amidino-phenyl)-3-[4-(2-methoxycarbonyl-ethyl)-phenyl]-imidazolidin-2,4-dion-hydrochlorid
Schmelzpunkt: über 260°C
$R_f$-Wert: 0,19 (Kieselgel; Methylenchlorid/Methanol = 9:1)

| | | | | | | | | |
|---|---|---|---|---|---|---|---|---|
| Ber.: | C | 57,62 | H | 5,08 | N | 13,44 | Cl | 8,50 |
| Gef.: | | 56,94 | | 5,03 | | 13,33 | | 8,99 |

(54) 3-(4-Amidino-phenyl)-1-[4-(2-methoxycarbonyl-ethyl)-phenyl]-imidazolidin-2,4-dion-hydrochlorid

(55) 1-(4-Amidino-phenyl)-4,4-dimethyl-3-[4-(2-methoxycarbonyl-ethyl)-phenyl]-imidazolidin-2,5-dion-hydrochlorid

(56) 3-(4-Amidino-phenyl)-4,4-dimethyl-1-[4-(2-methoxycarbonyl-ethyl)-phenyl]-imidazolidin-2,5-dion-hydrochlorid

(57) 2-(4-Amidino-phenyl)-5-[4-(2-methoxycarbonyl-ethyl)-phenyl]-3,4-dihydro-2H,5H-1,2,5-thiadiazol-1,1-dioxid-hydrochlorid
Schmelzpunkt: 245-248°C (Zers.)
$R_f$-Wert: 0,44 (Reversed Phase Platte RP8; Methanol/10%ige Natriumchloridlösung = 6:4)

(58) 1-(4-Amidino-phenyl)-3-[4-(2-methoxycarbonyl-ethyl)-phenyl]-3H-imidazol-2-on-hydrochlorid
Schmelzpunkt: 240°C (Zers., sintert ab 208°C)
$R_f$-Wert: 0,33 (Kieselgel; Methylenchlorid/Methanol = 5:1)

(59) 1-(4-Amidino-phenyl)-3-[4-(2-methoxycarbonyl-ethyl)-phenyl]-2-methylimino-imidazolidin-hydrochlorid

(60) 1-(4-Amidino-phenyl)-3-[4-(2-methoxycarbonyl-ethyl)-phenyl]-2-phenylimino-imidazolidin-hydrochlorid

(61) 1-(4-Amidino-phenyl)-3-[4-(2-methoxycarbonyl-ethyl)-phenyl]-2-(3-pyridyl-imino)-imidazolidin-hydrochlorid

(62) 3-(4-Amidino-phenyl)-1-[4-(2-methoxycarbonyl-ethyl)-phenyl]-4-trifluormethyl-3H-imidazol-2-on-hydrochlorid

(63) 1-(4-Amidino-phenyl)-3-[4-(2-methoxycarbonyl-ethyl)-phenyl]-4-phenyl-3H-imidazol-2-on-hydrochlorid

(64) 1-(4-Amidino-phenyl)-3-[4-(2-methoxycarbonyl-ethyl)-phenyl]-4-phenyl-imidazolidin-2-on-hydrochlorid

(65) 1-[(4-Amidino-phenyl)-carbonylmethyl]-3-(3-methoxycarbonyl-propyl)-imidazolidin-2-on-hydrochlorid

(66) 1-[2-(4-Amidino-phenyl)-ethyl]-3-(3-methoxycarbonyl-propyl)-imidazolidin-2-on-hydrochlorid

(67) 1-(4-Amidino-phenyl)-3-[4-(2-methoxycarbonyl-ethyl)-phenyl]-4H-1,2,4-triazol-5-on-hydrochlorid

(68) 1-(4-Amidino-phenyl)-3-[4-(2-methoxycarbonyl-ethyl)-phenyl]-4-methyl-4H-1,2,4-triazol-5-on-hydrochlorid

(69) 3-(4-Amidino-phenyl)-1-[4-(2-methoxycarbonyl-ethyl)-phenyl]-4H-1,2,4-triazol-5-on-hydrochlorid

(70) 3-(4-Amidino-phenyl)-1-[4-(2-methoxycarbonyl-ethyl)-phenyl]-4-methyl-4H-1,2,4-triazol-5-on-hydrochlorid

(71) 1-(4-Amidino-phenyl)-3-(4-methoxycarbonyl-butyl)-imidazolidin-2-on-hydrochlorid
$R_f$-Wert: 0,72 (Kieselgel; Methylenchlorid/Methanol = 2:1)

(72) 1-(4-Amidino-phenyl)-3-(2-methoxycarbonylthio-ethyl)-imidazolidin-2-on-hydrochlorid

(73) 1-(4-Amidino-phenyl)-3-(2-methoxycarbonylmethylsulfonylethyl)-imidazolidin-2-on-hydrochlorid

(74) 1-(4-Amidino-phenyl)-3-(2-methoxycarbonyloxy-ethyl)-imidazolidin-2-on-hydrochlorid

(75) 1-(4-Amidino-phenyl)-3-(2-methoxycarbonylmethylaminoethyl)-imidazolidin-2-on-hydrochlorid-acetat
Schmelzpunkt: 197°C (Zers., sintert ab 172°C)
$R_f$-Wert: 0,44 (Kieselgel; Methylenchlorid/Methanol/konz. Ammoniak = 8:2:0,1 bei zweimaliger Entwicklung)

(76) 1-[2-(N-Acetyl-N-methoxycarbonylmethyl-amino)-ethyl]-3-(4-amidino-phenyl)-imidazolidin-2-on-hydrochlorid

(77) 1-(4-Amidino-phenyl)-3-[2-(N-benzoyl-N-methoxycarbonylmethyl-amino)-ethyl]-imidazolidin-2-on-hydrochlorid

(78) 1-(4-Amidino-phenyl)-3-[1-(2-methoxycarbonyl-ethyl)-2-oxo-1H-4-pyridyl]-imidazolidin-2-on-hydrochlorid

(79) 1-(4'-Amidino-3-methansulfonylamino-4-biphenylyl)-3-(2-methoxycarbonyl-ethyl)-imidazolidin-2-on-hydrochlorid

(80) 4-(4-Amidino-phenyl)-1-[4-(2-methoxycarbonyl-ethyl)-phenyl]-3H-imidazol-2-on-hydrochlorid

(81) 4-(4-Amidino-phenyl)-1-[4-(2-methoxycarbonyl-ethyl)-phenyl]-3-methyl-3H-imidazol-2-on-hydrochlorid
Schmelzpunkt: 242-246°C (Zers.)
$R_f$-Wert: 0,50 (Kieselgel; Methylenchlorid/Methanol = 4:1)

(82) 4-(4-Amidino-phenyl)-1-[4-(2-methoxycarbonyl-ethyl)-phenyl]-3-phenyl-3H-imidazol-2-on-hydrochlorid

(83) 4-(4-Amidino-phenyl)-1-[4-(2-methoxycarbonyl-ethyl)-phenyl]-3H-imidazol-2-thion-hydrochlorid

(84) 4-(4-Amidino-phenyl)-1-[4-(2-methoxycarbonyl-ethyl)-phenyl]-3-methyl-3H-imidazol-2-thion-hydrochlorid

(85) 4-(4-Amidino-phenyl)-1-[4-(2-methoxycarbonyl-ethyl)-phenyl]-3-phenyl-3H-imidazol-2-thion-hydrochlorid

(86) 4-(4-Amidino-phenyl)-1-[4-(2-methoxycarbonyl-ethyl)-phenyl]-5-methyl-3H-imidazol-2-on-hydrochlorid

(87) 4-(4-Amidino-phenyl)-3,5-dimethyl-1-[4-(2-methoxycarbonyl-ethyl)-phenyl]-3H-imidazol-2-on-hydrochlorid

(88) 4-(4-Amidino-phenyl)-1-[4-(2-methoxycarbonyl-ethyl)-phenyl]-5-methyl-3-phenyl-3H-imidazol-2-on-hydrochlorid

(89) 4-(4'-Amidino-4-biphenylyl)-3,5-dimethyl-1-(2-methoxycarbonyl-ethyl)-3H-imidazol-2-on-hydrochlorid

(90) 4-(4'-Amidino-4-biphenylyl)-1-(2-methoxycarbonyl-ethyl)-5-methyl-3-phenyl-3H-imidazol-2-on-hydrochlorid

(91) 4-(4'-Amidino-4-biphenylyl)-1-(2-methoxycarbonyl-ethyl)-5-methyl-3H-imidazol-2-on-hydrochlorid

(92) 1-(4-Amidino-phenyl)-4-[4-(2-methoxycarbonyl-ethyl)-phenyl]-3H-imidazol-2-on-hydrochlorid

(93) 1-(4-Amidino-phenyl)-4-[4-(2-methoxycarbonyl-ethyl)-phenyl]-3-methyl-3H-imidazol-2-on-hydrochlorid

(94) 1-(4-Amidino-phenyl)-4-[4-(2-methoxycarbonyl-ethyl)-phenyl]-3-phenyl-3H-imidazol-2-on-hydrochlorid

(95) 1-(4-Amidino-phenyl)-4-[4-(2-methoxycarbonyl-ethyl)-phenyl]-3H-imidazol-2-thion-hydrochlorid

(96) 1-(4-Amidino-phenyl)-4-[4-(2-methoxycarbonyl-ethyl)-phenyl]-3-methyl-3H-imidazol-2-thion-hydrochlorid

(97) 1-(4-Amidino-phenyl)-4-[4-(2-methoxycarbonyl-ethyl)-phenyl]-3-phenyl-3H-imidazol-2-thion-hydrochlorid

(98) 1-(4-Amidino-phenyl)-4-[4-(2-methoxycarbonyl-ethyl)-phenyl]-5-methyl-3-phenyl-3H-imidazol-2-on-hydrochlorid

(99) 1-(4-Amidino-phenyl)-3,5-dimethyl-4-[4-(2-methoxycarbonyl-ethyl)-phenyl]-3H-imidazol-2-on-hydrochlorid

(100) 1-(4-Amidino-phenyl)-4-[4-(2-methoxycarbonyl-ethyl)-phenyl]-5-methyl-3H-imidazol-2-on-hydrochlorid

(101) 4-(4'-Amidino-4-biphenylyl)-1-(2-methoxycarbonylethyl)-3H-imidazol-2-thion-hydrochlorid

(102) 4-(4'-Amidino-4-biphenylyl)-1-(2-methoxycarbonylethyl)-3-methyl-3H-imidazol-2-thion-hydrochlorid

(103) 4-(4'-Amidino-4-biphenylyl)-1-(2-methoxycarbonylethyl)-3-phenyl-3H-imidazol-2-thion-hydrochlorid

(104) 4-(4'-Amidino-4-biphenylyl)-1-(2-methoxycarbonylethyl)-3H-imidazol-2-on-hydrochlorid
Schmelzpunkt: 278-282°C (Zers.)
$R_f$-Wert: 0,30 (Kieselgel; Methylenchlorid/Methanol = 4:1)

(105) 4-(4'-Amidino-4-biphenylyl)-1-(2-methoxycarbonylethyl)-3-methyl-3H-imidazol-2-on-hydrochlorid
Schmelzpunkt: 258-260°C (Zers.)
$R_f$-Wert: 0,56 (Kieselgel; Methylenchlorid/Ethanol = 4:1)

(106) 4-(4'-Amidino-4-biphenylyl)-1-(2-methoxycarbonylethyl)-3-phenyl-3H-imidazol-2-on-hydrochlorid
Schmelzpunkt: 250-253°C
$R_f$-Wert: 0,55 (Kieselgel; Methylenchlorid/Ethanol = 4:1)

(107) 1-(4-Amidino-phenyl)-3-[4-(3-methoxycarbonyl-propyl)-phenyl]-imidazolidin-2-on-hydrochlorid

(108) 1-(4-Amidino-phenyl)-3-[1-(2-methoxycarbonyl-ethyl)-4-piperidinyl]-imidazolidin-2-on-hydrochlorid

(109) 2-(4-Amidino-4'-biphenylyl)-4-(2-methoxycarbonylethyl)-4H-1,2,4-triazol-3-on-hydrochlorid

(110) 2-(4-Amidino-4'-biphenylyl)-4-(2-methoxycarbonylethyl)-5-methyl-4H-1,2,4-triazol-3-on-hydrochlorid

(111) 4-(4-Amidino-4'-biphenylyl)-2-(2-methoxycarbonyl ethyl)-4H-1,2,4-triazol-3-on-hydrochlorid

(112) 4-(4-Amidino-4'-biphenylyl)-2-(2-methoxycarbonylethyl)-5-methyl-4H-1,2,4-triazol-3-on-hydrochlorid

(113) 1-[4-(2-Methoxycarbonyl-ethyl)-phenyl]-3-(4-methylamidino-phenyl)-imidazolidin-2-on
Der Iminoester wird in absolutem Methanol aufgenommen und mit einem 20-fachen Überschuß einer methanolischen
Methylaminlösung umgesetzt

(114) 1-(4-n-Butylamidino-phenyl)-3-[4-(2-methoxycarbonylethyl)-phenyl]-imidazolidin-2-on
Herstellung analog (113) mit n-Butylamin

(115) 1-(4-Amidino-phenyl)-3-[4-(2-methoxycarbonyl-ethyl)-phenyl]-4-methyl-3H-imidazol-2-on-hydrochlorid
Schmelzpunkt: 248°C (Zers.)
$R_f$-Wert: 0,40 (Reversed Phase Platte RP8; Methanol/5%ige Natriumchloridlösung = 6:4)

(116) 3-(4-Amidino-phenyl)-1-(4-methoxycarbonyl-butyl)-3H-imidazo[4,5-b]pyridin-2-on-dihydrochlorid
$R_f$-Wert: 0,34 (Kieselgel; Methylenchlorid/Methanol = 19:1)

(117) 4-(4-Amidino-phenyl)-2-[4-(2-methoxycarbonyl-ethyl)-phenyl]-5-methyl-4H-1,2,4-triazol-3-on-hydrochlorid
Schmelzpunkt: 273-275°C
$R_f$-Wert: 0,55 (Kieselgel; Methylenchlorid/Methanol = 4:1)

(118) 2-(4-Amidino-phenyl)-4-[4-(2-methoxycarbonyl-ethyl)-phenyl]-5-methyl-4H-1,2,4-triazol-3-on-hydrochlorid
Schmelzpunkt: 272-274°C
$R_f$-Wert: 0,37 (Reversed Phase Platte RP8; Methanol/5%ige Natriumchloridlösung = 6:4)

(119) 2-(4-Amidino-phenyl)-5-ethyl-4-[4-(2-methoxycarbonylethyl)-phenyl]-4H-1,2,4-triazol-3-on-hydrochlorid
Schmelzpunkt: über 250°C
$R_f$-Wert: 0,36 (Reversed Phase Platte RP8; Methanol/10%ige Natriumchloridlösung = 6:4)

| | | | | | | | | |
|---|---|---|---|---|---|---|---|---|
| Ber. x HCl: | C | 58,67 | H | 5,63 | N | 16,29 | Cl | 8,25 |
| Gef.: | | 58,01 | | 5,65 | | 16,26 | | 9,14 |

(120) 3-(4'-Amidino-4-biphenylyl)-1-(2-methoxycarbonylethyl)-3H-imidazo[4,5-b]pyridin-2-on-hydrochlorid
$R_f$-Wert: 0,61 (Kieselgel; Methylenchlorid/Methanol = 8:2)

(121) 1-(4-Amidino-phenyl)-3-[4-(2-ethoxycarbonyl-ethyl)-phenyl]-3H-imidazol-2-on-hydrochlorid
Hergestellt aus 1-(4-Cyan-phenyl)-3-[4-(2-methoxycarbonyl-ethyl)-phenyl]-3H-imidazol-2-on, wobei der intermediär gebildete Iminoester durch Umsetzung mit ethanolischer Salzsäure erhalten wird.
$R_f$-Wert: 0,54 (Reversed Phase Platte RP8; Methanol/5%ige Natriumchloridlösung = 6:4)

(122) 3-(4-Amidino-phenyl)-1-[4-(2-methoxycarbopyl-ethyl)-phenyl]-4-methyl-3H-imidazol-2-on-hydrochlorid-hydrat
Schmelzpunkt: 95-100°C
$R_f$-Wert: 0,57 (Kieselgel; Methylenchlorid/Methanol = 4:1)

(123) 2-(4-Amidino-phenyl)-4-[4-(2-methoxycarbonyl-ethyl)-phenyl]-4H-1,2,4-triazol-3-on-hydrochlorid
Schmelzpunkt: 275-277°C
$R_f$-Wert: 0,55 (Reversed Phase Platte RP8; Methanol/5%ige Natriumchloridlösung = 6:4)

(124) 4-(Amidino-phenyl)-2-[4-(2-methoxycarbonyl-ethyl)-phenyl]-4H-1,2,4-triazol-3-on-hydrochlorid
Schmelzpunkt: 289-291°C (Zers.)
$R_f$-Wert: 0,49 (Reversed Phase Platte RP8; Methanol/5%ige Natriumchloridlösung = 6:4)

(125) 1-[1-(4-Amidino-phenyl)-4-piperidinyl]-3-(2-methoxycarbonyl-ethyl)-3H-imidazol-2-on-hydrochlorid
Schmelzpunkt: über 275°C
$R_f$-Wert: 0,53 (Reversed Phase Platte RP8; Methanol/10%ige Natriumchloridlösung = 6:4)

(126) 1-(4-Amidino-phenyl)-3-[4-(2-methoxycarbonyl-ethenyl)-phenyl]-3H-imidazol-2-on-hydrochlorid
Schmelzpunkt: 253-264°C
$R_f$-Wert: 0,28 (Reversed Phase Platte RP8; Methanol/10%ige Natriumchloridlösung = 6:4)

(127) 1-(4-Amidino-phenyl)-3-[4-(2-dibenzylamino-2-methoxycarbonyl-ethyl)-phenyl]-3H-imidazol-2-on-hydrochlorid
$R_f$-Wert: 0,37 (Kieselgel; Methylenchlorid/Methanol/konz. Ammoniak = 4:1:0,25)

(128) 1-(4-Amidino-phenyl)-3-[4-(2-amino-2-methoxycarbonylethyl)-phenyl]-3H-imidazol-2-on-dihydrochlorid
$R_f$-Wert: 0,18 (Kieselgel; Methylenchlorid/Methanol/konz. Ammoniak = 16:4:1)

(129) 1-(4-Amidino-phenyl)-3-[4-(2-dimethylamino-2-methoxycarbonyl-ethyl)-phenyl]-3H-imidazol-2-on

(130) 1-(4-Amidino-phenyl)-3-[4-(2-methoxycarbonyl-1-methoxycarbonylmethyl-ethyl)-phenyl]-imidazolidin-2-on

(131) 1-(4-Amidino-phenyl)-3-(3,4-bis-methoxycarbonylmethyloxy-phenyl)-imidazolidin-2-on

(132) 1-(4-Amidino-phenyl)-3-[4-(2-methoxycarbonyl-ethyl)-phenyl]-4-methyl-imidazolidin-2-on

(133) 1-(4-Amidino-phenyl)-3-[4-(2-methoxycarbonyl-2-methylpropyl)-phenyl]-imidazolidin-2-on

(134) 1-(4-Amidino-phenyl)-3-[4-(2-phosphono-ethyl)-phenyl]-imidazolidin-2-on

(135) 1-(4-Amidino-phenyl)-3-[4-[2-(O-methyl-phosphono)-ethyl]-phenyl]-imidazolidin-2-on

(136) 1-(4-Amidino-phenyl)-3-[4-[2-(5-tetrazolyl)-ethyl]-phenyl]-imidazolidin-2-on
Schmelzpunkt:

(137) 1-(4-Amidino-phenyl)-3-[4-(2-hydroxy-2-methoxycarbonylethyl)-phenyl]-3H-imidazol-2-on

(138) 1-(4-Amidino-phenyl)-3-[4-(2-methoxy-2-methoxycarbonylethyl)-phenyl]-3H-imidazol-2-on

(139) 2-(4-Amidino-phenyl)-4-[4-(2-methoxycarbonyl-2-methylpropyl)-phenyl]-5-methyl-4H-1,2,4-triazol-3-on

(140) 2-(4-Amidino-phenyl)-4-[4-(2-phosphono-ethyl)-phenyl]-5-methyl-4H-1,2,4-triazol-3-on

(141) 2-(4-Amidino-phenyl)-4-[4-[2-(0-methyl-phosphono)-ethyl]-phenyl]-5-methyl-4H-1,2,4-triazol-3-on

(142) 2-(4-Amidino-phenyl)-4-[4-(2-methoxycarbonyl-ethyl)-phenyl]-5-trifluormethyl-4H-1,2,4-triazol-3-on

(143) 2-(4-Amidino-phenyl)-4-[4-(2-methoxycarbonyl-ethyl)-phenyl]-5-phenyl-4H-1,2,4-triazol-3-on

(144) 2-(4-Amidino-phenyl)-4-[4-[2-(5-tetrazolyl)-ethyl]-phenyl]-5-methyl-4H-1,2,4-triazol-3-on

Beispiel 3

1-(4'-Amidino-3-methylsulfinyl-4-biphenylyl)-3-(2-methoxycarbonyl-ethyl)-imidazolidin-2-on

Herstellung aus 1-(4'-Amidino-3-methylthio-4-biphenylyl)-3-(2-methoxycarbonyl-ethyl)-imidazolidin-2-on durch Oxidation mit Brom in Eisessig in Gegenwart von Natriumacetat bei Raumtemperatur.

Analog werden folgende Verbindungen erhalten:

(1) 1-(4-Amidino-2-methylsulfinyl-phenyl)-3-[4-(2-methoxycarbonyl-ethyl)-phenyl]-imidazolidin-2-on

(2) 1-(4-Amidino-phenyl)-3-[4-(2-methoxycarbonyl-ethyl)-2-methylsulfinyl-phenyl]-imidazolidin-2-on

(3) 1-(4-Amidino-phenyl)-3-(2-methoxycarbonylmethylsulfinylethyl)-imidazolidin-2-on

Beispiel 4

1-(4-Cyano-phenyl)-3-[2-(4-methoxycarbonyl-phenyl)-ethyl]-imidazolidin-2-on

8,1 g 1-(4-Cyano-phenyl)-3-[2-(4-methoxycarbonyl-phenyl)-ethyl]-3H-imidazol-2-on werden in 750 ml Essigester gelöst und in Gegenwart von 2 g 10%iger Palladiumkohle 2,5 Stunden bei 50°C mit Wasserstoff von 5 bar behandelt. Man filtriert vom Katalysator ab, engt auf etwa 100 ml ein, fügt 100 ml tert.Butyl-methylether zu, kühlt im Eis-Acetonbad ab und wäscht den gebildeten Niederschlag nach dem Abfiltrieren mit tert.Butyl-methylether.
Ausbeute: 6,4 g (79 % der Theorie),
Schmelzpunkt: 194-197°C
$R_f$-Wert: 0,63 (Kieselgel; Methylenchlorid/Essigester/Cyclohexan = 3:1:1)

Analog werden folgende Verbindungen erhalten:

(1) 4-(4-Amidino-phenyl)-1-[4-(2-methoxycarbonyl-ethyl)-phenyl]-imidazolidin-2-on

(2) 4-(4-Amidino-phenyl)-1-[4-(2-methoxycarbonyl-ethyl)-phenyl]-3-methyl-imidazolidin-2-on

(3) 4-(4-Amidino-phenyl)-1-[4-(2-methoxycarbonyl-ethyl)-phenyl]-3-phenyl-imidazolidin-2-on

(4) 1-(4-Amidino-phenyl)-4-[4-(2-methoxycarbonyl-ethyl)-phenyl]-imidazolidin-2-on

(5) 1-(4-Amidino-phenyl)-4-[4-(2-methoxycarbonyl-ethyl)-phenyl]-3-methyl-imidazolidin-2-on

(6) 1-(4-Amidino-phenyl)-4-[4-(2-methoxycarbonyl-ethyl)-phenyl]-3-phenyl-imidazolidin-2-on

(7) 4-(4'-Amidino-4-biphenylyl)-1-(2-methoxycarbonyl-ethyl)-imidazolidin-2-on

(8) 4-(4'-Amidino-4-biphenylyl)-1-(2-methoxycarbonyl-ethyl)-3-methyl-imidazolidin-2-on

(9) 4-(4'-Amidino-4-biphenylyl)-1-(2-methoxycarbonyl-ethyl)-3-phenyl-imidazolidin-2-on-hydrochlorid
Man geht vom Hydrochlorid der freien Säure aus und reduziert in Methanol bei Raumtemperatur.
Schmelzpunkt: 225-235 (Zers.)

(10) 1-[4-(4-Amidino-phenyl)-cyclohexyl]-3-(2-methoxycarbonyl-ethyl)-imidazolidin-2-on

(11) 1-[1-(4-Amidino-phenyl)-4-piperidinyl]-3-(2-methoxycarbonyl-ethyl)-imidazolidin-2-on-hydrochlorid
Man verwendet verdünnte methanolische Salzsäure und arbeitet bei Raumtemperatur.
$R_f$-Wert: 0,47 (Reversed Phase Platte; Methanol/10%ige Natriumchloridlösung = 6:4)

(12) 1-[4-Cyano-phenyl)-3-[4-(2-methoxycarbonyl-ethyl)-phenyl]-imidazolidin-2-on-hydrochlorid
Schmelzpunkt: 160-162°C
$R_f$-Wert: 0,59 (Kieselgel; Cyclohexan/Essigester = 3:7)

(13) 4-[4'-Amidino-4-biphenylyl)-1-(2-carboxy-ethyl)-3-phenyl-imidazolidin-2-on-hydrochlorid
Man arbeitet in Ethanol bei Raumtemperatur.
Schmelzpunkt: 236-240°C

(14) 4-[4'-Amidino-4-biphenylyl)-1-(2-carboxy-ethyl)-3-methyl-imidazolidin-2-on-hydrochlorid
Man arbeitet in Ethanol bei Raumtemperatur.
$R_f$-Wert: 0,55 (Reversed Phase Platte RP18; Methanol/5%ige Natriumchloridlösung = 6:4)

(15) 4-[4'-Amidino-4-biphenylyl)-1-(2-carboxy-ethyl)-imidazolidin-2-on-hydrochlorid
Man arbeitet in Ethanol bei Raumtemperatur.
$R_f$-Wert: 0,61 (Reversed Phase Platte RP18; Methanol/5%ige Natriumchloridlösung = 6:4)

(16) 4-(4-Amidino-phenyl)-1-[4-(2-carboxy-ethyl)-phenyl]-3-methyl-imidazolidin-2-on-hydrochlorid
Man arbeitet in Dioxan/Wasser = 1:1).
Schmelzpunkt: 208-210°C

(17) 1-(4-Amidino-phenyl)-3-[4-(2-amino-2-methoxycarbonyl-ethyl)-phenyl]-imidazolidin-2-on-dihydrochlorid
Man arbeitet in Methanol bei Raumtemperatur.
$R_f$-Wert: 0,37 (Kieselgel; Methylenchlorid/Methanol/konz. Ammoniak = 8:4:1)

(18) 1-(4-Amidino-phenyl)-3-[4-(2-amino-2-carboxy-ethyl)-phenyl]-imidazolidin-2-on
Schmelzpunkt: 295°C (Zers.)
$R_f$-Wert: 0,07 (Kieselgel; Methylenchlorid/Methanol/konz. Ammoniak = 8:4:1)

Beispiel 5

1-(4-Ethoxycarbonylamidino-phenyl)-3-[4-(2-ethoxycarbonyl-ethyl)-phenyl]-3H-imidazol-2-on

Zu einer Mischung aus 0,3 g 1-(4-Amidino-phenyl)-3-[4-(2-ethoxycarbonyl-ethyl)-phenyl]-3H-imidazol-2-on-hydrochlorid, 0,07 ml Chlorameisensäure-ethylester und 40 ml Methylenchlorid werden bei Raumtemperatur 10 ml 0,2N Natronlauge unter kräftigem Rühren zugetropft. Nach 0,5-stündigem Rühren bei Raumtemperatur wird die Methylenchloridphase abgetrennt und zur Trockne eingedampft.

Ausbeute: 0,29 g (90 % der Theorie),
$R_f$-Wert: 0,48 (Kieselgst; Methylenchlorid/Methanol = 15:1)

| | | | | | | |
|---|---|---|---|---|---|---|
| Ber.: | C | 63,99 | H | 5,82 | N | 12,44 |
| Gef.: | | 64,11 | | 5,98 | | 12,35 |

Analog werden folgende Verbindungen erhalten:

(1) 1-(4-Methoxycarbonylamidino-phenyl)-3-[4-(2-methoxycarbonyl-ethyl)-phenyl]-imidazolidin-2-on
Schmelzpunkt: über 260°C
$R_f$-Wert: 0,73 (Kieselgel; Methylenchlorid/Methanol = 95:5)

(2) 1-(4-Benzyloxycarbonylamidino-phenyl)-3-[4-(2-methoxycarbonyl-ethyl)-phenyl]-imidazolidin-2-on

(3) 1-(4-Isopropyloxycarbonylamidino-phenyl)-3-[4-(2-methoxycarbonyl-ethyl)-phenyl]-imidazolidin-2-on

(4) 1-(4-Isobutyloxycarbonylamidino-phenyl)-3-[4-(2-methoxycarbonyl-ethyl)-phenyl]-imidazolidin-2-on

(5) 1-(4-Ethoxycarbonylamidino-phenyl)-3-[4-(2-methoxycarbonyl-ethyl)-phenyl]-imidazolidin-2-on

(6) 4-(4-Methoxycarbonylamidino-phenyl)-2-[4-(2-methoxycarbonyl-ethyl)-phenyl]-4H-1,2,4-triazol-3-on
Schmelzpunkt: 296-298°C
$R_f$-Wert: 0,46 (Kieselgel; Methylenchlorid/Methanol = 15:1)

(7) 4-[4-(2-Isopropyloxycarbonyl-ethyl)-phenyl]-2-(4-methoxycarbonylamidino-phenyl)-5-methyl-4H-1,2,4-triazol-3-on
Schmelzpunkt: 175-188°C (Zers.)
$R_f$-Wert: 0,28 (Kieselgel; Methylenchlorid/Methanol = 95:5)

(8) 1-(4-Methoxycarbonylamidino-phenyl)-3-[4-(2-methoxycarbonyl-ethyl)-phenyl]-3H-imidazol-2-on
Schmelzpunkt: über 260°C
$R_f$-Wert: 0,27 (Kieselgel; Methylenchlorid/Methanol = 95:5)

| | | | | | | |
|---|---|---|---|---|---|---|
| Ber.: | C | 62,55 | H | 5,25 | N | 13,26 |
| Gef.: | | 62,24 | | 5,33 | | 13,45 |

(9) 1-(4-Ethoxycarbonylamidino-phenyl)-3-[4-(2-ethoxycarbonyl-ethyl)-phenyl]-imidazolidin-2-on
Schmelzpunkt: über 335°C
$R_f$-Wert: 0,49 (Kieselgel; Methylenchlorid/Methanol = 15:1)

(10) 2-(4-Methoxycarbonylamidino-phenyl)-3-[4-(2-methoxycarbonyl-ethyl)-phenyl]-5-methyl-4H-1,2,4-triazol-3-on
Schmelzpunkt: 211-213°C (Zers.)
$R_f$-Wert: 0,54 (Kieselgel; Methylenchlorid/Methanol = 15:1)

(11) 2-(4-Methoxycarbonylamidino-phenyl)-3-[4-(2-methoxycarbonyl-ethyl)-phenyl]-4H-1,2,4-triazol-3-on
Schmelzpunkt: über 340°C
$R_f$-Wert: 0,45 (Kieselgel; Methylenchlorid/Methanol = 15:1)

(12) 1-(4-Methoxycarbonylamidino-phenyl)-3-[4-(2-methoxycarbonyl-ethyl)-cyclohexyl]-imidazolidin-2-on
$R_f$-Wert: 0,29 (Reversed Phase Platte RP8; Methanol/5%ige Natriumchloridlösung = 6:4)

(13) 1-(4-Methoxycarbonylamidino-phenyl)-3-[4-(2-methoxycarbonyl-ethyl)-phenyl]-imidazolidin-2,4-dion
Schmelzpunkt: 250°C (Zers., sintert ab 198°C)
$R_f$-Wert: 0,45 (Kieselgel; Besigester)

| | | | | | | |
|---|---|---|---|---|---|---|
| Ber.: | C | 60,27 | H | 5,06 | N | 12,78 |
| Gef.: | | 60,18 | | 5,12 | | 12,82 |

(14) 1-(4-Methoxycarbonylamidino-phenyl)-3-[4-(2-methoxycarbonyl-ethyl)-phenyl]-4-methyl-3H-imidazol-2-on
Schmelzpunkt: 212-213°C
$R_f$-Wert: 0,58 (Kieselgel; Methylenchlorid/Methanol = 9:1)

(15) 1-(4-Ethoxycarbonylamidino-phenyl)-3-[4-(2-methoxycarbonyl-ethyl)-phenyl]-4-methyl-3H-imidazol-2-on
Schmelzpunkt: 198-199°C
$R_f$-Wert: 0,58 (Kieselgel; Methylenchlorid/Methanol = 9:1)

(16) 2-(4-Methoxycarbonylamidino-phenyl)-5-[4-(2-methoxycarbonyl-ethyl)-phenyl]-3,4-dihydro-2H,5H-1,2,5-thiadiazol-1,1-dioxid
Schmelzpunkt: über 275°C
$R_f$-Wert: 0,23 (Kieselgel; Methylenchlorid/Methanol = 100:3)

(17) 2-(4-Ethoxycarbonylamidino-phenyl)-5-[4-(2-methoxycarbonyl-ethyl)-phenyl]-3,4-dihydro-2H,5H-1,2,5-thiadiazol-1,1-dioxid
Schmelzpunkt: über 275°C
$R_f$-Wert: 0,22 (Kieselgel; Methylenchlorid/Methanol = 100:3)

(18) 4-[4-(2-Isobutyloxycarbonyl-ethyl)-phenyl]-2-(4-methoxycarbonylamidino-phenyl)-5-methyl-4H-1,2,4-triazol-3-on
Schmelzpunkt: 200-201°C
$R_f$-Wert: 0,43 (Kieselgel; Methylenchlorid/Methanol = 95:5)

| | | | | | | |
|---|---|---|---|---|---|---|
| Ber.: | C | 62,62 | H | 6,09 | N | 14,60 |
| Gef.: | | 62,77 | | 6,20 | | 14,88 |

Beispiel 6

1-(4-Amidino-phenyl)-3-[4-(2-butyloxycarbonyl-ethyl)-phenyl]-imidazolidin-2-on-hydrochlorid

Herstellung aus 1-(4-Amidino-phenyl)-3-[4-(2-methoxycarbonylethyl)-phenyl]-imidazolidin-2-on-hydrochlorid durch dreitägiges Rühren bei Raumtemperatur mit gesättigter butanolischer Salzsäure.

Analog wird folgende Verbindung erhalten:
(1) 1-(4-Amidino-phenyl)-3-[4-[2-(2-phenyl-ethyloxycarbonyl)-ethyl]-phenyl]-imidazolidin-2-on-hydrochlorid

Beispiel 7

1-(1-Amidino-4-piperidinyl)-3-[4-(2-methoxycarbonyl-ethyl)-phenyl]-imidazolidin-2-on

Herstellung aus 1-[4-(2-Methoxycarbonyl-ethyl)-phenyl]-3-(4-piperidinyl)-imidazolidin-2-on und S-Ethylisothioharnstoff-hydrobromid durch vierstündiges Erwärmen auf 100°C in Dimethylformamid in Gegenwart von Natriumcarbonat.

Beispiel 8

1-(4-Aminomethyl-phenyl)-3-(4-methoxycarbonylmethyloxy-phenyl)-imidazolidin-2-on-hydrochlorid

2 g 1-(4-Cyano-phenyl)-3-(4-methoxycarbonylmethyloxy-phenyl)-imidazolidin-2-on werden in einer Mischung aus 40 ml Methanol und 4 ml methanolischer Salzsäure in Gegenwart von 0,5 g 10%-iger Palladiumkohle mit Wasserstoff von 5 bar 2,5 Stunden bei Raumtemperatur behandelt. Man versetzt mit 200 ml Methanol und 50 ml Wasser und filtriert heiß. Das Produkt kristallisiert beim Abkühlen aus.
Ausbeute: 1,38 g (62 % der Theorie),

Schmelzpunkt: über 250°C
$R_f$-Wert: 0,40 (Reversed Phase Platte RP8; Methanol/5%ige Natriumchloridlösung = 6:4)

| | | | | | | | | |
|---|---|---|---|---|---|---|---|---|
| Ber. x HCl: | C | 58,24 | H | 5,66 | N | 10,72 | Cl | 9,05 |
| Gef.: | | 58,04 | | 5,65 | | 10,92 | | 9,57 |

Analog werden folgende Verbindungen erhalten:

(1) 1-(4-Aminomethyl-phenyl)-3-[4-(2-methoxycarbonyl-ethyl)-phenyl]-3,4,5,6-tetrahydro-1H-pyrimidin-2-on-hydrochlorid Schmelzpunkt: 272-274°C (Zers.)
$R_f$-Wert: 0,30 (Kieselgel; Toluol/Dioxan/Methanol/konz. Ammoniak = 2:5:2:1)

(2) 1-(4-Aminomethyl-phenyl)-3-[4-(2-methoxycarbonyl-ethyl)-phenyl]-imidazolidin-2-on-hydrochlorid
Schmelzpunkt: über 250°C
$R_f$-Wert: 0,47 (Kieselgel; Toluol/Dioxan/Methanol/konz. Ammoniak = 4:10:4:1)

| | | | | | | | | |
|---|---|---|---|---|---|---|---|---|
| Ber. x HCl: | C | 61,61 | H | 6,20 | N | 10,78 | Cl | 9,09 |
| Gef.: | | 61,30 | | 6,29 | | 10,88 | | 9,12 |

(3) 1-[4-(2-Amino-2-methoxycarbonyl-ethyl)-phenyl]-3-(4-aminomethyl-phenyl)-imidazolidin-2-on-dihydrochlorid
$R_f$-Wert: 0,66 (Kieselgel; Methylenchlorid/Methanol/konz. Ammoniak = 16:4:1)

(4) 1-[4-(2-Amino-ethyl)-phenyl]-3-(4-methoxycarbonylmethylphenyl)-imidazolidin-2-on
Man arbeitet bei 40°C
Schmelzpunkt: über 250°C
$R_f$-Wert: 0,31 (Kieselgel; Toluol/Dioxan/Methanol/konz. Ammoniak = 4:10:4:1)

(5) 1-(4-Aminomethyl-2-methyl-phenyl)-3-[4-(2-methoxycarbonyl-ethyl)-phenyl]-imidazolidin-2-on
Schmelzpunkt: über 275°C
$R_f$-Wert: 0,38 (Reversed Phase Platte RP8; Methanol/5%ige Natriumchloridlösung = 6:4)

(6) 3-(4-Aminomethyl-phenyl)-1-[4-(2-methoxycarbonyl-ethyl)-phenyl]-4-methyl-3H-imidazol-2-on

(7) 1-(4'-Aminomethyl-4-biphenylyl)-3-(2-methoxycarbonyl-ethyl)-imidazolidin-2-on

(8) 1-(4-Aminomethyl-3-fluor-phenyl)-3-[4-(2-methoxycarbonyl-ethyl)-phenyl]-imidazolidin-2-on

(9) 1-(4-Aminomethyl-phenyl)-3-[3-(2-methoxycarbonyl-ethyl)-phenyl]-imidazolidin-2-on

(10) 1-(4-Aminomethyl-phenyl)-3-[4-(2-methoxycarbonyl-1-methoxycarbonylmethyl-ethyl)-phenyl]-imidazolidin-2-on

(11) 4-(4-Aminomethyl-phenyl)-2-[4-(2-methoxycarbonyl-ethyl)-phenyl]-4H-1,2,4-triazol-3-on

(12) 1-[4-(2-Amino-ethyl)-phenyl]-3-[4-(2-methoxycarbonyl-ethyl)-phenyl]-imidazolidin-2-on
Schmelzpunkt: über 200°C
$R_f$-Wert: 0,63 (Reversed Phase Platte RP8; Eisessig/Wasser = 1:1)

(13) 1-[4-(1-Amino-2-methyl-2-propyl)-phenyl]-3-[4-(2-methoxycarbonyl-ethyl)-phenyl]-imidazolidin-2-on

(14) 2-(4-Aminomethyl-phenyl)-4-[4-(2-methoxycarbonyl-ethyl)-phenyl]-5-methyl-4H-1,2,4-triazol-3-on

(15) 1-[4-(2-Amino-ethyl)-phenyl]-3-(4-methoxycarbonylmethyloxy-phenyl)-imidazolidin-2-on

(16) 1-[3-(2-Amino-ethyl)-phenyl]-3-[4-(2-methoxycarbonyl-ethyl)-phenyl)-imidazolidin-2-on-hydrochlorid
Man geht aus von 1-(3-Cyanomethyl-phenyl)-3-[4-(2-methoxycarbonyl-ethyl)-phenyl]-3H-imidazol-2-on und reduziert in einem Gemisch aus 50 ml Dioxan, 10 ml Methanol und 1 ml methanolischer Salzsäure.

Schmelzpunkt: über 260°C
$R_f$-Wert: 0,29 (Reversed Phase Platte RP8; Methanol/5%ige Natriumchloridlösung = 6:4)

(17) 1-(4-Aminomethyl-cyclohexyl)-3-[4-(2-methoxycarbonyl-ethyl)-phenyl]-imidazolidin-2-on

Beispiel 9

1-[2-(4-Amidino-phenyl)-2-hydroxy-ethyl]-3-(3-methoxycarbonyl-propyl)-imidazolidin-2-on

Herstellung durch Reduktion von 1-[(4-Amidino-phenyl)-carbonylmethyl]-3-(3-methoxycarbonyl-propyl)-imidazolidin-2-on-hydrochlorid mit Natriumborhydrid in Methanol bei 0-5°C.

Beispiel 10

1-(3-Guanidino-phenyl)-3-[4-(2-methoxycarbonyl-ethyl)-phenyl]-imidazolidin-2-on-hydrochlorid

Herstellung aus 1-(3-Amino-phenyl)-3-[4-(2-methoxycarbonylethyl)-phenyl]-imidazolidin-2-on-hydrochlorid durch dreistündiges Rückflußkochen mit Cyanamid in Dioxan.

Beispiel 11

1-(4'-Cyano-4-biphenylyl)-3-(2-methoxycarbonyl-ethyl)-imidazolidin-2-on

2 g 1-(2-Carboxy-ethyl)-3-(4'-cyano-4-biphenylyl)-imidazolidin-2-on werden in 150 ml Methanol suspendiert, mit 6 ml konzentrierter methanolischer Salzsäure versetzt und 16 Stunden bei Raumtemperatur gerührt. Der Niederschlag wird abfiltriert und durch Säulenchromatographie an Kieselgel (Elutionsmittel: Methylenchlorid/Essigester = 9:1) gereinigt.
Ausbeute: 0,5 g (23 % der Theorie),
Schmelzpunkt: 150-155°C
$R_f$-Wert: 0,50 (Kieselgel; Methylenchlorid/Essigester = 9:1)
Analog werden folgende Verbindungen erhalten:

(1) 1-(4'-Cyano-3'-fluor-4-biphenylyl)-3-(2-methoxycarbonyl-ethyl)-imidazolidin-2-on

(2) 1-(3'-Chlor-4'-cyano-4-biphenylyl)-3-(2-methoxycarbonyl-ethyl)-imidazolidin-2-on

(3) 1-(4'-Cyano-3-methoxy-4-biphenylyl)-3-(2-methoxycarbonyl-ethyl)-imidazolidin-2-on

(4) 1-(4'-Cyano-3-methylthio-4-biphenylyl)-3-(2-methoxycarbonyl-ethyl)-imidazolidin-2-on

(5) 1-(4'-Cyano-2,3-dimethyl-4-biphenylyl)-3-(2-methoxycarbonyl-ethyl)-imidazolidin-2-on

(6) 1-[4-(5-Cyano-2-pyridyl)-phenyl]-3-(2-methoxycarbonyl-ethyl)-imidazolidin-2-on

(7) 1-[4-(5-Cyano-2-pyrazinyl)-phenyl]-3-(2-methoxycarbonyl ethyl)-imidazolidin-2-on

(8) 1-[4-(5-Cyano-2-pyrimidinyl)-phenyl]-3-(2-methoxycarbonyl-ethyl)-imidazolidin-2-on

(9) 1-[6-(4-Cyano-phenyl)-3-pyridazinyl]-3-(2-methoxycarbonyl-ethyl)-imidazolidin-2-on

(10) 1-[2-(4-Cyano-phenyl)-5-pyrimidinyl]-3-(2-methoxycarbonyl-ethyl)-imidazolidin-2-on

(11) 2-(4-Amidino-phenyl)-4-[4-(2-isopropyloxycarbonyl ethyl)-phenyl]-5-methyl-4H-1,2,4-triazol-3-on-hydrochlorid Man verwendet isopropanolische Salzsäure
Schmelzpunkt: 255-257°C
$R_f$-Wert: 0,33 (Reversed Phase Platte RP8; Methanol/5%ige Natriumchloridlösung = 6:4)

(12) 2-(4-Amidino-phenyl)-4-[4-(2-isobutyloxycarbonyl-ethyl)-phenyl]-5-methyl-4H-1,2,4-triazol-3-on-hydrochlorid

Schmelzpunkt: über 250°C
$R_f$-Wert: 0,22 (Reversed Phase Platte RP8; Methanol/5%ige Natriumchloridlösung = 6:4)

| | | | | | | | | |
|---|---|---|---|---|---|---|---|---|
| Ber. x HCl: | C | 60,32 | H | 6,16 | N | 15,29 | Cl | 7,74 |
| Gef.: | | 60,19 | H | 6,28 | N | 15,37 | Cl | 7,78 |

(13) 2-(4-Amidino-phenyl)-5-ethyl-4-[4-(2-isopropyloxycarbonyl-ethyl)-phenyl]-4H-1,2,4-triazol-3-on
Schmelzpunkt: über 250°C
$R_f$-Wert: 0,27 (Reversed Phase Platte, RP8; Methanol/5%ige Natriumchloridlösung = 6:4)

(14) 1-(4-Amidino-phenyl)-3-[4-(2-isopropyloxycarbonyl-ethyl)-phenyl]-4-methyl-3H-imidazol-2-on
Schmelzpunkt: 246-249°C
$R_f$-Wert: 0,34 (Reversed Phase Platte, RP8; Methanol/5%ige Natriumchloridlösung = 6:4)

(15) 4-(4-Amidino-phenyl)-2-[4-(2-isopropyloxycarbonyl-ethyl)-phenyl]-4H-l,2,4-triazol-3-on

(16) 1-(4-Amidino-phenyl)-3-[4-(2-isopropyloxycarbonyl-ethyl)-phenyl]-imidazolidin-2-on

(17) 1-(4-Amidino-phenyl)-3-[4-(2-isopropyloxycarbonyl-ethyl)-phenyl]-3H-imidazol-2-on
Schmelzpunkt: über 250°C
$R_f$-Wert: 0,28 (Reversed Phase Platte, RP8; Methanol/5%ige Natriumchloridlösung = 6:4)

(18) 1-(4-Amidino-phenyl)-3-[4-(2-isopropyloxycarbonyl-ethyl)-phenyl]-imidazolidin-2,4-dion
Schmelzpunkt: über 250°C
$R_f$-Wert: 0,37 (Reversed Phase Platte, RP8; Methanol/5%ige Natriumchloridlösung = 6:4)

Beispiel 12

1-(2-Carboxy-ethyl)-3-(4'-cyano-4-biphenylyl)-imidazolidin-2-on

3,4 g 1-(3-Buten-1-yl)-3-(4'-cyano-4-biphenylyl)-imidazolidin-2-on werden in einer Mischung aus 20 ml Methylenchlorid und 20 ml Acetonitril gelöst und 25 mg Rutheniumtrichloridtrihydrat zugesetzt. Man fügt eine Mischung aus 16 g Natriummetaperjodat und 65 ml Wasser hinzu und rührt 2,5 Stunden. Danach versetzt man mit 100 ml Methylenchlorid und 20 ml Wasser, trennt die Phasen und wäscht die wäßrige Phase mehrmals mit Methylenchlorid. Die vereinigten organischen Phasen werden eingeengt und der pulvrige Rückstand ohne weitere Reinigung weiterverwendet.
Ausbeute: 2 g (56 % der Theorie),
$R_f$-Wert: 0,12 (Kieselgel; Cyclohexan/Essigester = 1:1)
Analog werden folgende Verbindungen erhalten:

(1) 1-(2-Carboxy-ethyl)-3-(4'-cyano-3'-fluor-4-biphenylyl)-imidazolidin-2-on

(2) 1-(2-Carboxy-ethyl)-3-(3'-chlor-4'-cyano-4-biphenylyl)-imidazolidin-2-on

(3) 1-(2-Carboxy-ethyl)-3-(4'-cyano-3-methoxy-4-biphenylyl)-imidazolidin-2-on

(4) 1-(2-Carboxy-ethyl)-3-(4'-cyano-3-methylthio-4-biphenylyl)-imidazolidin-2-on

(5) 1-(2-Carboxy-ethyl)-3-(4'-cyano-2,3-dimethyl-4-biphenylyl)-imidazolidin-2-on

(6) 1-(2-Carboxy-ethyl)-3-[4-(5-cyano-2-pyridyl)-phenyl]-imidazolidin-2-on

(7) 1-(2-Carboxy-ethyl)-3-[4-(5-cyano-2-pyrazinyl)-phenyl]-imidazolidin-2-on

(8) 1-(2-Carboxy-ethyl)-3-[4-(5-cyano-2-pyrimidinyl)-phenyl]-imidazolidin-2-on

(9) 1-(2-Carboxy-ethyl)-3-[6-(4-cyano-phenyl)-3-pyridazinyl]-imidazolidin-2-on

(10) 1-(2-Carboxy-ethyl)-3-[2-(4-cyano-phenyl)-5-pyrimidinyl]-imidazolidin-2-on

Beispiel 13

1-(4'-Cyano-4-biphenylyl)-3-methoxycarbonylmethyl-imidazolidin-2-on

4 g 1-(4'-Cyano-4-biphenylyl)-imidazolidin-2-on werden bei 50°C in 150 ml Dimethylformamid gelöst und portionsweise mit 0,73 g einer 95%igen Suspension von Natriumhydrid in Öl versetzt. Man läßt auf Raumtemperatur abkühlen, tropft anschließend eine Lösung von 1,7 ml Bromessigsäuremethylester in 15 ml Dimethylformamid zu und rührt 64 Stunden bei Raumtemperatur. Das Reaktionsgemisch wird auf 300 ml Wasser gegossen. Der ausgefallene Niederschlag wird säulenchromatographisch (Kieselgel; Methylenchlorid/Essigester = 9:1) gereinigt.
Ausbeute: 3,2 g (63 % der Theorie),
$R_f$-Wert: 0,54 (Kieselgel; Cyclohexan/Essigester = 1:2)
Analog werden folgende Verbindungen erhalten:

(1) 1-(4'-Cyano-4-biphenylyl)-3-methoxycarbonylmethyl-3,4,5,6-tetrahydro-1H-pyrimidin-2-on
Schmelzpunkt: 145-150°C
$R_f$-Wert: 0,28 (Kieselgel; Cyclohexan/Essigester = 1:2)

(2) 1-[2-(4'-Cyano-4-biphenylyl)-ethyl]-3-methoxycarbonyl-methyl-3H-benzimidazol-2-on
Als Base dient Kalium-tert.butylat. Das hierbei verwendete 4-Cyano-4'-(2-jodethyl)-biphenyl erhält man aus 4-(2-Bromethyl)-4'-cyano-biphenyl durch Umsetzen mit Natriumjodid in Aceton bei Raumtemperatur.
$R_f$-Wert: 0,89 (Kieselgel; Methylenchlorid/Methanol = 95:5)

(3) 1-[2-(4'-Cyano-4-biphenylyl)-ethyl]-3-methoxycarbonyl-methyl-imidazolidin-2-on

(4) 2-(4'-Cyano-4-biphenylyl)-5-(2-ethoxycarbonyl-ethyl)-3,4-dihydro-2H,5H-1,2,5-thiadiazol-1,1-dioxid
Man arbeitet in Dimethylformamid mit Kalium-tert.butylat und 1-Brom-2-chlor-ethan als Alkylierungsmittel
Schmelzpunkt: 183-185°C

(5) 1-(4-Cyano-phenyl)-3-(4-ethoxycarbonyl-butyl)-imidazolidin-2-on
$R_f$-Wert: 0,62 (Kieselgel; Cyclohexan/Essigester = 1:3)

(6) 3-(4-Cyano-phenyl)-1-(4-ethoxycarbonyl-butyl)-3H-imidazo-[4,5-b]pyridin-2-on
$R_f$-Wert: 0,78 (Kieselgel; Methylenchlorid/Methanol = 9:1)

Beispiel 14

1-(4'-Cyano-4-biphenylyl)-3-(2-ethoxycarbonyl-ethyl)-3,4,5,6-tetrahydro-1H-pyrimidin-2-on

1,9 g N-(4'-Cyano-4-biphenylyl)-N-(3-methansulfonyloxy-propyl)-N'-(2-ethoxycarbonyl-ethyl)-harnstoff werden in 2 ml Dimethylformamid gelöst, bei Raumtemperatur mit 0,18 g einer 55%igen Suspension von Natriumhydrid in Öl versetzt und 2 Stunden bei Raumtemperatur gerührt. Man versetzt mit 20 ml Wasser und reinigt den ausgefallenen Niederschlag säulenchromatographisch (Kieselgel; Essigester).
Ausbeute: 1,0 g (64 % der Theorie),
Schmelzpunkt: 137-138°C
$R_f$-Wert: 0,57 (Kieselgel; Cyclohexan/Essigester = 1:5)
Analog werden folgende Verbindungen erhalten:

(1) 1-(4-Cyano-phenyl)-3-[4-(2-methoxycarbonyl-ethyl)-phenyl]-imidazolidin-2-on
Schmelzpunkt: 160-162,5°C
$R_f$-Wert: 0,59 (Kieselgel; Cyclohexan/Essigester = 3:7)

(2) 1-(4-Cyano-phenyl)-3-[4-(2-methoxycarbonyl-ethyl)-phenyl]-3,4,5,6-tetrahydro-1H-pyrimidin-2-on
Schmelzpunkt: 149-152°C
$R_f$-Wert: 0,25 (Kieselgel; Cyclohexan/Essigester = 1:1)

(3) 1-(4-Cyano-3-fluor-phenyl)-3-[4-(2-methoxycarbonyl-ethyl)-phenyl]-imidazolidin-2-on

(4) 1-(3-Chlor-4-cyano-phenyl)-3-[4-(2-methoxycarbonyl-ethyl)-phenyl]-imidazolidin-2-on

(5) 1-(4-Cyano-2-methylthio-phenyl)-3-[4-(2-methoxycarbonyl-ethyl)-phenyl]-imidazolidin-2-on

(6) 1-(4-Cyano-2-methyl-phenyl)-3-[4-(2-methoxycarbonyl-ethyl)-phenyl]-imidazolidin-2-on

(7) 1-(4-Cyano-2-methoxy-phenyl)-3-[4-(2-methoxycarbonyl-ethyl)-phenyl]-imidazolidin-2-on

(8) 1-(4-Cyano-phenyl)-3-[2-fluor-4-(2-methoxycarbonyl-ethyl)-phenyl]-imidazolidin-2-on

(9) 1-[2-Chlor-4-(2-methoxycarbonyl-ethyl)-phenyl]-3-(4-cyano-phenyl)-imidazolidin-2-on

(10) 1-(4-Cyano-phenyl)-3-[2-methoxy-4-(2-methoxycarbonyl-ethyl)-phenyl]-imidazolidin-2-on

(11) 1-(4-Cyano-phenyl)-3-[4-(2-methoxycarbonyl-ethyl)-2-methyl-phenyl]-imidazolidin-2-on

(12) 1-(4-Cyano-phenyl)-3-[4-(2-methoxycarbonyl-ethyl)-2-methylthio-phenyl]-imidazolidin-2-on

(13) 1-(4-Cyano-phenyl)-3-[5-(2-methoxycarbonyl-ethyl)-2-pyridyl]-imidazolidin-2-on

(14) 1-(4-Cyano-phenyl)-3-[4-(2-methoxycarbonyl-ethyl)-cyclohexyl]-imidazolidin-2-on

(15) 1-(4-Cyano-phenyl)-3-(4-methoxycarbonylmethyloxy-phenyl)-imidazolidin-2-on

(16) 1-(4-tert.Butyloxycarbonylmethylthio-phenyl)-3-(4-cyanophenyl)-imidazolidin-2-on
Schmelzpunkt: 169-171°C

(17) 1-(4-Cyano-phenyl)-3-[4-(3-methoxycarbonyl-2-methyl-2-propyl)-phenyl]-imidazolidin-2-on
Man setzt Natriumjodid zu und verwendet Kalium-tert.butylat als Base.
Schmelzpunkt: 177-179°C

(18) 1-(4-Cyano-phenyl)-3-[2-(4-ethoxycarbonyl-phenyl)-ethyl]-imidazolidin-2-on

(19) 1-(4-Cyano-phenyl)-3-[4-(2-methoxycarbonyl-ethenyl)-phenyl]-imidazolidin-2-on

(20) 1-(5-Cyano-2-pyridyl)-3-[4-(2-methoxycarbonyl-ethyl)-phenyl]-imidazolidin-2-on

(21) 1-(4-Cyano-phenyl)-3-[4-(3-methoxycarbonyl-propyl)-phenyl]-imidazolidin-2-on

(22) 1-(4-Cyanomethyl-phenyl)-3-(4-methoxycarbonylmethyl-phenyl)-imidazolidin-2-on
Man setzt das Iodid ein [$R_f$-Wert: 0,66 (Kieselgel; Cyclohexan/Essigester = 3:7)], das aus dem entsprechenden Mesylat gewonnen wird.
Schmelzpunkt: 157-160°C
$R_f$-Wert: 0,30 (Kieselgel; Cyclohexan/Essigester = 4:6)

Beispiel 15

1-(4'-Cyano-4-biphenylyl)-3-(2-ethoxycarbonyl-ethyl)-3H-imidazol-2-on

Eine Mischung aus 2,8 g N-(4'-Cyano-4-biphenylyl)-N-(2,2-di-ethoxy-ethyl)-N'-(2-ethoxycarbonyl-ethyl)-harnstoff, 2,8 ml 2n Salzsäure und 28 ml Ethanol wird 45 Minuten zum Rückfluß erhitzt. Die Reaktionsmischung wird auf 0°C gekühlt, das ausgefallene Produkt abgesaugt und mit Ethanol von 0°C gewaschen.
Ausbeute: 1,4 g (63 % der Theorie),
Schmelzpunkt: 140-142°C
$R_f$-Wert: 0,40 (Kieselgel; Cyclohexan/Essigester = 1:1)

Analog werden folgende Verbindungen erhalten:

(1) 1-[4-(4-Cyano-phenyl)-cyclohexyl]-3-(2-ethoxycarbonyl-ethyl)-3H-imidazol-2-on

(2) 1-(4-Cyano-phenyl)-3-[4-(2-methoxycarbonyl-ethyl)-phenyl]-3H-imidazol-2-on
Man arbeitet mit methanolischer Salzsäure.
Schmelzpunkt: 153-155°C

(3) 1-(4-Cyano-phenyl)-3-[2-(4-methoxycarbonyl-phenyl)-ethyl]-3H-imidazol-2-on
Schmelzpunkt: 181-183°C

(4) 1-[1-(4-Cyano-phenyl)-4-piperidinyl]-3-(2-ethoxycarbonyl-ethyl)-3H-imidazol-2-on
Schmelzpunkt: 153-155°C

(5) 1-(4-Cyano-phenyl)-3-[4-(2-methoxycarbonyl-ethenyl)-phenyl]-3H-imidazol-2-on
Schmelzpunkt: 210-212°C
$R_f$-Wert: 0,76 (Kieselgel; Methylenchlorid/Essigester = 9:1)

(6) 1-(4-Cyano-phenyl)-3-[4-(2-dibenzylamino-2-methoxycarbonyl-ethyl)-phenyl]-3H-imidazol-2-on
$R_f$-Wert: 0,40 (Kieselgel; Cyclohexan/Essigester = 2:1)

(7) 1-[4-(2-Amino-2-methoxycarbonyl-ethyl)-phenyl]-3-(4-cyano-phenyl)-3H-imidazol-2-on-hydrochlorid
$R_f$-Wert: 0,82 (Kieselgel; Methylenchlorid/Methanol/konz. Ammoniak = 16:4:1)

Beispiel 16

1-(4'-Cyano-4-biphenylyl)-3-(2-ethoxycarbonyl-ethyl)-imidazolidin-2,4-dion

5 g N-(tert.Butyloxycarbonyl-methyl)-N-(4'-cyano-4-biphenylyl)-N'-(2-ethoxycarbonyl-ethyl)-harnstoff werden in 30 ml Methylenchlorid gelöst, mit 30 ml Trifluoressigsäure versetzt und 16 Stunden bei Raumtemperatur gerührt. Man dampft zur Trockene ein, digeriert mit Wasser und kristallisiert den Niederschlag aus tert.Butyl-methylether/Essigester = 1:1,3 um.
Ausbeute: 2,9 g (55 % der Theorie),
Schmelzpunkt: 177-178°C
$R_f$-Wert: 0,57 (Kieselgel; Methylenchlorid/Essigester = 7:1)
Analog wird folgende Verbindung erhalten:
(1) 1-(4-Aminomethyl-phenyl)-3-[4-[2-(5-tetrazolyl)-ethyl]-phenyl]-imidazolidin-2-on

Beispiel 17

1-(3-Brom-4'-cyano-4-biphenylyl)-3-(2-methoxycarbonyl-ethyl)-imidazolidin-2-on

Herstellung durch Umsetzung von 1-(4'-Cyano-4-biphenylyl)-3-(2-methoxycarbonyl-ethyl)-imidazolidin-2-on mit Brom in Eisessig bei Raumtemperatur.

Beispiel 18

1-(4-Amidino-phenyl)-3-(methoxycarbonylmethylsulfonyl-phenyl)-imidazolidin-2-on

2,1 g 1-(4-Amidino-phenyl)-3-(methoxycarbonylmethylthio-phenyl)-imidazolidin-2-on werden in 10 ml Ameisensäure suspendiert, mit 1,3 ml 30%igem Wasserstoffperoxid versetzt und 16 Stunden bei Raumtemperatur gerührt. Man filtriert vom ausgefallenen Niederschlag ab (siehe Beispiel (2)), zerstört überschüssiges Peroxid durch Zugabe von Natriumbisulfitlösung und dampft im Vakuum ein. Der Rückstand wird durch Kochen mit einer Mischung aus 100 ml Methylenchlorid und 60 ml Methanol extrahiert. Die erhaltene Lösung wird eingedampft und der Rückstand säulenchromatographisch gereinigt (Kieselgel; Methylenchlorid/Methanol = 9:1). Das Produkt enthält neben 1 Mol Wasser noch 0,5 Mol Salzsäure und 1 Mol Ameisensäure. Ausbeute: 0,25 g (10 % der Theorie)
$R_f$-Wert: 0,43 (Kieselgel; Methylenchlorid/Methanol = 8:2)
Analog werden folgende Verbindungen erhalten:

(1) 1-(4-Cyano-phenyl)-3-[4-(2-methoxycarbonyl-ethyl)-2-methylsulfonyl-phenyl]-imidazolidin-2-on

(2) 1-(4-Cyano-phenyl)-3-(4-methoxycarbonylmethylsulfonyl-phenyl)-imidazolidin-2-on

(Entsteht als Nebenprodukt bei Beispiel 18)
Schmelzpunkt: 246-249°C

(3) 1-(4-Cyano-2-methylsulfonyl-phenyl)-3-(4-(2-methoxycarbonyl-ethyl)-phenyl]-imidazolidin-2-on

(4) 1-(4'-Cyano-3-methylsulfonyl-4-biphenylyl)-3-(2-methoxycarbonyl-ethyl)-imidazolidin-2-on

(5) 1-(4-Amidino-phenyl)-3-(4-methoxycarbonylmethylsulfinylphenyl)-imidazolidin-2-on
Man arbeitet in Eisessig bei 10°C mit äquimolaren Mengen Wasserstoffperoxid
Schmelzpunkt: ab 215°C (Zers.)
$R_f$-Wert: 0,48 (Reversed Phase Platte RP8, Methanol/10%ige Natriumchloridlösung = 6:4)

Beispiel 19

1-(4'-Cyano-3-nitro-4-biphenylyl)-3-(2-methoxycarbonyl-ethyl)-imidazolidin-2-on

Herstellung durch Umsetzung von 1-(4'-Cyano-4-biphenylyl)-3-(2-methoxycarbonyl-ethyl)-imidazolidin-2-on mit rauchender Salpetersäure bei 0°C.

Beispiel 20

1-(3-Amino-4'-cyano-4-biphenylyl)-3-(2-methoxycarbonylethyl)-imidazolidin-2-on

Herstellung durch Reduktion von 1-(4'-Cyano-3-nitro-4-biphenylyl)-3-(2-methoxycarbonyl-ethyl)-imidazolidin-2-on mit Wasserstoff von 3 bar Druck in Gegenwart von 5%iger Palladiumkohle in Essigester bei Raumtemperatur.

Beispiel 21

1-(3-Acetamino-4'-cyano-4-biphenylyl)-3-(2-methoxycarbonyl-ethyl)-imidazolidin-2-on

Herstellung aus 1-(3-Amino-4'-cyano-4-biphenylyl)-3-(2-methoxycarbonyl-ethyl)-imidazolidin-2-on und Acetylchlorid in Methylenchlorid bei Raumtemperatur unter Verwendung von Ethyl-diisopropylamin.
Analog werden folgende Verbindungen erhalten:

(1) 1-(3-Benzoylamino-4'-cyano-4-biphenylyl)-3-(2-methoxycarbonyl-ethyl)-imidazolidin-2-on

(2) 1-(4'-Cyano-3-methansulfonylamino-4-biphenylyl)-3-(2-methoxycarbonyl-ethyl)-imidazolidin-2-on

Beispiel 22

1-(4-Cyano-phenyl)-3-[4-(2-phosphono-ethyl)-phenyl]-imidazolidin-2-on

Herstellung aus einer Mischung von 1-(4-Cyano-phenyl)-3-[4-[2-(dimethoxy-phosphoryl)-ethyl]-phenyl]-imidazolidin-2-on, Natriumiodid und Trimethylchlorsilan in Acetonitril durch Rühren bei 40°C.
Analog wird folgende Verbindung erhalten:
(1) 1-(4-Cyano-phenyl)-3-[4-[2-(0-methyl-phosphono)-ethyl]-phenyl]-imidazolidin-2-on
Man verwendet Natriumiodid alleine und arbeitet in Methylethylketon unter Rückfluß.

Beispiel 23

1-(4-Cyano-phenyl)-3-[4-(2-methoxycarbonyl-ethyl)-phenyl]-imidazolidin-2-thion

Herstellung durch Erhitzen von 1-(4-Cyano-phenyl)-3-[4-(2-methoxycarbonyl-ethyl)-phenyl]-imidazolidin-2-on mit 2,4-Bis-(4-methoxyphenyl)-1,3-dithia-2,4-diphosphetan-2,4-disulfid in Xylol.
Analog werden folgende Verbindungen erhalten:

(1) 4-(4-Cyano-phenyl)-1-[4-(2-methoxycarbonyl-ethyl]-phenyl]-3H-imidazol-2-thion

(2) 1-(4-Cyano-phenyl)-4-[4-(2-methoxycarbonyl-ethyl]-phenyl]-3H-imidazol-2-thion

Beispiel 24

4-(4'-Cyano-4-biphenylyl)-1-(2-methoxycarbonyl-ethyl)-3-methyl-3H-imidazol-2-on

Eine Mischung aus 8,4 g 4-Cyano-4'-[(2-methoxycarbonylethyl)-aminomethyl-carbonyl]-biphenyl-hydrochlorid, 2,8 ml Methylisocyanat und 50 ml Pyridin wird 3 Stunden zum Rückfluß erhitzt und anschließend in eine Mischung aus Eis und Salzsäure eingerührt. Man extrahiert mit Essigester, dampft die organische Phase ein und verreibt den Rückstand bis zur Kristallisation mit einer 1:1-Mischung aus Essigester und Ether.
Ausbeute: 2,1 g (35 % der Theorie),
Schmelzpunkt: 128-130°C

Analog werden folgende Verbindungen erhalten:

(1) 4-(4-Cyano-phenyl)-1-[4-(2-methoxycarbonyl-ethyl)-phenyl]-3H-imidazol-2-on
Man setzt Trimethylsilylisocyanat ein.

(2) 4-(4-Cyano-phenyl)-1-[4-(2-methoxycarbonyl-ethyl)-phenyl]-3-phenyl-3H-imidazol-2-on

(3) 4-(4-Cyano-phenyl)-1-[4-(2-methoxycarbonyl-ethyl)-phenyl]-3-methyl-3H-imidazol-2-thion
Man setzt Methylisothiocyanat ein.

(4) 4-(4-Cyano-phenyl)-1-[4-(2-methoxycarbonyl-ethyl)-phenyl]-3-phenyl-3H-imidazol-2-thion
Man setzt Phenylisothiocyanat ein.

(5) 4-(4-Cyano-phenyl)-1-[4-(2-methoxycarbonyl-ethyl)-phenyl]-5-methyl-3H-imidazol-2-on

(6) 4-(4-Cyano-phenyl)-3,5-dimethyl-1-[4-(2-methoxycarbonyl-ethyl)-phenyl]-3H-imidazol-2-on

(7) 4-(4-Cyano-phenyl)-1-[4-(2-methoxycarbonyl-ethyl)-phenyl]-5-methyl-3-phenyl-3H-imidazol-2-on

(8) 1-(4-Cyano-phenyl)-4-[4-(2-methoxycarbonyl-ethyl)-phenyl]-3H-imidazol-2-on

(9) 1-(4-Cyano-phenyl)-4-[4-(2-methoxycarbonyl-ethyl)-phenyl]-3-methyl-3H-imidazol-2-on

(10) 1-(4-Cyano-phenyl)-4-[4-(2-methoxycarbonyl-ethyl)-phenyl]-3-phenyl-3H-imidazol-2-on

(11) 1-(4-Cyano-phenyl)-4-[4-(2-methoxycarbonyl-ethyl)-phenyl]-3-methyl-3H-imidazol-2-thion

(12) 1-(4-Cyano-phenyl)-4-[4-(2-methoxycarbonyl-ethyl)-phenyl]-3-phenyl-3H-imidazol-2-thion

(13) 1-(4-Cyano-phenyl)-4-[4-(2-methoxycarbonyl-ethyl)-phenyl]-5-methyl-3-phenyl-3H-imidazol-2-on

(14) 1-(4-Cyano-phenyl)-3,5-dimethyl-4-[4-(2-methoxycarbonyl-ethyl)-phenyl]-3H-imidazol-2-on

(15) 1-(4-Cyano-phenyl)-4-[4-(2-methoxycarbonyl-ethyl)-phenyl]-5-methyl-3H-imidazol-2-on

(16) 4-(4'-Cyano-4-biphenylyl)-1-(2-methoxycarbonyl-ethyl)-3H-imidazol-2-on
Man verwendet Kaliumisocyanat und Wasser als Lösungsmittel. Schmelzpunkt: 235-240°C

(17) 4-(4-Cyano-phenyl)-1-[4-(2-methoxycarbonyl-ethyl)-phenyl]-3-methyl-3H-imidazol-2-on
Hergestellt aus dem in situ nach Beispiel IV, ohne Zusatz von Hilfsbase hergestellten 4-[(4-Cyano-phenacyl)-amino]-zimtsäure-methylester
Schmelzpunkt: 140-144°C

(18) 4-(4'-Cyano-4-biphenylyl)-1-(2-methoxycarbonyl-ethyl)-3-phenyl-3H-imidazol-2-on
Schmelzpunkt: 103-107°C

(19) 4-(4'-Cyano-4-biphenylyl)-1-(2-methoxycarbonyl-ethyl)-3H-imidazol-2-thion

(20) 4-(4'-Cyano-4-biphenylyl)-1-(2-methoxycarbonyl-ethyl)-3-methyl-3H-imidazol-2-thion

(21) 4-(4'-Cyano-4-biphenylyl)-1-(2-methoxycarbonyl-ethyl)-3-phenyl-3H-imidazol-2-thion

(22) 4-(4'-Cyano-4-biphenylyl)-1-(2-methoxycarbonyl-ethyl)-5-methyl-3H-imidazol-2-on

(23) 4-(4'-Cyano-4-biphenylyl)-3,5-dimethyl-1-(2-methoxycarbonyl-ethyl)-3H-imidazol-2-on

(24) 4-(4'-Cyano-4-biphenylyl)-1-(2-methoxycarbonyl-ethyl)-5-methyl-3-phenyl-3H-imidazol-2-on

Beispiel 25

1-(4-Cyano-phenyl)-3-[4-(2-methoxycarbonyl-ethyl)-cyclohexyl]-imidazolidin-2-on

Eine Mischung aus 2,25 g 1-[4-(2-Methoxycarbonyl-ethyl)-cyclohexyl]-imidazolidin-2-on, 2,25 g 4-Iod-benzonitril, 0,29 g Tris-[2-(2-methoxy-ethoxy)-ethyl]-amin, 2,46 g Kaliumcarbonat, 0,2 g Kupfer(I)chlorid, 0,2 g Kupfer(I)iodid und 60 ml Xylol wird unter Stickstoff 4 Stunden am Wasserabscheider erhitzt. Man läßt auf 50°C abkühlen, gibt 150 ml Essigester zu, filtriert den Niederschlag heiß ab und wäscht mit heißem Essigester nach. Die Essigesterphasen werden zur Trockene eingedampft und der Rückstand säulenchromatographisch über Kieselgel (Elutionsmittel: Cyclohexan/ Essigester = 1:1) gereinigt.
Ausbeute: 1,7 g (54 % der Theorie),
$R_f$-Wert: 0,56 (Kieselgel; Cyclohexan/Essigester = 1:1)
Analog wird folgende Verbindung erhalten:
(1) 2-(4-Cyano-phenyl)-5-[4-(2-methoxycarbonyl-ethyl)-phenyl]-3,4-dihydro-2H,5H-1,2,5-thiadiazol-1,1-dioxid
Man verwendet 4-Fluor-benzonitril und Natriumhydrid in N-Methylpyrrolidon ohne Kupfersalze.
Schmelzpunkt: 160-162°C

Beispiel 26

3-(4-Cyano-phenyl)-1-[4-(2-methoxycarbonyl-ethyl)-phenyl]-4-methyl-3H-imidazol-2-on

3 g N-(4-Cyano-phenyl)-N'-(2-hydroxy-propyl)-N'-[4-(2-methoxycarbonyl-ethyl)-phenyl]-harnstoff werden in einer Mischung aus 40 ml Methylenchlorid und 20 ml Dimethylsulfoxid gelöst und nacheinander mit 0,64 ml Pyridin, 0,61 ml Trifluoressigsäure und 4,9 g N,N'-Dicyclohexylcarbodiimid versetzt. Man rührt 4,5 Stunden bei Raumtemperatur, gibt weitere 5 ml Trifluoressigsäure zu und erwärmt eine Stunde auf 50°C. Man läßt 16 Stunden bei Raumtemperatur stehen, verdünnt mit 150 ml Methylenchlorid und wäscht mehrmals mit Wasser. Die Methylenchloridphase wird eingedampft und der Rückstand säulenchromatographisch über Kieselgel gereinigt (Elutionsmittel: Methylenchlorid/Cyclohexan/Essigester = 2:1:1).
Ausbeute: 0,8 g (28 % der Theorie),
$R_f$-Wert: 0,55 (Kieselgel; Methylenchlorid/Cyclohexan/Essigester = 3:1:1)
Analog wird folgende Verbindung erhalten:
(1) 1-(4-Cyano-phenyl)-3-[4-(2-methoxycarbonyl-ethyl)-phenyl]-4-methyl-3H-imidazol-2-on
Schmelzpunkt: 168-170°C
$R_f$-Wert: 0,55 (Kieselgel; Methylenchlorid/Cyclohexan/Essigester = 2:1:1)

Beispiel 27

4-(4-Cyano-phenyl)-2-[4-(2-ethoxycarbonyl-ethyl)-phenyl]-5-methyl-4H-1,2,4-triazol-3-on

1,75 g N-Acetylamino-N-[4-(2-ethoxycarbonyl-ethyl)-phenyl]-N'-(4-cyano-phenyl)-harnstoff werden 1,5 Stunden auf 180°C erhitzt. Man verreibt mit 8 ml Ethanol und filtriert das ausgefallene Produkt ab.
Ausbeute: 0,66 g (40 % der Theorie),
Schmelzpunkt: 170-172°C
$R_f$-Wert: 0,91 (Kieselgel; Essigester/Cyclohexan = 2:1)
Analog werden folgende Verbindungen erhalten:

(1) 4-(4-Cyano-phenyl)-2-[4-(2-methoxycarbonyl-ethyl)-phenyl]-4H-1,2,4-triazol-3-on
Schmelzpunkt: 213-215°C
$R_f$-Wert: 0,69 (Kieselgel; Methylenchlorid/Essigester = 9:1)

(2) 2-(4-Cyano-phenyl)-4-[4-(2-methoxycarbonyl-ethyl)-phenyl]-4H-1,2,4-triazol-3-on
$R_f$-Wert: 0,62 (Kieselgel; Methylenchlorid/Essigester = 9:1)

(3) 2-(4-Cyano-phenyl)-4-[4-(2-methoxycarbonyl-ethyl)-phenyl]-5-methyl-4H-1,2,4-triazol-3-on
Schmelzpunkt: 163-164°C
$R_f$-Wert: 0,65 (Kieselgel; Methylenchlorid/Essigester = 9:1)

(4) 1-(4-Cyano-phenyl)-3-[4-(2-methoxycarbonyl-ethyl)-phenyl]-imidazolidin-2,4-dion
Schmelzpunkt: 188-190°C
$R_f$-Wert: 0,57 (Kieselgel; Cyclohexan/Essigester = 4:6)

(5) 2-(4-Cyano-phenyl)-4-[4-(2-ethoxycarbonyl-ethyl)-phenyl]-5-ethyl-4H-1,2,4-triazol-4-on
Man kocht in Xylol unter Rückfluß in Gegenwart von Toluolsulfonsäure
Schmelzpunkt: 159-161°C
$R_f$-Wert: 0,68 (Kieselgel; Methylenchlorid/Essigester = 9:1)

Beispiel 28

1-[6-(4-Cyano-phenyl)-3-pyridazinyl]-3-(2-methoxycarbonyl-ethyl)-imidazolidin-2-on

2,2 g 1-[6-(4-Cyano-phenyl)-3-pyridazinyl]-imidazolidin-2-on werden in 160 ml Dimethylformamid mit 0,33 g einer 60%igen Suspension von Natriumhydrid in Öl 3 Stunden bei Raumtemperatur gerührt. Man fügt 2 ml Acrylsäuremethylester zu und rührt weitere 16 Stunden bei Raumtemperatur. Das Reaktionsgemisch wird auf eine Mischung aus 300 ml Wasser und 8 ml 1N Salzsäure gegossen, das ausgefallene Produkt abfiltriert, mit Methanol aufgekocht und nach dem Abkühlen wieder abfiltriert. Ausbeute: 1,8 g (60 % der Theorie),
$R_f$-Wert: 0,46 (Kieselgel; Methylenchlorid/Methanol = 15:1 nach zweimaliger Entwicklung)

Analog wird folgende Verbindung erhalten:
(1) 3-(4'-Cyano-4-biphenylyl)-1-(2-methoxycarbonyl-ethyl)-3H-imidazo[4,5-b]pyridin-2-on
$R_f$-Wert: 0,81 (Kieselgel; Methylenchlorid/Methanol = 19:1)

Beispiel 29

1-(2-tert.Butoxycarbonylmethylamino-ethyl)-3-(4-cyano-phenyl)-imidazolidin-2-on

Eine Lösung von 4,6 g 1-(4-Cyano-phenyl)-3-(2-methansulfonyloxy-ethyl)-imidazolidin-2-on in 75 ml Dimethylformamid wird unter Rühren bei Raumtemperatur mit 2,1 g Kaliumkarbonat und anschließend tropfenweise mit 2,1 ml Glycin-tert.butylester versetzt. Man rührt 16 Stunden bei Raumtemperatur und 30 Stunden bei 60°C. Das Lösungsmittel wird im Vakuum abdestilliert und der Rückstand in einer Mischung aus 150 ml Eiswasser und 100 ml Methylenchlorid aufgenommen. Die Methylenchloridphase wird abgetrennt und mit Wasser gewaschen. Die wäßrigen Phasen extrahiert man nochmals mit Methylenchlorid und dampft schließlich die vereinigten organischen Phasen ein. Der Rückstand wird über Kieselgel gereinigt (Elutionsmittel: Essigester/Methanol/konz.Ammoniak = 9,5:0,5:0,1).
Ausbeute: 1,4 g (27 % der Theorie),
Schmelzpunkt: 104-106°C
$R_f$-Wert: 0,32 (Kieselgel; Essigester/Methanol/konz. Ammoniak = 9:1:0,1)

Beispiel 30

1-[2-(N-Acetyl-N-carboxymethyl-amino)-ethyl]-3-(4-amidino-phenyl)-imidazolidin-2-on

Eine unter Eiskühlung hergestellte Mischung aus 0,3 g 1-(4-Amidino-phenyl)-3-(2-carboxymethylamino-ethyl)-imidazolidin-2-on, 20 ml Wasser und 2,35 ml Acetanhydrid läßt man unter Rühren auf Raumtemperatur kommen und rührt 30 Minuten nach. Man dampft im Vakuum zur Trockne ein und reinigt den Rückstand durch Chromatographie an Kieselgel (Elutionsmittel: Methanol/Eisessig/Wasser = 6:1:1)
Ausbeute: 0,2 g (57 % der Theorie),

Schmelzpunkt: 302-304°C (Zers.)
$R_f$-Wert: 0,54 (Kieselgel; Methanol/2N wäßriges Ammoniak = 4:1)

Beispiel 31

1-[4-(1-Amino-ethyl)-phenyl]-3-[4-(2-methoxycarbonyl-ethyl)-phenyl]-imidazolidin-2-on-hydrochlorid

0,5 g 1-[4-(1-Hydroxyimino-ethyl)-phenyl]-3-[4-(2-methoxycarbonyl-ethyl)-phenyl]-imidazolidin-2-on werden in einer Mischung aus 50 ml Methanol und 1 ml methanolischer Salzsäure in Gegenwart von 100 mg 10%iger Palladium/Kohle 3,5 Stunden bei Raumtemperatur mit Wasserstoff von 5 bar behandelt. Man dampft ein und nimmt mit einer Mischung aus 20 ml Methylenchlorid, 15 ml Methanol, 20 ml Wasser und 0,1 ml 6N Salzsäure auf. Die wäßrige Phase wird abgetrennt und auf etwa ein Drittel des Volumens eingeengt, wobei das Produkt auskristallisiert.
Ausbeute: 0,28 g (53 % der Theorie),
Schmelzpunkt: 264-266°C
$R_f$-Wert: 0,51 (Reversed Phase Platte RP8, Methanol/5%ige Natriumchloridlösung = 6:4)

Analog werden folgende Verbindungen erhalten:

(1) 1-(1-Amino-5-indanyl)-3-[4-(2-methoxycarbonyl-ethyl)-phenyl]-imidazolidin-2-on-hydrochlorid Schmelzpunkt: 223-226°C (sintert ab 208°C)

(2) 1-(1-Amino-1,2,3,4-tetrahydro-6-naphthyl)-3-[4-(2-methoxycarbonyl-ethyl)-phenyl]-imidazolidin-2-on-hydrochlorid

Beispiel 32

1-(4-Amino-cyclohexyl)-3-[4-(3-methoxycarbonyl-propyl)-phenyl]-imidazolidin-2-on

Herstellung aus 1-(4-Aminocarbonyl-cyclohexyl)-3-[4-(3-methoxycarbonyl-propyl)-phenyl]-imidazolidin-2-on durch Behandeln mit [Bis-(trifluoracetoxy)iod]benzol in Acetonitril/Wasser bei Raumtemperatur.

Analog werden folgende Verbindungen erhalten:

(1) 1-(4-Amino-cyclohexyl)-3-[4-(2-methoxycarbonyl-ethyl)-phenyl]-imidazolidin-2-on

(2) 1-[4-(2-Amino-2-propyl)-phenyl]-3-[4-(2-methoxycarbonyl-ethyl)-phenyl]-imidazolidin-2-on

Beispiel 33

1-[4-[(2-Methoxycarbonyl-ethyl)-aminocarbonyl]-phenyl]-3-(4-piperidinyl)-imidazolidin-2-on

Herstellung durch Behandeln von 1-(1-Benzyloxycarbonyl-4-piperidinyl)-3-[4-[(2-methoxycarbonyl-ethyl)-aminocarbonyl]-phenyl]-imidazolidin-2-on mit Wasserstoff von 3 bar in Gegenwart von 5%iger Palladiumkohle in Methanol.

Analog werden folgende Verbindungen erhalten:

(1) 1-(4-Amino-cyclohexyl)-3-[4-[(2-methoxycarbonyl-ethyl)-aminocarbonyl]-phenyl]-imidazolidin-2-on

(2) 1-[4-(2-Methoxycarbonyl-ethyl)-phenyl]-3-[4-(methylaminomethyl)-phenyl]-imidazolidin-2-on

(3) 1-[4-(2-Methoxycarbonyl-ethyl)-phenyl]-3-[4-(n-propylamino-methyl)-phenyl]-imidazolidin-2-on

Beispiel 34

1-[4-(1-Amino-cyclopropyl)-phenyl]-3-[4-(2-methoxycarbonyl-ethyl)-phenyl]-imidazolidin-2-on

Herstellung aus 1-[4-(1-tert.Butyloxycarbonylamino-cyclopropyl)-phenyl]-3-[4-(2-methoxycarbonyl-ethyl)-phenyl]-imidazolidin-2-on durch zweistündiges Rühren in einem 1:1-Gemisch aus Methylenchlorid und Trifluoressigsäure.

Analog wird folgende Verbindung erhalten:
(1) 1-[4-(1-Amino-cyclopentyl)-phenyl]-3-[4-[(2-methoxycarbonyl-ethyl)-phenyl]-imidazolidin-2-on

Beispiel 35

1-[4-(2-Methoxycarbonyl-ethyl)-phenyl]-3-[4-(methylamino-methyl)-phenyl]-imidazolidin-2-on

Herstellung aus 1-(4-Aminomethyl-phenyl)-3-[4-(2-methoxycarbonyl-ethyl)-phenyl]-imidazolidin-2-on durch Alkylierung mit Methyljodid in Dimethylsulfoxid.

Analog werden folgende Verbindungen erhalten:

(1) 1-[4-(2-Methoxycarbonyl-ethyl)-phenyl]-3-[4-(n-propylamino-methyl)-phenyl]-imidazolidin-2-on

(2) 1-[4-[(2-Methoxycarbonyl-ethyl)-aminocarbonyl]-phenyl]-3-(1-methyl-4-piperidinyl)-imidazolidin-2-on

Beispiel 36

1-(4-Cyano-2-methyl-phenyl)-3-[4-(2-methoxycarbonyl-ethyl)-phenyl]-imidazolidin-2-on

3,0 g 1-(4-Brom-2-methyl-phenyl)-3-[4-(2-methoxycarbonyl-ethyl)-phenyl]-imidazolidin-2-on und 1,3 g Kupfer(I)cyanid werden in 10 ml Dimethylformamid 10 Stunden bei einer Badtemperatur von 175°C erhitzt. Das Dimethylformamid wird im Vakuum abgedampft, der Rückstand mit Chloroform digeriert und abfiltriert. Die Chloroformlösung wird mit Wasser und gesättigter Kochsalzlösung gewaschen und eingeengt. Der Rückstand wird säulenchromatographisch gereinigt (Kieselgel; Methylenchlorid/Essigester = 100:2).
Ausbeute: 1,4 g (54 % der Theorie),
Schmelzpunkt: 151-153°C

Beispiel 37

1-[4-(Dimethylamino-methyl)-phenyl]-3-[4-(2-methoxycarbonyl-ethyl)-phenyl]-imidazolidin-2-on

Herstellung aus 1-(4-Aminomethyl-phenyl)-3-[4-(2-methoxycarbonyl-ethyl)-phenyl]-imidazolidin-2-on durch Behandeln mit Formaldehyd und Natriumcyanborhydrid.

Beispiel 38

Trockenampulle mit 2,5 mg Wirkstoff pro 1 ml

Zusammensetzung:

| | |
|---|---|
| Wirkstoff | 2,5 mg |
| Mannitol | 50,0 mg |
| Wasser für Injektionszwecke ad | 1,0 ml |

Herstellung:
Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

Beispiel 39

Trockenampulle mit 35 mg Wirkstoff pro 2 ml

Zusammensetzung:

| | |
|---|---|
| Wirkstoff | 35,0 mg |
| Mannitol | 100,0 mg |

Wasser für Injektionszwecke ad 2,0 ml

Herstellung:
Wirkstoff und Mannitol werden in Wasser gelöst. Nach Abfüllung wird gefriergetrocknet.

Die Auflösung zur gebrauchsfertigen Lösung erfolgt mit Wasser für Injektionszwecke.

Beispiel 40

Tablette mit 50 mg Wirkstoff

Zusammensetzung:

| | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

Herstellung:
(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

Beispiel 41

Tablette mit 350 mg Wirkstoff

Zusammensetzung:

| | |
|---|---|
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

Herstellung:
(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

Beispiel 42

Kapseln mit 50 mg Wirkstoff

Zusammensetzung:

| | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| | 160,0 mg |

Herstellung:
(1) wird mit (3) verrieben. Diese Verreibung wird der Mischung aus (2) und (4) unter intensiver Mischung zugegeben. Diese Pulvermischung wird auf einer Kapselabfüllmaschine in Hartgelatine-Steckkapseln Größe 3 abgefüllt.

Beispiel 43

Kapseln mit 350 mg Wirkstoff

Zusammensetzung:

| | |
|---|---|
| (1) Wirkstoff | 350,0 mg |
| (2) Maisstärke getrocknet | 46,0 mg |
| (3) Milchzucker pulverisiert | 30,0 mg |
| (4) Magnesiumstearat | 4,0 mg |
| | 430,0 mg |

Herstellung:
(1) wird mit (3) verrieben. Diese Verreibung wird der Mischung aus (2) und (4) unter intensiver Mischung zugegeben. Diese Pulvermischung wird auf einer Kapselabfüllmaschine in Hartgelatine-Steckkapseln Größe 0 abgefüllt.

**Patentansprüche**

**1.** Cyclische Harnstoffderivate der allgemeinen Formel

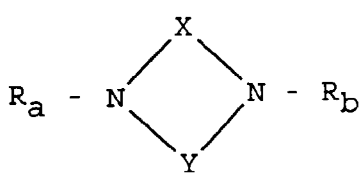

, (I)

in der

X eine gegebenenfalls am Stickstoffatom durch eine Alkyl-, Aralkyl-, Aryl-, Heteroaryl- oder Cyanogruppe substituierte Carbiminogruppe, eine Carbonyl-, Thiocarbonyl-, Sulfinyl- oder Sulfonylgruppe,

Y eine gegebenenfalls durch $R_c$ oder $R_d$ oder $R_c$ und $R_d$ substituierte geradkettige Alkylen- oder Alkenylengruppe mit jeweils 2 bis 4 Kohlenstoffatomen, in denen jedes Kohlenstoffatom durch ein Fluor-, Chlor- oder Bromatom, durch eine Alkyl-, Trifluormethyl-, Aralkyl-, Aryl-, Heteroaryl- oder Alkylcarbonylgruppe mono- oder

disubstituiert sein kann, wobei die Substituenten gleich oder verschieden sein können und zusätzlich eine oder zwei Methylengruppen jeweils durch eine Carbonylgruppe ersetzt sein können, oder eine gegebenenfalls durch $R_c$ oder $R_d$ oder $R_c$ und $R_d$ substituierte 1,2-Cycloalkylengruppe mit 4 bis 7 Kohlenstoffatomen,

eine 1,2-Cycloalkenylengruppe mit 4 bis 7 Kohlenstoffatomen oder eine 1,2-Phenylengruppe, in der eine oder zwei Methingruppen durch ein Stickstoffatom ersetzt sein können und die im Kohlenstoffgerüst durch ein Fluor-, Chlor- oder Bromatom, durch eine Alkylgruppe mit 1 bis 4 Kohlenstoffatomen, durch eine Trifluormethyl-, Hydroxy-, Alkoxy-, Alkylsulfenyl-, Alkylsulfinyl-, Alkylsulfonyl-, Alkylcarbonyl-, Arylcarbonyl-, Alkoxycarbonyl-, Carboxy-, Nitro-, $(R_1)_2N$-, $(R_1)_2NCO$- oder $(R_1)_2NSO_2$- Gruppe, wobei die Reste $R_1$ gleich oder verschieden sein können und jeweils ein Wasserstoffatom, eine Alkyl-, Aralkyl-, Aryl-oder Heteroarylgruppe bedeuten, oder durch eine durch eine Alkylcarbonyl-, Arylcarbonyl-, Aralkylcarbonyl-, Heteroarylcarbonyl-, Alkylsulfonyl-, Aralkylsulfonyl- oder Arylsulfonylgruppe substituierte $R_1$NH-Gruppe substituiert sein kann und in der zusätzlich eine oder zwei -CH=CH-Gruppen jeweils durch eine -CO-$NR_1$-Gruppe ersetzt sein können,

eine gegebenenfalls durch $R_c$ oder $R_d$ substituierte -CO-NH-, -NH-CO-, -CH=N- oder -N=CH-Gruppe,

einer der Reste $R_a$ bis $R_d$ eine Gruppe der Formel

A - B - C -,

in der

A eine geradkettige oder verzweigte Aminoalkylgruppe mit 1 bis 5 Kohlenstoffatomen, eine Amino-, Amidino- oder Guanidinogruppe, wobei in jeder der vorstehend erwähnten Gruppen jeweils an einem der Stickstoffatome ein oder zwei Wasserstoffatome jeweils durch eine Alkylgruppe mit 1 bis 4 Kohlenstoffatomen oder ein Wasserstoffatom durch eine Alkoxycarbonylgruppe mit insgesamt 2 bis 5 Kohlenstoffatomen, durch eine Alkylcarbonyl-, Arylcarbonyl-, Aryloxycarbonyl- oder Aralkoxycarbonylgruppe ersetzt sein kann, eine Cyanogruppe, eine Cyanoalkylgruppe mit 1 bis 4 Kohlenstoffatomen im Alkylteil oder auch, falls A an ein Stickstoffatom der Reste B oder C gebunden ist, das nicht Teil einer Lactamgruppe ist, ein Wasserstoffatom oder eine Alkylgruppe,

B eine Bindung,

eine Alkylen- oder Alkenylengruppe,

eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, $(R_1)_2N$-, $(R_1)_2NCO$-, $(R_1)_2NSO_2$- oder Nitrogruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1$NH-Gruppen mono-oder disubstituiert sein kann, wobei die Substituenten gleich oder verschieden sein können,

eine Pyridinylen-, Pyrimidinylen-, Pyrazinylen-, Pyridazinylen-oder Triazinylengruppe, die jeweils im Kohlenstoffgerüst durch eine Alkylgruppe substituiert sein können, wobei zusätzlich eine oder zwei -CH=N-Gruppen jeweils durch eine -CO-$NR_1$-Gruppe ersetzt sein können und eines der Stickstoffatome statt an den Rest $R_1$ auch an den Rest C gebunden sein kann, sofern dieser nicht mit einem Heteroatom oder einer Carbonylgruppe an den Rest B anschließt,

eine gegebenenfalls durch eine Alkyl-, Aralkyl- oder Arylgruppe substituierte Cyclopropylengruppe,

eine gegebenenfalls durch eine Alkyl-, Aralkyl- oder Arylgruppe substituierte Cycloalkylengruppe mit 4 bis 5 Kohlenstoffatomen, in der eine CH-Einheit durch ein Stickstoffatom und zusätzlich eine zum Stickstoffatom benachbarte Methylengruppe durch eine Carbonylgruppe ersetzt sein kann,

eine gegebenenfalls durch eine Alkyl-, Aralkyl- oder Arylgruppe substituierte Cycloalkylengruppe mit 6 oder 7 Kohlenstoffatomen, in der eine oder zwei zueinander in 1,4-Stellung befindliche CH-Einheiten je

durch ein Stickstoffatom ersetzt sein können, wobei zusätzlich jeweils eine oder zwei der zu einem Stickstoffatom benachbarten Methylengruppen durch eine Carbonylgruppe ersetzt sein können,

eine Biphenylengruppe, die durch Fluor-, Chlor- oder Bromatome, durch Alkyl-, Trifluormethyl-, Hydroxy-, Alkoxy-, Alkylsulfenyl-, Alkylsulfinyl-, Alkylsulfonyl-, Alkylcarbonyl-$NR_1$- oder Alkylsulfonyl-$NR_1$-Gruppen mono- oder disubstituiert sein kann, wobei die Substituenten gleich oder verschieden sein können und $R_1$ wie eingangs definiert ist, und

C eine gegebenenfalls durch eine Hydroxy-, Alkoxy- oder $(R_1)_2N$-Gruppe substituierte Alkylen- oder Alkenylengruppe,

eine über die Carbonylgruppe mit dem Rest B verbundene Alkylencarbonylgruppe,

eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, $(R_1)_2N$-, $(R_1)_2NCO$-, $(R_1)_2NSO_2$- oder Nitrogruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1NH$-Gruppen mono- oder disubstituiert sein kann, wobei die Substituenten gleich oder verschieden sein können,

eine Indanylen- oder 1,2,3,4-Tetrahydronaphthylengruppe, in denen jeweils der gesättigte Ring an den Rest A und der aromatische Ring an das cyclische Harnstoffgerüst gebunden ist, eine Pyridinylen-, Pyrimidinylen-, Pyrazinylen-, Pyridazinylen- oder Triazinylengruppe, die jeweils im Kohlenstoffgerüst durch eine Alkylgruppe substituiert sein können, wobei zusätzlich eine oder zwei -CH=N-Gruppen jeweils durch eine -CO-$NR_1$-Gruppe ersetzt sein können und eines der Stickstoffatome statt an den Rest $R_1$ auch an den Rest B gebunden sein kann, solange dieser nicht eine Bindung bedeutet oder nicht mit einem Heteroatom an den Rest C anschließt,

eine gegebenenfalls durch eine Alkyl-, Aralkyl- oder Arylgruppe substituierte Cycloalkylengruppe mit 4 bis 5 Kohlenstoffatomen, in der eine CH-Einheit durch ein Stickstoffatom und zusätzlich eine zum Stickstoffatom benachbarte Methylengruppe durch eine Carbonylgruppe ersetzt sein kann, oder

eine gegebenenfalls durch eine Alkyl-, Aralkyl- oder Arylgruppe substituierte Cycloalkylengruppe mit 6 oder 7 Kohlenstoffatomen, in der eine oder zwei zueinander in 1,4-Stellung befindliche CH-Einheiten je durch ein Stickstoffatom ersetzt sein können, wobei zusätzlich jeweils eine oder zwei der zu einem Stickstoffatom benachbarten Methylengruppen durch eine Carbonylgruppe ersetzt sein können,

ein zweiter der Reste $R_a$ bis $R_d$ eine Gruppe der Formel

$$F - E - D -,$$

in der

D eine Alkylengruppe mit 1 bis 5 Kohlenstoffatomen oder eine Alkenylengruppe mit 2 bis 5 Kohlenstoffatomen,

eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, Carboxyalkoxy-, Alkoxycarbonylalkoxy-, Aralkoxycarbonylalkoxy-, $(R_1)_2N$-, $(R_1)_2NCO$-, $(R_1)_2NSO_2$- oder Nitrogruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1NH$-Gruppen mono- oder disubstituiert sein kann, wobei die Substituenten gleich oder verschieden sein können,

eine Pyridinylen-, Pyrimidinylen-, Pyrazinylen-, Pyridazinylen-oder Triazinylengruppe, die jeweils im Kohlenstoffgerüst durch eine Alkylgruppe substituiert sein können, wobei zusätzlich eine oder zwei -CH=N-Gruppen jeweils durch eine -CO-$NR_1$-Gruppe ersetzt sein können und eines der Stickstoffatome statt an den Rest $R_1$ auch an den Rest E gebunden sein kann, sofern dieser keine Bindung darstellt oder nicht mit einem Heteroatom an den Rest D gebunden ist,

eine gegebenenfalls durch eine Alkyl-, Aralkyl- oder Arylgruppe substituierte Cycloalkylengruppe mit 4 bis 5 Kohlenstoffatomen, in der eine CH-Einheit durch ein Stickstoffatom und zusätzlich eine zum Stickstoffatom benachbarte Methylengruppe durch eine Carbonylgruppe ersetzt sein kann,

eine gegebenenfalls durch eine Alkyl-, Aralkyl- oder Arylgruppe substituierte Cycloalkylengruppe mit 6 oder 7 Kohlenstoffatomen, in der eine oder zwei zueinander in 1,4-Stellung befindliche CH-Einheiten je durch ein Stickstoffatom ersetzt sein können, wobei zusätzlich jeweils eine oder zwei der zu einem Stickstoffatom benachbarten Methylengruppen durch eine Carbonylgruppe ersetzt sein können, oder

eine durch den Rest W unterbrochene Alkylengruppe mit 2 bis 6 Kohlenstoffatomen, in der W ein Sauerstoff- oder Schwefelatom, eine Sulfinyl-, Sulfonyl-, $R_1$N-, (Alkylcarbonyl)N-, (Aralkylcarbonyl)N-, (Arylcarbonyl)N-, (Heteroarylcarbonyl)N-, (Alkyl-(Alkylsulfonyl)N-, (Arylsulfonyl)N-, Aminocarbonyl- oder Carbonylaminogruppe darstellt,

E eine Bindung,

eine Alkylengruppe mit 1 bis 5 Kohlenstoffatomen oder eine Alkenylengruppe mit 2 bis 5 Kohlenstoffatomen, die jeweils durch eine oder zwei Alkylgruppen, durch eine Hydroxy-, Alkoxy-, Amino-, Alkylamino-, Aralkylamino-, Dialkylamino-, Bis-(aralkyl)amino-, Carboxyalkyl-, Alkoxycarbonylalkyl- oder Aralkoxycarbonylalkylgruppe substituiert sein können,

eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, $(R_1)_2$N-, $(R_1)_2$NCO-, $(R_1)_2NSO_2$- oder Nitrogruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1$NH-Gruppen mono-oder disubstituiert sein kann, wobei die Substituenten gleich oder verschieden sein können,

eine Pyridinylen-, Pyrimidinylen-, Pyrazinylen-, Pyridazinylen-oder Triazinylengruppe, die jeweils im Kohlenstoffgerüst durch eine Alkylgruppe substituiert sein können, wobei zusätzlich eine oder zwei -CH=N-Gruppen jeweils durch eine -CO-$NR_1$-Gruppe ersetzt sein können und eines der Stickstoffatome statt an den Rest $R_1$ auch an den Rest D gebunden sein kann,

eine gegebenenfalls durch eine Alkyl-, Aralkyl- oder Arylgruppe substituierte Cycloalkylengruppe mit 4 bis 5 Kohlenstoffatomen, in der eine CH-Einheit durch ein Stickstoffatom und zusätzlich eine zum Stickstoffatom benachbarte Methylengruppe durch eine Carbonylgruppe ersetzt sein kann,

eine gegebenenfalls durch eine Alkyl-, Aralkyl- oder Arylgruppe substituierte Cycloalkylengruppe mit 6 oder 7 Kohlenstoffatomen, in der eine oder zwei zueinander in 1,4-Stellung befindliche CH-Einheiten je durch ein Stickstoffatom ersetzt sein können, wobei zusätzlich jeweils eine oder zwei der zu einem Stickstoffatom benachbarten Methylengruppen durch eine Carbonylgruppe ersetzt sein können,

eine über den Arylteil mit dem Rest D verknüpfte Alkylenarylengruppe oder

eine über den Rest W mit dem Rest D verknüpfte Alkylengruppe, in der W wie eingangs definiert ist, und

F eine Carbonylgruppe, die durch eine Hydroxy- oder Alkoxygruppe mit 1 bis 6 Kohlenstoffatomen substituiert ist, wobei ein Alkoxyrest mit 1 bis 3 Kohlenstoffatomen in 1-, 2- oder 3-Stellung durch eine Aryl- oder Heteroarylgruppe oder in 2- oder 3-Stellung durch eine Pyrrolidin-2-on-1-yl-, Morpholino-, Thiomorpholino- oder 1-oxido-thiomorpholinogruppe substituiert sein kann, eine Sulfo-, Phosphono-, O-Alkylphosphono- oder Tetrazol-5-ylgruppe darstellen, wobei, sofern A eine Cyanogruppe oder eine gegebenenfalls am Stickstoffatom benzoylierte oder benzyloxycarbonylierte Amino- oder Aminoalkylgruppe darstellt, der kürzeste Abstand zwischen dem Stickstoffatom dieser Gruppen und dem Rest F mindestens 10 Bindungen beträgt,

der dritte der Reste $R_a$ bis $R_d$ ein Wasserstoffatom, eine Alkyl-, Aralkyl-, Aryl- oder Heteroarylgruppe oder auch, wenn der dritte der Reste $R_a$ bis $R_d$ mit einem ungesättigten Kohlenstoffatom des Restes Y verbunden ist, eine Alkoxy-, Alkylsulfenyl-oder $(R_1)_2$N-Gruppe und

der vierte der Reste $R_a$ bis $R_d$ ein Wasserstoffatom, eine Alkyl-, Aralkyl-, Aryl- oder Heteroarylgruppe oder $R_a$ oder $R_b$ zusammen mit einem benachbarten Rest $R_c$ oder $R_d$ auch eine Bindung mit der Maßgabe darstellen, daß die A-B-C-Gruppe keine 4-Piperidinylgruppe darstellt, wenn gleichzeitig die F-E-D-Gruppe eine Alkoxycarbonylalkylgruppe und Y eine durch ein Fluor-, Chlor- oder Bromatom, durch eine Alkyl- oder Trifluormethylgruppe substituierte 1,2-Phenylengruppe bedeuten,

deren Tautomere, deren Stereoisomere einschließlich deren Gemische und deren Additionssalze,

wobei, soweit nichts anderes erwähnt wurde,

die vorstehend erwähnten Alkyl-, Alkylen-, Alkenylen- oder Alkoxyteile jeweils 1 bis 3 Kohlenstoffatome enthalten können, sowie

unter dem vorstehend erwähnten Begriff "eine Arylgruppe" eine Phenylgruppe, die durch eine Trifluormethyl-, Carboxy-, $(R_1)_2NCO$-, Alkoxycarbonyl-, Alkylcarbonyl-, Alkylsulfenyl-, Alkylsulfinyl-, Alkylsulfonyl-, Nitro-, $(R_1)_2N$-, Alkylcarbonyl-$NR_1$- Aralkylcarbonyl-$NR_1$-, Arylcarbonyl-$NR_1$-, Heteroarylcarbonyl-$NR_1$-, Alkylsulfonyl-$NR_1$-, Aralkylsulfonyl-$NR_1$-, Arylsulfonyl-$NR_1$- oder $(R_1)_2N$-sulfonyl-Gruppe monosubstituiert oder durch Fluor-, Chlor- oder Bromatome, durch Hydroxy-, Alkoxy- oder Alkylgruppen mit 1 bis 4 Kohlenstoffatomen mono-, di-oder trisubstituiert sein kann, und

unter dem vorstehend erwähnten Begriff "eine Heteroarylgruppe" ein 5-gliedriger heteroaromatischer Ring, welcher ein Sauerstoff-, Schwefel- oder Stickstoffatom, ein Stickstoffatom und ein Sauerstoff-, Schwefel- oder Stickstoffatom oder zwei Stickstoffatome und ein Sauerstoff-, Schwefel- oder Stickstoffatom enthält oder ein 6-gliedriger heteroaromatischer Ring, welcher ein, zwei oder drei Stickstoffatome enthält und in dem zusätzlich eine oder zwei -CH=N-Gruppen durch eine -CO-$NR_1$-Gruppe ersetzt sein können, wobei die vorstehend erwähnten heteroaromatischen Ringe zusätzlich durch eine oder zwei Alkylgruppen oder durch ein Fluor-, Chlor- oder Bromatom, durch eine Hydroxy- oder Alkoxygruppe substituiert sein können, zu verstehen ist.

**2.** Cyclische Harnstoffderivate der allgemeinen Formel I gemäß Anspruch 1, in der

X eine gegebenenfalls am Stickstoffatom durch eine Alkyl-, Aralkyl-, Aryl-, Heteroaryl- oder Cyanogruppe substituierte Carbiminogruppe, eine Carbonyl-, Thiocarbonyl-, Sulfinyl- oder Sulfonylgruppe,

Y eine gegebenenfalls durch $R_c$ oder $R_d$ oder $R_c$ und $R_d$ substituierte geradkettige Alkylen- oder Alkenylengruppe mit jeweils 2 oder 3 Kohlenstoffatomen, die durch ein Fluor-, Chlor- oder Bromatom, durch eine Alkyl-, Trifluormethyl-, Aralkyl-, Aryl-, Heteroaryl- oder Alkylcarbonylgruppe mono- oder disubstituiert sein kann, wobei die Substituenten gleich oder verschieden sein können und zusätzlich eine oder zwei Methylengruppen jeweils durch eine Carbonylgruppe ersetzt sein können, oder eine gegebenenfalls durch $R_c$ oder $R_d$ oder $R_c$ und $R_d$ substituierte 1,2-Cyclohexylengruppe,

eine 1,2-Cyclohexenylengruppe oder eine 1,2-Phenylengruppe, in der eine oder zwei CH-Gruppen jeweils durch ein Stickstoffatom ersetzt sein können und die im Kohlenstoffgerüst durch ein Fluor-, Chlor- oder Bromatom, durch eine Alkylgruppe mit 1 bis 4 Kohlenstoffatomen, durch eine Trifluormethyl-, Hydroxy-, Alkoxy-, Alkylsulfenyl-, Alkylsulfinyl-, Alkylsulfonyl-, Alkylcarbonyl-, Arylcarbonyl-, Alkoxycarbonyl-, Carboxy-, Nitro-, $(R_1)_2N$-, $(R_1)_2NCO$- oder $(R_1)_2NSO_2$-Gruppe, wobei die Reste $R_1$ gleich oder verschieden sein können und jeweils ein Wasserstoffatom, eine Alkyl-, Aralkyl-, Aryl- oder Heteroarylgruppe bedeuten, oder durch eine durch eine Alkylcarbonyl-, Arylcarbonyl-, Aralkylcarbonyl-, Heteroarylcarbonyl-, Alkylsulfonyl-, Aralkylsulfonyl- oder Arylsulfonylgruppe substituierte $R_1NH$-Gruppe substituiert sein kann, und in der zusätzlich eine oder zwei -CH=CH-Gruppen jeweils durch eine -CO-$NR_1$-Gruppe ersetzt sein können,

eine gegebenenfalls durch $R_c$ oder $R_d$ substituierte -CO-NH-, -NH-CO-, -CH=N- oder -N=CH-Gruppe darstellen,

einer der Reste $R_a$ bis $R_d$ eine Gruppe der Formel

A - B - C -,

in der

A eine geradkettige oder verzweigte Aminoalkylgruppe mit 1 bis 5 Kohlenstoffatomen, eine Amino-, Amidino- oder Guanidinogruppe, wobei in jeder der vorstehend erwähnten Gruppen jeweils an einem der Stickstoffatome ein oder zwei Wasserstoffatome jeweils durch eine Alkylgruppe mit 1 bis 4 Kohlenstoffatomen oder ein Wasserstoffatom durch eine Alkoxycarbonylgruppe mit insgesamt 2 bis 5 Kohlenstoffatomen, durch eine Alkylcarbonyl-, Arylcarbonyl-, Aryloxycarbonyl- oder Aralkoxycarbonylgruppe ersetzt sein kann, eine Cyanogruppe, eine Cyanoalkylgruppe mit 1 bis 4 Kohlenstoffatomen im Alkylteil oder auch, falls A an ein Stickstoffatom der Reste B oder C gebunden ist, das nicht Teil einer Lactamgruppe ist, ein Wasserstoffatom oder eine Alkylgruppe,

B eine Bindung,

eine Alkylen- oder Alkenylengruppe,

eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, $(R_1)_2N$-, $(R_1)_2NCO$-, $(R_1)_2NSO_2$- oder Nitrogruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1NH$-Gruppen mono- oder disubstituiert sein kann, wobei die Substituenten gleich oder verschieden sein können,

eine Pyridinylen-, Pyrimidinylen-, Pyrazinylen- oder Pyridazinylengruppe, die jeweils im Kohlenstoffgerüst durch eine Alkylgruppe substituiert sein können, wobei zusätzlich eine oder zwei -CH=N-Gruppen jeweils durch eine -CO-$NR_1$-Gruppe ersetzt sein können und eines der Stickstoffatome statt an den Rest $R_1$ auch an den Rest C gebunden sein kann, sofern dieser nicht mit einem Heteroatom oder einer Carbonylgruppe an den Rest B anschließt,

eine Cycloalkylengruppe mit 3 bis 5 Kohlenstoffatomen,

eine Cyclohexylengruppe, in der eine oder zwei CH-Einheiten, die sich zueinander in 1,4-Stellung befinden, durch Stickstoffatome ersetzt sein können, wobei zusätzlich jeweils eine oder zwei der zu einem Stickstoffatom benachbarten Methylengruppen durch eine Carbonylgruppe ersetzt sein können, oder

eine Biphenylengruppe und

C eine gegebenenfalls durch eine Hydroxygruppe substituierte Alkylen- oder Alkenylengruppe,

eine über die Carbonylgruppe mit dem Rest B verbundene Alkylencarbonylgruppe,

eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, $(R_1)_2N$-, $(R_1)_2NCO$-, $(R_1)_2NSO_2$- oder Nitrogruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1NH$-Gruppen mono- oder disubstituiert sein kann, wobei die Substituenten gleich oder verschieden sein können,

eine Indanylen- oder 1,2,3,4-Tetrahydronaphthylengruppe, in denen jeweils der gesättigte Ring an den Rest A und der aromatische Ring an das cyclische Harnstoffgerüst gebunden ist,

eine Pyridinylen-, Pyrimidinylen-, Pyrazinylen- oder Pyridazinylengruppe, die jeweils im Kohlenstoffgerüst durch eine Alkylgruppe substituiert sein können, wobei zusätzlich eine oder zwei -CH=N-Gruppen jeweils durch eine -CO-$NR_1$-Gruppe ersetzt sein können und eines der Stickstoffatome statt an den Rest $R_1$ auch an den Rest B gebunden sein kann, solange dieser keine Bindung bedeutet oder nicht mit einem Heteroatom an den Rest C anschließt,

eine Cyclohexylengruppe, in der eine oder zwei CH-Einheiten, die sich zueinander in 1,4-Stellung befinden, durch Stickstoffatome ersetzt sein können, wobei zusätzlich jeweils eine oder zwei der zu einem

Stickstoffatom benachbarten Methylengruppen durch eine Carbonylgruppe ersetzt sein und die Stickstoffatome nicht an ein Stickstoffatom des cyclischen Harnstoffs gebunden sein können,

ein zweiter der Reste $R_a$ bis $R_d$ eine Gruppe der Formel

$$F - E - D -,$$

in der

D eine Alkylengruppe mit 1 bis 5 Kohlenstoffatomen oder eine Alkenylengruppe mit 2 bis 5 Kohlenstoffatomen,

eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, Carboxyalkoxy-, Alkoxycarbonylalkoxy-, Aralkoxycarbonylalkoxy-, $(R_1)_2N$-, $(R_1)_2NCO$-, $(R_1)_2NSO_2$- oder Nitrogruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1NH$-Gruppen mono- oder disubstituiert sein kann, wobei die Substituenten gleich oder verschieden sein können,

eine Pyridinylen-, Pyrimidinylen-, Pyrazinylen- oder Pyridazinylengruppe, die jeweils im Kohlenstoffgerüst durch eine Alkylgruppe substituiert sein kann, wobei zusätzlich eine oder zwei -CH=N-Gruppen jeweils durch eine -CO-$NR_1$-Gruppe ersetzt sein können und eines der Stickstoffatome statt an den Rest $R_1$ auch an den Rest E gebunden sein kann, sofern dieser keine Bindung darstellt oder nicht mit einem Heteroatom an den Rest D gebunden ist,

eine Cyclohexylengruppe, in der eine oder zwei CH-Einheiten, die sich zueinander in 1,4-Stellung befinden, durch Stickstoffatome ersetzt sein können, wobei zusätzlich jeweils eine oder zwei der zu einem Stickstoffatom benachbarte Methylengruppen durch eine Carbonylgruppe ersetzt sein können, oder

eine durch den Rest W unterbrochene Alkylengruppe mit 3 bis 6 Kohlenstoffatomen, in der W ein Sauerstoff- oder Schwefelatom, eine Sulfinyl-, Sulfonyl-, $R_1N$-, (Alkylcarbonyl)N-, (Aralkylcarbonyl)N-, (Arylcarbonyl)N-, (Heteroarylcarbonyl)N-, (Alkylsulfonyl)N- oder (Arylsulfonyl)N-Gruppe darstellt und die mit einem Stickstoffatom des cyclischen Harnstoffs verknüpfte Alkylengruppe 2 oder 3 Kohlenstoffatome enthält,

E eine Bindung,

eine Alkylengruppe mit 1 bis 5 Kohlenstoffatomen oder eine Alkenylengruppe mit 2 bis 5 Kohlenstoffatomen, die jeweils durch eine oder zwei Alkylgruppen, durch eine Hydroxy-, Alkoxy-, Amino-, Alkylamino-, Aralkylamino-, Dialkylamino-, Bis-(aralkyl)amino-, Carboxyalkyl-, Alkoxycarbonylalkyl- oder Aralkoxycarbonylalkylgruppe substituiert sein können,

eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, $(R_1)_2N$-, $(R_1)_2NCO$-, $(R_1)_2NSO_2$- oder Nitrogruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1NH$-Gruppen mono- oder disubstituiert sein kann, wobei die Substituenten gleich oder verschieden sein können,

eine Pyridinylen-, Pyrimidinylen-, Pyrazinylen- oder Pyridazinylengruppe, die jeweils im Kohlenstoffgerüst durch eine Alkylgruppe substituiert sein können, wobei zusätzlich eine oder zwei -CH=N-Gruppen jeweils durch eine -CO-$NR_1$-Gruppe ersetzt sein können und eines der Stickstoffatome statt an dem Rest $R_1$ auch an den Rest D gebunden sein kann,

eine Cyclohexylengruppe, in der eine oder zwei CH-Einheiten, die sich zueinander in 1,4-Stellung befinden, durch Stickstoffatome ersetzt sein können, wobei zusätzlich jeweils eine oder zwei der zu einem Stickstoffatom benachbarten Methylengruppen durch eine Carbonylgruppe ersetzt sein können,

eine über den Arylrest mit dem Rest D verknüpfte Alkylenarylengruppe oder

eine über den Rest W' mit dem Rest D verknüpfte Alkylengruppe, in der W' ein Sauerstoff- oder Schwefelatom, eine Sulfinyl-, Sulfonyl-, $R_1$N-, (Alkylcarbonyl)N-, (Aralkylcarbonyl)N-, (Arylcarbonyl)N-, (Heteroarylcarbonyl)N-, (Alkylsulfonyl)N-, (Arylsulfonyl)N- oder Aminocarbonylgruppe, in der das Stickstoffatom an die Alkylengruppe gebunden ist, darstellt,

F eine Carbonylgruppe, die durch eine Hydroxy- oder Alkoxygruppe mit 1 bis 6 Kohlenstoffatomen substituiert ist, wobei ein Alkoxyrest mit 1 bis 3 Kohlenstoffatomen in 1-, 2- oder 3-Stellung durch eine Aryl- oder Heteroarylgruppe oder in 2- oder 3-Stellung durch eine Pyrrolidin-2-on-1-yl-, Morpholino-, Thiomorpholino- oder 1-Oxido-thiomorpholinogruppe substituiert sein kann, eine Sulfo-, Phosphono-, O-Alkylphosphono- oder Tetrazol-5-ylgruppe darstellen, wobei, sofern A eine Cyanogruppe oder eine gegebenenfalls am Stickstoffatom benzoylierte oder benzyloxycarbonylierte Amino- oder Aminoalkylgruppe darstellt, der kürzeste Abstand zwischen dem Stickstoffatom dieser Gruppen und dem Rest F mindestens 10 Bindungen beträgt,

der dritte der Reste $R_a$ bis $R_d$ ein Wasserstoffatom, eine Alkyl-, Aralkyl-, Aryl- oder Heteroarylgruppe oder auch, wenn der dritte der Reste $R_a$ bis $R_d$ mit einem ungesättigten Kohlenstoffatom des Restes Y verbunden ist, eine Alkoxy-, Alkylsulfenyloder $(R_1)_2$N-Gruppe und

der vierte der Reste $R_a$ bis $R_d$ ein Wasserstoffatom, eine Alkyl-, Aralkyl-, Aryl- oder Heteroarylgruppe oder $R_a$ oder $R_b$ zusammen mit einem benachbarten Rest $R_c$ oder $R_d$ auch eine Bindung mit der Maßgabe darstellen, daß die A-B-C-Gruppe keine 4-Piperidinylgruppe darstellt, wenn gleichzeitig die F-E-D-Gruppe eine Alkoxycarbonylalkylgruppe und Y eine durch ein Fluor-, Chlor- oder Bromatom, durch eine Alkyl- oder Trifluormethylgruppe substituierte 1,2-Phenylengruppe bedeuten,

deren Tautomere, deren Stereoisomere einschließlich deren Gemische und deren Additionssalze.

**3.** Cyclische Harnstoffderivate der allgemeinen Formel I gemäß Anspruch 1, in der

X eine gegebenenfalls am Stickstoffatom durch eine Methyl-, Phenyl- oder Pyridylgruppe substituierte Carbiminogruppe, eine Carbonyl-, Thiocarbonyl- oder Sulfonylgruppe,

Y eine gegebenenfalls durch $R_c$ oder $R_d$ oder $R_c$ und $R_d$ substituierte geradkettige Alkylen- oder Alkenylengruppe mit jeweils 2 oder 3 Kohlenstoffatomen, die durch ein Chloratom, durch eine oder zwei Methylgruppen oder durch eine Trifluormethyl-, Phenyl- oder Acetylgruppe substituiert sein kann, wobei zusätzlich eine Methylengruppe durch eine Carbonylgruppe ersetzt sein kann,

eine gegebenenfalls durch $R_c$ oder $R_d$ substituierte -CO-NH-, -NH-CO-, -CH=N- oder -N=CH-Gruppe, eine 1,2-Phenylen- oder 2,3-Pyridinylengruppe,

einer der Reste $R_a$ bis $R_d$ eine Gruppe der Formel

A - B - C -,

in der

A eine geradkettige oder verzweigte Aminoalkylgruppe mit 1 bis 4 Kohlenstoffatomen, eine Amino-, Amidino- oder Guanidinogruppe, wobei in jeder der vorstehend erwähnten Gruppen jeweils an einem der Stickstoffatome ein oder zwei Wasserstoffatome jeweils durch eine Alkylgruppe mit 1 bis 4 Kohlenstoffatomen oder ein Wasserstoffatom durch eine Alkoxycarbonylgruppe mit insgesamt 2 bis 5 Kohlenstoffatomen oder durch eine Benzyloxycarbonylgruppe ersetzt sein kann, oder auch, falls A an ein Stickstoffatom des Restes C gebunden ist, das nicht Teil einer Lactamgruppe ist, ein Wasserstoffatom oder eine Methylgruppe,

B eine Bindung,

eine Phenylengruppe, die durch eine oder zwei Methylgruppen, durch ein Fluor-, Chlor- oder Bromatom, durch eine Methoxy-, Methylsulfenyl-, Methylsulfinyl-, Methylsulfonyl-, Nitro-, Amino-, Acetylamino-, Benzoylamino- oder Methansulfonylaminogruppe substituiert sein kann,

eine Cycloalkylengruppe mit 3 bis 6 Kohlenstoffatomen,

eine Pyridinylen-, Pyrimidinylen-, Pyrazinylen-, Pyridazinylen-oder Biphenylengruppe,

C eine gegebenenfalls durch eine Hydroxygruppe substituierte Ethylengruppe,

eine über die Carbonylgruppe mit dem Rest B verbundene Methylencarbonylgruppe,

eine Phenylengruppe, die durch eine oder zwei Methylgruppen, durch ein Fluor-, Chlor- oder Bromatom, durch eine Methoxy-, Methylsulfenyl-, Methylsulfinyl-, Methylsulfonyl-, Nitro-, Amino-, Acetylamino-, Benzoylamino- oder Methansulfonylaminogruppe substituiert sein kann,

eine Indanylen- oder 1,2,3,4-Tetrahydronaphthylengruppe, in denen jeweils der gesättigte Ring an den Rest A und der aromatische Ring an das cyclische Harnstoffgerüst gebunden ist,

eine Pyridinylen-, Pyrimidinylen-, Pyrazinylen-, Pyridazinylen-, Cyclohexylen- oder Piperidinylengruppe, wobei das Stickstoffatom nicht an ein Stickstoffatom des cyclischen Harnstoffs gebunden sein kann,

ein zweiter der Reste $R_a$ bis $R_d$ eine Gruppe der Formel

$$F - E - D -,$$

in der

D eine Alkylengruppe mit 1 bis 4 Kohlenstoffatomen,

eine Phenylengruppe, die durch ein Fluor-, Chlor- oder Bromatom, durch eine Methyl-, Methoxy-, Methylsulfenyl-, Methylsulfinyl-, Methylsulfonyl-, Carboxymethoxy-, Methoxycarbonylmethoxy-, Nitro-, Amino-, Acetylamino-, Benzoylamino- oder Methansulfonylaminogruppe substituiert sein kann,

eine Pyridinylen-, Cyclohexylen- oder Piperidinylengruppe, wobei zusätzlich in einer Pyridinylengruppe eine -CH=N-Gruppe durch eine -CO-NH-Gruppe ersetzt sein kann, wobei das Stickstoffatom statt an das Wasserstoffatom auch den Rest E gebunden sein kann, sofern dieser keine Bindung darstellt oder nicht mit einem Heteroatom an den Rest D gebunden ist, oder

eine durch den Rest W unterbrochene Alkylengruppe mit 3 bis 5 Kohlenstoffatomen, in der W ein Sauerstoff- oder Schwefelatom, eine Sulfinyl-, Sulfonyl-, Imino-, Methylimino-, Acetylimino-, Benzoylimino- oder Methansulfonyliminogruppe darstellt und die mit dem cyclischen Harnstoff verknüpfte Alkylengruppe 2 oder 3 Kohlenstoffatome enthält,

E eine Bindung,

eine gegebenenfalls durch eine oder zwei Methylgruppen, durch eine Hydroxy-, Methoxy-, Amino-, Dimethylamino-, Dibenzylamino-, Carboxymethyl- oder Methoxycarbonylmethylgruppe substituierte Alkylengruppe mit ein bis drei Kohlenstoffatomen oder eine Alkenylengruppe mit zwei oder drei Kohlenstoffatomen,

eine Phenylengruppe oder

eine durch den Rest W' mit der Gruppe D verknüpfte Alkylengruppe mit 1 oder 2 Kohlenstoffatomen, in der W' ein Sauerstoff-oder Schwefelatom, eine Sulfinyl-, Sulfonyl- oder Aminocarbonylgruppe, wobei die Aminogruppe an die Alkylengruppe gebunden ist, darstellt, und

F eine Carbonylgruppe, die durch eine Hydroxygruppe, durch eine Alkoxygruppe mit 1 bis 4 Kohlenstoffatomen oder durch eine Phenylalkoxygruppe mit 1 oder 2 Kohlenstoffatomen im Alkoxyteil substituiert ist, eine Phosphono-, O-Methylphosphono- oder Tetrazol-5-yl-gruppe darstellen, wobei, sofern A eine gegebenenfalls am Stickstoffatom benzyloxycarbonylierte Amino- oder Aminoalkylgruppe darstellt, der kürzeste Abstand zwischen dem Stickstoffatom dieser Gruppe und dem Rest F mindestens 10 Bindungen beträgt,

der dritte der Reste $R_a$ bis $R_d$ ein Wasserstoffatom, eine Methyl-, Ethyl- oder Phenylgruppe und

der vierte der Reste $R_a$ bis $R_d$ ein Wasserstoffatom oder eine Methylgruppe bedeuten,

deren Tautomere, deren Stereoisomere einschließlich deren Gemische und deren Additionssalze.

**4.** Cyclische Harnstoffderivate der allgemeinen Formel I gemäß Anspruch 1, in der

X eine Carbonyl- oder Sulfonylgruppe,

Y eine gegebenenfalls durch $R_c$ oder $R_d$ oder $R_c$ und $R_d$ substituierte geradkettige Alkylen- oder Alkenylengruppe mit jeweils 2 oder 3 Kohlenstoffatomen, die durch eine oder zwei Methylgruppen oder durch eine Trifluormethyl- oder Phenylgruppe substituiert sein kann, wobei zusätzlich eine Methylengruppe durch eine Carbonylgruppe ersetzt sein kann, oder eine gegebenenfalls durch $R_c$ oder $R_d$ substituierte -N=CH- oder -CH=N-Gruppe,

einer der Reste $R_a$ bis $R_d$ eine Gruppe der Formel

A - B - C -,

in der

A eine geradkettige oder verzweigte Aminoalkylgruppe mit 1 bis 4 Kohlenstoffatomen, eine Amino- oder Amidinogruppe, wobei in jeder der vorstehend erwähnten Gruppen jeweils an einem der Stickstoffatome ein Wasserstoffatom durch eine Alkoxycarbonylgruppe mit insgesamt 2 bis 5 Kohlenstoffatomen oder durch eine Benzyloxycarbonylgruppe ersetzt sein kann,

B eine Bindung,

eine Phenylengruppe, die durch ein Fluor- oder Chloratom substituiert sein kann,

eine Cyclopropylen-, Pyridinylen-, Pyrimidinylen-, Pyrazinylen-oder Pyridazinylengruppe,

C eine Phenylengruppe, die durch eine oder zwei Methylgruppen, durch ein Fluor-, Chlor- oder Bromatom, durch eine Methoxy-, Methylsulfenyl-, Methylsulfinyl-, Methylsulfonyl-, Amino-, Acetylamino-, Benzoylamino- oder Methansulfonylaminogruppe substituiert sein kann, oder auch, wenn A eine Aminogruppe und B eine Bindung darstellen, eine Indanylen- oder 1,2,3,4-Tetrahydronaphthylengruppe, in denen jeweils der gesättigte Ring an den Rest A und der aromatische Ring an das cyclische Harnstoffgerüst gebunden ist,

eine Pyridinylen-, Pyrimidinylen-, Pyrazinylen-, Pyridazinylen-, Cyclohexylen- oder Piperidinylengruppe, wobei das Stickstoffatom nicht an ein Stickstoffatom des cyclischen Harnstoffs gebunden sein kann,

ein zweiter der Reste $R_a$ bis $R_d$ eine Gruppe der Formel

F - E - D -,

in der

D eine Alkylengruppe mit 1 bis 4 Kohlenstoffatomen,

eine Phenylengruppe, die durch ein Fluor-, Chlor- oder Bromatom, durch eine Methyl-, Methoxy-, Methylsulfenyl-, Methylsulfinyl- oder Methylsulfonylgruppe substituiert sein kann,

eine Pyridinylen-, Cyclohexylen- oder Piperidinylengruppe, wobei zusätzlich in einer Pyridinylengruppe die -CH=N-Gruppe durch eine -CO-NH-Gruppe ersetzt sein kann und das Stickstoffatom statt an das Wasserstoffatom auch an den Rest E gebunden sein kann, sofern dieser keine Bindung darstellt oder nicht mit einem Heteroatom an den Rest D gebunden ist, oder

eine $-CH_2CH_2-N(COCH_3)-CH_2$-Gruppe, in der der Ethylenteil an den cyclischen Harnstoff gebunden ist,

E eine Bindung,

eine gegebenenfalls durch eine oder zwei Methylgruppen oder durch eine Amino- oder Dibenzylaminogruppe substituierte Ethylengruppe,

eine Ethenylen- oder Phenylengruppe oder

eine durch den Rest W' mit der Gruppe D verknüpfte Methylengruppe, in der W' ein Sauerstoff- oder Schwefelatom, eine Sulfinyl- oder Sulfonylgruppe darstellt, und

F eine Carbonylgruppe, die durch eine Hydroxygruppe oder durch eine Alkoxygruppe mit 1 bis 4 Kohlenstoffatomen substituiert ist, eine Phosphono-, O-Methylphosphono- oder Tetrazol-5-yl-Gruppe darstellen, wobei, sofern A eine gegebenenfalls am Stickstoffatom benzyloxycarbonylierte Amino- oder Aminoalkylgruppe darstellt, der kürzeste Abstand zwischen dem Stickstoffatom dieser Gruppe und dem Rest F mindestens 10 Bindungen beträgt,

der dritte der Reste $R_a$ bis $R_d$ ein Wasserstoffatom, eine Methyl-, Ethyl- oder Phenylgruppe und

der vierte der Reste $R_a$ bis $R_d$ ein Wasserstoffatom oder eine Methylgruppe bedeuten,

deren Tautomere, deren Stereoisomere einschließlich deren Gemische und deren Additionssalze.

**5.** Cyclische Harnstoffderivate der allgemeinen Formel I gemäß Anspruch 1, in der

X eine Carbonyl- oder Sulfonylgruppe,

Y eine gegebenenfalls durch $R_c$ oder $R_d$ und gegebenenfalls durch eine Methyl- oder Phenylgruppe substituierte Ethylen- oder Ethenylengruppe oder eine gegebenenfalls durch eine Methylgruppe substituierte Carbonylmethylen- oder Methylencarbonylgruppe, oder eine gegebenenfalls durch $R_c$ oder $R_d$ substituierte -CH=N- oder -N=CH-Gruppe,

einer der Reste $R_a$ bis $R_d$ eine Gruppe der Formel

A - B - C -,

in der

A eine gegebenenfalls durch eine Alkoxycarbonylgruppe mit insgesamt 2 bis 5 Kohlenstoffatomen substituierte Aminomethyl-, Aminoethyl- oder Amidinogruppe,

B eine Bindung oder eine 1,4-Phenylengruppe und

C eine gegebenenfalls durch eine Methylgruppe substituierte 1,4-Phenylengruppe, eine 3,6-Pyridazinylen- oder 1,4-Piperidinylengruppe, wobei jeweils das Stickstoffatom nicht an ein Stickstoffatom des cyclischen Harnstoffs gebunden sein kann,

oder, wenn A eine Aminogruppe und B eine Bindung darstellen, eine Indanylengruppe, in der der gesättigte Ring an den Rest A und der aromatische Ring an das cyclische Harnstoffgerüst gebunden ist,

ein zweiter der Reste $R_a$ bis $R_d$ eine Gruppe der Formel

F - E - D -,

in der

D eine Alkylengruppe mit 1 bis 4 Kohlenstoffatomen, eine 1,4-Phenylen- oder 1,4-Cyclohexylengruppe,

E eine Bindung,

eine gegebenenfalls durch eine Amino- oder Dibenzylaminogruppe substituierte Ethylengruppe oder eine Ethenylengruppe,

eine 1,4-Phenylengruppe oder

eine durch den Rest W' mit der Gruppe D verknüpfte Methylengruppe, in der W' ein Sauerstoff- oder Schwefelatom, eine Sulfinyl- oder Sulfonylgruppe darstellt,

F eine Carbonylgruppe, die durch eine Hydroxygruppe oder durch eine Alkoxygruppe mit 1 bis 4 Kohlenstoffatomen substituiert ist, darstellen, wobei, sofern A eine Aminomethylgruppe darstellt, der kürzeste Abstand zwischen dem Stickstoffatom dieser Gruppe und dem Rest F mindestens 10 Bindungen beträgt,

der dritte der Reste $R_a$ bis $R_d$ ein Wasserstoffatom oder eine Methyl-, Ethyl- oder Phenylgruppe und

der vierte der Reste $R_a$ bis $R_d$ ein Wasserstoffatom oder eine Methylgruppe bedeuten,

deren Tautomere, deren Stereoisomere einschließlich deren Gemische und deren Additionssalze.

**6.** Cyclische Harnstoffderivate der allgemeinen Formel I gemäß Anspruch 1, in der

$R_a$ bis $R_d$, X und Y wie in den Ansprüchen 1 bis 5 definiert sind, und in denen sich zwischen den Anknüpfungsstellen derjenigen der Reste $R_a$ bis $R_d$, die die A-B-C- und F-E-D-Gruppen darstellen, am cyclischen Harnstoff ein weiteres Ringglied befindet,

deren Tautomere, deren Stereoisomere einschließlich deren Gemische und deren Additionssalze.

**7.** Folgende neue Verbindungen der allgemeinen Formel I:

(a) 1-(4-Amidino-phenyl)-3-[4-(2-carboxy-ethyl)-phenyl]-imidazolidin-2-on,

(b) 1-(4-Amidino-phenyl)-3-[4-(2-carboxy-ethyl)-cyclohexyl]-imidazolidin-2-on,

(c) 1-(4-Amidino-phenyl)-3-[4-(2-carboxy-ethyl)-phenyl]-imidazolidin-2,4-dion,

(d) 2-(4-Amidino-phenyl)-5-[4-(2-carboxy-ethyl)-phenyl]-3,4-dihydro-2H,5H-1,2,5-thiadiazol-1,1-dioxid,

(e) 1-(4-Amidino-phenyl)-3-[4-(2-carboxy-ethyl)-phenyl]-3H-imidazol-2-on,

(f) 1-(4-Amidino-phenyl)-3-[4-(2-carboxy-ethyl)-phenyl]-4-methyl-3H-imidazol-2-on,

(g) 2-(4-Amidino-phenyl)-4-[4-(2-carboxy-ethyl)-phenyl]-5-methyl-4H-1,2,4-triazol-3-on,

(h) 2-(4-Amidino-phenyl)-4-[4-(2-carboxy-ethyl)-phenyl]-5-ethyl-4H-1,2,4-triazol-3-on,

(i) 2-(4-Amidino-phenyl)-4-[4-(2-carboxyethyl)-phenyl]-4H-1,2,4-triazol-3-on,

(j) 4-(4-Amidino-phenyl)-2-[4-(2-carboxy-ethyl)-phenyl]-4H-1,2,4-triazol-3-on,

(k) 1-(4-Amidino-phenyl)-3-[4-(2-methoxycarbonyl-ethyl)-phenyl]-4-methyl-3H-imidazol-2-on,

(l) 2-(4-Amidino-phenyl)-4-[4-(2-methoxycarbonyl-ethyl)-phenyl]-5-methyl-4H-1,2,4-triazol-3-on,

(m) 2-(4-Amidino-phenyl)-5-ethyl-4-[4-(2-methoxycarbonyl-ethyl)-phenyl]-4H-1,2,4-triazol-3-on,

(n) 2-(4-Amidino-phenyl)-4-[4-(2-methoxycarbonyl-ethyl)-phenyl]-4H-1,2,4-triazol-3-on,

(o) 2-(4-Methoxycarbonylamidino-phenyl)-3-[4-(2-methoxycarbonyl-ethyl)-phenyl]-5-methyl-4H-1,2,4-triazol-3-on,

(p) 2-(4-Methoxycarbonylamidino-phenyl)-3-[4-(2-methoxycarbonyl-ethyl)-phenyl]-4H-1,2,4-triazol-3-on,

(q) 4-[4-(2-Isobutyloxycarbonyl-ethyl)-phenyl]-2- (4-methoxycarbonylamidino-phenyl)-5-methyl-4H-l,2,4-triazol-3-on und

(r) 2-(4-Amidino-phenyl)-4-[4-(2-isobutyloxycarbonyl-ethyl)-phenyl]-5-methyl-4H-1,2,4-triazol-3-on,

(s) 1-(4-Amidino-phenyl)-3-[4-(2-methoxycarbonyl-ethyl)-cyclohexyl]-imidazolidin-2-on,

(t) 1-(4-Amidino-phenyl)-3-[4-(2-methoxycarbonyl-ethyl)-phenyl]-imidazolidin-2,4-dion,

(u) 1-(4-Amidino-phenyl)-3-[4-(2-isopropyloxycarbonyl-ethyl)-phenyl]-imidazolidin-2,4-dion,

deren Tautomere, deren Stereoisomere einschließlich deren Gemische und deren Additionssalze.

**8.** Physiologisch verträgliche Additionssalze der Verbindungen nach mindestens einem der Ansprüche 1 bis 7 mit anorganischen oder organischen Säuren oder Basen.

**9.** Arzneimittel, enthaltend eine Verbindung der allgemeinen Formel I, in der $R_a$, $R_b$, X und Y mit der Maßgabe wie in den Ansprüchen 1 bis 7 definiert sind, daß B keine Cyano- oder Cyano-$C_{1-4}$-alkylgruppe darstellt, oder ein physiologisch verträgliches Additionssalz hiervon neben gegebenenfalls einem oder mehreren inerten Trägerstoffen und/oder Verdünnungsmitteln.

**10.** Verwendung einer Verbindung der allgemeinen Formel I, in der $R_a$, $R_b$, X und Y mit der Maßgabe wie in den Ansprüchen 1 bis 7 definiert sind, daß B keine Cyano- oder Cyano-$C_{1-4}$-alkylgruppe darstellt, oder ein physiologisch verträgliches Additionssalz hiervon zur Herstellung eines Arzneimittels, das zur Bekämpfung bzw. Verhütung von Krankheiten, bei denen kleinere oder größere Zell-Aggregate auftreten oder Zell-Matrixinteraktionen eine Rolle spielen, geeignet ist.

**11.** Verfahren zur Herstellung eines Arzneimittels gemäß Anspruch 9, dadurch gekennzeichnet, daß auf nichtchemischem Wege eine Verbindung der allgemeinen Formel I, in der $R_a$, $R_b$, X und Y mit der Maßgabe wie in den Ansprüchen 1 bis 7 definiert sind, daß B keine Cyano- oder Cyano-$C_{1-4}$-alkylgruppe darstellt, oder ein physiologisch verträgliches Additionssalz hiervon in einen oder mehrere inerte Trägerstoffe und/oder Verdünnungsmittel eingearbeitet wird.

**12.** Verfahren zur Herstellung der cyclischen Harnstoffderivate gemäß den Ansprüchen 1 bis 8, dadurch gekennzeichnet, daß

a) zur Herstellung von Verbindungen der allgemeinen Formel I, in der F eine Carboxygruppe darstellt, eine Verbindung der allgemeinen Formel

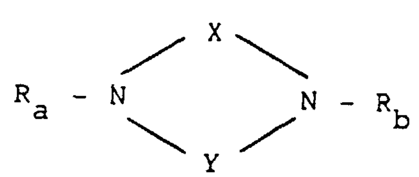

,(II)

in der

$R_a$, $R_b$, X und Y mit der Maßgabe wie in den Ansprüchen 1 bis 7 definiert sind, daß einer der Reste $R_a$ bis $R_d$ eine Gruppe der Formel

$$F' - E - D -$$

darstellt, in der
E und D wie in den Ansprüchen 1 bis 7 definiert sind und
F' eine mittels Hydrolyse, Behandeln mit Säuren, Thermolyse oder Hydrogenolyse in eine Carboxylgruppe überführbare Gruppe bedeutet, in eine Verbindung der allgemeinen Formel I, in der F eine Carboxylgruppe darstellt, umgewandelt wird oder

b) zur Herstellung von Verbindungen der allgemeinen Formel I, in der A eine gegebenenfalls durch eine Alkylgruppe substituierte $H_2N$-C(=NH)-Gruppe darstellt, eine gegebenenfalls im Reaktionsgemisch gebildete Verbindung der allgemeinen Formel

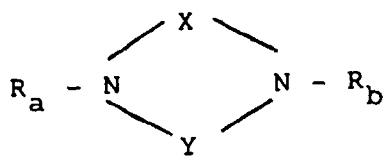

,(III)

in der

$R_a$, $R_b$, X und Y mit der Maßgabe wie in den Ansprüchen 1 bis 7 definiert sind, daß einer der Reste $R_a$ bis $R_d$ eine Gruppe der Formel

$$Z_1 - C(=NH) - B -$$

darstellt, in der
B und C wie in den Ansprüchen 1 bis 7 definiert sind und
$Z_1$ eine Alkoxy-, Aralkoxy-, Alkylthio-, Aralkylthio- oder Aminogruppe darstellt, mit einem Amin der allgemeinen Formel

$$R_4 - NH_2 \quad ,(IV)$$

in der
$R_4$ ein Wasserstoffatom oder eine Alkylgruppe mit 1 bis 4 Kohlenstoffatomen darstellt, oder mit deren Säureadditionssalzen umgesetzt wird oder

c) zur Herstellung von Verbindungen der allgemeinen Formel I, in der mindestens einer der Reste B, C, D oder E eine Sulfinyl- oder Sulfonylgruppe enthält, eine Verbindung der allgemeinen Formel

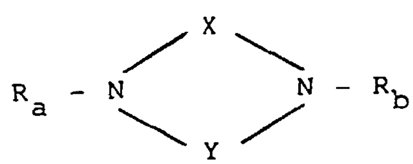

,(V)

in der

$R_a$, $R_b$, X und Y mit der Maßgabe wie in den Ansprüchen 1 bis 7 definiert sind, daß mindestens einer der Reste Y, B, C, D oder E eine Sulfenyl- oder Sulfinylgruppe enthält, oxidiert wird oder

d) zur Herstellung von Verbindungen der allgemeinen Formel I, in der Y eine gegebenenfalls durch $R_c$ oder $R_d$ oder $R_c$ und $R_d$ substituierte geradkettige Alkylengruppe mit 2 bis 4 Kohlenstoffatomen darstellt, die durch Alkyl-, Trifluormethyl-, Aralkyl-, Aryl- oder Heteroarylgruppen mono- oder disubstituiert sein kann, eine Verbindung der allgemeinen Formel

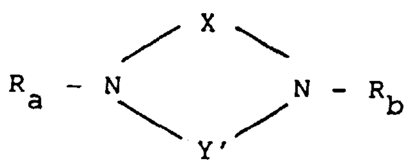

,(VI)

in der

$R_a$, $R_b$ und X wie in den Ansprüchen 1 bis 7 definiert sind und
Y' eine gegebenenfalls durch $R_c$ oder $R_d$ oder $R_c$ und $R_d$ substituierte geradkettige Alkenylengruppe mit 2 bis 4 Kohlenstoffatomen darstellt, die durch Alkyl-, Trifluormethyl-, Aralkyl-, Aryl- oder Heteroarylgruppen mono- oder di- substituiert sein kann, hydriert wird oder

e) zur Herstellung von Verbindungen der allgemeinen Formel I, in der A eine durch eine Alkoxycarbonylgruppe mit insgesamt 2 bis 5 Kohlenstoffatomen, durch eine Aralkoxycarbonyl-, Aryloxycarbonyl-, Alkylcarbonyl- oder Arylcarbonylgruppe substituierte Aminoalkyl-, Amidino- oder Guanidinogruppe darstellt, eine Verbindung der allgemeinen Formel

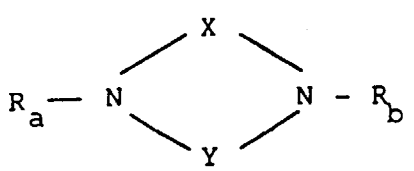

,(VII)

in der

$R_a$, $R_b$, X und Y mit der Maßgabe wie in den Ansprüchen 1 bis 7 definiert sind, daß einer der Reste $R_a$ bis $R_d$ eine Gruppe der Formel

A' - B - C -

darstellt, in der
B und C wie in den Ansprüchen 1 bis 7 definiert sind und
A' eine $H_2N$-$C_{1-5}$alkyl-, $H_2N$-C(=NH)- oder $H_2N$-C(=NH)-NH-Gruppe darstellt, mit einer Verbindung der allgemeinen Formel

$$Z_2 - R_5 \qquad ,(VIII)$$

in der
$R_5$ eine Alkoxycarbonylgruppe mit insgesamt 2 bis 5 Kohlenstoffatomen, eine Aralkoxycarbonyl-, Aryloxycarbonyl-, Alkylcarbonyl- oder Arylcarbonylgruppe und
$Z_2$ eine nukleophile Austrittsgruppe darstellen, umgesetzt wird

f) zur Herstellung von Verbindungen der allgemeinen Formel I, in der F eine durch eine Alkoxygruppe mit 1 bis 6 Kohlenstoffatomen substituierte Carbonylgruppe darstellt, wobei ein Alkoxyrest mit 1 bis 3 Kohlenstoffatomen in 1-, 2- oder 3-Stellung durch eine Aryl- oder Heteroarylgruppe oder in 2- oder 3-Stellung durch eine Pyrrolidin-2-on-1-yl-, Morpholino-, Thiomorpholino- oder 1-Oxido-thiomorpholinogruppe substituiert sein kann, eine Verbindung der allgemeinen Formel

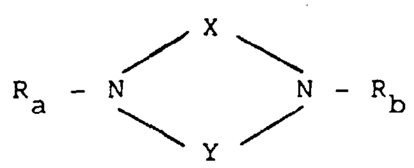

,(IX)

in der

$R_a$, $R_b$, X und Y mit der Maßgabe wie in den Ansprüchen 1 bis 7 definiert sind, daß einer der Reste $R_a$ bis $R_d$ eine Gruppe der Formel

F'' - E - D -

darstellt, in der
E und D wie in den Ansprüchen 1 bis 7 definiert sind und
F'' eine Carboxy- oder Alkoxycarbonylgruppe darstellt, mit einem Alkohol der allgemeinen Formel

$$HO - R_6 \qquad ,(X)$$

in der
$R_6$ eine Alkylgruppe mit 1 bis 6 Kohlenstoffatomen, die in 1-, 2- oder 3-Stellung durch eine Aryl- oder Heteroarylgruppe oder in 2- oder 3-Stellung durch eine Pyrrolidin-2-on-1-yl-, Morpholino-, Thiomorpholino- oder 1-Oxido-thiomorpholinogruppe substituiert sein kann, darstellt, umgesetzt wird oder

g) zur Herstellung von Verbindungen der allgemeinen Formel I, in der A eine $NH_2$-C(=NH)-Gruppe und B oder, falls B eine Bindung darstellt, C eine gegebenenfalls durch eine Alkyl-, Aralkyl- oder Arylgruppe substituierte Cycloalkylengruppe mit 4 oder 5 Kohlenstoffatomen, in der eine CH-Einheit durch ein Stickstoffatom ersetzt ist, oder eine gegebenenfalls durch eine Alkyl-, Aralkyl- oder Arylgruppe substituierte Cycloalkylengruppe mit 6 oder 7 Kohlenstoffatomen, in der eine oder zwei zueinander in 1,4-Stellung befindliche CH-Einheiten jeweils durch ein Stickstoffatom ersetzt sind, wobei B oder, falls B eine Bindung darstellt, C über eines der genannten Stickstoffatome mit dem Rest A verknüpft ist, darstellen, eine Verbindung der allgemeinen Formel

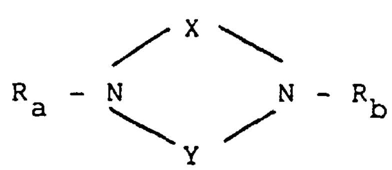

,(XI)

in der

$R_a$, $R_b$, X und Y mit der Maßgabe wie in den Ansprüchen 1 bis 7 definiert sind, daß einer der Reste $R_a$ bis $R_d$ eine Gruppe der Formel

H - B' - C - oder H - C' -

darstellt, in der

C wie in den Ansprüchen 1 bis 7 definiert ist und

B' oder C' eine gegebenenfalls durch eine Alkyl-, Aralkyl- oder Arylgruppe substituierte Cycloalkylengruppe mit 4 oder 5 Kohlenstoffatomen, in der eine CH-Einheit durch ein Stickstoffatom ersetzt ist, oder eine gegebenenfalls durch eine Alkyl-, Aralkyl- oder Arylgruppe substituierte Cycloalkylengruppe mit 6 oder 7 Kohlenstoffatomen, in der eine oder zwei zueinander in 1,4-Stellung befindliche CH-Einheiten jeweils durch ein Stickstoffatom ersetzt sind, wobei das Wasserstoffatom mit einem Stickstoffatom des Restes B' oder C' verknüpft ist, bedeuten, mit einer Verbindung der allgemeinen Formel

$$Z_3\text{-C(=NH)-NH}_2 \qquad ,(XII)$$

in der

$Z_3$ eine nukleophile Austrittsgruppe darstellt, umgesetzt wird oder

h) zur Herstellung von Verbindungen der allgemeinen Formel I, in der A eine $H_2N\text{-}CH_2\text{-}V$-Gruppe, in der V eine Bindung oder eine geradkettige oder verzweigte Alkylengruppe mit 1 bis 4 Kohlenstoffatomen bedeutet, darstellt, eine Verbindung der allgemeinen Formel

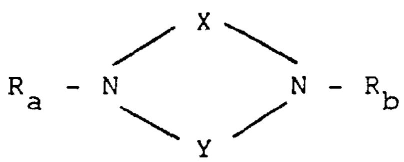

,(XIII)

in der

$R_a$, $R_b$, X und Y mit der Maßgabe wie in den Ansprüchen 1 bis 7 definiert sind, daß einer der Reste $R_a$ bis $R_d$ eine Gruppe der Formel

NC - V - B - C -

darstellt, in der

B und C wie in den Ansprüchen 1 bis 7 definiert sind und

V eine Bindung oder eine geradkettige oder verzweigte Alkylengruppe mit 1 bis 4 Kohlenstoffatomen darstellt, reduziert wird oder

i) zur Herstellung von Verbindungen der allgemeinen Formel I, in der C eine durch eine Hydroxygruppe substituierte Alkylengruppe darstellt, eine Verbindung der allgemeinen Formel

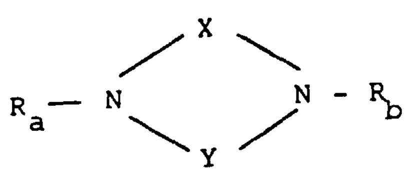

,(XIV)

in der

$R_a$, $R_b$, X und Y mit der Maßgabe wie in den Ansprüchen 1 bis 7 definiert sind, daß einer der Reste $R_a$ bis $R_d$ eine Gruppe der Formel

A - B - C'' -

darstellt, in der
A und B wie in den Ansprüchen 1 bis 7 definiert sind und
C'' eine Alkylengruppe darstellt, in der eine Methylengruppe durch eine Carbonylgruppe ersetzt ist, reduziert wird oder

j) zur Herstellung von Verbindungen der allgemeinen Formel I, in der A eine $H_2N$-C(=NH)-NH-Gruppe darstellt, eine Verbindung der allgemeinen Formel

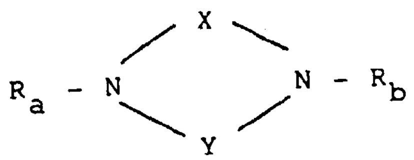
,(XV)

in der

$R_a$, $R_b$, X und Y mit der Maßgabe wie in den Ansprüchen 1 bis 7 definiert sind, daß einer der Reste $R_a$ bis $R_d$ eine Gruppe der Formel

$$H_2N - B - C -$$

darstellt, in der
B und C wie in den Ansprüchen 1 bis 7 definiert sind, mit Cyanamid oder dessen Säureadditionssalz oder mit einem S-Alkyl-isothioharnstoff, O-Methylisothioharnstoff oder 1-Amidino-3,5-dimethylpyrazol umgesetzt wird oder

k) eine Verbindung der allgemeinen Formel

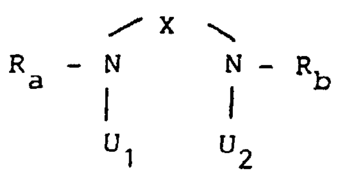
,(XVI)

in der

$R_a$, $R_b$ und X wie in den Ansprüchen 1 bis 7 definiert sind,
einer der Reste $U_1$ oder $U_2$ ein Wasserstoffatom und der andere der Reste $U_1$ oder $U_2$ eine Gruppe der Formel

$$- Y'' - Z_4,$$

in der
Y'' eine gegebenenfalls durch $R_c$ oder $R_d$ oder $R_c$ und $R_d$ substituierte geradkettige Alkylen- oder Alkenylengruppe mit jeweils 2 bis 4 Kohlenstoffatomen, in denen jedes Kohlenstoffatom durch eine Alkyl-, Trifluormethyl-, Aralkyl-, Aryl-, Heteroaryl- oder Alkylcarbonylgruppe mono- oder disubstituiert sein kann, wobei die Substituenten gleich oder verschieden sein können, oder eine gegebenenfalls durch $R_c$ oder $R_d$ oder $R_c$ und $R_d$ substituierte 1,2-Cycloalkylengruppe mit 4 bis 7 Kohlenstoffatomen oder eine 1,2-Cycloalkenylengruppe mit 4 bis 7 Kohlenstoffatomen, eine gegebenenfalls durch die Reste $R_c$ oder $R_d$ substituierte -CH=N-Gruppe, in der der Stickstoffatom mit einem der Stickstoffatome in Formel XVI verknüpft ist, oder eine gegebenenfalls durch $R_c$ oder $R_d$ substituierte $-CH_2$-NH-Gruppe und
$Z_4$ eine nukleophile Austrittsgruppe

oder zusammen mit einer benachbarten Methylengruppe des Restes Y'' eine Carbonyl-, Carboxy-, Alkoxycarbonyl-, Aralkoxycarbonyl-, Aryloxycarbonyl- oder Dialkoxymethylgruppe bedeuten, cyclisiert wird oder

l) zur Herstellung von Verbindungen der allgemeinen Formel I, in der $R_a$ oder $R_c$ eine E-F-D-Gruppe darstellt, eine gegebenenfalls im Reaktionsgemisch gebildeten Verbindung der gemeinen Formel

$$R_c - CO - CHR_d - NR_a - CO - NHR_b \quad ,(XVII)$$

in der
$R_a$ bis $R_d$ wie in den Ansprüchen 1 bis 7 definiert sind, cyclisiert und gegebenenfalls eine so erhaltene Verbindung hydriert wird oder

m) zur Herstellung von Verbindungen der allgemeinen Formel I, in der X eine Carbonylgruppe darstellt, eine Verbindung der allgemeinen Formel

$$R_a - NH - Y - NH - R_b \quad ,(XVIII)$$

in der

$R_a$, $R_b$ und Y wie in den Ansprüchen 1 bis 7 definiert sind, mit einer Verbindung der allgemeinen Formel

$$Z_5 - CO - Z_6 \quad ,(XIX)$$

in der
$Z_5$ und $Z_6$, die gleich oder verschieden sein können, nukleophile Austrittsgruppen darstellen, umgesetzt wird oder

n) zur Herstellung von Verbindungen der allgemeinen Formel I, in der $R_a$ bis $R_d$ wie in den Ansprüchen 1 bis 7 mit der Maßgabe definiert sind, daß mindestens einer der Reste $R_a$ und $R_b$ kein Wasserstoffatom darstellt, eine Verbindung der allgemeinen Formel

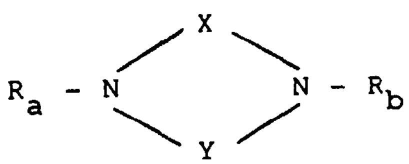

,(XX)

in der

X und Y wie in den Ansprüchen 1 bis 7 definiert sind, einer der Reste $R_a$ oder $R_b$ ein Wasserstoffatom darstellt und
der andere der Reste $R_a$ oder $R_b$ wie in den Ansprüchen 1 bis 7 definiert ist, mit einer Verbindung der allgemeinen Formel

$$Z_7 - R' \quad ,(XXI)$$

in der
R' mit Ausnahme eines Wasserstoffatoms die für $R_a$ oder $R_b$ in den Ansprüchen 1 bis 7 erwähnten Bedeutungen besitzt und $Z_7$ eine nukleophile Austrittsgruppe

oder, falls $R_a$ oder $R_b$ eine -D-COO-alkylgruppe mit der Maßgabe darstellt, daß sich zwischen dem Stickstoffatom des cyclischen Harnstoffs und der Alkoxycarbonylgruppe zwei Kohlenstoffatome befinden, mit einer Verbindung der allgemeinen Formel

$$- D' -COO\text{-alkyl} \qquad ,(XXII)$$

in der
D' die für D in den Ansprüchen 1 bis 7 erwähnten Bedeutungen mit der Maßgabe besitzt, daß der Alkoxycarbonylgruppe unmittelbar eine Kohlenstoff-Kohlenstoff-Doppel- oder Dreifachbindung vorausgeht, umgesetzt wird oder

o) zur Herstellung von Verbindungen der allgemeinen Formel I, in der F eine Carboxy-, Alkoxycarbonyl-, Aralkoxycarbonyl- oder Aryloxycarbonylgruppe darstellt, eine Verbindung der allgemeinen Formel

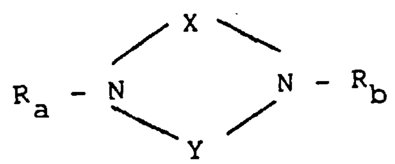

,(XXIII)

in der
$R_a$, $R_b$, X und Y mit der Maßgabe wie in den Ansprüchen 1 bis 7 definiert sind, daß einer der Reste $R_a$ bis $R_d$ eine Gruppe der Formel

$$CH_2 = CH - E - D -$$

darstellt, wobei E und D wie in den Ansprüchen 1 bis 7 definiert sind, oxidiert und gegebenenfalls eine so erhaltene Verbindung verestert wird oder

p) zur Herstellung von Verbindungen der allgemeinen Formel I, in der F eine O-Alkyl-phosphonogruppe darstellt, eine Verbindung der allgemeinen Formel

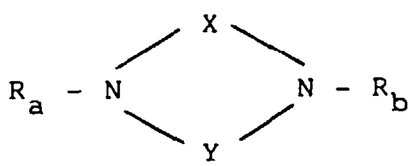

,(XXIV)

in der
$R_a$, $R_b$, X und Y mit der Maßgabe wie in den Ansprüchen 1 bis 7 definiert sind, daß F eine Dialkoxyphosphorylgruppe darstellt, mit einem Alkalijodid umgesetzt wird oder

q) zur Herstellung von Verbindungen der allgemeinen Formel I, in der F eine Phosphonogruppe darstellt, eine Verbindung der allgemeinen Formel

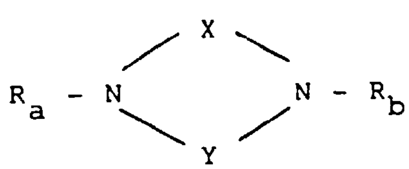

,(XXV)

in der
$R_a$, $R_b$, X und Y mit der Maßgabe wie in den Ansprüchen 1 bis 7 definiert sind, daß F eine O-Alkylphosphono- oder Dialkoxyphosphorylgruppe darstellt, mit einem Alkalijodid in Gegenwart von einem Trialkylhalogensilan umgesetzt wird oder

r) zur Herstellung von Verbindungen der allgemeinen Formel I, in der W eine $R_1$N-Gruppe darstellt, eine Verbindung der allgemeinen Formel

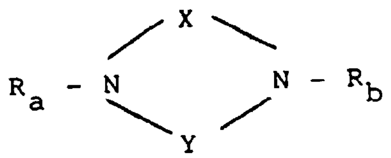
,(XXVI)

in der

$R_a$, $R_b$, X und Y mit der Maßgabe wie in den Ansprüchen 1 bis 7 definiert sind, daß einer der Reste $R_a$ bis $R_d$ eine $Z_8$-D"-Gruppe darstellt, wobei D" eine Alkylengruppe mit 1 bis 3 Kohlenstoffatomen und $Z_8$ eine nukleophile Austrittsgruppe oder einen Sulfonsäureesterrest darstellen, mit einer Verbindung der allgemeinen Formel

$R_1$NH - E' - F ,(XXVII)

in der
F und $R_1$ wie in den Ansprüchen 1 bis 7 definiert sind und
E' eine Alkylengruppe mit 1 bis 3 Kohlenstoffatomen bedeutet, umgesetzt wird oder

s) zur Herstellung von Verbindungen der allgemeinen Formel I, in der A eine Amino- oder Aminoalkylgruppe darstellt, eine Verbindung der allgemeinen Formel

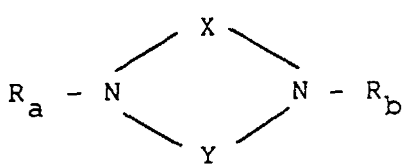
,(XXVIII)

in der

$R_a$, $R_b$, X und Y mit der Maßgabe wie in den Ansprüchen 1 bis 7 definiert sind, daß einer der Reste $R_a$ bis $R_d$ eine $H_2$N-CO-T-B-C-Gruppe darstellt, wobei B und C wie in den Ansprüchen 1 bis 7 definiert sind und
T eine Bindung oder eine Alkylengruppe mit 1 bis 5 Kohlenstoffatomen darstellt, mit einer Phenyljod(III) verbindung der allgemeinen Formel

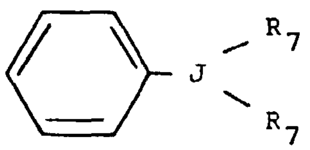
,(XXIX)

in der
$R_7$ jeweils den Acylrest einer organischen Carbonsäure darstellt umgesetzt wird oder

t) zur Herstellung von Verbindungen der allgemeinen Formel I, in der A eine durch eine oder zwei Alkylgruppen am Stickstoffatom substituierte Amino- oder Aminoalkylgruppe darstellt, eine Verbindung der allgemeinen Formel

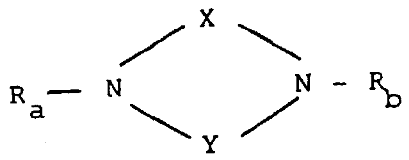
,(XXX)

in der

$R_a$, $R_b$, X und Y mit der Maßgabe wie in den Ansprüchen 1 bis 7 definiert sind, daß einer der Reste $R_a$ bis $R_d$ eine Gruppe der Formel

A'' - B - C -

darstellt, in der
B und C wie in den Ansprüchen 1 bis 7 definiert sind und
A'' eine Amino-, Alkylamino-, Aminoalkyl- oder Alkylaminoalkylgruppe darstellt, mit einer Verbindung der allgemeinen Formel

$$Z_9 - (R_8\text{-}C\text{-}R_9) - Z_{10} \qquad ,(XXXI)$$

in der
$R_8$ und $R_9$, die gleich oder verschieden sein können, Wasserstoffatome oder Alkylgruppen,
eine der Gruppen $Z_9$ oder $Z_{10}$ eine nukleophile Austrittsgruppe und
die andere der Gruppen $Z_9$ oder $Z_{10}$ ein Wasserstoffatom oder eine Alkylgruppe oder
$Z_9$ und $Z_{10}$ zusammen ein Sauerstoffatom bedeuten, umgesetzt wird oder

u) zur Herstellung von Verbindungen der allgemeinen Formel I, in der A eine Cyanogruppe darstellt, eine Verbindung der allgemeinen Formel

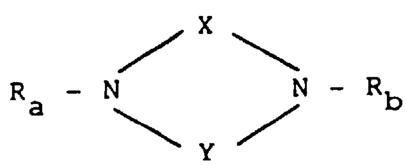
,(XXXII)

in der

$R_a$, $R_b$, X und Y mit der Maßgabe wie in den Ansprüchen 1 bis 7 definiert sind, daß einer der Reste $R_a$ bis $R_d$ eine Gruppe der Formel

A''' - B - C -

darstellt, in der
B und C wie in den Ansprüchen 1 bis 7 definiert sind und
A''' ein Halogenatom darstellt, mit Kupfer(I)cyanid umgesetzt wird oder

v) zur Herstellung von Verbindungen der allgemeinen Formel I, in der A eine Aminoalkylgruppe, in der die Aminogruppe nicht an ein quartäres Kohlenstoffatom gebunden ist, oder eine Aminogruppe darstellt, die an eine CH- oder $CH_2$-Gruppe des Restes B oder C gebunden ist, darstellt, eine Verbindung der allgemeinen Formel

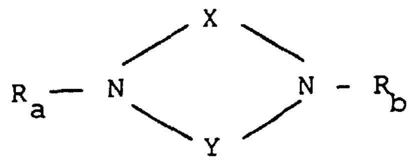
,(XXXIII)

in der

$R_a$, $R_b$, X und Y mit der Maßgabe wie in den Ansprüchen 1 bis 7 definiert sind, daß einer der Reste $R_a$ bis $R_d$ eine Gruppe der Formel

A'''' - B - C -

darstellt, in der
B und C wie in den Ansprüchen 1 bis 7 definiert sind und
A'''' eine N-Hydroxy-iminogruppe enthält, reduziert wird und

gewünschtenfalls anschließend eine so erhaltene Verbindung der allgemeinen Formel I mittels Bromierung in eine entsprechende Bromverbindung der allgemeinen Formel I übergeführt wird oder

eine so erhaltene Verbindung der allgemeinen Formel I mittels Nitrierung in eine entsprechende Nitroverbindung der allgemeinen Formel I übergeführt wird oder

eine so erhaltene Verbindung der allgemeinen Formel I, die eine Nitrogruppe enthält, mittels Reduktion in eine entsprechende Aminoverbindung übergeführt wird oder

eine so erhaltene Verbindung der allgemeinen Formel I, die eine $R_1$NH-Gruppe enthält oder in der W eine Iminogruppe darstellt, mittels Acylierung oder Sulfonierung in eine entsprechende Verbindung der allgemeinen Formel I, die eine durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1$NH-Gruppen enthält, übergeführt wird oder

eine so erhaltene Verbindung der allgemeinen Formel I, in der X eine Carbonylgruppe darstellt, mittels einem Schwefelungsmittel in eine entsprechende Thiocarbonylverbindung übergeführt wird und

erforderlichenfalls ein während der Umsetzungen a) bis v) zum Schutze von reaktiven Gruppen verwendeter Schutzrest abgespalten wird und/oder

gewünschtenfalls eine so erhaltene Verbindung der allgemeinen Formel I in ihre cis-/trans-Isomere, in ihre Enantiomeren und/oder Diastereomeren aufgetrennt wird und/oder

eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

## Claims

**1.** Cyclic urea derivatives of general formula

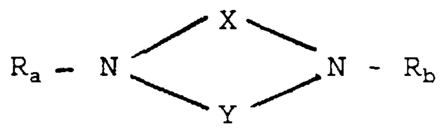

, (I)

wherein

X represents a carbimino group optionally substituted at the nitrogen atom by an alkyl, aralkyl, aryl, heteroaryl or cyano group, or a carbonyl, thiocarbonyl, sulphinyl or sulphonyl group,

Y represents a straight-chained $C_{2-4}$-alkylene or alkenylene group optionally substituted by $R_c$ or $R_d$ or $R_c$ and $R_d$, wherein every carbon atom may be mono- or disubstituted by a fluorine, chlorine or bromine atom or by an alkyl, trifluoromethyl, aralkyl, aryl, heteroaryl or alkylcarbonyl group, whilst the substituents may be identical or different and, in addition, one or two methylene groups may each be replaced by a carbonyl group, or a 1,2-cycloalkylene group having 4 to 7 carbon atoms optionally substituted by $R_c$ or $R_d$ or $R_c$ and $R_d$,

a 1,2-cycloalkenylene group having 4 to 7 carbon atoms or a 1,2-phenylene group in which one or two methine groups may be substituted by a nitrogen atom and which may be substituted in the carbon skeleton by a fluorine, chlorine or bromine atom, by a $C_{1-4}$-alkyl group, by a trifluoromethyl, hydroxy, alkoxy, alkylsulphenyl, alkylsulphinyl, alkylsulphonyl, alkylcarbonyl, arylcarbonyl, alkoxycarbonyl, carboxy, nitro, $(R_1)_2N$-, $(R_1)_2NCO$- or $(R_1)_2NSO_2$ group, wherein the groups $R_1$ may be identical or different and may each represent a hydrogen atom or an alkyl, aralkyl, aryl or heteroaryl group, or by a $R_1NH$ group substituted by an alkylcarbonyl, arylcarbonyl, aralkylcarbonyl, heteroarylcarbonyl, alkylsulphonyl, aralkylsulphonyl or arylsulphonyl group, and wherein, additionally, one or two -CH=CH groups may each be replaced by a $-CO-NR_1$ group,

a -CO-NH-, -NH-CO-, -CH=N- or -N=CH- group optionally substituted by $R_c$ or $R_d$,

one of the groups $R_a$ to $R_d$ represents a group of formula

A - B - C -,

wherein

A represents a straight-chained or branched $C_{1-5}$-aminoalkyl group, an amino, amidino or guanidino group, whilst in each of the above-mentioned groups at one of the nitrogen atoms, one or two hydrogen atoms may be replaced by a $C_{1-4}$-alkyl or one hydrogen atom may be replaced by a $C_{2-5}$-alkoxycarbonyl group or by an alkylcarbonyl, arylcarbonyl, aryloxycarbonyl or aralkoxycarbonyl group, or A may represent a cyano group, a cyanoalkyl group having 1 to 4 carbon atoms in the alkyl moiety or, if A is bound to a nitrogen atom of groups B or C which is not part of a lactam group, it may represent a hydrogen atom or an alkyl group,

B represents a bond,

an alkylene or alkenylene group,

a phenylene group which may be mono- or disubstituted by fluorine, chlorine or bromine atoms, by $C_{1-4}$-alkyl groups, by trifluoromethyl, hydroxy, alkoxy, alkylsulphenyl, alkylsulphinyl, alkylsulphonyl, $(R_1)_2N$-, $(R_1)_2NCO$-, $(R_1)_2NSO_2$- or nitro groups or by $R_1NH$ groups substituted by an alkylcarbonyl, aralkylcarbonyl, arylcarbonyl, heteroarylcarbonyl, alkylsulphonyl, aralkylsulphonyl or arylsulphonyl group, whilst the substituents may be identical or different,

a pyridinylene, pyrimidinylene, pyrazinylene, pyridazinylene or triazinylene group, which may be alkyl-substituted in the carbon skeleton, whilst additionally one or two -CH=N-groups may be replaced by a $-CO-NR_1$ group and one of the nitrogen atoms, instead of being bound to the group $R_1$, may also be bound to the group C, provided that the latter is not attached to group B by a heteroatom or a carbonyl group,

a cyclopropylene group optionally substituted by an alkyl, aralkyl or aryl group,

a $C_{4-5}$-cycloaykylene group optionally substituted by an alkyl, aralkyl or aryl group, wherein a CH-unit may be replaced by a nitrogen atom and additionally a methylene group adjacent to the nitrogen atom may be replaced by a carbonyl group,

a $C_{6-7}$-cycloalkylene group optionally substituted by an alkyl, aralkyl or aryl group, wherein one or two CH-units in the 1,4-position relative to one another may each be replaced by a nitrogen atom, whilst additionally one or two of the methylene groups adjacent to a nitrogen atom may be replaced by a carbonyl group,

a biphenylene group which may be mono- or disubstituted by fluorine, chlorine or bromine atoms or by alkyl, trifluoromethyl, hydroxy, alkoxy, alkylsulphenyl, alkylsulphinyl, alkylsulphonyl, alkylcarbonyl-$NR_1$ or alkylsulphonyl-$NR_1$ groups, whilst the substituents may be identical or different and $R_1$ is as hereinbefore defined, and

C represents an alkylene or alkenylene group optionally substituted by a hydroxy, alkoxy or $(R_1)_2N$-group,

an alkylenecarbonyl group connected to the group B via the carbonyl group,

a phenylene group which may be mono- or disubstituted by fluorine, chlorine or bromine atoms, by $C_{1-4}$-alkyl groups, by trifluoromethyl, hydroxy, alkoxy, alkylsulphenyl, alkylsulphinyl, alkylsulphonyl, $(R_1)_2N$-, $(R_1)_2NCO$-, $(R_1)_2NSO_2$- or nitro groups or by $R_1NH$-groups substituted by an alkylcarbonyl, aralkylcarbonyl, arylcarbonyl, heteroarylcarbonyl, alkylsulphonyl, aralkylsulphonyl or arylsulphonyl group, whilst the substituents may be identical or different,

an indanylene or 1,2,3,4-tetrahydronaphthylene group, wherein in each case the saturated ring is bound to the group A and the aromatic ring is bound to the cyclic urea skeleton,

a pyridinylene, pyrimidinylene, pyrazinylene, pyridazinylene or triazinylene group which may be substituted in the carbon skeleton by an alkyl group, whilst additionally one or two -CH=N-groups may each be replaced by a -CO-$NR_1$-group and one of the nitrogen atoms, instead of being bound to the group $R_1$, may also be bound to the group B, provided that the latter is not a bond or does not adjoin the group C with a heteroatom,

a $C_{4-5}$-cycloalkylene group optionally substituted by an alkyl, aralkyl or aryl group wherein a CH-unit may be replaced by a nitrogen atom and in addition a methylene group adjacent to the nitrogen atom may be replaced by a carbonyl group, or

a $C_{6-7}$-cycloalkylene group optionally substituted by an alkyl, aralkyl or aryl group, wherein one or two CH-units located in the 1,4-position relative to each other may each be replaced by a nitrogen atom, whilst additionally one or two of the methylene groups adjacent to a nitrogen atom may be replaced by a carbonyl group,

a second of the groups $R_a$ to $R_d$ represents a group of the formula

F - E - D -,

wherein

D represents a $C_{1-5}$-alkylene group or a $C_{2-5}$-alkenylene group,

a phenylene group which may be mono- or disubstituted by fluorine, chlorine or bromine atoms, by $C_{1-4}$-alkyl groups, by trifluoromethyl, hydroxy, alkoxy, alkylsulphenyl, alkylsulphinyl, alkylsulphonyl, carboxyalkoxy, alkoxycarbonylalkoxy, aralkoxycarbonylalkoxy, $(R_1)_2N$-, $(R_1)_2NCO$-, $(R_1)_2NSO_2$- or nitro groups or by $R_1NH$-groups substituted by an alkylcarbonyl, aralkylcarbonyl, arylcarbonyl, heteroarylcarbonyl, alkylsulphonyl, aralkylsulphonyl or arylsulphonyl group, the substituents being identical or different,

a pyridinylene, pyrimidinylene, pyrazinylene, pyridazinylene or triazinylene group which may be alkyl-substituted in the carbon skeleton, whilst additionally one or two -CH=N-groups may be replaced by a $-CO-NR_1$-group and one of the nitrogen atoms, instead of being bound to the group $R_1$, may also be bound to the group E, provided that the latter is not a bond or is not bound to the group D by means of a heteroatom,

a $C_{4-5}$-cycloalkylene group optionally substituted by an alkyl, aralkyl or aryl group, wherein a CH-unit may be replaced by a nitrogen atom and in addition a methylene group adjacent to the nitrogen atom may be replaced by a carbonyl group,

a $C_{6-7}$-cycloalkylene group optionally substituted by an alkyl, aralkyl or aryl group, wherein one or two CH-units in the 1,4-position relative to each other may each be replaced by a nitrogen atom, whilst additionally one or two of the methylene groups adjacent to the nitrogen atom may be replaced by a carbonyl group, or

a $C_{2-6}$-alkylene group interrupted by the group W, wherein W represents an oxygen or sulphur atom, a sulphinyl, sulphonyl, $R_1N$, (alkylcarbonyl)N-, (aralkylcarbonyl)N-, (arylcarbonyl)N-, (heteroarylcarbonyl) N-, (alkylsulphonyl)N-, (arylsulphonyl)N-, aminocarbonyl or carbonylamino group,

E represents a bond,

a $C_{1-5}$-alkylene group or a $C_{2-5}$-alkenylene group, each of which may be substituted by one or two alkyl groups, a hydroxy, alkoxy, amino, alkylamino, aralkylamino, dialkylamino, bis(aralkyl)amino, carboxyalkyl, alkoxycarbonylalkyl or aralkoxycarbonylalkyl group,

a phenylene group which may be mono- or disubstituted by fluorine, chlorine or bromine atoms, by $C_{1-4}$-alkyl groups, by trifluoromethyl, hydroxy, alkoxy, alkylsulphenyl, alkylsulphinyl, alkylsulphonyl, $(R_1)_2N$-, $(R_1)_2NCO$-, $(R_1)_2NSO_2$- or nitro groups or by $R_1NH$-groups substituted by an alkylcarbonyl, aralkylcarbonyl, arylcarbonyl, heteroarylcarbonyl, alkylsulphonyl, aralkylsulphonyl or arylsulphonyl group, the substituents being identical or different,

a pyridinylene, pyrimidinylene, pyrazinylene, pyridazinylene or triazinylene group which may be alkyl-substituted in the carbon skeleton, whilst additionally one or two -CH=N-groups may each be replaced by a $-CO-NR_1$-group and one of the nitrogen atoms, instead of being bound to the group $R_1$, may also be bound to the group D,

a $C_{4-5}$-cycloalkylene group optionally substituted by an alkyl, aralkyl or aryl group, wherein a CH-unit may be replaced by a nitrogen atom and in addition a methylene group adjacent to the nitrogen atom may be replaced by a carbonyl group,

a $C_{6-7}$-cycloalkylene group optionally substituted by an alkyl, aralkyl or aryl group, wherein one or two CH-units in the 1,4-position relative to each other may each be replaced by a nitrogen atom, whilst additionally one or two of the methylene groups adjacent to a nitrogen atom may be replaced by a carbonyl group,

an alkylenearylene group linked to the group D via the aryl moiety or

an alkylene group linked to the group D via the group W, where W is as hereinbefore defined, and

F represents a carbonyl group substituted by a hydroxy or $C_{1-6}$-alkoxy group, whilst a $C_{1-3}$-alkoxy group may be substituted in the 1-, 2- or 3-position by an aryl or heteroaryl group or in the 2- or 3-position by a pyrrolidin-2-on-1-yl, morpholino, thiomorpholino or 1-oxido-thiomorpholino group, or F may represent a sulpho, phosphono, O-alkylphosphono or tetrazol-5-yl group, whilst if A represents a cyano group or an amino or aminoalkyl group optionally benzoylated or benzyloxycarbonylated at the nitrogen atom, the shortest spacing between the nitrogen atom of these groups and group F is at least 10 bonds,

the third of the groups $R_a$ to $R_d$ represents a hydrogen atom, an alkyl, aralkyl, aryl or heteroaryl group or, if the third of the groups $R_a$ to $R_d$ is connected to an unsaturated carbon atom of group Y, it may represent

an alkoxy, alkylsulphenyl or $(R_1)_2N$-group and

the fourth of the groups $R_a$ to $R_d$ represents a hydrogen atom, an alkyl, aralkyl, aryl or heteroaryl group or $R_a$ or $R_b$ together with an adjacent group $R_c$ or $R_d$ may also represent a bond, with the proviso that the A-B-C group cannot represent a 4-piperidinyl group if at the same time the F-E-D group denotes an alkoxycarbonylalkyl group and Y denotes a 1,2-phenylene group substituted by a fluorine, chlorine or bromine atom or an alkyl or trifluoromethyl group,

the tautomers, the stereoisomers including the mixtures thereof and the addition salts thereof,

and, unless otherwise specified,

the above-mentioned alkyl, alkylene, alkenylene or alkoxy moieties may each contain 1 to 3 carbon atoms, and

the term "an aryl group" used above denotes a phenyl group which may be monosubstituted by a trifluoromethyl, carboxy, $(R_1)_2NCO$-, alkoxycarbonyl, alkylcarbonyl, alkylsulphenyl, alkylsulphinyl, alkylsulphonyl, nitro, $(R_1)_2N$-, alkylcarbonyl-$NR_1$, aralkylcarbonyl-$NR_1$, arylcarbonyl-$NR_1$, heteroarylcarbonyl-$NR_1$, alkylsulphonyl-$NR_1$, aralkylsulphonyl-$NR_1$, arylsulphonyl-$NR_1$ or $(R_1)_2N$-sulphonyl group or may be mono-, di- or trisubstituted by fluorine, chlorine or bromine atoms or by hydroxy, alkoxy or alkyl groups having 1 to 4 carbon atoms, and

the term "a heteroaryl group" used above denotes a 5-membered heteroaromatic ring which contains an oxygen, sulphur or nitrogen atom, a nitrogen atom and an oxygen, sulphur or nitrogen atom or two nitrogen atoms and an oxygen, sulphur or nitrogen atom or a 6-membered heteroaromatic ring which contains one, two or three nitrogen atoms and in which, additionally, one or two -CH=N-groups may be replaced by a -CO-$NR_1$-group, whilst the above-mentioned heteroaromatic rings may additionally be substituted by one or two alkyl groups or by a fluorine, chlorine or bromine atom or by a hydroxy or alkoxy group.

**2.** Cyclic urea derivatives of general formula I according to claim 1 wherein

X represents a carbimino group optionally substituted at the nitrogen atom by an alkyl, aralkyl, aryl, heteroaryl or cyano group, or a carbonyl, thiocarbonyl, sulphinyl or sulphonyl group,

Y represents a straight-chained $C_{2-3}$-alkylene or alkenylene group optionally substituted by $R_c$ or $R_d$ or $R_c$ and $R_d$, which may be mono- or disubstituted by a fluorine, chlorine or bromine atom or by an alkyl, trifluoromethyl, aralkyl, aryl, heteroaryl or alkylcarbonyl group, whilst the substituents may be identical or different and, in addition, one or two methylene groups may be replaced by a carbonyl group, or a 1,2-cyclohexylene group optionally substituted by $R_c$ or $R_d$ or $R_c$ and $R_d$,

a 1,2-cyclohexenylene group or a 1,2-phenylene group wherein one or two CH-groups may each be replaced by a nitrogen atom and which may be substituted in the carbon skeleton by a fluorine, chlorine or bromine atom, by a $C_{1-4}$-alkyl group, by a trifluoromethyl, hydroxy, alkoxy, alkylsulphenyl, alkylsulphinyl, alkylsulphonyl, alkylcarbonyl, arylcarbonyl, alkoxycarbonyl, carboxy, nitro, $(R_1)_2N$-, $(R_1)_2NCO$- or $(R_1)_2NSO_2$-group, wherein the groups $R_1$ may be identical or different and may each represent a hydrogen atom, an alkyl, aralkyl, aryl or heteroaryl group, or by a $R_1NH$-group substituted by an alkylcarbonyl, arylcarbonyl, aralkylcarbonyl, heteroarylcarbonyl, alkylsulphonyl, aralkylsulphonyl or arylsulphonyl group, and wherein, additionally, one or two -CH=CH-groups may each be replaced by a -CO-$N_1$-group,

a -CO-NH-, -NH-CO-, -CH=N- or -N=CH-group optionally substituted by $R_c$ or $R_d$,

one of the groups $R_a$ to $R_d$ represents a group of the formula

$$A - B - C -,$$

wherein

A represents a straight-chained or branched $C_{1-5}$-aminoalkyl group, an amino, amidino or guanidino group, whilst in each of the above-mentioned groups, at one of the nitrogen atoms, one or two hydrogen atoms may be replaced by a $C_{1-4}$-alkyl group or a hydrogen atom may be replaced by a $C_{2-5}$-alkoxycarbonyl group, by an alkylcarbonyl, arylcarbonyl, aryloxycarbonyl or aralkoxycarbonyl group, a cyano group, a cyanoalkyl group having 1 to 4 carbon atoms in the alkyl moiety or, if A is bound to a nitrogen atom of groups B or C which is not part of a lactam group, it may also represent a hydrogen atom or an alkyl group,

B represents a bond,

an alkylene or alkenylene group,

a phenylene group which may be mono- or disubstituted by fluorine, chlorine or bromine atoms, by $C_{1-4}$-alkyl groups, by trifluoromethyl, hydroxy, alkoxy, alkylsulphenyl, alkylsulphinyl, alkylsulphonyl, $(R_1)_2N$-, $(R_1)_2NCO$-, $(R_1)_2NSO_2$- or nitro groups or by $R_1NH$-groups substituted by an alkylcarbonyl, aralkylcarbonyl, arylcarbonyl, heteroarylcarbonyl, alkylsulphonyl, aralkylsulphonyl or arylsulphonyl group, whilst the substituents may be identical or different,

a pyridinylene, pyrimidinylene, pyrazinylene or pyridazinylene group which may be substituted in the carbon skeleton by an alkyl group, whilst additionally one or two -CH=N-groups may be replaced by a -CO-$NR_1$-group and one of the nitrogen atoms, instead of being bound to the group $R_1$, may also be bound to the group C, provided that the latter does not adjoin the group B with a heteroatom or a carbonyl group,

a $C_{3-5}$-cycloalkylene group,

a cyclohexylene group wherein one or two CH-units in the 1,4-position relative to each other may be replaced by nitrogen atoms, whilst additionally one or two of the methylene groups adjacent to a nitrogen atom may be replaced by a carbonyl group, or

a biphenylene group and

C represents an alkylene or alkenylene group optionally substituted by a hydroxy group,

an alkylenecarbonyl group connected to the group B via the carbonyl group,

a phenylene group which may be mono- or disubstituted by fluorine, chlorine or bromine atoms, by $C_{1-4}$-alkyl groups, by trifluoromethyl, hydroxy, alkoxy, alkylsulphenyl, alkylsulphinyl, alkylsulphonyl, $(R_1)_2N$-, $(R_1)_2NCO$-, $(R_1)_2NSO_2$- or nitro groups or by $R_1NH$-groups substituted by an alkylcarbonyl, aralkylcarbonyl, arylcarbonyl, heteroarylcarbonyl, alkylsulphonyl, aralkylsulphonyl or arylsulphonyl group, the substituents being identical or different,

an indanylene or 1,2,3,4-tetrahydronaphthylene group wherein, in each case, the saturated ring is bound to the group A and the aromatic ring is bound to the cyclic urea skeleton,

a pyridinylene, pyrimidinylene, pyrazinylene or pyridazinylene group, each of which may be substituted in the carbonyl skeleton by an alkyl group, whilst additionally one or two -CH=N-groups may each be replaced by a -CO-$NR_1$-group and one of the nitrogen atoms, instead of being bound to the group $R_1$, may also be bound to the group B, provided that the latter does not represent a bond or is not adjacent to the group C with a heteroatom,

a cyclohexylene group wherein one or two CH-units in the 1,4-position relative to each other may be replaced by nitrogen atoms, whilst additionally one or two of the methylene group adjacent to a nitrogen atom may be replaced by a carbonyl group and the nitrogen atoms may not be bound to a nitrogen atom of the cyclic urea,

a second of the groups $R_a$ to $R_d$ represents a group of the formula

F - E - D -,

wherein

D represents a $C_{1-5}$-alkylene group or a $C_{2-5}$-alkenylene group,

a phenylene group which may be mono- or disubstituted by fluorine, chlorine or bromine atoms, by $C_{1-4}$-alkyl groups, by trifluoromethyl, hydroxy, alkoxy, alkylsulphenyl, alkylsulphinyl, alkylsulphonyl, carboxyalkoxy, alkoxycarbonylalkoxy, aralkoxycarbonylalkoxy, $(R_1)_2N$-, $(R_1)_2NCO$-, $(R_1)_2NSO_2$- or nitro groups or by $R_1NH$-groups substituted by an alkylcarbonyl, aralkylcarbonyl, arylcarbonyl, heteroarylcarbonyl, alkylsulphonyl, aralkylsulphonyl or arylsulphonyl group, the substituents being identical or different,

a pyridinylene, pyrimidinylene, pyrazinylene or pyridazinylene group, each of which may be substituted by an alkyl group in the carbon skeleton, whilst additionally one or two -CH=N-groups may each be replaced by a -CO-$NR_1$-group and one of the nitrogen atoms instead of being bound to the group $R_1$ may also be bound to the group E, provided that the latter does not represent a bond or is not bound by a heteroatom to the group D,

a cyclohexylene group wherein one or two CH-units in the 1,4-position relative to each other may be replaced by nitrogen atoms, whilst additionally one or two of the methylene groups adjacent to a nitrogen atom may be replaced by a carbonyl group, or

a $C_{3-6}$-alkylene group interrupted by the group W, wherein W represents an oxygen or sulphur atom, a sulphinyl, sulphonyl, $R_1N$-, (alkylcarbonyl)N-, (aralkylcarbonyl)N-, (arylcarbonyl)N-, (heteroarylcarbonyl) N-, (alkylsulphonyl)N- or (arylsulphonyl)N-group and the alkylene group linked to a nitrogen atom of the cyclic urea contains 2 or 3 carbon atoms,

E represents a bond,

a $C_{1-5}$-alkylene group or a $C_{2-5}$-alkenylene group, each of which may be substituted by one or two alkyl groups, by a hydroxy, alkoxy, amino, alkylamino, aralkylamino, dialkylamino, bis(aralkyl)amino, carboxyalkyl, alkoxycarbonylalkyl or aralkoxycarbonylalkyl group,

a phenylene group which may be mono- or disubstituted by fluorine, chlorine or bromine atoms, by $C_{1-4}$-alkyl groups, by trifluoromethyl, hydroxy, alkoxy, alkylsulphenyl, alkylsulphinyl, alkylsulphonyl, $(R_1)_2N$-, $(R_1)_2NCO$-, $(R_1)_2NSO_2$- or nitro groups or by $R_1$-NH-groups substituted by an alkylcarbonyl, aralkylcarbonyl, arylcarbonyl, heteroarylcarbonyl, alkylsulphonyl, aralkylsulphonyl or arylsulphonyl group, whilst the substituents may be identical or different,

a pyridinylene, pyrimidinylene, pyrazinylene or pyridazinylene group, each of which may be alkyl-substituted in the carbon skeleton, whilst additionally one or two -CH=N-groups may each be replaced by a -CO-$NR_1$-group and one of the nitrogen atoms, instead of being bound to the group $R_1$, may also be bound to the group D,

a cyclohexylene group wherein one or two CH-units in the 1,4-position relative to each other may be replaced by nitrogen atoms, whilst additionally one or two of the methylene groups adjacent to a nitrogen atom may be replaced by a carbonyl group,

an alkylenearylene group linked to the group D via the aryl group or

an alkylene group linked to the group D via the group W', wherein W' represents an oxygen or sulphur atom, a sulphinyl, sulphonyl, $R_1N$-, (alkycarbonyl) N-, (aralkylcarbonyl)N-, (arylcarbonyl)N-, (heteroarylcarbonyl)N-, (alkylsulphonyl)N-, (arylsulphonyl)N- or aminocarbonyl group wherein the nitrogen atom is bound to the alkylene group,

F represents a carbonyl group which is substituted by a hydroxy or $C_{1-6}$-alkoxy group, whilst a $C_{1-3}$-alkoxy group may be substituted in the 1-, 2- or 3-position by an aryl or heteroaryl group or in the 2- or 3-position by a pyrrolidin-2-on-1-yl, morpholino, thiomorpholino or 1-oxido-thiomorpholino group, or F may represent a sulpho, phosphono, O-alkylphosphono or tetrazol-5-yl group, whilst if A represents a cyano group or an amino or aminoalkyl group optionally benzoylated or benzyloxycarbonylated at the nitrogen atom, the shortest distance between the nitrogen atom of these groups and the group F is at least 10 bonds,

the third of the groups $R_a$ to $R_d$ is a hydrogen atom, an alkyl, aralkyl, aryl or heteroaryl group or, if the third of the groups $R_a$ to $R_d$ is bound to an unsaturated carbon atom of group Y, it may represent an alkoxy, alkylsulphenyl or $(R_1)_2$N-group and

the fourth of groups $R_a$ to $R_d$ represents a hydrogen atom or an alkyl, aralkyl, aryl or heteroaryl group, or $R_a$ or $R_b$ together with an adjacent group $R_c$ or $R_d$ also denotes a bond, with the proviso that the A-B-C group cannot represent a 4-piperidinyl group if at the same time the F-E-D group denotes an alkoxycarbonylalkyl group and Y denotes a 1,2-phenylene group substituted by a fluorine, chlorine or bromine atom or an alkyl or trifluoromethyl group,

the tautomers, the stereoisomers including the mixtures thereof and the addition salts thereof.

**3.** Cyclic urea derivatives of general formula I according to claim 1, wherein

X represents a carbimino group optionally substituted at the nitrogen atom by a methyl, phenyl or pyridyl group, or a carbonyl, thiocarbonyl or sulphonyl group,

Y represents a straight-chained alkylene or alkenylene group, each having 2 or 3 carbon atoms, optionally substituted by $R_c$ or $R_d$ or $R_c$ and $R_d$, which may be substituted by a chlorine atom, by one or two methyl groups or by a trifluoromethyl, phenyl or acetyl group, whilst additionally a methylene group may be replaced by a carbonyl group,

a -CO-NH-, -NH-CO-, -CH=N- or -N=CH-group, optionally substituted by $R_c$ or $R_d$, or a 1,2-phenylene or 2,3-pyridinylene group,

one of the groups $R_a$ to $R_d$ represents a group of formula

A - B - C -,

wherein

A represents a straight-chained or branched $C_{1-4}$-aminoalkyl group, an amino-, amidino or guanidino group, whilst in each of the above-mentioned groups, at one of the nitrogen atoms, one or two hydrogen atoms may each be replaced by a $C_{1-4}$-alkyl group or a hydrogen atom may be replaced by an alkoxycarbonyl group having a total of 2 to 5 carbon atoms or by a benzyloxycarbonyl group, or, if A is bound to a nitrogen atom of group C which is not part of a lactam group, it may represent a hydrogen atom or a methyl group,

B represents a bond,

a phenylene group which may be substituted by one or two methyl groups, by a fluorine, chlorine or bromine atom, by a methoxy, methylsulphenyl, methylsulphinyl, methylsulphonyl, nitro, amino, acetylamino, benzoylamino or methanesulphonylamino group,

a $C_{3-6}$-cycloalkylene group,

a pyridinylene, pyrimidinylene, pyrazinylene, pyridazinylene or biphenylene group,

C represents an ethylene group optionally substituted by a hydroxy group,

a methylenecarbonyl group linked to the group B via the carbonyl group,

a phenylene group which may be substituted by one or two methyl groups, by a fluorine, chlorine or bromine atom, by a methoxy, methylsulphenyl, methylsulphinyl, methylsulphonyl, nitro, amino, acetylamino, benzoylamino or methanesulphonylamino group,

an indanylene or 1,2,3,4-tetrahydronaphthylene group wherein the saturated ring is bound to the group A and the aromatic ring is bound to the cyclic urea skeleton,

a pyridinylene, pyrimidinylene, pyrazinylene, pyridazinylene, cyclohexylene or piperidinylene group, wherein the nitrogen atom may not be bound to a nitrogen atom of the cyclic urea,

a second of groups $R_a$ to $R_d$ represents a group of formula

F - E - D -,

wherein

D represents a $C_{1-4}$-alkylene group,

a phenylene group which may be substituted by a fluorine, chlorine or bromine atom, by a methyl, methoxy, methylsulphenyl, methylsulphinyl, methylsulphonyl, carboxymethoxy, methoxycarbonylmethoxy, nitro, amino, acetylamino, benzoylamino or methanesulphonylamino group,

a pyridinylene, cyclohexylene or piperidinylene group, whilst additionally in a pyridinylene group a -CH=N-group may be replaced by a -CO-NH-group, whilst the nitrogen atom, instead of being bound to the hydrogen atom, may also be bound to the group E, provided that the latter is not a bond or is not bound by a heteroatom to group D, or

a $C_{3-5}$-alkylene group interrupted by the group W, wherein W represents an oxygen or sulphur atom, a sulphinyl, sulphonyl, imino, methylimino, acetylimino, benzoylimino or methanesulphonylimino group and the alkylene group linked to the cyclic urea contains 2 or 3 carbon atoms,

E represents a bond,

a $C_{1-3}$-alkylene group optionally substituted by one or two methyl groups or by a hydroxy, methoxy, amino, dimethylamino, dibenzylamino, carboxymethyl or methoxycarbonylmethyl group or a $C_{2-3}$-alkenylene group,

a phenylene group or

a $C_{1-2}$-alkylene group linked to group D by the group W', wherein W' represents an oxygen or sulphur atom, a sulphinyl, sulphonyl or aminocarbonyl group, the amino group being bound to the alkylene group, and

F represents a carbonyl group which is substituted by a hydroxy group, by a $C_{1-4}$-alkoxy group or by a phenylalkoxy group having 1 or 2 carbon atoms in the alkoxy part, a phosphono, O-methylphosphono or tetrazol-5-yl group, whilst if A represents an amino or aminoalkyl group optionally benzyloxycarbonylated at the nitrogen atom, the shortest distance between the nitrogen atom of this group and group F is at least 10 bonds,

the third of the groups $R_a$ to $R_d$ represents a hydrogen atom, a methyl, ethyl or phenyl group and

the fourth of groups $R_a$ to $R_d$ represents a hydrogen atom or a methyl group,

the tautomers, the stereoisomers, including the mixtures thereof, and the addition salts thereof.

**4.** Cyclic urea derivatives of general formula I according to claim 1, wherein

X represents a carbonyl or sulphonyl group,

Y represents a straight-chained alkylene or alkenylene group each having 2 or 3 carbon atoms, optionally substituted by $R_c$ or $R_d$ or $R_c$ and $R_d$, which may be substituted by one or two methyl groups or by a trifluoromethyl or phenyl group, whilst additionally a methylene group may be replaced by a carbonyl group, or an

-N=CH- or -CH=N-group optionally substituted by $R_c$ or $R_d$,
one of the groups $R_a$ to $R_d$ represents a group of the formula

A - B - C -,

wherein

A represents a straight-chained or branched $C_{1-4}$-aminoalkyl group, an amino or amidino group, whilst in each of the above-mentioned groups, at one of the nitrogen atoms, a hydrogen atom may be replaced by an alkoxycarbonyl group having a total of 2 to 5 carbon atoms or by a benzyloxycarbonyl group,

B represents a bond,

a phenylene group which may be substituted by a fluorine or chlorine atom,

a cyclopropylene, pyridinylene, pyrimidinylene, pyrazinylene or pyridazinylene group,

C represents a phenylene group which may be substituted by one or two methyl groups, by a fluorine, chlorine or bromine atom, by a methoxy, methylsulphenyl, methylsulphinyl, methylsulphonyl, amino, acetylamino, benzoylamino or methanesulphonylamino group, or, if A represents an amino group and B represents a bond, an indanylene or 1,2,3,4-tetrahydronaphthylene group, wherein the saturated ring is bound to the group A and the aromatic ring is bound to the cyclic urea skeleton,

a pyridinylene, pyrimidinylene, pyrazinylene, pyridazinylene, cyclohexylene or piperidinylene group, whilst the nitrogen atom may not be bound to a nitrogen atom of the cyclic urea,

a second of the groups $R_a$ to $R_d$ represents a group of formula

F - E - D -,

wherein

D represents a $C_{1-4}$-alkylene group,

a phenylene group which may be substituted by a fluorine, chlorine or bromine atom or by a methyl, methoxy, methylsulphenyl, methylsulphinyl or methylsulphonyl group,

a pyridinylene, cyclohexylene or piperidinylene group, whilst additionally in a pyridinylene group the -CH=N-group may be replaced by a -CO-NH-group and the nitrogen atom, instead of being bound to the hydrogen atom, may also be bound to the group E, provided that the latter is not a bond or is not bound to the group D by a heteroatom, or

a $-CH_2CH_2-N(COCH_3)-CH_2-$group wherein the ethylene moiety is bound to the cyclic urea,

E represents a bond,

an ethylene group optionally substituted by one or two methyl groups or by an amino or dibenzylamino group,

an ethenylene or phenylene group or

a methylene group linked to group D by the group W', wherein W' represents an oxygen or sulphur atom or a sulphinyl or sulphonyl group, and

F represents a carbonyl group which is substituted by a hydroxy group or by a $C_{1-4}$-alkoxy group, or F may represent a phosphono, O-methylphosphono or tetrazol-5-yl group, whilst if A represents an amino or aminoalkyl group optionally benzyloxycarbonylated at the nitrogen atom, the shortest distance between the nitrogen atom of this group and the group F is at least 10 bonds,

the third of the groups $R_a$ to $R_d$ represents a hydrogen atom or a methyl, ethyl or phenyl group and

the fourth of the groups $R_a$ to $R_d$ represents a hydrogen atom or a methyl group,

the tautomers, the stereoisomers including the mixtures thereof and the addition salts thereof.

**5.** Cyclic urea derivatives of general formula I according to claim 1, wherein

X represents a carbonyl or sulphonyl group,

Y represents an ethylene or ethenylene group optionally substituted by $R_c$ or $R_d$ and optionally substituted by a methyl or phenyl group, or a carbonylmethylene or methylenecarbonyl group optionally substituted by a methyl group, or a -CH=N- or -N=CH-group optionally substituted by $R_c$ or $R_d$,
one of the groups $R_a$ to $R_d$ represents a group of formula

A - B - C -,

wherein

A represents an aminomethyl, aminoethyl or amidino group optionally substituted by an alkoxycarbonyl group with a total of 2 to 5 carbon atoms,

B represents a bond or a 1,4-phenylene group and

C represents an optionally methyl-substituted 1,4-phenylene, 3,6-pyridazinylene or 1,4-piperidinylene group, whilst the nitrogen atom may not be bound to a nitrogen atom of the cyclic urea,

or, if A denotes an amino group and B is a bond, C may represent an indanylene group in which the saturated ring is bound to the group A and the aromatic ring is bound to the cyclic urea structure,

a second of the groups $R_a$ to $R_d$ represents a group of the formula

F - E - D -,

wherein

D represents a $C_{1-4}$-alkylene group, a 1,4-phenylene or 1,4-cyclohexylene group,

E represents a bond,

an ethylene group optionally substituted by an amino or dibenzylamino group or an ethenylene group,

a 1,4-phenylene group or

a methylene group linked by the group W' to the group D, wherein W' represents an oxygen or sulphur atom or a sulphinyl or sulphonyl group,

F represents a carbonyl group which may be substituted by a hydroxy group or by a $C_{1-4}$-alkoxy group, whilst if A represents an aminomethyl group, the shortest distance between the nitrogen atom of this group and the group F is at least 10 bonds,

the third of the groups $R_a$ to $R_d$ represents a hydrogen atom or a methyl, ethyl or phenyl group and

the fourth of the groups $R_a$ to $R_d$ represents a hydrogen atom or a methyl group,

the tautomers, the stereoisomers including the mixtures thereof and the addition salts thereof.

**6.** Cyclic urea derivatives of general formula I according to claim 1, wherein

$R_a$ to $R_d$, X and Y are defined as in claims 1 to 5, and wherein there is an additional ring member between the linking points of those groups $R_a$ to $R_d$ which represent the A-B-C- and F-E-D-groups, at the cyclic urea,

the tautomers, the stereoisomers including the mixtures thereof and the addition salts thereof.

**7.** The following new compounds of general formula I:

(a) 1-(4-amidino-phenyl)-3-[4-(2-carboxy-ethyl)-phenyl]-imidazolidin-2-one,

(b) 1-(4-amidino-phenyl)-3-[4-(2-carboxy-ethyl)-cyclohexyl]-imidazolidin-2-one,

(c) 1-(4-amidino-phenyl)-3-[4-(2-carboxy-ethyl)-phenyl]-imidazolidin-2,4-dione,

(d) 2-(4-amidino-phenyl)-5-[4-(2-carboxy-ethyl)-phenyl]-3,4-dihydro-2H,5H-1,2,5-thiadiazol-1,1-dioxide,

(e) 1-(4-amidino-phenyl)-3-[4-(2-carboxy-ethyl)-phenyl]-3H-imidazol-2-one,

(f) 1-(4-amidino-phenyl)-3-[4-(2-carboxy-ethyl)-phenyl]-4-methyl-3H-imidazol-2-one,

(g) 2-(4-amidino-phenyl)-4-[4-(2-carboxy-ethyl)-phenyl]-5-methyl-4H-1,2,4-triazol-3-one,

(h) 2-(4-amidino-phenyl)-4-[4-(2-carboxy-ethyl)-phenyl]-5-ethyl-4H-1,2,4-triazol-3-one,

(i) 2-(4-amidino-phenyl)-4-[4-(2-carboxyethyl)-phenyl]-4H-1,2,4-triazol-3-one,

(j) 4-(4-amidino-phenyl)-2-[4-(2-carboxy-ethyl)-phenyl]-4H-1,2,4-triazol-3-one,

(k) 1-(4-amidino-phenyl)-3-[4-(2-methoxycarbonyl-ethyl)-phenyl]-4-methyl-3H-imidazol-2-one,

(l) 2-(4-amidino-phenyl)-4-[4-(2-methoxycarbonyl-ethyl)-phenyl]-5-methyl-4H-1,2,4-triazol-3-one,

(m) 2-(4-amidino-phenyl)-5-ethyl-4-[4-(2-methoxycarbonyl-ethyl)-phenyl]-4H-1,2,4-triazol-3-one,

(n) 2-(4-amidino-phenyl)-4-[4-(2-methoxycarbonyl-ethyl)-phenyl]-4H-1,2,4-triazol-3-one,

(o) 2-(4-methoxycarbonylamidino-phenyl)-3-[4-(2-methoxycarbonyl-ethyl)-phenyl]-5-methyl-4H-1,2,4-triazol-3-one,

(p) 2-(4-methoxycarbonylamidino-phenyl)-3-[4-(2-methoxycarbonyl-ethyl)-phenyl]-4H-1,2,4-triazol-3-one,

(q) 4-[4-(2-isobutyloxycarbonyl-ethyl)-phenyl]-2-(4-methoxycarbonylamidino-phenyl)-5-methyl-4H-1,2,4-triazol-3-one and

(r) 2-(4-amidino-phenyl)-4-[4-(2-isobutyloxycarbonyl-ethyl)-phenyl]-5-methyl-4H-1,2,4-triazol-3-one,

(s) 1-(4-amidino-phenyl)-3-[4-(2-methoxycarbonyl-ethyl)-cyclohexyl]-imidazolidin-2-one,

(t) 1-(4-amidino-phenyl)-3-[4-(2-methoxycarbonyl-ethyl)-phenyl]-imidazolidin-2,4-dione,

(u) 1-(4-amidino-phenyl)-3-[4-(2-isopropyloxycarbonylethyl)-phenyl]-imidazolidin-2,4-dione,

the tautomers, the stereoisomers including the mixtures thereof and the addition salts thereof.

**8.** Physiologically acceptable addition salts of the compounds according to at least one of claims 1 to 7 with organic or inorganic acids or bases.

**9.** Pharmaceutical compositions containing a compound of general formula I wherein $R_a$, $R_b$, X and Y are defined as in claims 1 to 7 with the proviso that B does not represent a cyano or cyano-$C_{1-4}$-alkyl group, or a physiologically acceptable addition salt thereof, optionally together with one or more inert carriers and/or diluents.

**10.** Use of a compound of general formula I wherein $R_a$, $R_b$, X and Y are defined as in claims 1 to 7 with the proviso that B does not represent a cyano or cyano-$C_{1-4}$-alkyl group, or a physiologically acceptable addition salt thereof, for preparing a pharmaceutical composition which is suitable for treating or preventing diseases in which smaller or larger cell aggregates occur or cell-matrix interactions are involved.

**11.** Process for preparing a pharmaceutical composition according to claim 9, characterised in that a compound of general formula I wherein $R_a$, $R_b$, X and Y are defined as in claims 1 to 7, with the proviso that B does not represent a cyano or cyano-$C_{1-4}$-alkyl group, or a physiologically acceptable addition salt thereof, is incorporated into one or more inert carriers and/or diluents by a non-chemical method.

**12.** Process for preparing the cyclic urea derivatives according to claims 1 to 8, characterised in that

a) in order to prepare compounds of general formula I wherein F represents a carboxy group, a compound of general formula

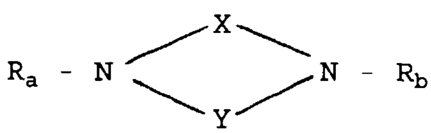

, (II)

wherein

$R_a$, $R_b$, X and Y are defined as in claims 1 to 7 with the proviso that one of the groups $R_a$ to $R_d$ represents a group of the formula

F' - E - D - ,

wherein
E and D are defined as in claims 1 to 7 and
F' represents a group which may be converted into a carboxyl group by hydrolysis, treatment with acids, thermolysis or hydrogenolysis, is converted into a compound of general formula I wherein F represents a carboxyl group or

b) in order to prepare compounds of general formula I wherein A represents an $H_2N$-C(=NH)- group optionally substituted by an alkyl group, a compound of formula

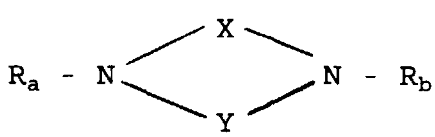

, (III)

optionally formed in the reaction mixture
wherein

$R_a$, $R_b$, X and Y are defined as in claims 1 to 7, with the proviso that one of the groups $R_a$ to $R_d$ represents a group of the formula

$Z_1$ - C(=NH) - B - ,

wherein
B and C are defined as in claims 1 to 7 and
$Z_1$ represents an alkoxy, aralkoxy, alkylthio, aralkylthio or amino group, is reacted with an amine of general formula

$$R_4 - NH_2 \qquad ,(IV)$$

wherein
$R_4$ represents a hydrogen atom or a $C_{1-4}$-alkyl group, or with the acid addition salts thereof, or

c) in order to prepare compounds of general formula I wherein at least one of the groups B, C, D or E represents a sulphinyl or sulphonyl group, a compound of general formula

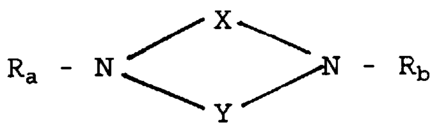
, (V)

wherein
$R_a$, $R_b$, X and Y are defined as in claims 1 to 7, with the proviso that at least one of the groups Y, B, C, D or E contains a sulphenyl or sulphinyl group, is oxidised or

d) in order to prepare compounds of general formula I wherein Y represents a straight-chained $C_{2-4}$-alkylene group optionally substituted by $R_c$ or $R_d$ or $R_c$ and $R_d$, which may be mono- or disubstituted by alkyl, trifluoromethyl, aralkyl, aryl or heteroaryl groups, a compound of general formula

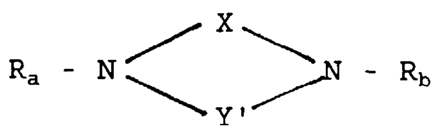
, (VI)

wherein

$R_a$, $R_b$ and X are defined as in claims 1 to 7 and
Y' represents a straight-chained $C_{2-4}$-alkenylene group optionally substituted by $R_c$ or $R_d$ or $R_c$ and $R_d$, which may be mono- or disubstituted by alkyl, trifluoromethyl, aralkyl, aryl or heteroaryl groups, is hydrogenated or

e) in order to prepare compounds of general formula I wherein A represents an aminoalkyl, amidino or guanidino group substituted by an alkoxycarbonyl group having a total of 2 to 5 carbon atoms or by an aralkoxycarbonyl, aryloxycarbonyl, alkylcarbonyl or arylcarbonyl group, a compound of general formula

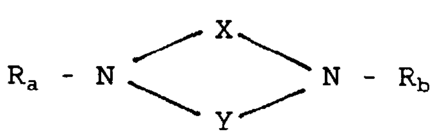
, (VII)

wherein

$R_a$, $R_b$, X and Y are as defined in claims 1 to 7 with the proviso that one of the groups $R_a$ to $R_d$ represents a group of formula

$$A' - B - C - ,$$

wherein
B and C are as defined in claims 1 to 7 and
A' represents an $H_2N$-$C_{1-5}$alkyl-, $H_2N$-C(=NH)- or $H_2N$-C(=NH)-NH- group, with a compound of general formula

$$Z_2 - R_5 \qquad ,(VIII)$$

wherein

$R_5$ represents an alkoxycarbonyl group having a total of 2 to 5 carbon atoms, an aralkoxycarbonyl, aryloxycarbonyl, alkylcarbonyl or arylcarbonyl group and
$Z_2$ represents a nucleophilic leaving group, or

f) in order to prepare compounds of general formula I wherein F represents a carbonyl group substituted by a $C_{1-6}$-alkoxy group, wherein an alkoxy group with 1 to 3 carbon atoms in the 1-, 2- or 3-position may be substituted by an aryl or heteroaryl group or in the 2-or 3-position by a pyrrolidin-2-on-1-yl, morpholino, thiomorpholino or l-oxido-thiomorpholino group, a compound of general formula

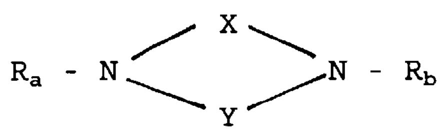

, (IX)

wherein

$R_a$, $R_b$, X and Y are as defined in claims 1 to 7, with the proviso that one of the groups $R_a$ to $R_d$ represents a group of formula

$$F'' - E - D - ,$$

wherein
E and D are as defined in claims 1 to 7 and
F" represents a carboxy or alkoxycarbonyl group, is reacted with an alcohol of general formula

$$HO - R_6 \qquad ,(X)$$

wherein
$R_6$ represents a $C_{1-6}$-alkyl group which may be substituted in the 1-, 2- or 3-position by an aryl or heteroaryl group or in the 2- or 3-position by a pyrrolidin-2-on-1-yl, morpholino, thiomorpholino or 1-oxido-thiomorpholino group, or

g) in order to prepare compounds of general formula I wherein A represents an $NH_2$-(=NH)- group and B or, if B represents a bond, C represents a $C_{4-5}$-cycloalkylene group optionally substituted by an alkyl, aralkyl or aryl group, wherein a CH-unit is replaced by a nitrogen atom, or a $C_{6-7}$-cycloalkylene group optionally substi-

tuted by an alkyl, aralkyl or aryl group, wherein one or two CH-units in the 1,4-position relative to each other are each replaced by a nitrogen atom, whilst B or, if B is a bond, C is linked to the group A via one of the above-mentioned nitrogen atoms, a compound of general formula

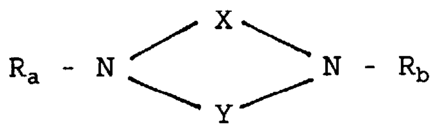

, (XI)

wherein

$R_a$, $R_b$, X and Y are as defined in claims 1 to 7, with the proviso that one of the groups $R_a$ to $R_d$ represents a group of formula

H - B' - C - or H - C' -,

wherein
C is as defined in claims 1 to 7 and
B' or C' represents a $C_{4-5}$-cycloalkylene group optionally substituted by an alkyl, aralkyl or aryl group, wherein a CH-unit is replaced by a nitrogen atom, or a $C_{6-7}$-cycloalkylene group optionally substituted by an alkyl, aralkyl or aryl group, wherein one or two CH-units in the 1,4-position relative to each other are each replaced by a nitrogen atom, the hydrogen atom being linked to a nitrogen atom of the group B' or C', is reacted with a compound of general formula

$Z_3$-C(=NH)-$NH_2$ ,(XII)

wherein
$Z_3$ represents a nucleophilic leaving group, or

h) in order to prepare compounds of general formula I, wherein A represents an $H_2N$-$CH_2$-V- group, wherein V represents a bond or a straight-chained or branched $C_{1-4}$-alkylene group, a compound of general formula

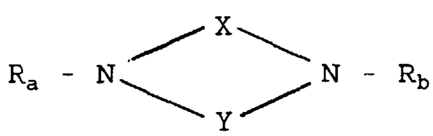

, (XIII)

wherein

$R_a$, $R_b$, X and Y are as defined in claims 1 to 7 with the proviso that one of the groups $R_a$ to $R_d$ represents a group of the formula

NC - V - B - C - ,

wherein
B and C are as defined in claims 1 to 7 and
V represents a bond or a straight-chained or branched $C_{1-4}$-alkylene, is reduced, or

i) in order to prepare compounds of general formula I wherein C represents an alkylene group substituted by a hydroxy group, a compound of general formula

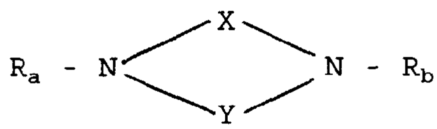

, (XIV)

wherein

$R_a$, $R_b$, X and Y are as defined in claims 1 to 7, with the proviso that one of the groups $R_a$ to $R_d$ represents a group of the formula

A - B - C" -,

wherein
A and B are defined as in claims 1 to 7 and
C" represents an alkylene group wherein a methylene group is replaced by a carbonyl group, is reduced or

j) in order to prepare compounds of general formula I wherein A represents an $H_2N$-C(=NH)-NH- group, a compound of general formula

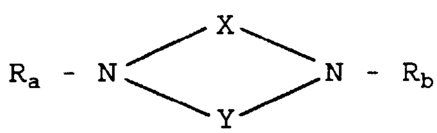

, (XV)

wherein

$R_a$, $R_b$, X and Y are as defined in claims 1 to 7 with the proviso that one of the groups $R_a$ to $R_d$ represents a group of the formula

$H_2N$ - B - C -,

wherein
B and C are defined as in claims 1 to 7, is reacted with cyanamide or an acid addition salt thereof or with an S-alkyl-isothiourea, O-methylisothiourea or 1-amidino-3,5-dimethylpyrazole, or

k) a compound of general formula

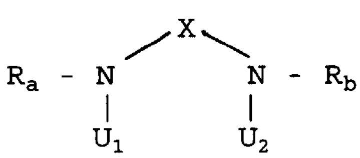

, (XVI)

wherein

$R_a$, $R_b$ and X are as defined in claims 1 to 7,
one of the groups $U_1$ or $U_2$ represents a hydrogen atom and the other group $U_1$ or $U_2$ represents a group of the formula

- Y" - $Z_4$,

wherein
Y" represents a straight-chained alkylene or alkenylene group each having 2 to 4 carbon atoms, optionally substituted by $R_c$ or $R_d$ or $R_c$ and $R_d$, wherein each carbon atom may be mono- or disubstituted by an alkyl, trifluoromethyl, aralkyl, aryl, heteroaryl or alkylcarbonyl group, whilst the substituents may be identical or different, or a 1,2-cycloalkylene group having 4 to 7 carbon atoms optionally substituted by $R_c$ or $R_d$ or $R_c$ and $R_d$ or a 1,2-cycloalkenylene group having 4 to 7 carbon atoms, a -CH=N- group optionally substituted by the groups $R_c$ or $R_d$, wherein the nitrogen atom is linked to one of the nitrogen atoms in formula XVI, or a $-CH_2$-NH- group optionally substituted by $R_c$ or $R_d$ and
$Z_4$ represents a nucleophilic leaving group or together with an adjacent methylene group of the group Y", a carbonyl, carboxy, alkoxycarbonyl, aralkoxycarbonyl, aryloxycarbonyl or dialkoxymethyl group is cyclised or

l) in order to prepare compounds of general formula I wherein $R_a$ or $R_c$ represents an E-F-D- group, a compound of general formula

$$R_c - CO - CHR_d - NR_a - CO - NHR_b \qquad ,(XVII)$$

optionally formed in the reaction mixture, wherein $R_a$ to $R_d$ are defined as in claims 1 to 7, is cyclised and optionally a compound thus obtained is hydrogenated, or

m) in order to prepare compounds of general formula I wherein X represents a carbonyl group, a compound of general formula

$$R_a - NH - Y - NH - R_b \qquad ,(XVIII)$$

wherein
$R_a$, $R_b$ and Y are defined as in claims 1 to 7, is reacted with a compound of general formula

$$Z_5 - CO - Z_6 \qquad ,(XIX)$$

wherein
$Z_5$ and $Z_6$, which may be identical or different, represent nucleophilic leaving groups, or

n) in order to prepare compounds of general formula I wherein $R_a$ to $R_d$ are defined as in claims 1 to 7, with the proviso that at least one of the groups $R_a$ and $R_b$ does not represent a hydrogen atom, a compound of general formula

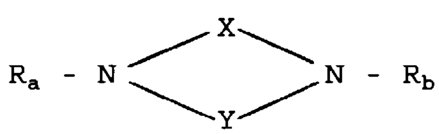

, (XX)

wherein

X and Y are defined as in claims 1 to 7,
one of the groups $R_a$ or $R_b$ represents a hydrogen atom and the other group $R_a$ or $R_b$ is defined as in claims 1 to 7, is reacted with a compound of general formula

$$Z_7 - R' \qquad ,(XXI)$$

wherein

R' has the meanings given in claims 1 to 7 for $R_a$ or $R_b$, with the exception of a hydrogen atom, and $Z_7$ represents a nucleophilic leaving group

or if $R_a$ or $R_b$ represents a -D-COO-alkyl group with the proviso that there are two carbon atoms between the nitrogen atom of the cyclic urea and the alkoxycarbonyl group, with a compound of general formula

- D' -COO-alkyl ,(XXII)

wherein
D' has the meanings given for D in claims 1 to 7, with the proviso that the alkoxycarbonyl group is immediately preceded by a carbon-carbon double or triple bond, or

o) in order to prepare compounds of general formula I wherein F represents a carboxy, alkoxycarbonyl, aralkoxycarbonyl or aryloxycarbonyl group, a compound of general formula

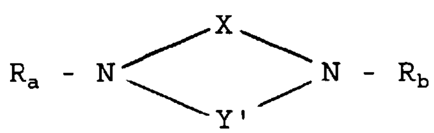

, (XXIII)

wherein
$R_a$, $R_b$, X and Y are defined as in claims 1 to 7, with the proviso that one of the groups $R_a$ to $R_d$ represents a group of the formula

$$CH_2 = CH - E - D -,$$

wherein E and D are defined as in claims 1 to 7, is oxidised and optionally a compound thus obtained is esterified or

p) in order to prepare compounds of general formula I wherein F represents an O-alkyl-phosphono group, a compound of general formula

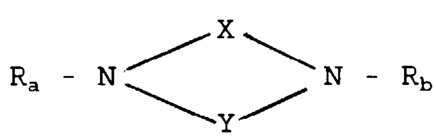

, (XXIV)

wherein
$R_a$, $R_b$, X and Y are defined as in claims 1 to 7, with the proviso that F represents a dialkoxyphosphoryl group, is reacted with an alkali metal iodide or

q) in order to prepare compounds of general formula I wherein F represents a phosphono group, a compound of general formula

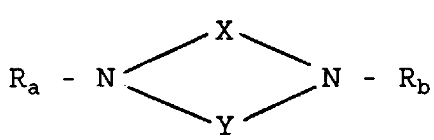

, (XXV)

wherein
$R_a$, $R_b$, X and Y are defined as in claims 1 to 7, with the proviso that F represents an O-alkylphosphono- or dialkoxyphosphoryl group, is reacted with an alkali metal iodide in the presence of a trialkylhalosilane, or

r) in order to prepare compounds of general formula I wherein W represents an $R_1N$ group, a compound of general formula

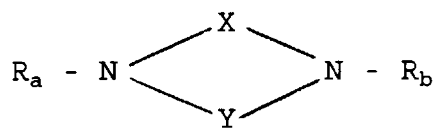

, (XXVI)

wherein
$R_a$, $R_b$, X and Y are defined as in claims 1 to 7, with the proviso that one of the groups $R_a$ to $R_d$ represents a $Z_8$-D"- group, wherein D" represents a $C_{1-3}$-alkylene group and $Z_8$ represents a nucleophilic leaving group or a sulphonic acid ester group is reacted with a compound of general formula

$R_1NH$ - E' - F ,(XXVII)

wherein

F and $R_1$ are defined as in claims 1 to 7 and
E' represents a $C_{1-3}$-alkylene group, or

s) in order to prepare compounds of general formula I wherein A represents an amino or aminoalkyl group, a compound of general formula

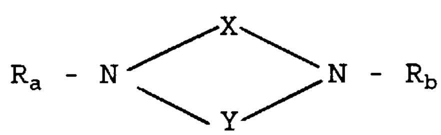

, (XXVIII)

wherein
$R_a$, $R_b$, X and Y are defined as in claims 1 to 7, with the proviso that one of the groups $R_a$ to $R_d$ represents an $H_2N$-CO-T-B-C group, where B and C are defined as in claims 1 to 7 and T represents a bond or a $C_{1-5}$-alkylene group, is reacted with a phenyl iodine(III) compound of general formula

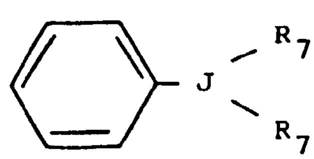

, (XXIX)

wherein
$R_7$ in each case represents the acyl group of an organic carboxylic acid, or

t) in order to prepare compounds of general formula I wherein A represents an amino or aminoalkyl group substituted by one or two alkyl groups at the nitrogen atom, a compound of general formula

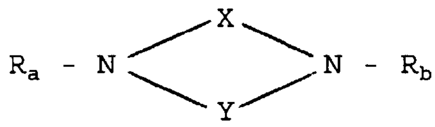 , (XXX)

wherein

$R_a$, $R_b$, X and Y are defined as in claims 1 to 7, with the proviso that one of the groups $R_a$ to $R_d$ represents a group of the formula

A" - B - C - ,

wherein

B and C are defined as in claims 1 to 7 and

A" represents an amino, alkylamino, aminoalkyl or alkylaminoalkyl group, is reacted with a compound of general formula

$$Z_9 - (R_8\text{-}C\text{-}R_9) - Z_{10} \qquad ,(XXXI)$$

wherein

$R_8$ and $R_9$, which may be identical or different, represent hydrogen atoms or alkyl groups,

one of the groups $Z_9$ or $Z_{10}$ represents a nucleophilic leaving group and

the other group $Z_9$ or $Z_{10}$ represents a hydrogen atom or an alkyl group or

$Z_9$ and $Z_{10}$ together represent an oxygen atom, or

u) in order to prepare compounds of general formula I wherein A represents a cyano group, a compound of general formula

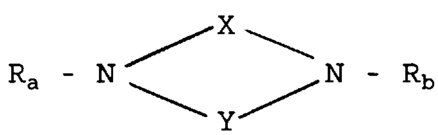 , (XXXII)

wherein

$R_a$, $R_b$, X and Y are defined as in claims 1 to 7, with the proviso that one of the groups $R_a$ to $R_d$ represents a group of the formula

A"' - B - C - ,

wherein

B and C are defined as in claims 1 to 7 and

A"' represents a halogen atom, is reacted with copper(I)cyanide or

v) in order to prepare compounds of general formula I wherein A represents an aminoalkyl group where the amino group is not bound to a quaternary carbon atom, or an amino group which is bound to a CH- or $CH_2$ group of group B or C, a compound of general formula

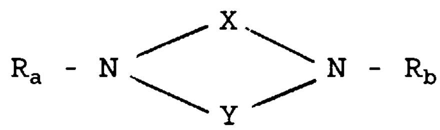
, (XXXIII)

wherein

$R_a$, $R_b$, X and Y are defined as in claims 1 to 7, with the proviso that one of the groups $R_a$ to $R_d$ represents a group of the formula

A"" - B - C - ,

wherein
B and C are defined as in claims 1 to 7 and
A"" contains an N-hydroxy-imino group, is reduced

and subsequently, if desired, a compound of general formula I thus obtained is converted by bromination into a corresponding bromine compound of general formula I or

a compound of general formula I thus obtained is converted by nitrogenation into a corresponding nitro compound of general formula I or

a compound of general formula I thus obtained which contains a nitro group is converted by reduction into a corresponding amino compound or

a compound of general formula I thus obtained which contains an $R_1$NH group or wherein W represents an imino group, is converted by acylation or sulphonation into a corresponding compound of general formula I which contains an $R_1$NH group substituted by an alkylcarbonyl, aralkylcarbonyl, arylcarbonyl, heteroarylcarbonyl, alkylsulphonyl, aralkylsulphonyl or arylsulphonyl group, or

a compound of general formula I thus obtained wherein X represents a carbonyl group is converted by means of a sulphurising agent into a corresponding thiocarbonyl compound and

if necessary any protecting group used during reactions a) to v) in order to protect reactive groups is cleaved and/or

if desired a compound of general formula I thus obtained is separated into the cis-/trans-isomers, enantiomers and/or diastereomers thereof, and/or

a compound of general formula I thus obtained is converted into the salts thereof, more particularly for pharmaceutical use into the physiologically acceptable salts thereof with an organic or inorganic acid or base.

**Revendications**

**1.** Dérivés cycliques de l'urée de formule générale

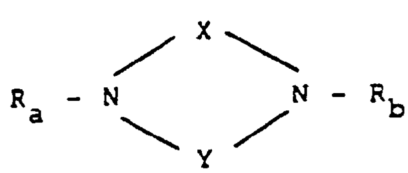
(I)

dans laquelle

X représente un groupe carbimino éventuellement substitué sur l'atome d'azote par un groupe alkyle, aralkyle, aryle, hétéroaryle ou cyano, un groupe carbonyle, thiocarbonyle, sulfinyle ou sulfonyle,
Y représente un groupe alkylène ou alcénylène linéaire, de 2 à 4 atomes de carbone dans chaque cas, éventuellement substitué par $R_c$ ou $R_d$ ou $R_c$ et $R_d$, dans lesquels chaque atome de carbone peut être mono- ou disubstitué par un atome de fluor, de chlore ou de brome, par un groupe alkyle, trifluorométhyle, aralkyle, aryle, hétéroaryle ou alkylcarbonyle, les substituants pouvant être identiques ou différents et en outre un ou deux groupes méthylène pouvant être remplacés dans chaque cas par un groupe carbonyle, ou un groupe 1,2-cycloalkylène de 4 à 7 atomes de carbone éventuellement substitué par $R_c$ ou $R_d$ ou $R_c$ et $R_d$,

un groupe 1,2-cycloalcénylène de 4 à 7 atomes de carbone ou un groupe 1,2-phénylène dans lequel un ou deux groupes méthine peuvent être remplacés par un atome d'azote et qui peut être substitué dans le squelette carboné par un atome de fluor, de chlore ou de brome, par un groupe alkyle de 1 à 4 atomes de carbone, par un groupe trifluorométhyle, hydroxyle, alcoxy, alkylsulfényle, alkylsulfinyle, alkylsulfonyle, alkylcarbonyle, arylcarbonyle, alcoxycarbonyle, carboxyle, nitro, $(R_1)_2N$-, $(R_1)_2NCO$- ou $(R_1)_2NSO_2$-, les restes $R_1$ pouvant être identiques ou différents et représentant dans chaque cas un atome d'hydrogène, un groupe alkyle, aralkyle, aryle ou hétéroaryle, ou par un groupe $R_1NH$- substitué par un groupe alkylcarbonyle, arylcarbonyle, aralkylcarbonyle, hétéroarylcarbonyle, alkylsulfonyle, aralkylsulfonyle ou arylsulfonyle et dans lequel en outre un ou deux groupes -CH=CH- peuvent être remplacés dans chaque cas par un groupe -CO-$NR_1$-,
un groupe -CO-NH-, -NH-CO-, -CH=N- ou -N=CH-éventuellement substitué par $R_c$ ou $R_d$,
l'un des restes $R_a$ à $R_d$ représente un groupe de formule

A - B - C -

dans laquelle

A représente un groupe aminoalkyle linéaire ou ramifié de 1 à 5 atomes de carbone, un groupe amino, amidino ou guanidino, où, dans chacun des groupes cités précédemment, sur l'un des atomes d'azote un ou deux atomes d'hydrogène pouvant être remplacés dans chaque cas par un groupe alkyle de 1 à 4 atomes de carbone ou un atome d'hydrogène pouvant être remplacé par un groupe alcoxycarbonyle de 2 à 5 atomes de carbone au total, par un groupe alkylcarbonyle, arylcarbonyle, aryloxycarbonyle ou aralcoxycarbonyle, un groupe cyano, un groupe cyanoalkyle de 1 à 4 atomes de carbone dans la partie alkyle ou encore, lorsque A est lié à un atome d'azote des restes B ou C qui ne fait pas partie d'un groupe lactame, un atome d'hydrogène ou un groupe alkyle,
B représente une liaison,

un groupe alkylène ou alcénylène, un groupe phénylène qui peut être mono- ou disubstitué par des atomes de fluor, de chlore ou de brome, par des groupes alkyle de 1 à 4 atomes de carbone, par des groupes trifluorométhyle, hydroxyle, alcoxy, alkylsulfényle, alkylsulfinyle, alkylsulfonyle, $(R_1)_2N$-, $(R_1)_2NCO$-, $(R_1)_2NSO_2$- ou nitro ou par des groupe $R_1NH$- substitués par un groupe alkylcarbonyle, aralkylcarbonyle, arylcarbonyle, hétéroarylcarbonyle, alkylsulfonyle, aralkylsulfonyle ou arylsulfonyle, les substituants pouvant être identiques ou différents,
un groupe pyridinylène, pyrimidinylène, pyrazinylène, pyridazinylène ou triazinylène, qui peuvent être substitués dans chaque cas dans le squelette carboné par un groupe alkyle, un ou deux groupes -CH=N- pouvant en outre être remplacés dans chaque cas par un groupe -CO-$NR_1$-et l'un des atomes d'azote, au lieu d'être lié au reste $R_1$, pouvant aussi l'être au reste C dans la mesure où celui-ci n'est pas lié au reste B par un hétéroatome ou un groupe carbonyle,
un groupe cyclopropylène éventuellement substitué par un groupe alkyle, aralkyle ou aryle,
un groupe cycloalkylène de 4 à 5 atomes de carbone éventuellement substitué par un groupe alkyle, aralkyle ou aryle, dans lequel une unité CH peut être remplacée par un atome d'azote et en outre un groupe méthylène voisin de l'atome d'azote peut être remplacé par un groupe carbonyle,
un groupe cycloalkylène de 6 ou 7 atomes de carbone éventuellement substitué par un groupe alkyle, aralkyle ou aryle, dans lequel une ou deux unités CH situées en position 1,4 l'une par rapport à l'autre peuvent être remplacées chacune par un atome d'azote, un ou deux des groupes méthylène voisins d'un atome d'azote pouvant en outre être remplacés dans chaque cas par un groupe carbonyle,

un groupe biphénylène qui peut être mono- ou disubstitué par des atomes de fluor, de chlore ou de brome, par des groupes alkyle, trifluorométhyle, hydroxyle, alcoxy, alkylsulfényle, alkylsulfinyle, alkylsulfonyle, alkylcarbonyl-$NR_1$- ou alkylsulfonyl-$NR_1$-, les substituants pouvant être identiques ou différents et $R_1$ étant défini comme au début, et

C représente un groupe alkylène ou alcénylène éventuellement substitué par un groupe hydroxyle, alcoxy ou $(R_1)_2N$-,

un groupe alkylènecarbonyle lié au reste B par le groupe carbonyle,
un groupe phénylène qui peut être mono- ou disubstitué par des atomes de fluor, de chlore ou de brome, par des groupes alkyle de 1 à 4 atomes de carbone, par des groupes trifluorométhyle, hydroxyle, alcoxy, alkylsulfényle, alkylsulfinyle, aikylsulfonyle, $(R_1)_2N$-, $(R_1)_2NCO$-, $(R_1)_2NSO_2$- ou nitro ou par des groupes $R_1NH$- substitués par un groupe alkylcarbonyle, aralkylcarbonyle, arylcarbonyle, hétéroarylcarbonyle, alkylsulfonyle, aralkylsulfonyle ou arylsulfonyle, les substituants pouvant être identiques ou différents,
un groupe indanylène ou 1,2,3,4-tétrahydronaphtylène, dans lesquels dans chaque cas le cycle saturé est lié au reste A et le cycle aromatique est lié au squelette cyclique de l'urée, un groupe pyridinylène, pyrimidinylène, pyrazinylène, pyridazinylène ou triazinylène, qui peuvent être substitués dans chaque cas dans le squelette carboné par un groupe alkyle, un ou deux groupes -CH=N- pouvant en outre être remplacés dans chaque cas par un groupe -CO-$NR_1$-et l'un des atomes d'azote, au lieu d'être lié au reste $R_1$, pouvant aussi l'être au reste B dans la mesure où celui-ci ne représente pas une liaison ou n'est pas lié au reste C par un hétéroatome,
un groupe cycloalkylène de 4 à 5 atomes de carbone éventuellement substitué par un groupe alkyle, aralkyle ou aryle, dans lequel une unité CH peut être remplacée par un atome d'azote et en outre un groupe méthylène voisin de l'atome d'azote peut être remplacé par un groupe carbonyle, ou
un groupe cycloalkylène de 6 ou 7 atomes de carbone éventuellement substitué par un groupe alkyle, aralkyle ou aryle, dans lequel une ou deux unités CH situées en position 1,4 l'une par rapport à l'autre peuvent être remplacées chacune par un atome d'azote, un ou deux des groupes méthylène voisins d'un atome d'azote pouvant en outre être remplacés dans chaque cas par un groupe carbonyle,
un deuxième des restes $R_a$ à $R_d$ représente un groupe de formule

F - E - D -

dans laquelle

D représente un groupe alkylène de 1 à 5 atomes de carbone

ou un groupe alcénylène de 2 à 5 atomes de carbone, un groupe phénylène qui peut être mono- ou disubstitué par des atomes de fluor, de chlore ou de brome, par des groupes alkyle de 1 à 4 atomes de carbone, par des groupes trifluorométhyle, hydroxyle, alcoxy, alkylsulfényle, alkylsulfinyle, alkylsulfonyle, carboxyalcoxy, alcoxycarbonylalcoxy, aralcoxycarbonylalcoxy, $(R_1)_2N$-, $(R_1)_2NCO$-, $(R_1)_2NSO_2$- ou nitro ou par des groupes $R_1NH$-substitués par un groupe alkylcarbonyle, aralkylcarbonyle, arylcarbonyle, hétéroarylcarbonyle, alkylsulfonyle, aralkylsulfonyle ou arylsulfonyle, les substituants pouvant être identiques ou différents,
un groupe pyridinylène, pyrimidinylène, pyrazinylène, pyridazinylène ou triazinylène, qui peuvent être substitués dans chaque cas dans le squelette carboné par un groupe alkyle, un ou deux groupes -CH=N- pouvant en outre être remplacés dans chaque cas par un groupe -CO-$NR_1$-et l'un des atomes d'azote, au lieu d'être lié au reste $R_1$, pouvant aussi l'être au reste E dans la mesure où celui-ci ne représente pas une liaison ou n'est pas lié au reste D par un hétéroatome,
un groupe cycloalkylène de 4 à 5 atomes de carbone éventuellement substitué par un groupe alkyle, aralkyle ou aryle, dans lequel une unité CH peut être remplacée par un atome d'azote et en outre un groupe méthylène voisin de l'atome d'azote peut être remplacé par un groupe carbonyle,
un groupe cycloalkylène de 6 ou 7 atomes de carbone éventuellement substitué par un groupe alkyle, aralkyle ou aryle, dans lequel une ou deux unités CH situées en position 1,4 l'une par rapport à l'autre peuvent être remplacées chacune par un atome d'azote, un ou deux des groupes méthylène voisins d'un atome d'azote pouvant en outre être remplacés dans chaque cas par un groupe carbonyle, ou
un groupe alkylène de 2 à 6 atomes de carbone, interrompu par le reste W, dans lequel W représente un atome d'oxygène ou de soufre, un groupe sulfinyle, sulfonyle, $R_1N$-, (alkylcarbonyl)N-, (aralkylcarbonyl)

N-, (arylcarbonyl)N-, (hétéroarylcarbonyl)N-, (alkylsulfonyl)N-, (arylsulfonyl)-N-, aminocarbonyle ou carbonylamino,

E représente une liaison,

un groupe alkylène de 1 à 5 atomes de carbone ou un groupe alcénylène de 2 à 5 atomes de carbone, qui peuvent être substitués dans chaque cas par un ou deux groupes alkyle, par un groupe hydroxyle, alcoxy, amino, alkylamino, aralkylamino, dialkylamino, bis(aralkyl)amino, carboxyalkyle, alcoxycarbonylalkyle ou aralcoxycarbonylalkyle,
un groupe phénylène qui peut être mono- ou disubstitué par des atomes de fluor, de chlore ou de brome, par des groupes alkyle de 1 à 4 atomes de carbone, par des groupes trifluorométhyle, hydroxyle, alcoxy, alkylsulfényle, alkylsulfinyle, alkylsulfonyle, $(R_1)_2N$-, $(R_1)_2NCO$-, $(R_1)_2NSO_2$- ou nitro ou par des groupes $R_1NH$- substitués par un groupe alkylcarbonyle, aralkylcarbonyle, arylcarbonyle, hétéroarylcarbonyle, alkylsulfonyle, aralkylsulfonyle ou arylsulfonyle, les substituants pouvant être identiques ou différents,
un groupe pyridinylène, pyrimidinylène, pyrazinylène, pyridazinylène ou triazinylène, qui peuvent être substitués dans chaque cas dans le squelette carboné par un groupe alkyle, un ou deux groupes -CH=N- pouvant en outre être remplacés dans chaque cas par un groupe -CO-$NR_1$-et l'un des atomes d'azote, au lieu d'être lié au reste $R_1$, pouvant aussi l'être au reste D,
un groupe cycloalkylène de 4 à 5 atomes de carbone éventuellement substitué par un groupe alkyle, aralkyle ou aryle, dans lequel une unité CH peut être remplacée par un atome d'azote et en outre un groupe méthylène voisin de l'atome d'azote peut être remplacé par un groupe carbonyle,
un groupe cycloalkylène de 6 ou 7 atomes de carbone éventuellement substitué par un groupe alkyle, aralkyle ou aryle, dans lequel une ou deux unités CH situées en position 1,4 l'une par rapport à l'autre peuvent être remplacées chacune par un atome d'azote, un ou deux des groupes méthylène voisins d'un atome d'azote pouvant en outre être remplacés dans chaque cas par un groupe carbonyle,
un groupe alkylène-arylène lié au reste D par la partie aryle ou
un groupe alkylène lié au reste D par le reste W, dans lequel W est défini comme au début, et

F représente un groupe carbonyle qui est substitué par un groupe hydroxyle ou alcoxy de 1 à 6 atomes de carbone, un reste alcoxy de 1 à 3 atomes de carbone pouvant être substitué en position 1, 2 ou 3 par un groupe aryle ou hétéroaryle ou en position 2 ou 3 par un groupe pyrrolidin-2-on-1-yle, morpholino, thiomorpholino ou 1-oxydo-thiomorpholino, un groupe sulfo, phosphono, O-alkylphosphono ou tétrazol-5-yle, où, dans la mesure où A représente un groupe cyano ou un groupe amino ou aminoalkyle éventuellement benzoylé ou benzyloxycarbonylé sur l'atome d'azote, la plus courte distance entre l'atome d'azote de ces groupes et le reste F est d'au moins 10 liaisons,

le troisième des restes $R_a$ à $R_d$ représente un atome d'hydrogène, un groupe alkyle, aralkyle, aryle ou hétéroaryle, ou encore, lorsque le troisième des restes $R_a$ à $R_d$ est lié à un atome de carbone insaturé du reste Y, un groupe alcoxy, alkylsulfényle ou $(R_1)_2N$- et
le quatrième des restes $R_a$ à $R_d$ représente un atome d'hydrogène, un groupe alkyle, aralkyle, aryle ou hétéroaryle, ou $R_a$ ou $R_b$ représentent aussi avec un reste $R_c$ ou $R_d$ voisin une liaison avec la condition que le groupe A-B-C- ne représente pas un groupe 4-pipéridinyle, lorsque, en même temps, le groupe F-E-D- représente un groupe alcoxycarbonylalkyle et Y représente un groupe 1,2-phénylène substitué par un atome de fluor, de chlore ou de brome, par un groupe alkyle ou trifluorométhyle,
leurs tautomères, leurs stéréoisomères y compris leurs mélanges et leurs sels d'addition,

où, sauf indication contraire,

les parties alkyle, alkylène, alcénylène ou alcoxy citées précédemment peuvent contenir dans chaque cas 1 à 3 atomes de carbone,
le terme cité précédemment « un groupe aryle » signifiant un groupe phényle qui peut être monosubstitué par un groupe trifluorométhyle, carboxyle, $(R_1)_2NCO$-, alcoxycarbonyle, alkylcarbonyle, alkylsulfényle, alkylsulfinyle, alkylsulfonyle, nitro, $(R_1)_2N$-, alkylcarbonyl-$NR_1$-, aralkylcarbonyl-$NR_1$-, arylcarbonyl-$NR_1$-, hétéroaryl-carbonyl-$NR_1$-, alkylsulfonyl-$NR_1$-, aralkylsulfonyl-$NR_1$-, arylsulfonyl-$NR_1$- ou $(R_1)_2N$-sulfonyle ou di- ou trisubstitué par des atomes de fluor, de chlore ou de brome, par des groupes hydroxyle, alcoxy ou alkyle de 1 à 4 atomes de carbone, et
le terme cité précédemment « un groupe hétéroaryle » signifiant un cycle hétéroaromatique à 5 chaînons qui contient un atome d'oxygène, de soufre ou d'azote, un atome d'azote et un atome d'oxygène, de soufre

ou d'azote ou deux atomes d'azote et un atome d'oxygène, de soufre ou d'azote ou un cycle hétéroaromatique à 6 chaînons qui contient un, deux ou trois atomes d'azote et dans lequel en outre un ou deux groupes -CH=N- peuvent être remplacés par un groupe $-CO-NR_1-$, les cycles hétéroaromatiques cités précédemment pouvant en outre être substitués par un ou deux groupes alkyle ou par un atome de fluor, de chlore ou de brome, par un groupe hydroxyle ou alcoxy.

**2.** Dérivés cycliques de l'urée de formule générale I selon la revendication 1, dans laquelle

X représente un groupe carbimino éventuellement substitué sur l'atome d'azote par un groupe alkyle, aralkyle, aryle, hétéroaryle ou cyano, un groupe carbonyle, thiocarbonyle, sulfinyle ou sulfonyle,
Y représente un groupe alkylène ou alcénylène linéaire de 2 ou 3 atomes de carbone dans chaque cas, éventuellement substitué par $R_c$ ou $R_d$ ou $R_c$ et $R_d$, qui peut être mono- ou disubstitué par un atome de fluor, de chlore ou de brome, par un groupe alkyle, trifluorométhyle, aralkyle, aryle, hétéroaryle ou alkylcarbonyle, les substituants pouvant être identiques ou différents et en outre un ou deux groupes méthylène pouvant être remplacés dans chaque cas par un groupe carbonyle, ou un groupe 1,2-cyclohexylène éventuellement substitué par $R_c$ ou $R_d$ ou $R_c$ et $R_d$,

un groupe 1,2-cyclohexénylene ou un groupe 1,2-phénylène dans lequel un ou deux groupes CH peuvent être remplacés dans chaque cas par un atome d'azote et qui peut être substitué dans le squelette carboné par un atome de fluor, de chlore ou de brome, par un groupe alkyle de 1 à 4 atomes de carbone, par un groupe trifluorométhyle, hydroxyle, alcoxy, alkylsulfényle, alkylsulfinyle, alkylsulfonyle, alkylcarbonyle, arylcarbonyle, alcoxycarbonyle, carboxyle, nitro, $(R_1)_2N-$, $(R_1)_2NCO-$ ou $(R_1)_2NSO_2-$, les restes $R_1$ pouvant être identiques ou différents et représentant dans chaque cas un atome d'hydrogène, un groupe alkyle, aralkyle, aryle ou hétéroaryle, ou par un groupe $R_1NH-$ substitué par un groupe alkylcarbonyle, arylcarbonyle, aralkylcarbonyle, hétéroarylcarbonyle, alkylsulfonyle, aralkylsulfonyle ou arylsulfonyle et dans lequel en outre un ou deux groupes - CH=CH- peuvent être remplacés dans chaque cas par un groupe $-CO-NR_1-$,
un groupe -CO-NH-, -NH-CO-, -CH=N- ou -N=CH-éventuellement substitué par $R_c$ ou $R_d$,
l'un des restes $R_a$ à $R_d$ représente un groupe de formule

A - B - C -

dans laquelle

A représente un groupe aminoalkyle linéaire ou ramifié de 1 à 5 atomes de carbone, un groupe amino, amidino ou guanidino, où, dans chacun des groupes cités précédemment, sur l'un des atomes d'azote un ou deux atomes d'hydrogène pouvant être remplacés dans chaque cas par un groupe alkyle de 1 à 4 atomes de carbone ou un atome d'hydrogène pouvant être remplacé par un groupe alcoxycarbonyle de 2 à 5 atomes de carbone au total, par un groupe alkylcarbonyle, arylcarbonyle, aryloxycarbonyle ou aralcoxycarbonyle, un groupe cyano, un groupe cyanoalkyle de 1 à 4 atomes de carbone dans la partie alkyle ou encore, lorsque A est lié à un atome d'azote des restes 3 ou C qui ne fait pas partie d'un groupe lactame, un atome d'hydrogène ou un groupe alkyle,
B représente une liaison,

un groupe alkylène ou alcénylène,
un groupe phénylène qui peut être mono- ou disubstitué par des atomes de fluor, de chlore ou de brome, par des groupes alkyle de 1 à 4 atomes de carbone, par des groupes trifluorométhyle, hydroxyle, alcoxy, alkylsulfényle, alkylsulfinyle, alkylsulfonyle, $(R_1)_2N-$, $(R_1)_2NCO-$, $(R_1)_2NSO_2-$ ou nitro ou par des groupes $R_1NH-$ substitués par un groupe alkylcarbonyle, aralkylcarbonyle, arylcarbonyle, hétéroarylcarbonyle, alkylsulfonyle, aralkylsulfonyle ou arylsulfonyle, les substituants pouvant être identiques ou différents,
un groupe pyridinylène, pyrimidinylène, pyrazinylène ou pyridazinylène, qui peuvent être substitués dans chaque cas dans le squelette carboné par un groupe alkyle, un ou deux groupes -CH=N- pouvant en outre être remplacés dans chaque cas par un groupe $-CO-NR_1-$ et l'un des atomes d'azote, au lieu d'être lié au reste $R_1$, pouvant aussi l'être au reste C dans la mesure où celui-ci n'est pas lié au reste B par un hétéroatome ou un groupe carbonyle,
un groupe cycloalkylène de 3 à 5 atomes de carbone,
un groupe cyclohexylène dans lequel une ou deux unités CH situées en position 1,4 l'une par rapport à

l'autre peuvent être remplacées par des atomes d'azote, un ou deux des groupes méthylène voisins d'un atome d'azote pouvant en outre être remplacés dans chaque cas par un groure carbonyle, ou
un groupe biphénylène et

C représente un groupe alkylène ou alcénylène éventuellement substitué par un groupe hydroxyle,

un groupe alkylènecarbonyle lié au reste B par le groupe carbonyle,
un groupe phénylène qui peut être mono- ou disubstitué par des atomes de fluor, de chlore ou de brome, par des groupes alkyle de 1 à 4 atomes de carbone, par des groupes trifluorométhyle, hydroxyle, alcoxy, alkylsulfényle, alkylsulfinyle, alkylsulfonyle, $(R_1)_2N$-, $(R_1)_2NCO$-, $(R_1)_2NSO_2$- ou nitro ou par des groupes $R_1NH$- substitués par un groupe alkylcarbonyle, aralkylcarbonyle, arylcarbonyle, hétéroarylcarbonyle, alkylsulfonyle, aralkylsulfonyle ou arylsulfonyle, les substituants pouvant être identiques ou différents,
un groupe indanylène ou l,2,3,4-tétrahydronaphtylène, dans lesquels dans chaque cas le cycle saturé est lié au reste A et le cycle aromatique est lié au squelette cyclique de l'urée,
un groupe pyridinylène, pyrimidinylène, pyrazinylène ou pyridazinylène, qui peuvent être substitués dans chaque cas dans le squelette carboné par un groupe alkyle, un ou deux groupes -CH=N- pouvant en outre être remplacés dans chaque cas par un groupe -CO-$NR_1$- et l'un des atomes d'azote, au lieu d'être lié au reste $R_1$, pouvant aussi l'être au reste B dans la mesure où celui-ci ne représente pas une liaison ou n'est pas lié au reste C par un hétéroatome,
un groupe cyclohexylène dans lequel une ou deux unités CH situées en position 1,4 l'une par rapport à l'autre peuvent être remplacées par des atomes d'azote, un ou deux des groupes méthylène voisins d'un atome d'azote pouvant en outre être remplacés dans chaque cas par un groupe carbonyle et les atomes d'azote ne pouvant pas être liés à un atome d'azote de l'urée cyclique,
un deuxième des restes $R_a$ à $R_d$ représente un groupe de formule

F - E - D -

dans laquelle

D représente un groupe alkylène de 1 à 5 atomes de carbone ou un groupe alcénylène de 2 à 5 atomes de carbone,

un groupe phénylène qui peut être mono- ou disubstitué par des atomes de fluor, de chlore ou de brome, par des groupes alkyle de 1 à 4 atomes de carbone, par des groupes trifluorométhyle, hydroxyle, alcoxy, alkylsulfényle, alkylsulfinyle, alkylsulfonyle, carboxyalcoxy, alcoxycarbonylalcoxy, aralcoxycarbonylalcoxy, $(R_1)_2N$-, $(R_1)_2NCO$-, $(R_1)_2NSO_2$- ou nitro ou par des groupes $R_1NH$-substitués par un groupe alkylcarbonyle, aralkylcarbonyle, arylcarbonyle, hétéroarylcarbonyle, alkylsulfonyle, aralKylsulfonyle ou arylsulfonyle, les substituants pouvant être identiques ou différents,
un groupe pyridinylène, pyrimidinylène, pyrazinylène ou pyridazinylène, qui peuvent être substitués dans chaque cas dans le squelette carboné par un groupe alkyle, un ou deux groupes -CH=N- pouvant en outre être remplacés dans chaque cas par un groupe -CO-$NR_1$- et l'un des atomes d'azote, au lieu d'être lié au reste $R_1$, pouvant aussi l'être au reste E dans la mesure où celui-ci ne représente pas une liaison ou n'est pas lié au reste D par un hétéroatome,
un groupe cyclohexylène dans lequel une ou deux unités CH situées en position 1,4 l'une par rapport à l'autre peuvent être remplacées par des atomes d'azote, un ou deux des groupes méthylène voisins d'un atome d'azote pouvant en outre être remplacés dans chaque cas par un groupe carbonyle, ou
un groupe alkylène de 3 à 6 atomes de carbone, interrompu par le reste W, dans lequel W représente un atome d'oxygène ou de soufre, un groupe sulfinyle, sulfonyle, $R_1N$-, (alkylcarbonyl)N-, (aralkylcarbonyl) N-, (arylcarbonyl)N-, (hétéroarylcarbonyl)N-, (alkylsulfonyl)N- ou (aryl-sulfonyl)N- et le groupe alkylène lié à un atome d'azote de l'urée cyclique contient 2 ou 3 atomes de carbone,

E représente une liaison,

un groupe alkylène de 1 à 5 atomes de carbone ou un groupe alcénylène de 2 à 5 atomes de carbone, qui peuvent être substitués dans chaque cas par un ou deux groupes alkyle, par un groupe hydroxyle, alcoxy, amino, alkylamino, aralkylamino, dialkylamino, bis(aralkyl)amino, carboxyalkyle, alcoxycarbonylalkyle ou aralcoxycarbonylalkyle,

un groupe phénylène qui peut être mono- ou disubstitué par des atomes de fluor, de chlore ou de brome, par des groupes alkyle de 1 à 4 atomes de carbone, par des groupes trifluorométhyle, hydroxyle, alcoxy, alkylsulfényle, alkylsulfinyle, alkylsulfonyle, $(R_1)_2N$-, $(R_1)_2NCO$-, $(R_1)_2NSO_2$- ou nitro ou par des groupes $R_1NH$- substitués par un groupe alkylcarbonyle, aralkylcarbonyle, arylcarbonyle, hétéroarylcarbonyle, alkylsulfonyle, aralkylsulfonyle ou arylsulfonyle, les substituants pouvant être identiques ou différents,
un groupe pyridinylène, pyrimidinylène, pyrazinylène ou pyridazinylène, qui peuvent être substitués dans chaque cas dans le squelette carboné par un groupe alkyle, un ou deux groupes -CH=N- pouvant en outre être remplacés dans chaque cas par un groupe -CO-$NR_1$- et l'un des atomes d'azote, au lieu d'être lié au reste $R_1$, pouvant aussi l'être au reste D,
un groupe cyclohexylène dans lequel une ou deux unités CH situées en position 1,4 l'une par rapport à l'autre peuvent être remplacées par des atomes d'azote, un ou deux des groupes méthylène voisins d'un atome d'azote pouvant en outre être remplacés dans chaque cas par un groupe carbonyle,
un groupe alkylène-arylène lié au reste D par le reste aryle ou
un groupe alkylène lié au reste D par le reste W', dans lequel W' représente un atome d'oxygène ou de soufre, un groupe sulfinyle, sulfonyle, $R_1N$-, (alkylcarbonyl)N-, (aralkylcarbonyl)N-, (arylcarbonyl)N-, (hétéroarylcarbonyl)N-, (alkylsulfonyl)N-, (arylsulfonyl)N- ou aminocarbonyle, dans lequel l'atome d'azote est lié au groupe alkylène,

F représente un groupe carbonyle qui est substitué par un groupe hydroxyle ou alcoxy de 1 à 6 atomes de carbone, un reste alcoxy de 1 à 3 atomes de carbone pouvant être substitué en position 1, 2 ou 3 par un groupe aryle ou hétéroaryle ou en position 2 ou 3 par un groupe pyrrolidin-2-on-1-yle, morpholino, thiomorpholino ou 1-oxydo-thiomorpholino, un groupe sulfo, phosphono, O-alkylphosphono ou tétrazol-5-yle, où, dans la mesure où A représente un groupe cyano ou un groupe amino ou aminoalkyle éventuellement benzoylé ou benzyloxycarbonylé sur l'atome d'azote, la plus courte distance entre l'atome d'azote de ces groupes et le reste F est d'au moins 10 liaisons,

le troisième des restes $R_a$ à $R_d$ représente un atome d'hydrogène, un groupe alkyle, aralkyle, aryle ou hétéroaryle, ou encore, lorsque le troisième des restes $R_a$ à $R_d$ est lié à un atome de carbone insaturé du reste Y, un groupe alcoxy, alkylsulfényle ou $(R_1)_2N$- et
le quatrième des restes $R_a$ à $R_d$ représente un atome d'hydrogène, un groupe alkyle, aralkyle, aryle ou hétéroaryle, ou $R_a$ ou $R_b$ représentent aussi avec un reste $R_c$ ou $R_d$ voisin une liaison avec la condition que le groupe A-B-C- ne représente pas un groupe 4-pipéridinyle, lorsque, en même temps, le groupe F-E-D- représente un groupe alcoxycarbonylalkyle et Y représente un groupe 1,2-phénylène substitué par un atome de fluor, de chlore ou de brome, par un groupe alkyle ou trifluorométhyle,
leurs tautomères, leurs stéréoisomères y compris leurs mélanges et leurs sels d'addition.

**3.** Dérivés cycliques de l'urée de formule générale I selon la revendication 1, dans laquelle

X représente un groupe carbimino éventuellement substitué sur l'atome d'azote par un groupe méthyle, phényle ou pyridyle, un groupe carbonyle, thiocarbonyle ou sulfonyle,
Y représente un groupe alkylène ou alcénylène linéaire de 2 ou 3 atomes de carbone dans chaque cas, éventuellement substitué par $R_c$ ou $R_d$ ou $R_c$ et $R_d$, qui peut être substitué par un atome de chlore, par un ou deux groupes méthyle ou par un groupe trifluorométhyle, phényle ou acétyle, un groupe méthylène pouvant en outre être remplacé par un groupe carbonyle,

un groupe -CO-NH-, -NH-CO-, -CH=N- ou -N=CH-éventuellement substitué par $R_c$ ou $R_d$, un groupe 1,2-phénylène ou 2,3-pyridinylène,
l'un des restes $R_a$ à $R_d$ représente un groupe de formule

$$A - B - C -$$

dans laquelle

A représente un groupe aminoalkyle linéaire ou ramifié de 1 à 4 atomes de carbone, un groupe amino, amidino ou guanidino, où, dans chacun des groupes cités précédemment, sur l'un des atomes d'azote un ou deux atomes d'hydrogène pouvant être remplacés dans chaque cas par un groupe alkyle de 1 à 4 atomes de carbone ou un atome d'hydrogène pouvant être remplacé par un groupe alcoxycarbonyle de 2 à 5 atomes de carbone

au total ou par un groupe benzyloxycarbonyle, ou encore, lorsque A est lié à un atome d'azote du reste C qui ne fait pas partie d'un groupe lactame, un atome d'hydrogène ou un groupe méthyle,
B représente une liaison,

un groupe phénylène qui peut être substitué par un ou deux groupes méthyle, par un atome de fluor, de chlore ou de brome, par un groupe méthoxy, méthylsulfényle, méthylsulfinyle, méthylsulfonyle, nitro, amino, acétylamino, benzoylamino ou méthanesulfonylamino,
un groupe cycloalkylène de 3 à 6 atomes de carbone,
un groupe pyridinylène, pyrimidinylène, pyrazinylène, pyridazinylène ou biphénylène,

C représente un groupe éthylène éventuellement substitué par un groupe hydroxyle,

un groupe méthylènecarbonyle lié au reste B par le groupe carbonyle,
un groupe phénylène qui peut être substitué par un ou deux groupes méthyle, par un atome de fluor, de chlore ou de brome, par un groupe méthoxy, méthylsulfényle, méthylsulfinyle, méthylsulfonyle, nitro, amino, acétylamino, benzoylamino ou méthanesulfonylamino, un groupe indanylène ou 1,2,3,4-tétrahydronaphtylène, dans lesquels dans chaque cas le cycle saturé est lié au reste A et le cycle aromatique est lié au squelette cyclique de l'urée,
un groupe pyridinylène, pyrimidinylène, pyrazinylène, pyridazinylène, cyclohexylène ou pipéridinylène, l'atome d'azote ne pouvant pas être lié à un atome d'azote de l'urée cyclique,
un deuxième des restes $R_a$ à $R_d$ représente un groupe de formule

F - E - D -

dans laquelle

D représente un groupe alkylène de 1 à 4 atomes de carbone,

un groupe phénylène qui peut être substitué par un atome de fluor, de chlore ou de brome, par un groupe méthyle, méthoxy, méthylsulfényle, méthylsulfinyle, méthylsulfonyle, carboxyméthoxy, méthoxycarbonylméthoxy, nitro, amino, acétylamino, benzoylamino ou méthanesulfonylamino,
un groupe pyridinylène, cyclohexylène ou pipéridinylène, un groupe -CH=N- pouvant en outre être remplacé dans un groupe pyridinylène par un groupe -CO-NH-, l'atome d'azote, au lieu d'être lié à l'atome d'hydrogène, pouvant aussi l'être au reste E dans la mesure où celui-ci ne représente pas une liaison ou n'est pas lié au reste D par un hétéroatome,
un groupe alkylène de 3 à 5 atomes de carbone, interrompu par le reste W, dans lequel W représente un atome d'oxygène ou de soufre, un groupe sulfinyle, sulfonyle, imino, méthylimino, acétylimino, benzoylimino ou méthanesulfonylimino et le groupe alkylène lié à l'urée cyclique contient 2 ou 3 atomes de carbone,

E représente une liaison,

un groupe alkylène de 1 à 3 atomes de carbone éventuellement substitué par un ou deux groupes méthyle, par un groupe hydroxyle, méthoxy, amino, diméthylamino, dibenzylamino, carboxyméthyle ou méthoxycarbonylméthyle, ou un groupe alcénylène de 2 ou 3 atomes de carbone,
un groupe phénylène ou
un groupe alkylène de 1 ou 2 atomes de carbone lié au groupe D par le reste W', dans lequel W' représente un atome d'oxygène ou de soufre, un groupe sulfinyle, sulfonyle ou aminocarbonyle, le groupe amino étant lié au groupe alkylène, et

F représente un groupe carbonyle qui est substitué par un groupe hydroxyle, par un groupe alcoxy de 1 à 4 atomes de carbone ou par un groupe phénylalcoxy de 1 ou 2 atomes de carbone dans la partie alcoxy, un groupe phosphono, O-méthylphosphono ou tétrazol-5-yle, où, dans la mesure où A représente un groupe amino ou aminoalkyle éventuellement benzyloxycarbonylé sur l'atome d'azote, la plus courte distance entre l'atome d'azote de ce groupe et le reste F est d'au moins 10 liaisons,

le troisième des restes $R_a$ à $R_d$ représente un atome d'hydrogène, un groupe méthyle, éthyle ou phényle et le quatrième des restes $R_a$ à $R_d$ représente un atome d'hydrogène ou un groupe méthyle,

leurs tautomères, leurs stéréoisomères y compris leurs mélanges et leurs sels d'addition.

**4.** Dérivés cycliques de l'urée de formule générale I selon la revendication 1, dans laquelle

X représente un groupe carbonyle ou sulfonyle,
Y représente un groupe alkylène ou alcénylène linéaire de 2 ou 3 atomes de carbone dans chaque cas, éventuellement substitué par $R_c$ ou $R_d$ ou $R_c$ et $R_d$, qui peut être substitué par un ou deux groupes méthyle ou par un groupe trifluorométhyle ou phényle, un groupe méthylène pouvant en outre être remplacé par un groupe carbonyle, ou un groupe -N=CH- ou -CH=N- éventuellement substitué par $R_c$ ou $R_d$,
l'un des restes $R_a$ à $R_d$ représente un groupe de formule

A - B - C -

dans laquelle
A représente un groupe aminoalkyle linéaire ou ramifié de 1 à 4 atomes de carbone, un groupe amino ou amidino, dans chacun des groupes cités précédemment, sur l'un des atomes d'azote un atome d'hydrogène pouvant être remplacé dans chaque cas par un groupe alcoxycarbonyle de 2 à 5 atomes de carbone au total ou par un groupe benzyloxycarbonyle,
B représente une liaison,

un groupe phénylène qui peut être substitué par un atome de fluor ou de chlore,
un groupe cyclopropylène, pyridinylène, pyrimidinylène, pyrazinylène ou pyridazinylène,

C représente un groupe phénylène qui peut être substitué par un ou deux groupes méthyle, par un atome de fluor, de chlore ou de brome, par un groupe méthoxy, méthylsulfényle, méthylsulfinyle, méthylsulfonyle, amino, acétylamino, benzoylamino ou méthanesulfonylamino, ou encore, lorsque A représente un groupe amino et B représente une liaison, un groupe indanylène ou 1,2,3,4-tétrahydronaphtylène, dans lesquels dans chaque cas le cycle saturé est lié au reste A et le cycle aromatique est lié au squelette cyclique de l'urée,

un groupe pyridinylène, pyrimidinylène, pyrazinylène, pyridazinylène, cyclohexylène ou pipéridinylène, l'atome d'azote ne pouvant pas être lié à un atome d'azote de l'urée cyclique,
un deuxième des restes $R_a$ à $R_d$ représente un groupe de formule

F - E - D -

dans laquelle

D représente un groupe alkylène de 1 à 4 atomes de carbone,

un groupe phénylène qui peut être substitué par un atome de fluor, de chlore ou de brome, par un groupe méthyle, méthoxy, méthylsulfényle, méthylsulfinyle ou méthylsulfonyle,
un groupe pyridinylène, cyclohexylène ou pipéridinylène, le groupe -CH=N- pouvant en outre être remplacé dans un groupe pyridinylène par un groupe -CO-NH et l'atome d'azote, au lieu d'être lié à l'atome d'hydrogène, pouvant aussi l'être au reste E dans la mesure où celui-ci ne représente pas une liaison ou n'est pas lié au reste D par un hétéroatome, ou
un groupe $-CH_2CH_2-N(COCH_3)-CH_2-$ dans lequel la partie éthylène est liée à l'urée cyclique,

E représente une liaison,

un groupe éthylène éventuellement substitué par un ou deux groupes méthyle ou par un groupe amino ou dibenzylamino,
un groupe éthénylène ou phénylène ou
un groupe méthylène lié au groupe D par le reste W', dans lequel W' représente un atome d'oxygène ou de soufre, un groupe sulfinyle ou sulfonyle, et

F représente un groupe carbonyle qui est substitué par un groupe hydroxyle ou par un groupe alcoxy de 1 à

4 atomes de carbone, un groupe phosphono, O-méthylphosphono ou tétrazol-5-yle, où, dans la mesure où A représente un groupe amino ou aminoalkyle éventuellement benzyloxycarbonylé sur l'atome d'azote, la plus courte distance entre l'atome d'azote de ce groupe et le reste F est d'au moins 10 liaisons,

le troisième des restes $R_a$ à $R_d$ représente un atome d'hydrogène, un groupe méthyle, éthyle ou phényle et le quatrième des restes $R_a$ à $R_d$ représente un atome d'hydrogène ou un groupe méthyle,
leurs tautomères, leurs stéréoisomères y compris leurs mélanges et leurs sels d'addition.

**5.** Dérivés cycliques de l'urée de formule générale I selon la revendication 1, dans laquelle

X représente un groupe carbonyle ou sulfonyle,
Y représente un groupe éthylène ou éthénylène éventuellement substitué par $R_c$ ou $R_d$ et éventuellement par un groupe méthyle ou phényle ou un groupe carbonylméthylène ou méthylènecarbonyle éventuellement substitué par un groupe méthyle, ou un groupe -CH=N- ou-N=CH- éventuellement substitué par $R_c$ ou $R_d$,
l'un des restes $R_a$ à $R_d$ représente un groupe de formule

A - B - C -

dans laquelle
A représente un groupe aminométhyle, aminoéthyle ou amidino éventuellement substitué par un groupe alcoxycarbonyle de 2 à 5 atomes de carbone au total,
B représente une liaison ou un groupe 1,4-phénylène et
C représente un groupe 1,4-phénylène éventuellement substitué par un groupe méthyle, un groupe 3,6-pyridazinylène ou 1,4-pipéridinylène, où, dans chaque cas, l'atome d'azote ne peut pas être lié à un atome d'azote de l'urée cyclique, ou, lorsque A représente un groupe amino et B représente une liaison, un groupe indanylène dans lequel le cycle saturé est lié au reste A et le cycle aromatique est lié au squelette cyclique de l'urée, un deuxième des restes $R_a$ à $R_d$ représente un groupe de formule

F - E - D -

dans laquelle
D représente un groupe alkylène de 1 à 4 atomes de carbone, un groupe 1,4-phénylène ou 1,4-cyclohexylène,
E représente une liaison,

un groupe éthylène éventuellement substitué par un groupe amino ou dibenzylamino ou un groupe éthénylène,
un groupe 1,4-phénylène ou
un groupe méthylène lié au groupe D par le reste W', dans lequel W' représente un atome d'oxygène ou de soufre, un groupe sulfinyle ou sulfonyle,

F représente un groupe carbonyle qui est substitué par un groupe hydroxyle ou par un groupe alcoxy de 1 à 4 atomes de carbone, où, dans la mesure où A représente un groupe aminométhyle, la plus courte distance entre l'atome d'azote de ce groupe et le reste F est d'au moins 10 liaisons,

le troisième des restes $R_a$ à $R_d$ représente un atome d'hydrogène ou un groupe méthyle, éthyle ou phényle et
le quatrième des restes $R_a$ à $R_d$ représente un atome d'hydrogène ou un groupe méthyle,
leurs tautomères, leurs stéréoisomères y compris leurs mélanges et leurs sels d'addition.

**6.** Dérivés cycliques de l'urée de formule générale I selon la revendication 1, dans laquelle

$R_a$ à $R_d$, X et Y sont définis comme dans les revendications 1 à 5, et dans lesquels un autre élément cyclique se trouve sur l'urée cyclique entre les points de fixation de ceux des restes $R_a$ à $R_d$ qui représentent les groupes A-B-C- et F-E-D-,
leurs tautomères, leurs stéréoisomères y compris leurs mélanges et leurs sels d'addition.

**7.** Nouveaux composés de formule générale I suivants :

(a) 1-(4-amidino-phényl)-3-[4-(2-carboxy-éthyl)phényl]-imidazolidin-2-one,

(b) 1-(4-amidino-phényl)-3-[4-(2-carboxy-éthyl)cyclohexyl]imidazolidin-2-one,

(c) 1-(4-amidino-phényl)-3-[4-(2-carboxy-éthyl)-phényl]-imidazolidin-2,4-dione,

(d) 2-(4-amidino-phényl)-5-[4-(2-carboxy-éthyl)-phényl]-3,4-dihydro-2H,5H-1,2,5-thiadiazol-1,1-dioxyde,

(e) 1-(4-amidino-phényl)-3-[4-(2-carboxy-éthyl)-phényl]-3H-imidazol-2-one,

(f) 1-(4-amidino-phényl)-3-[4-(2-carboxy-éthyl)-phényl]-4-méthyl-3H-imidazol-2-one,

(g) 2-(4-amidino-phényl)-4-[4-(2-carboxy-éthyl)-phényl]-5-méthyl-4H-1,2,4-triazol-3-one,

(h) 2-(4-amidino-phényl)-4-[4-(2-carboxy-éthyl)-phényl]-5-éthyl-4H-1,2,4-triazol-3-one,

(i) 2-(4-amidino-phényl)-4-[4-(2-carboxyéthyl)-phényl]-4H-1,2,4-triazol-3-one,

(j) 4-(4-amidino-phényl)-2-[4-(2-carboxy-éthyl)-phényl]-4H-1,2,4-triazol-3-one,

(k) 1-(4-amidino-phényl)-3-[4-(2-méthoxycarbonyl-éthyl)-phényl]-4-méthyl-3H-imidazol-2-one,

(l) 2-(4-amidino-phényl)-4-[4-(2-méthoxycarbonyl-éthyl)-phényl]-5-méthyl-4H-1,2,4-triazol-3-one,

(m) 2-(4-amidino-phényl)-5-éthyl-4-[4-(2-méthoxycarbonyl-éthyl)-phényl]-4H-1,2,4-triazol-3-one,

(n) 2-(4-amidino-phényl)-4-[4-(2-méthoxycarbonyl-éthyl)-phényl]-4H-1,2,4-triazol-3-one,

(o) 2-(4-méthoxycarbonylamidino-phényl)-3-[4-(2-méthoxycarbonyl-éthyl)-phényl]-5-méthyl-4H-1,2,4-triazol-3-one,

(p) 2-(4-méthoxycarbonylamidino-phényl)-3-[4-(2-méthoxycarbonyl-éthyl)-phényl]-4H-1,2,4-triazol-3-one,

(q) 4-[4-(2-isobutyloxycarbonyl-éthyl)phényl]-2-(4-méthoxycarbonylamidino-phényl)-5-méthyl-4H-1,2,4-triszol-3-one et

(r) 2-(4-amidino-phényl)-4-[4-(2-isobutyloxycarbonyléthyl) phényl]-5-méthyl-4H-1,2,4-triazol-3-one,

(s) 1-(4-amidino-phényl)-3-[4-(2-méthoxycarbonyl-éthyl)-cyclohexyl]-imidazolidin-2-one,

(t) 1-(4-amidino-phényl)-3-[4-(2-méthoxvcarbonyl-éthyl)-phényl]-imidazolidin-2,4-dione,

(u) 1-(4-amidino-phényl)-3-[4-(2-isopropyloxycarbonyléthyl)-phényl]-imidazolidin-2,4-dione,

leurs tautomères, leurs stéréoisomères y compris leurs mélanges et leurs sels d'addition.

**8.** Sels d'addition physiologiquement acceptables des composés selon au moins l'une des revendications 1 à 7 avec des acides ou des bases inorganiques ou organiques.

**9.** Médicament contenant un composé de formule générale I dans laquelle $R_a$, $R_b$, X et Y sont définis comme dans les revendications 1 à 7 avec la condition que B ne représente pas un groupe cyano ou cyano(alkyle en $C_{1-4}$), ou un sel d'addition physiologiquement acceptable de celui-ci outre éventuellement un ou plusieurs supports et/ou diluants inertes.

**10.** Utilisation d'un composé de formule générale I dans laquelle $R_a$, $R_b$, X et Y sont définis comme dans les revendications 1 à 7 avec la condition que B ne représente pas un groupe cyano ou cyano(alkyle en $C_{1-4}$), ou d'un sel

d'addition physiologiquement acceptable de celui-ci pour la préparation d'un médicament qui convient pour la lutte contre ou la prévention de maladies dans lesquelles il apparaît des agrégats cellulaires de taille relativement petite ou relativement grande ou dans lesquelles des interactions cellules-matrices jouent un rôle.

**11.** Procédé de préparation d'un médicament selon la revendication 9 caractérisé en ce que, par voie non chimique, un composé de formule générale I dans laquelle $R_a$, $R_b$, X et Y sont définis comme dans les revendications 1 à 7 avec la condition que B ne représente pas un groupe cyano ou cyano(alkyle en $C_{1-4}$), ou un sel d'addition physiologiquement acceptable de celui-ci est incorporé dans un ou plusieurs supports et/ou diluants inertes.

**12.** Procédé de préparation des dérivés cycliques de l'urée selon les revendications 1 à 8 caractérisé en ce que

a) pour la préparation de composés de formule générale I dans laquelle F représente un groupe carboxyle, un composé de formule générale

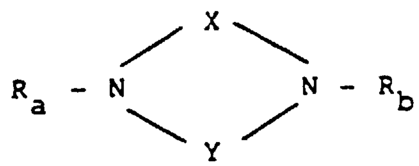

(II)

dans laquelle

$R_a$, $R_b$, X et Y sont définis comme dans les revendications 1 à 7 avec la condition que l'un des restes $R_a$ à $R_d$ représente un groupe de formule

F' - E - D -

dans laquelle
E et D sont définis comme dans les revendications 1 à 7 et
F' représente un groupe qui peut être converti en un groupe carboxyle par hydrolyse, traitement avec des acides, thermolyse ou hydrogénolyse, est converti en un composé de formule générale I dans laquelle F représente un groupe carboxyle ou

b) pour la préparation de composés de formule générale I dans laquelle A représente un groupe $H_2N-C(=NH)-$ éventuellement substitué par un groupe alkyle, un composé, éventuellement formé dans le mélange réactionnel, de formule générale

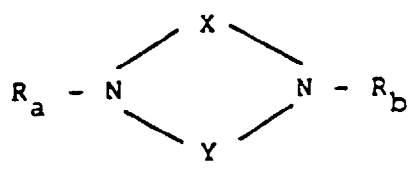

(III)

dans laquelle

$R_a$, $R_b$, X et Y sont définis comme dans les revendications 1 à 7 avec la condition que l'un des restes $R_a$ à $R_d$ représente un groupe de formule

$Z_1$ - C(=NH) - B -

dans laquelle
B et C sont définis comme dans les revendications 1 à 7 et

$Z_1$ représente un groupe alcoxy, aralcoxy, alkylthio, aralkylthio ou amino, est mis à réagir avec une amine de formule générale

$$R_4 - NH_2 \quad (IV)$$

dans laquelle
$R_4$ représente un atome d'hydrogène ou un groupe alkyle de 1 à 4 atomes de carbone, ou avec ses sels d'addition d'acide ou

c) pour la préparation de composés de formule générale I dans laquelle au moins l'un des restes B, C, D ou E contient un groupe sulfinyle ou sulfonyle, un composé de formule générale

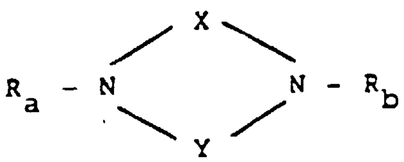

(V)

dans laquelle
$R_a$, $R_b$, X et Y sont définis comme dans les revendications 1 à 7 avec la condition qu'au moins l'un des restes Y, B, C, D ou E contienne un groupe sulfényle ou sulfinyle, est oxydé ou
d) pour la préparation de composés de formule générale I dans laquelle Y représente un groupe alkylène linéaire de 2 à 4 atomes de carbone éventuellement substitué par $R_c$ ou $R_d$ ou $R_c$ et $R_d$, qui peut être mono- ou disubstitué par des groupes alkyle, trifluorométhyle, aralkyle, aryle ou hétéroaryle, un composé de formule générale

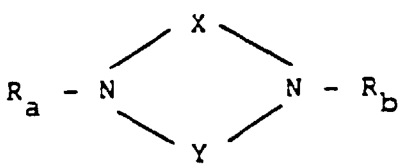

(VI)

dans laquelle

$R_a$, $R_b$ et X sont définis comme dans les revendications 1 à 7 et
Y' représente un groupe alcénylène linéaire de 2 à 4 atomes de carbone éventuellement substitué par $R_c$ ou $R_d$ ou $R_c$ et $R_d$, qui peut être mono- ou disubstitué par des groupes alkyle, trifluorométhyle, aralkyle, aryle ou hétéroaryle, est hydrogéné ou

e) pour la préparation de composés de formule générale I dans laquelle A représente un groupe aminoalkyle, amidino ou guanidino substitué par un groupe alcoxycarbonyle de 2 à 5 atomes de carbone au total, par un groupe aralcoxycarbonyle, aryloxycarbonyle, alkylcarbonyle ou arylcarbonyle, un composé de formule générale

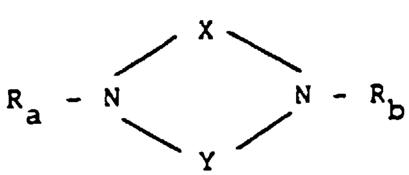

(VII)

dans laquelle

$R_a$, $R_b$, X et Y sont définis comme dans les revendications 1 à 7 avec la condition que l'un des restes $R_a$ à $R_d$ représente un groupe de formule

A' - B - C -

dans laquelle
B et C sont définis comme dans les revendications 1 à 7 et A' représente un groupe $H_2N$-alkyle en $C_{1-5}$, $H_2N$-C(=NH)- ou $H_2N$-C(=NH)-NH-, est mis à réagir avec un composé de formule générale

$Z_2$ - $R_5$ (VIII)

dans laquelle
$R_5$ représente un groupe alcoxycarbonyle de 2 à 5 atomes de carbone au total, un groupe aralcoxycarbonyle, aryloxycarbonyle, alkylcarbonyle ou arylcarbonyle, et
$Z_2$ représente un groupe partant nucléophile

f) pour la préparation de composés de formule générale I dans laquelle F représente un groupe carbonyle substitué par un groupe alcoxy de 1 à 6 atomes de carbone, un reste alcoxy de 1 à 3 atomes de carbone pouvant être substitué en position 1, 2 ou 3 par un groupe aryle ou hétéroaryle ou en position 2 ou 3 par un groupe pyrrolidin-2-on-1-yle, morpholino, thiomorpholino ou 1-oxydo-thiomorpholino, un composé de formule générale

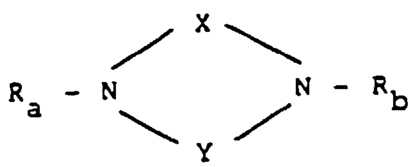

(IX)

dans laquelle

$R_a$, $R_b$, X et Y sont définis comme dans les revendications 1 à 7 avec la condition que l'un des restes $R_a$ à $R_d$ représente un groupe de formule

F" - E - D -

dans laquelle
E et D sont définis comme dans les revendications 1 à 7 et
F" représente un groupe carboxyle ou alcoxycarbonyle, est mis à réagir avec un alcool de formule générale

HO - $R_6$ (X)

dans laquelle
$R_6$ représente un groupe alkyle de 1 à 6 atomes de carbone, qui peut être substitué en position 1, 2 ou 3 par un groupe aryle ou hétéroaryle ou en position 2 ou 3 par un groupe pyrrolidin-2-on-1-yle, morpholino, thiomorpholino ou 1-oxydo-thiomorpholino, ou

g) pour la préparation de composés de formule générale I dans laquelle A représente un groupe $H_2N$-C(=NH)- et B ou, si B représente une liaison, C représente un groupe cycloalkylène de 4 ou 5 atomes de carbone éventuellement substitué par un groupe alkyle, aralkyle ou aryle, dans lequel une unité CH est remplacée par

un atome d'azote, ou un groupe cycloalkylène de 6 ou 7 atomes de carbone, éventuellement substitué par un groupe alkyle, aralkyle ou aryle, dans lequel une ou deux unités CH situées en position 1,4 l'une par rapport à l'autre sont remplacées dans chaque cas par un atome d'azote, B ou, si B représente une liaison, C étant lié au reste A par l'un des atomes d'azote cités, un composé de formule générale

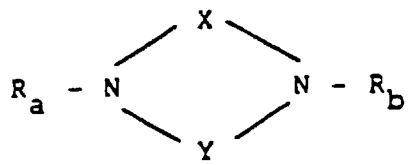
(XI)

dans laquelle

$R_a$, $R_b$, X et Y sont définis comme dans les revendications 1 à 7 avec la condition que l'un des restes $R_a$ à $R_d$ représente un groupe de formule

H - B' - C - ou H - C' -

dans laquelle
C est défini comme dans les revendications 1 à 7 et B' ou C' représente un groupe cycloalkylène de 4 ou 5 atomes de carbone, éventuellement substitué par un groupe alkyle, aralkyle ou aryle, dans lequel une unité CH est remplacée par un atome d'azote, ou un groupe cycloalkylène de 6 ou 7 atomes de carbone, éventuellement substitué par un groupe alkyle, aralkyle ou aryle, dans lequel une ou deux unités CH situées en position 1,4 l'une par rapport à l'autre sont remplacées dans chaque cas par un atome d'azote, l'atome d'hydrogène étant lié à un atome d'azote du reste B' ou C', est mis à réagir avec un composé de formule générale

$$Z_3\text{-C(=NH)-NH}_2 \qquad \text{(XII)}$$

dans laquelle
$Z_3$ représente un groupe partant nucléophile, ou

h) pour la préparation de composés de formule générale I dans laquelle A représente un groupe $H_2N\text{-}CH_2\text{-}V\text{-}$ dans lequel V représente une liaison ou un groupe alkylène linéaire ou ramifié de 1 à 4 atomes de carbone, un composé de formule générale

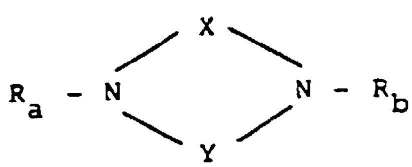
(XIII)

dans laquelle

$R_a$, $R_b$, X et Y sont définis comme dans les revendications 1 à 7 avec la condition que l'un des restes $R_a$ à $R_d$ représente un groupe de formule

NC - V - B - C -

dans laquelle

B et C sont définis comme dans les revendications 1 à 7 et V représente une liaison ou un groupe alkylène linéaire ou ramifié de 1 à 4 atomes de carbone, est réduit ou

i) pour la préparation de composés de formule générale I dans laquelle C représente un groupe alkylène substitué par un groupe hydroxyle, un composé de formule générale

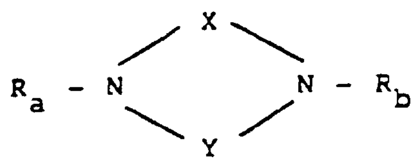

(XIV)

dans laquelle

$R_a$, $R_b$, X et Y sont définis comme dans les revendications 1 à 7 avec la condition que l'un des restes $R_a$ à $R_d$ représente un groupe de formule

A - B - C" -

dans laquelle
A et B sont définis comme dans les revendications 1 à 7 et C" représente un groupe alkylène dans lequel un groupe méthylène est remplacé par un groupe carbonyle, est réduit ou

j) pour la préparation de composés de formule générale I dans laquelle A représente un groupe $H_2N$-C(=NH)-NH-, un composé de formule générale

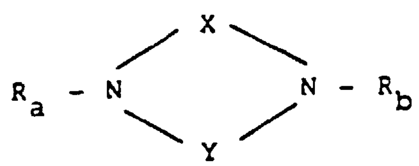

(XV)

dans laquelle

$R_a$, $R_b$, X et Y sont définis comme dans les revendications 1 à 7 avec la condition que l'un des restes $R_a$ à $R_d$ représente un groupe de formule

$H_2N$ - B - C -

dans laquelle
B et C sont définis comme dans les revendications 1 à 7, est mis à réagir avec le cyanamide ou son sel d'addition d'acide ou avec une S-alkyl-isothiourée, la O-méthylisothiourée ou le 1-amidino-3,5-diméthylpyrazole ou

k) un composé de formule générale

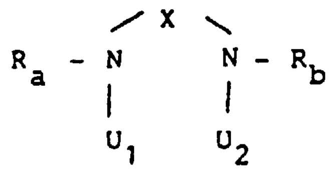

(XVI)

dans laquelle

$R_a$, $R_b$ et X sont définis comme dans les revendications 1 à 7,
l'un des restes $U_1$ et $U_2$ représente un atome d'hydrogène et l'autre des restes $U_1$ et $U_2$ représente un groupe de formule

$$- Y'' - Z_4$$

dans laquelle
Y" représente un groupe alkylène ou alcénylène linéaire de 2 à 4 atomes de carbone dans chaque cas, éventuellement substitué par $R_c$ ou $R_d$ ou $R_c$ et $R_d$, dans lesquels chaque atome de carbone peut être mono- ou disubstitué par un groupe alkyle, trifluorométhyle, aralkyle, aryle, hétéroaryle ou alkylcarbonyle, les substituants pouvant être identiques ou différents, ou un groupe 1,2-cycloalkylène de 4 à 7 atomes de carbone, éventuellement substitué par $R_c$ ou $R_d$ ou $R_c$ et $R_d$, ou un groupe 1,2-cycloalcénylène de 4 à 7 atomes de carbone, un groupe -CH=N- éventuellement substitué par le reste $R_c$ ou $R_d$, dans lequel l'atome d'azote est lié à l'un des atomes d'azote de la formule XVI, ou un groupe $-CH_2-NH-$ éventuellement substitué par $R_c$ ou $R_d$ et
$Z_4$ représente un groupe partant nucléophile ou
avec un groupe méthylène voisin du reste Y" un groupe carbonyle, carboxyle, alcoxycarbonyle, aralcoxycarbonyle, aryloxycarbonyle ou dialcoxyméthyle, est cyclisé ou

l) pour la préparation de composés de formule générale I dans laquelle $R_a$ ou $R_c$ représente un groupe E-F-D-, un composé, éventuellement formé dans le mélange réactionnel, de formule générale

$$R_c - CO - CHR_d - NR_a - CO - NHR_b \qquad (XVII)$$

dans laquelle
$R_a$ à $R_d$ sont définis comme dans les revendications 1 à 7, est cyclisé et, éventuellement, un composé ainsi obtenu est hydrogéné ou
m) pour la préparation de composés de formule générale I dans laquelle X représente un groupe carbonyle, un composé de formule générale

$$R_a\,NH - Y - NH - R_b \qquad (XVIII)$$

dans laquelle

$R_a$, $R_b$ et Y sont définis comme dans les revendications 1 à 7, est mis à réagir avec un composé de formule générale

$$Z_5 - CO - Z_6 \qquad (XIX)$$

dans laquelle
$Z_5$ et $Z_6$, qui peuvent être identiques ou différents, représentent des groupes partants nucléophiles, ou

n) pour la préparation de composés de formule générale I dans laquelle $R_a$ à $R_d$ sont définis comme dans les

revendications 1 à 7, avec la condition qu'au moins l'un des restes $R_a$ et $R_b$ ne représente pas un atome d'hydrogène, un composé de formule générale

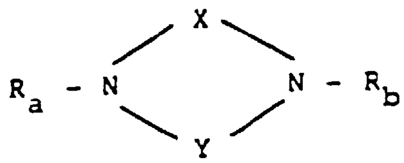

(XX)

dans laquelle

X et Y sont définis comme dans les revendications 1 à 7, l'un des restes $R_a$ et $R_b$ représente un atome d'hydrogène et l'autre des restes $R_a$ et $R_b$ est défini comme dans les revendications 1 à 7, est mis à réagir avec un composé de formule générale

$$Z_7 - R' \quad (XXI)$$

dans laquelle
R' possède les significations citées dans les revendications 1 à 7 pour $R_a$ ou $R_b$ à l'exception d'un atome d'hydrogène et $Z_7$ représente un groupe partant nucléophile ou
lorsque $R_a$ ou $R_b$ représente un groupe -D-COO-alkyle avec la condition qu'il existe deux atomes de carbone entre l'atome d'azote de l'urée cyclique et le groupe alcoxycarbonyle, est mis à réagir avec un composé de formule générale

$$- D' - COO - alkyle \quad (XXII)$$

dans laquelle
D' possède les significations indiquées pour D dans les revendications 1 à 7 avec la condition qu'une double ou triple liaison carbone-carbone précède immédiatement le groupe alcoxycarbonyle, ou

o) pour la préparation de composés de formule générale I dans laquelle F représente un groupe carboxyle, alcoxycarbonyle, aralcoxycarbonyle ou aryloxycarbonyle, un composé de formule générale

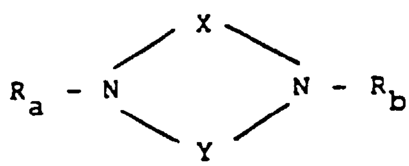

(XXIII)

dans laquelle
$R_a$, $R_b$, X et Y sont définis comme dans les revendications 1 à 7 avec la condition que l'un des restes $R_a$ à $R_d$ représente un groupe de formule

$$CH_2 = CH - E - D -$$

où E et D sont définis comme dans les revendications 1 à 7, est oxydé et, éventuellement, un composé ainsi obtenu est estérifié ou
p) pour la préparation de composés de formule générale I dans laquelle F représente un groupe O-alkyl-phosphono, un composé de formule générale

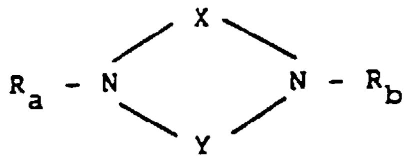

(XXIV)

dans laquelle
$R_a$, $R_b$, X et Y sont définis comme dans les revendications 1 à 7 avec la condition que F représente un groupe dialcoxyphosphoryle, est mis à réagir avec un iodure alcalin ou
q) pour la préparation de composés de formule générale I dans laquelle F représente un groupe phosphono, un composé de formule générale

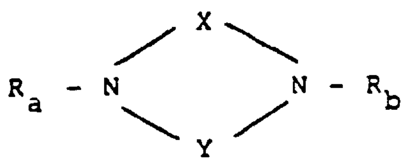

(XXV)

dans laquelle
$R_a$, $R_b$, X et Y sont définis comme dans les revendications 1 à 7 avec la condition que F représente un groupe O-alkylphosphono ou dialcoxyphosphoryle, est mis à réagir avec un iodure alcalin en présence d'un trialkylhalogénosilane ou
r) pour la préparation de composés de formule générale I dans laquelle W représente un groupe $R_1N$-, un composé de formule générale

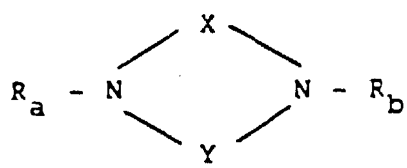

(XXVI)

dans laquelle

$R_a$, $R_b$, X et Y sont définis comme dans les revendications 1 à 7 avec la condition que l'un des restes $R_a$ à $R_d$ représente un groupe $Z_8$-D"-, D" représentant un groupe alkylène de 1 à 3 atomes de carbone et $Z_8$ représentant un groupe partant nucléophile ou un reste d'ester d'acide sulfonique, est mis à réagir avec un composé de formule générale

$$R_1NH - E' - F \qquad (XXVII)$$

dans laquelle

F et $R_1$ sont définis comme dans les revendications 1 à 7 et
E' représente un groupe alkylène de 1 à 3 atomes de carbone, ou

s) pour la préparation de composés de formule générale I dans laquelle A représente un groupe amino ou aminoalkyle, un composé de formule générale

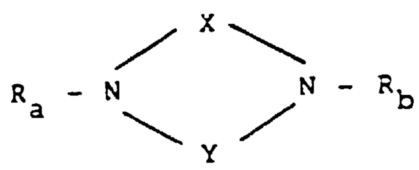

(XXVIII)

dans laquelle

$R_a$, $R_b$, X et Y sont définis comme dans les revendications 1 à 7 avec la condition que l'un des restes $R_a$ à $R_d$ représente un groupe $H_2N$-CO-T-B-C-, B et C étant définis comme dans les revendications 1 à 7 et T représentant une liaison ou un groupe alkylène de 1 à 5 atomes de carbone, est mis à réagir avec un composé de phényliode (III) de formule générale

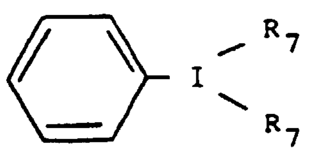

**(XXIX)**

dans laquelle
$R_7$ représente dans chaque cas le reste acyle d'un acide carboxylique organique ou

t) pour la préparation de composés de formule générale I dans laquelle A représente un groupe amino ou aminoalkyle substitué par un ou deux groupes alkyle sur l'atome d'azote, un composé de formule générale

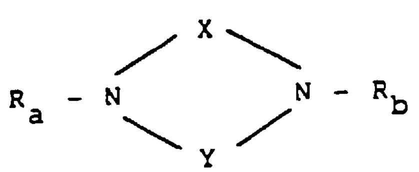

(XXX)

dans laquelle

$R_a$, $R_b$, X et Y sont définis comme dans les revendications 1 à 7 avec la condition que l'un des restes $R_a$ à $R_d$ représente un groupe de formule

A" - B - C -

dans laquelle
B et C sont définis comme dans les revendications 1 à 7 et
A" représente un groupe amino, alkylalalino, aminoalkyle ou alkylaminoalkyle, est mis à réagir avec un composé de formule générale

$$Z_9 - (R_8\text{-}C\text{-}R_9) - Z_{10} \qquad \text{(XXXI)}$$

dans laquelle
$R_8$ et $R_9$, qui peuvent être identiques ou différents, représentent des atomes d'hydrogène ou des groupes alkyle, l'un des groupes $Z_9$ et $Z_{10}$ représente un groupe partant nucléophile et

l'autre des groupes $Z_9$ et $Z_{10}$ représente un atome d'hydrogène ou un groupe alkyle ou
$Z_9$ et $Z_{10}$ représentent ensemble un atome d'oxygène, ou

u) pour la préparation de composés de formule générale I dans laquelle A représente un groupe cyano, un composé de formule générale

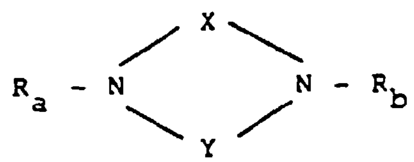 (XXXII)

dans laquelle

$R_a$, $R_b$, X et Y sont définis comme dans les revendications 1 à 7 avec la condition que l'un des restes $R_a$ à $R_d$ représente un groupe de formule

A"' - B - C -

dans laquelle
B et C sont définis comme dans les revendications 1 à 7 et
A'" représente un atome d'halogène, est mis à réagir avec le cyanure de cuivre (I) ou

v) pour la préparation de composés de formule générale I dans laquelle A représente un groupe aminoalkyle dans lequel le groupe amino n'est pas lié à un atome de carbone quaternaire, ou un groupe amino qui est lié à un groupe CH- ou $CH_2$- du reste B ou C, un composé de formule générale

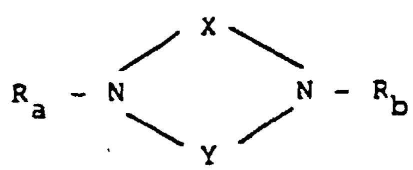 (XXXIII)

dans laquelle

$R_a$, $R_b$, X et Y sont définis comme dans les revendications 1 à 7 avec la condition que l'un des restes $R_a$ à $R_d$ représente un groupe de formule

A"" - B - C -

dans laquelle
B et C sont définis comme dans les revendications 1 à 7 et A"" représente un groupe N-hydroxy-imino, est réduit et si on le souhaite, un composé de formule générale I ainsi obtenu est ensuite converti par bromation en un composé bromé de formule générale I correspondant ou

un composé de formule générale I ainsi obtenu est ensuite converti par nitration en un composé nitré de formule générale I correspondant ou
un composé de formule générale I ainsi obtenu qui contient un groupe nitro est ensuite converti par réduction en un composé aminé correspondant ou
un composé de formule générale I ainsi obtenu qui contient un groupe $R_1$NH- ou dans lequel W représente un groupe imino est ensuite converti par acylation ou sulfonation en un composé de formule générale I correspondant qui contient in groupe $R_1$NH- substitué par un groupe alkylcarbonyle,

aralkylcarbonyle, arylcarbonyle, hétéroarylcarbonyle, alkylsulfonyle, aralkylsulfonyle ou arylsulfonyle, ou
un composé de formule générale I ainsi obtenu dans lequel X représente un groupe carbonyle est ensuite converti au moyen d'un agent de sulfuration en un composé thiocarbonylé correspondant et, si nécessaire, un reste protecteur utilisé pendant les réactions a) à v) pour la protection de groupes réactifs est clivé et/ou
si on le souhaite un composé de formule générale I ainsi obtenu est résolu en ses isomères cis/trans, en ses énantiomères et/ou diastéréoisomères et/ou
un composé de formule générale I ainsi obtenu est converti en ses sels, en particulier pour l'utilisation pharmaceutique en ses sels physiologiquement acceptables avec un acide ou une base inorganique ou organique.